(19) Europäisches Patentamt / European Patent Office / Office européen des brevets

(11) EP 4 674 848 A1

(12) # EUROPEAN PATENT APPLICATION
published in accordance with Art. 153(4) EPC

(43) Date of publication:
**07.01.2026 Bulletin 2026/02**

(21) Application number: **24764003.0**

(22) Date of filing: **29.02.2024**

(51) International Patent Classification (IPC):
***C07D 403/14*** (2006.01) ***C09K 11/06*** (2006.01)
***H10K 50/11*** (2023.01) ***H10K 50/12*** (2023.01)
***H10K 50/15*** (2023.01) ***H10K 50/16*** (2023.01)
***H10K 85/60*** (2023.01) ***H10K 101/20*** (2023.01)
***H10K 101/30*** (2023.01) ***C07D 519/00*** (2006.01)

(52) Cooperative Patent Classification (CPC):
**C09K 11/06; C07D 403/14; C07D 519/00; H10K 50/11; H10K 50/12; H10K 50/15; H10K 50/16; H10K 85/60;** C09K 2211/1007; C09K 2211/1014; C09K 2211/1029; C09K 2211/1033; C09K 2211/1037; C09K 2211/1044; C09K 2211/1048; (Cont.)

(86) International application number:
**PCT/JP2024/007515**

(87) International publication number:
**WO 2024/181526 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.03.2023 JP 2023031074**

(71) Applicant: **Kyulux, Inc.**
**Fukuoka-shi, Fukuoka 819-0388 (JP)**

(72) Inventors:
- **YAMANE, Yu**
  **Fukuoka-shi, Fukuoka 819-0388 (JP)**
- **CHO, Yong Joo**
  **Fukuoka-shi, Fukuoka 819-0388 (JP)**
- **HAMASAKI, Taro**
  **Fukuoka-shi, Fukuoka 819-0388 (JP)**
- **SHIMAMURA, Naomi**
  **Fukuoka-shi, Fukuoka 819-0388 (JP)**
- **SUZUKI, Yoshitake**
  **Fukuoka-shi, Fukuoka 819-0388 (JP)**
- **KANAHARA, Kousei**
  **Fukuoka-shi, Fukuoka 819-0388 (JP)**
- **HWANG, Songhye**
  **Fukuoka-shi, Fukuoka 819-0388 (JP)**

(74) Representative: **Zimmermann & Partner Patentanwälte mbB**
**P.O. Box 330 920**
**80069 München (DE)**

(54) **COMPOUND, LIGHT-EMITTING MATERIAL, AND LIGHT-EMITTING ELEMENT**

(57) When a compound represented by the following general formula is used, an organic light-emitting device having excellent characteristics can be provided. One or more of $R^1$ to $R^5$ represent a cyano group, and two or more represent a donor group; $X^1$ to $X^3$ represent N or C(R); R represents H, D, or a substituent; one or more of $Ar^1$ and $Ar^2$ represent a group bonding via a benzene ring that has a donor group bonding via a nitrogen atom; and $L^1$ represents a single bond or a linking group.

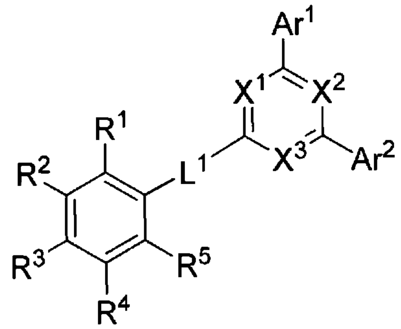

EP 4 674 848 A1

(52) Cooperative Patent Classification (CPC): (Cont.)
C09K 2211/1051; C09K 2211/1059; H10K 2101/20;
H10K 2101/30

## Description

### Technical Field

The present invention relates to a compound useful as a light-emitting material, and a light-emitting device using the compound.

### Background Art

Studies for enhancing the light emission efficiency of light-emitting devices such as organic electroluminescent devices (organic EL devices) are being made actively. In particular, various kinds of efforts have been made for increasing light emission efficiency by newly developing and combining an electron transporting material, a hole transporting material, and a light-emitting material to constitute an organic electroluminescent device. Among them, there are seen some reports relating to an organic electroluminescent device that utilizes a delayed fluorescent material.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light-emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. On the other hand, in a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher light emission efficiency.

Since such a principle has been clarified, various studies have led to the discovery of various delayed fluorescent materials. Among these, many compounds in which a benzene ring is substituted with a donor group and an acceptor group are included. For example, a compound having a skeleton, in which the benzene ring is substituted with a carbazol-9-yl group of a donor group and with a cyano group and a substituted triazinyl group of acceptor groups, has been proposed (see PTL 1).

### Citation List

### Patent Literature

PTL 1:WO2019/191665A1

### Summary of Invention

### Technical Problem

Even when a material emits delayed fluorescence, one having extremely good characteristics and having no problem in practical use has not been provided. Accordingly, it would be even more useful when a delayed fluorescent material with further more superior properties can be provided. However, the improvement of delayed fluorescent materials is in the stage of trial and error, and it is not easy to generalize the chemical structure of useful light-emitting materials.

Under such circumstances, the present inventors have conducted research for the purpose of providing a compound more useful as a delayed fluorescent material for a light-emitting device. Then, the present inventors have conducted intensive studies for the purpose of deriving and generalizing a general formula of a compound more useful as a delayed fluorescent material.

### Solution to Problem

As a result of intensive studies for achieving the above object, the present inventors have found that a compound having a structure that satisfies a specific requirement is useful as a light-emitting material. The present invention has been proposed based on these findings, and specifically has the following configuration.

[1] A compound represented by the following general formula (1):

General Formula (1)

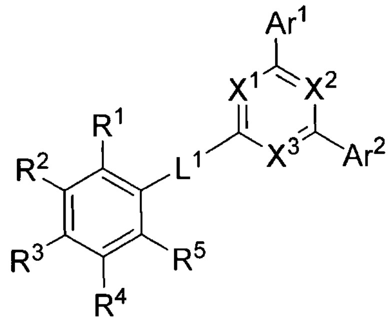

[In the general formula (1), $R^1$ to $R^5$ each independently represent a hydrogen atom, a deuterium atom, a cyano group, a substituted or unsubstituted alkyl group, a substituted or unsubstituted aryl group, or a donor group. One or more of $R^1$ to $R^5$ represent a cyano group, and two or more of $R^1$ to $R^5$ represent a donor group. $X^1$ to $X^3$ each independently represent N or C(R), and at least one of $X^1$ to $X^3$ represent N. R represents a hydrogen atom, a deuterium atom, or a substituent. $Ar^1$ and $Ar^2$ each independently represent a substituted or unsubstituted aryl group, or a substituted or unsubstituted heteroaryl group, and at least one of $Ar^1$ and $Ar^2$ represents a group bonding via a benzene ring that has a donor group bonding via a nitrogen atom but has no acceptor group bonding thereto. $L^1$ represents a single bond or a divalent linking group.]

[2] The compound according to [1], in which only one of $R^1$ to $R^5$ represents a cyano group.

[3] The compound according to [2], in which $R^2$ represents a cyano group.

[4] A compound according to any one of [1] to [3], in which $Ar^1$ represents a group having a structure represented by the following general formula (c):

General Formula (c)

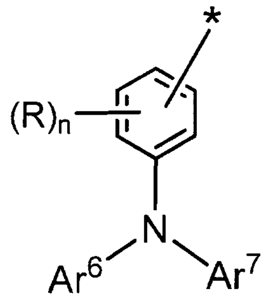

[In the general formula (c), R's each independently represent a deuterium atom or a donor group, $Ar^6$ and $Ar^7$ each independently represent a substituted or unsubstituted aryl group, or a substituted or unsubstituted heteroaryl group, $Ar^6$ and $Ar^7$ may bond to each other to form a cyclic structure, $Ar^6$ and R may bond to each other to form a cyclic structure, and $Ar^7$ and R may bond to each other to form a cyclic structure, n represents an integer of 0 to 4, and * indicates a bonding position.]

[5] A compound according to any one of [1] to [3], in which $Ar^1$ represents a group having a structure represented by the following general formula (d):

General Formula (d)

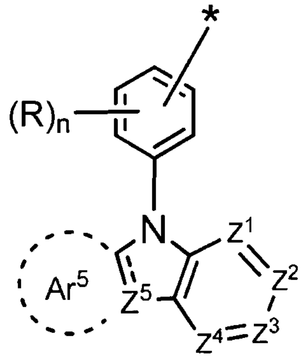

[In the general formula (d), R's each independently represent a deuterium atom or a donor group. $Z^1$ represents C-$R^{14}$ or N, $Z^2$ represents C-$R^{15}$ or N, $Z^3$ represents C-$R^{16}$ or N, and $Z^4$ represents C-$R^{17}$ or N, $Z^5$ represents C or N, and $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each may bond to each other to form a cyclic structure. n represents an integer of 0 to 4. * indicates a bonding position.]

[6] The compound according to any one of [1] to [3], in which $Ar^1$ represents a group having a structure represented by the following general formula (f):

General Formula (f)

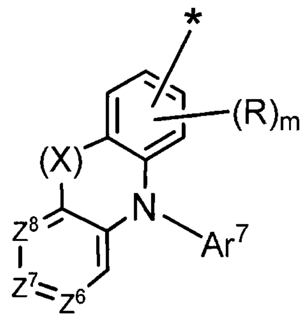

[In the general formula (f), R's each independently represent a deuterium atom or a donor group. $Z^6$ represents C-$R^{18}$ or N, $Z^7$ represents C-$R^{19}$ or N, $Z^8$ represents C-$R^{20}$ or N, and $R^{18}$ and $R^{19}$ and $R^{19}$ and $R^{20}$ each may bond to each other to form a cyclic structure. X represents a single bond when absent, or represents a linking group having a linking chain of one or two atoms. $Ar^7$ represents a substituted or unsubstituted aryl group, or a substituted or unsubstituted heteroaryl group. m represents an integer of 0 to 3. * indicates a bonding position.]

[7] The compound according to any one of [1] to [6], in which $Ar^2$ represents a substituted or unsubstituted aryl group having no donor group.

[8] The compound according to any one of [1] to [6], in which $Ar^2$ represents a donor group bonding via a nitrogen atom.

[9] The compound according to any one of [1] to [6], in which $Ar^2$ represents a group having a structure represented by the following general formula (f).

[10] The compound according to any one of [1] to [8], in which three of $R^1$ to $R^5$ represent a donor group.

[11] The compound according to any one of [1] to [10], in which the donor group is a substituted or unsubstituted carbazol-9-yl group.

[12] The compound according to any one of [1] to [10], in which $X^1$ to $X^3$ represent N.

[13] The compound according to any one of [1] to [12], in which $L^1$ represents a single bond.

[14] The compound according to any one of [1] to [13], in which $R^1$ represents a hydrogen atom.

[15] The compound according to any one of [1] to [14], in which the compound has at least one deuterium atom.

[16] A light-emitting material including: the compound according to any one of [1] to [15].

[17] A delayed fluorescent substance including: the compound according to any one of [1] to [15].

[18] A film including: the compound according to any one of [1] to [15].

[19] An organic semiconductor device including: the compound according to any one of [1] to [15].

[20] An organic light-emitting device including: the compound according to any one of [1] to [15].

[21] The organic light-emitting device according to [20], in which the device includes a layer containing the compound,

and the layer also contains a host material.

[22] The organic light-emitting device according to [21], in which the layer containing the compound further contains a delayed fluorescent material in addition to the compound and the host material, and the delayed fluorescent material has a lowest excited singlet energy lower than a lowest excited singlet energy of the host material and higher than a lowest excited singlet energy of the compound.

[23] The organic light-emitting device according to [21] or [22], in which the device includes the layer containing the compound, and the layer also contains a light-emitting material having a structure different from a structure of the compound.

[24] The organic light-emitting device according to [21] or [22], in which an amount of light emitted from the compound is largest among the materials contained in the device.

[25] The organic light-emitting device according to [23], in which an amount of light emitted from the light-emitting material is larger than an amount of light emitted from the compound.

[26] The organic light-emitting device according to any one of [20] to [25], which is an organic electroluminescent device.

[27] The organic light-emitting device according to any one of [20] to [26], which emits delayed fluorescence.

Advantageous Effects of Invention

The compound of the present invention shows excellent light emission characteristics. The compound of the present invention is useful as a material for an organic light-emitting device.

Description of Embodiments

The contents of the present invention will be described in detail below. The constitutional elements may be described below with reference to representative embodiments and specific examples of the present invention, but the present invention is not limited to the embodiments and the specific examples. In the description herein, a numerical range expressed as "to" means a range that includes the numerical values described before and after "to" as the lower limit and the upper limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the present description, the term "substituent" means an atom or an atomic group except a hydrogen atom and a deuterium atom. Meanwhile, the term "substituted or unsubstituted" means that a hydrogen atom can be substituted with a deuterium atom or a substituent.

[Compound Represented by General Formula (1)]

The compound represented by the following general formula (1) is described.

General Formula (1)

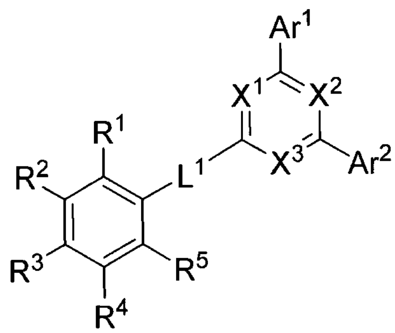

In the general formula (1), $R^1$ to $R^5$ each independently represent a hydrogen atom, a deuterium atom, a cyano group, a substituted or unsubstituted alkyl group, a substituted or unsubstituted aryl group, or a donor group. One or more of $R^1$ to $R^5$ represent a cyano group, and two or more of $R^1$ to $R^5$ represent a donor group. $X^1$ to $X^3$ each independently represent N or C(R), and at least one of $X^1$ to $X^3$ represents N. R represents a hydrogen atom, a deuterium atom or a substituent. $Ar^1$ and $Ar^2$ each independently represent a substituted or unsubstituted aryl group, or a substituted or unsubstituted heteroaryl group, and at least one of $Ar^1$ and $Ar^2$ represents a group bonding via a benzene ring that has a

donor group bonding via a nitrogen atom but has no acceptor group bonding thereto. As referred to herein, the expression "has a donor group bonding via a nitrogen atom" means that the donor group directly bonds to the benzene ring via the nitrogen atom constituting the donor group. The expression "has no acceptor group" means that the acceptor group does not directly bond to the benzene ring.

The alkyl group that $R^1$ to $R^5$ can take can be linear, branched or cyclic. Two or more of a linear moiety, a cyclic moiety and a branched moiety can be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent can be further substituted with, for example, a deuterium atom, an aryl group, an alkoxy group, an aryloxy group, and a halogen atom. In one aspect of the present invention, the substituent for the alkyl group is one or more selected from the group consisting of an aryl group and a deuterium atom. In one preferred aspect of the present invention, the alkyl group is unsubstituted, and, for example, can be selected from the group consisting of a methyl group, an ethyl group, an isopropyl group and a tert-butyl group.

The aryl group that $R^1$ to $R^5$ and $Ar^1$ and $Ar^2$ can take each can be a monocyclic ring or can be a fused ring of two or more kinds of rings. In the case of a fused ring, the number of fused rings is preferably 2 to 6, and can be selected from, for example, 2 to 4. Specific examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group can be selected from Substituent Group A, can be selected from Substituent Group B, can be selected from Substituent Group C, can be selected from Substituent Group D, or can be selected from Substituent Group E. In one aspect of the present invention, the substituent for the aryl group is one or more selected from the group consisting of an alkyl group, an aryl group and a deuterium atom. In one preferred aspect of the present invention, the aryl group is substituted with at least one deuterium atom. In one aspect of the present invention, the aryl group is unsubstituted.

Specific examples of the substituted or unsubstituted aryl group that $R^1$ to $R^5$ and $Ar^1$ and $Ar^2$ can take are shown below. The aryl group which can be employed in the present invention should not be limitatively interpreted by the following specific examples. In the following specific examples, * indicates a bonding position. A methyl group is not shown. Consequently, Ar2 to Ar7 represent structures substituted with a methyl group.

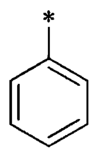
Ar1

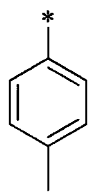
Ar2

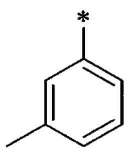
Ar3

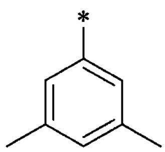
Ar4

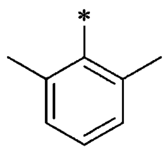
Ar5

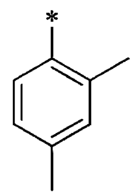
Ar6

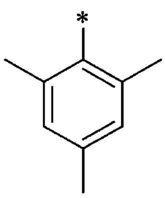
Ar7

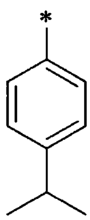
Ar8

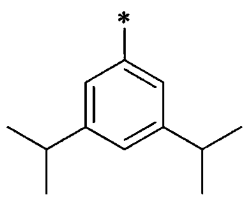
Ar9

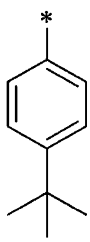
Ar10

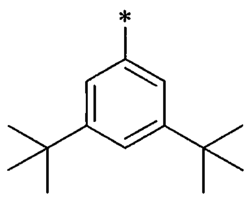
Ar11

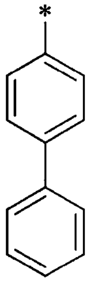
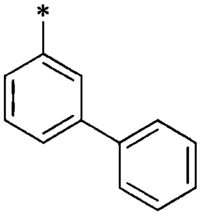
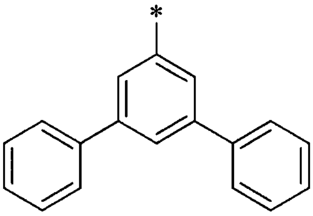
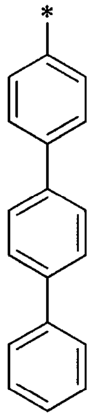
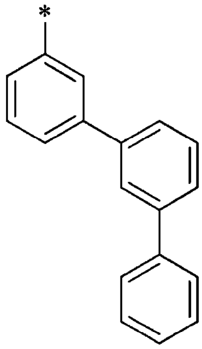

Ar12 Ar13 Ar14 Ar15 Ar16

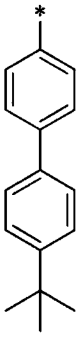
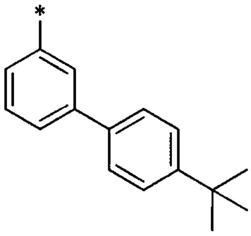
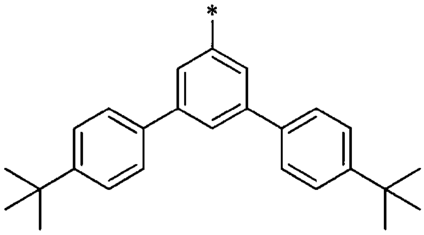
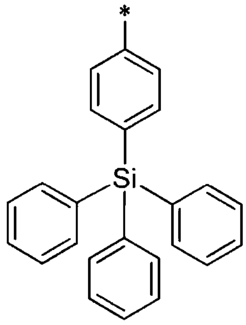

Ar17 Ar18 Ar19 Ar20

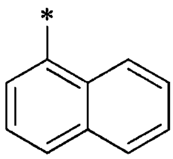
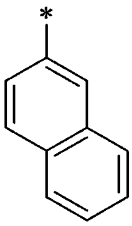
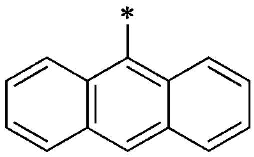
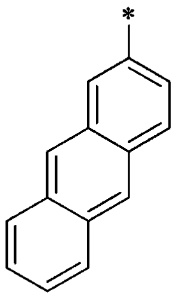
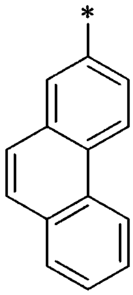

Ar21 Ar22 Ar23 Ar24 Ar25

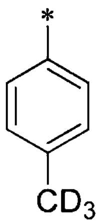
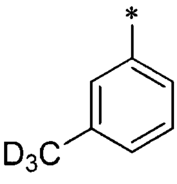
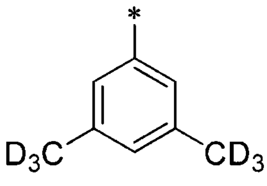
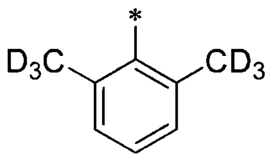
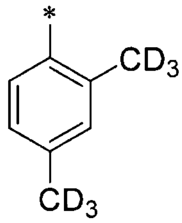

Ar26 Ar27 Ar28 Ar29 Ar30

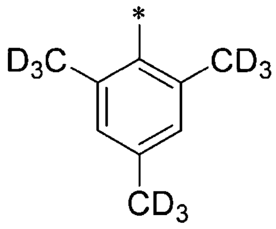

Ar31

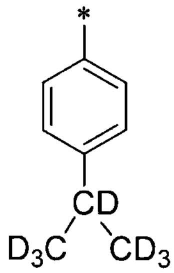

Ar32

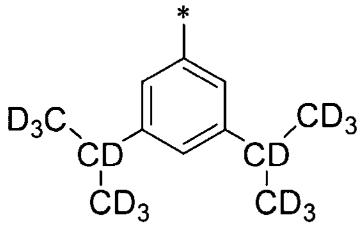

Ar33

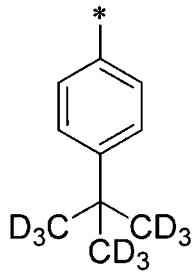

Ar34

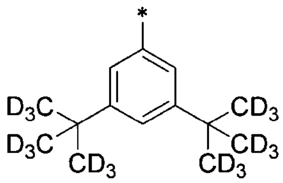

Ar35

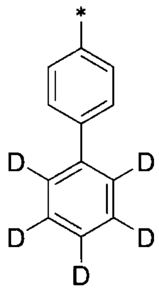

Ar36

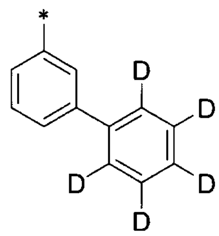

Ar37

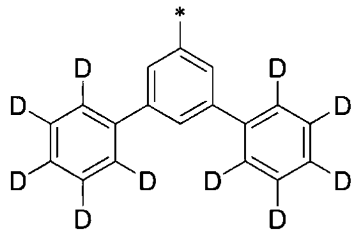

Ar38

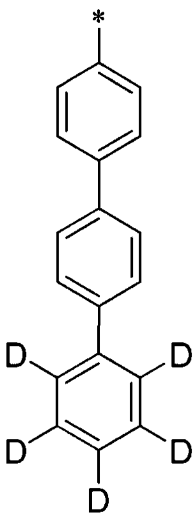

Ar39

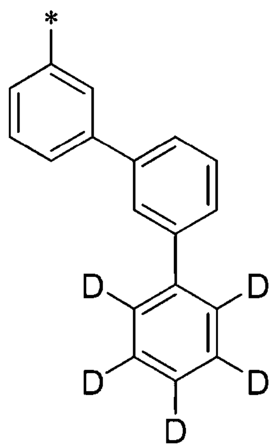

Ar40

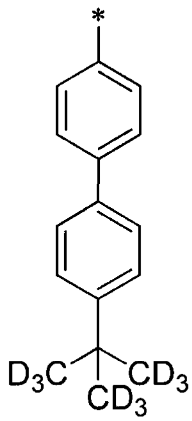

Ar41

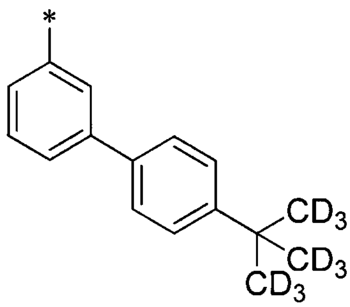

Ar42

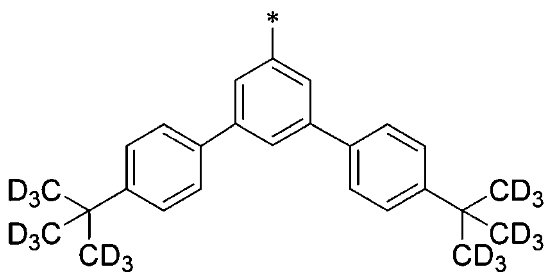

Ar43

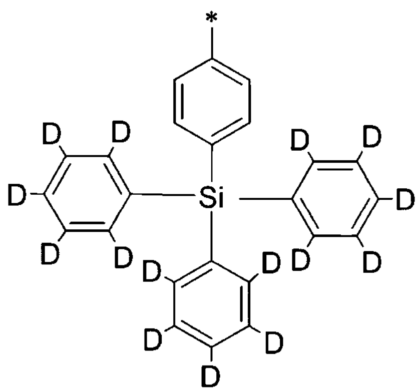

Ar44

In addition to the above-mentioned specific examples, groups obtained by substituting all hydrogen atoms present in Ar1 to Ar44 with deuterium atoms are disclosed as Ar45 to Ar88 in that order.

In one aspect of the present invention, the aryl group that $R^1$ to $R^5$ can take is selected from the group consisting of Ar1 to Ar88. In one aspect of the present invention, the aryl group that $R^1$ to $R^5$ can take is Ar1 or Ar45. In one aspect of the present invention, the aryl group that $R^1$ to $R^5$ can take is selected from the group consisting of Ar2 to Ar11, Ar26 to Ar35, Ar46 to Ar55, and Ar70 to Ar79. In one aspect of the present invention, the aryl group that $R^1$ to $R^5$ can take is selected from the group consisting of Ar12 to Arl9, Ar36 to Ar43, Ar56 to Ar63, and Ar80 to Ar87. In one aspect of the present invention, the aryl group that $R^1$ to $R^5$ can take is selected from the group consisting of Ar21 to Ar26 and Ar65 to Ar70. In one preferred aspect of the present invention, the aryl group that $R^1$ to $R^5$ can take is selected from the group consisting of Ar1, Ar12 to Ar14, Ar23, Ar36 to Ar38, Ar45, Ar56 to Ar58, Ar67, and Ar80 to Ar82.

In one aspect of the present invention, the aryl group that $Ar^1$ and $Ar^2$ can take is selected from the group consisting of Ar1 to Ar88. In one aspect of the present invention, the aryl group that $Ar^1$ and $Ar^2$ can take is Ar1 or Ar45. In one aspect of the present invention, the aryl group that $Ar^1$ or $Ar^2$ can take is selected from the group consisting of Ar2 to Ar11, Ar26 to Ar35, Ar46 to Ar55, and Ar70 to Ar79. In one aspect of the present invention, the aryl group that $Ar^1$ or $Ar^2$ can take is selected from the group consisting of Ar12 to Arl9, Ar36 to Ar43, Ar56 to Ar63, and Ar80 to Ar87. In one preferred aspect of the present invention, the aryl group that $R^1$ to $R^5$ can take is selected from the group consisting of Ar1, Ar12 to Ar14, Ar36 to Ar38, Ar45, Ar56 to Ar58, and Ar80 to Ar82.

At least two of $R^1$ to $R^5$ in the general formula (1) represent a donor group. The donor group that $R^1$ to $R^5$ can take does not include a substituted or unsubstituted aryl group.

The "donor group" can be selected from groups having a negative Hammett's σp value. The "acceptor group" can be selected from groups having a positive Hammett's σp value. The Hammett's σp value is proposed by L. P. Hammett and quantifies the influence of a substituent on the reaction rate or equilibrium of a para-substituted benzene derivative. Specifically, the value is a constant (σp) specific to the substituent in the following formula that is established between substituents and reaction rate constants or equilibrium constants in para-substituted benzene derivatives:

$$\log(k/k_0) = \rho\sigma p$$

or

$\log(K/K_0) = \rho\sigma p$. In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and ρ represents a reaction constant to be determined by the kind and the condition of reaction. Regarding the description relating to the "Hammett's σp value" and the numerical value of each substituent in the present invention, reference can be made to the description relating to σp value in Hansch, C. et. al., Chem. Rev., 91, 165-195 (1991).

The donor group that $R^1$ to $R^5$ can take preferably has σp of -0.3 or less, more preferably -0.5 or less, and still more preferably -0.7 or less. For example, the value can be selected from a range of -0.9 or less, or from a range of -1.1 or less.

The donor group in the present invention is preferably a group containing a substituted amino group. The donor group can be a substituted amino group, or can be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a substituted or unsubstituted heteroaryl group, more preferably a substituted or unsubstituted aryl group or a substituted or unsubstituted heteroaryl group. Particularly, the substituted amino group is preferably a substituted or unsubstituted diarylamino group, or a substituted or unsubstituted diheteroarylamino group. As referred to herein, the two aryl groups constituting the diarylamino group can bond to each other, and the two heteroaryl groups constituting the diheteroarylamino group can bond to each other.

The donor group that $R^1$ to $R^5$ can take is preferably a group represented by the following general formula (a).

General Formula (a)

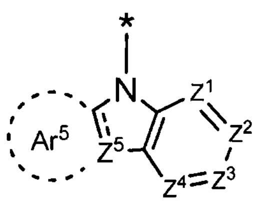

In the general formula (a), $Z^1$ represents C-$R^{14}$ or N, $Z^2$ represents C-$R^{15}$ or N, $Z^3$ represents C-$R^{16}$ or N, and $Z^4$

represents C-$R^{17}$ or N. $Z^5$ represents C or N, $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each can bond to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent can be selected from Substituent Group A, can be selected from Substituent Group B, can be selected from Substituent Group C, can be selected from Substituent Group D, or can be selected from Substituent Group E. When two or more of $R^{14}$ to $R^{17}$ represent substituents, the two or more substituents can be the same or different. Zero to two of $R^{14}$ to $R^{17}$ preferably represent a substituent, and for example, one can be a substituent, or zero can be a substituent ($R^{14}$ to $R^{17}$ represent a hydrogen atom or a deuterium atom).

$R^{14}$ and $R^{15}$, $R^{15}$ and $R^{16}$, and $R^{16}$ and $R^{17}$ each can bond to each other to form a cyclic structure. The cyclic structure can be any of an aromatic ring, an heteroaromatic ring, an aliphatic hydrocarbon ring, and an aliphatic heterocyclic ring, and can be a ring obtained by fusing these rings. The structure is preferably an aromatic ring or a heteroaromatic ring. Examples of the aromatic ring include a substituted or unsubstituted benzene ring. Another benzene ring can be further fused to the benzene ring, and a heterocyclic ring such as a pyridine ring can be fused to the benzene ring. The heteroaromatic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein can be unsubstituted, can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. It is preferable that a substituted or unsubstituted aryl group bonds to the nitrogen atom constituting the pyrrole ring of indole, and examples of the substituent include a substituent selected from any of Substituent Group A to Substituent Group E. The cyclic structure can be a substituted or unsubstituted cyclopentadiene ring. In one aspect of the present invention, a pair of $R^{14}$ and $R^{15}$, $R^{15}$ and $R^{16}$, and $R^{16}$ and $R^{17}$ bonds to each other to form a cyclic structure. In one aspect of the present invention, none of $R^{14}$ and $R^{15}$, $R^{15}$ and $R^{16}$, and $R^{16}$ and $R^{17}$ bonds to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ represents C, and $Ar^5$ represents a substituted or unsubstituted aromatic ring or a substituted or unsubstituted heteroaromatic ring. In one aspect of the present invention, $Z^5$ represents N, and $Ar^5$ represents a substituted or unsubstituted heteroaromatic ring.

Examples of the aromatic ring that $Ar^5$ can take include a benzene ring. Another benzene ring can be further fused to the benzene ring, and a heterocyclic ring such as a pyridine ring can be fused to the benzene ring. The heteroaromatic ring that $Ar^5$ can take is preferably a 5- to 7-membered ring, and for example, a 5-membered ring or a 6-membered ring can be employed. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ represents C, and the heteroaromatic ring is a furan ring of a substituted or unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ represents N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole and benzimidazole referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E.

When $Z^5$ in the general formula (a) represents C, a group represented by the following general formula (b) is preferred.

General Formula (b)

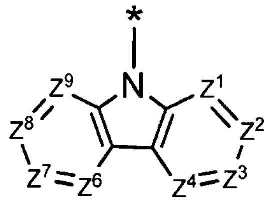

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{18}$ and $R^{19}$, $R^{19}$ and $R^{20}$, and $R^{20}$ and $R^{21}$ each can bond to each other to form a cyclic structure.

Regarding $Z^1$ to $Z^4$ and $R^{14}$ to $R^{17}$ in the general formula (b), reference can be made to the corresponding description of the general formula (a). $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and regarding the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

The donor group that $R^1$ to $R^5$ can take is preferably a substituted or unsubstituted carbazol-9-yl group. The carbazol-9-yl group referred to herein can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. One or more rings can be fused to the two benzene rings constituting the carbazol-9-yl group. In one preferred aspect of the present invention, the donor group that $R^1$ to $R^5$ can take is a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2 to 7-positions, more preferably at least one of 3 to 6-positions, and still more preferably a 3-position and a 6-position.

In one aspect of the present invention, the donor group that $R^1$ to $R^5$ can take is a carbazol-9-yl group fused with one or more rings, and hereinafter, this will be referred to as a "ring-fused carbazol-9-yl group". The ring-fused carbazol-9-yl group that $R^1$ to $R^5$ can take can be unsubstituted, or can be substituted with a substituent selected from Substituent Group A, can be substituted with a substituent selected from Substituent Group B, can be substituted with a substituent selected from Substituent Group C, can be substituted with a substituent selected from Substituent Group D, and can be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group and an aryl group or with a group formed by combining two or more thereof.

The number of rings constituting the fused ring in the ring-fused carbazol-9-yl group is 4 or more, preferably 5 or more, more preferably 5 to 9, and still more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group that bonds via the nitrogen atom constituting the ring skeleton of carbazole, and has a structure in which a ring is fused to at least one of the two benzene rings constituting carbazole. The fused ring can be any of an aromatic hydrocarbon ring, an aromatic heterocyclic ring, an aliphatic hydrocarbon ring, and an aliphatic heterocyclic ring, and can be a ring obtained by further fusing these rings. An aromatic hydrocarbon ring and an aromatic heterocyclic ring are preferable. Examples of the aromatic hydrocarbon ring include a substituted or unsubstituted benzene ring. Another benzene ring can be further fused to the benzene ring, and a heterocyclic ring such as a pyridine ring can be fused to the benzene ring. The aromatic heterocyclic ring means a ring exhibiting aromaticity including a heteroatom as a ring skeleton-constituting atom, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) bonds to the nitrogen atom of the pyrrole ring, and it is more

preferable that an aryl group which can be substituted with an alkyl group or an aryl group is bonded. In the present invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indolo structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

The ring-fused carbazol-9-yl group employable herein includes a benzofuro[2,3-a]carbazol-9-yl group, a benzofuro[3,2-a]carbazol-9-yl group, a benzofuro[2,3-b]carbazol-9-yl group, a benzofuro[3,2-b]carbazol-9-yl group, a benzofuro[2,3-c]carbazol-9-yl group, and a benzofuro[3,2-c]carbazol-9-yl group. In addition, the ring-fused carbazol-9-yl group also employable herein includes a benzothieno[2,3-a]carbazol-9-yl group, a benzothieno[3,2-a]carbazol-9-yl group, a benzothieno[2,3-b]carbazol-9-yl group, a benzothieno[3,2-b]carbazol-9-yl group, a benzothieno[2,3-c]carbazol-9-yl group, and a benzothieno[3,2-c]carbazol-9-yl group. The ring-fused carbazol-9-yl group also employable herein includes an indolo[2,3-a]carbazol-9-yl group, an indolo[3,2-a]carbazol-9-yl group, an indolo[2,3-b]carbazol-9-yl group, an indolo[3,2-b]carbazol-9-yl group, an indolo[2,3-c]carbazol-9-yl group, and an indolo[3,2-c]carbazol-9-yl group.

The number of the substituents substituted on the ring-fused carbazol-9-yl group is preferably 1 to 10, more preferably 1 to 6, even more preferably 1 to 4, and can be, for example 1, or can be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the heteroatom present in the ring-fused carbazol-9-yl group. In one preferred aspect of the present invention, the compound has at least one substituent only on the para-position of the benzene ring viewed from the heteroatom present in the ring-fused carbazol-9-yl group. In one preferred aspect of the present invention, the compound has substituents on all the substitutable para-positions of the benzene ring viewed from the heteroatom present in the ring-fused carbazol-9-yl group.

Specific examples of the donor group that $R^1$ to $R^5$ in the general formula (1) can take are shown below. The donor group which can be employed in the present invention shall not be construed as being limited by the following specific examples. In the following specific examples, Ph indicates a phenyl group ($C_6H_5$), and * indicates a bonding position. A methyl group is not shown, and for example, D2 has one methyl group. A deuterated methyl group is expressed as $CD_3$. $C_6D_5$ represents a phenyl group in which all hydrogen atoms are deuterated. D represents a deuterium atom.

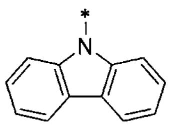
D1

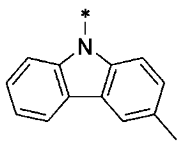
D2

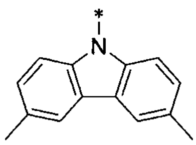
D3

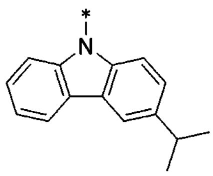
D4

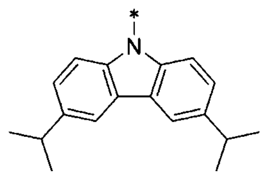
D5

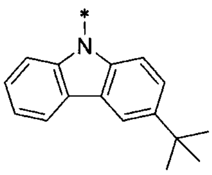
D6

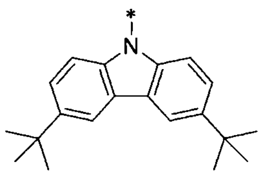
D7

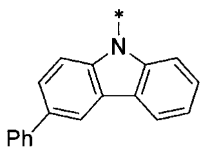
D8

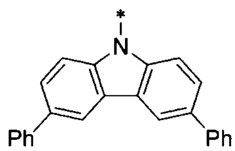
D9

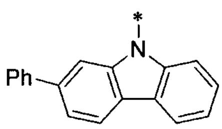
D10

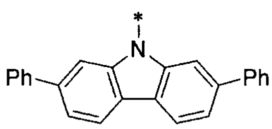
D11

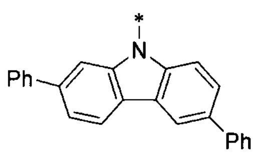
D12

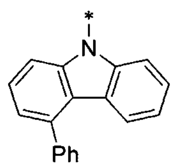
D13

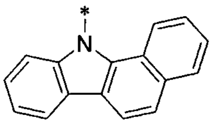 D14

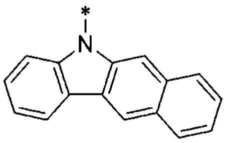 D15

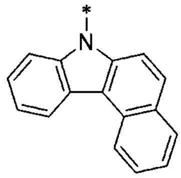 D16

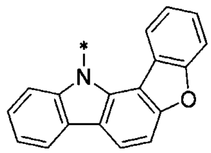 D17

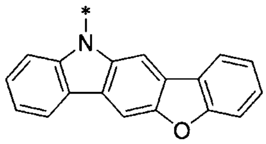 D18

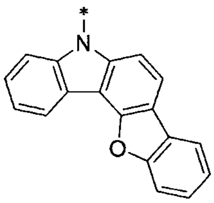 D19

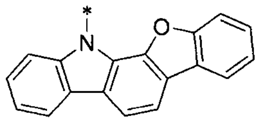 D20

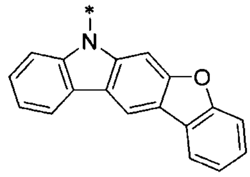 D21

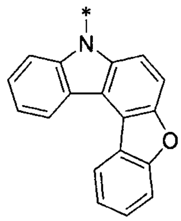 D22

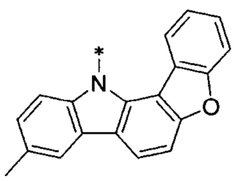 D23

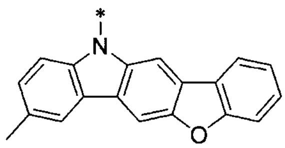 D24

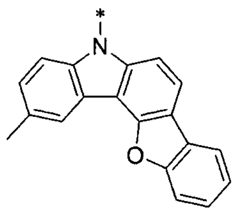 D25

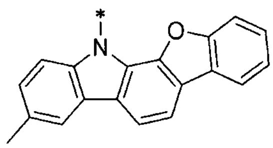 D26

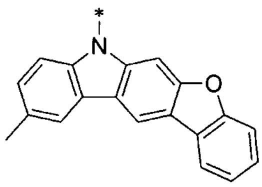 D27

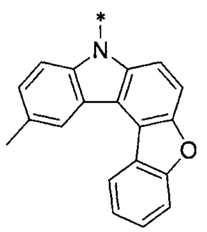 D28

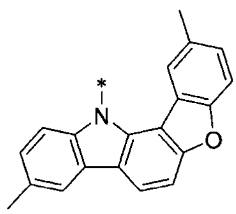 D29

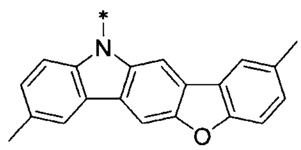 D30

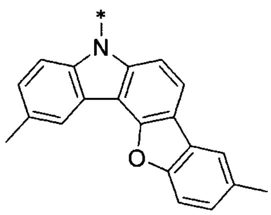 D31

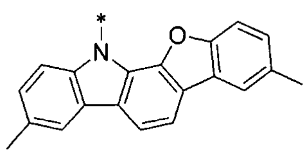 D32

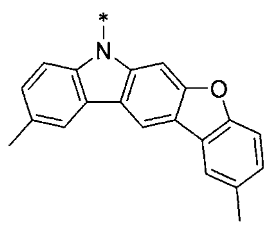 D33

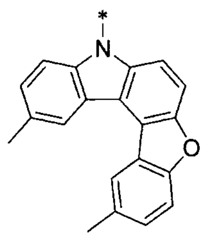 D34

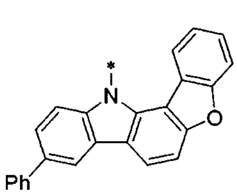
D35

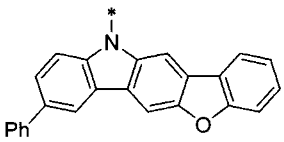
D36

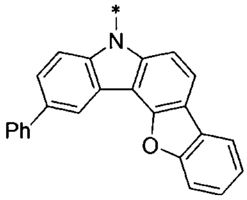
D37

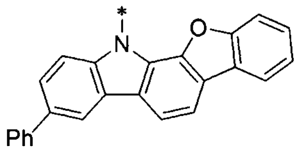
D38

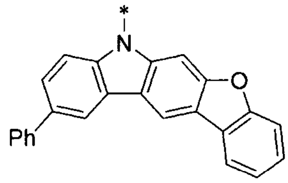
D39

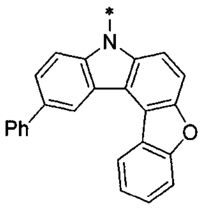
D40

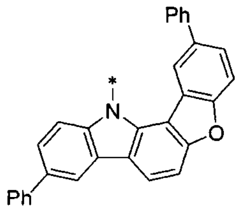
D41

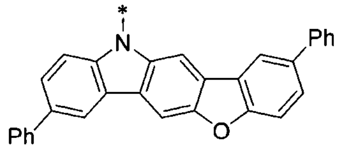
D42

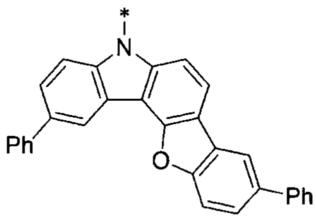
D43

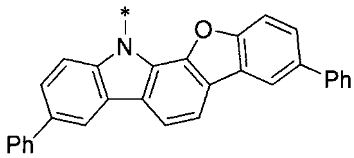
D44

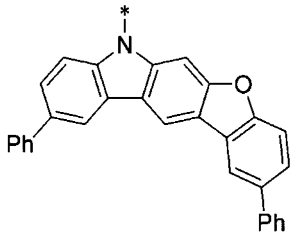
D45

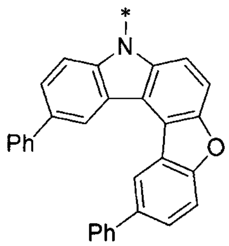
D46

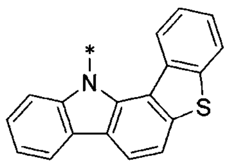
D47

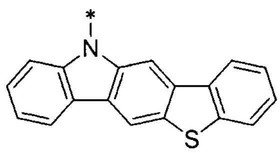
D48

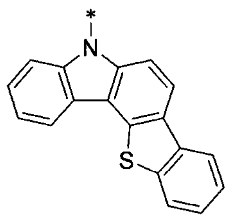
D49

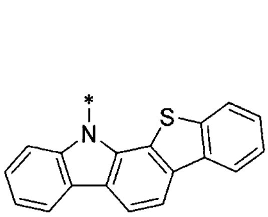
D50

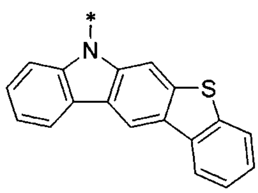
D51

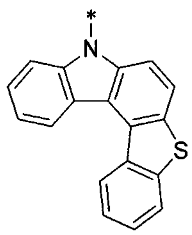
D52

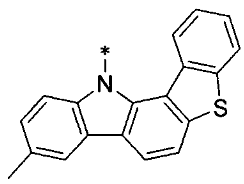
D53

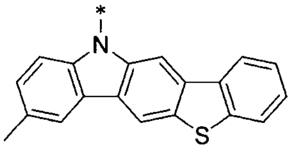
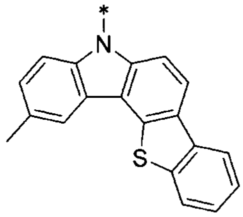
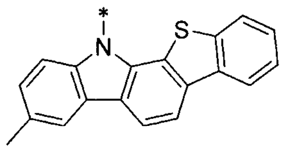
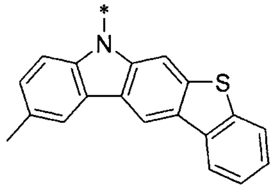

D54 D55 D56 D57

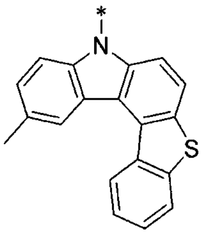
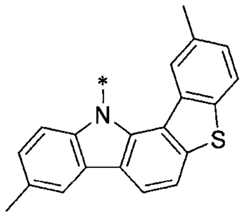
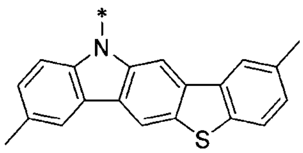
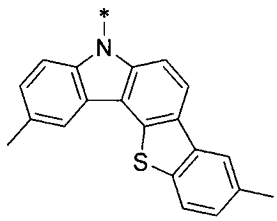

D58 D59 D60 D61

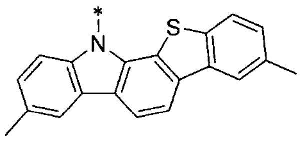
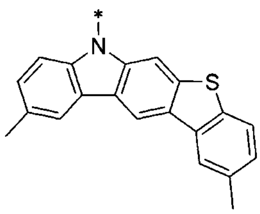
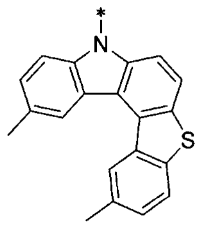
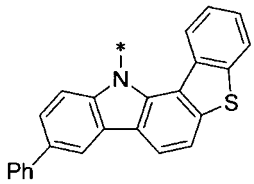

D62 D63 D64 D65

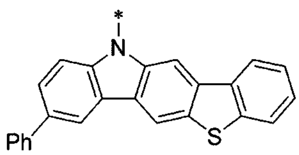
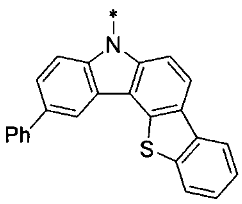
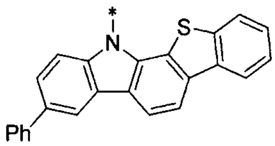
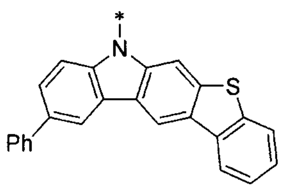

D66 D67 D68 D69

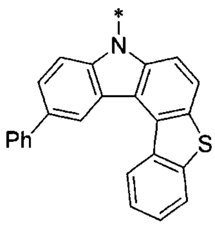
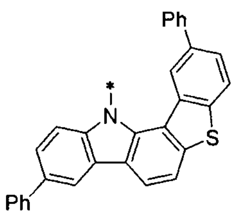
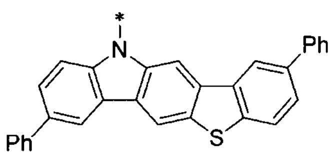
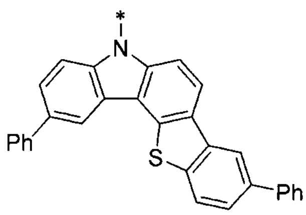

D70 D71 D72 D73

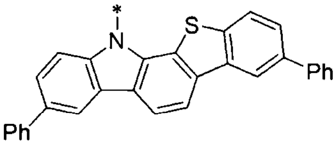
D74

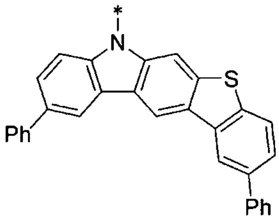
D75

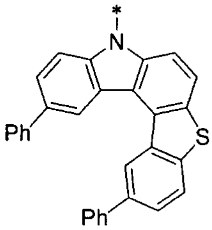
D76

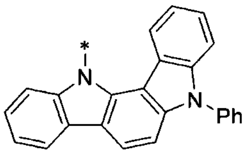
D77

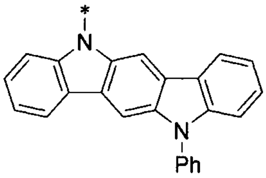
D78

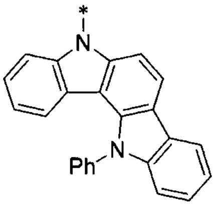
D79

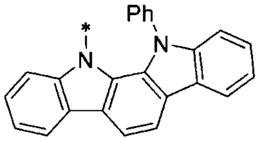
D80

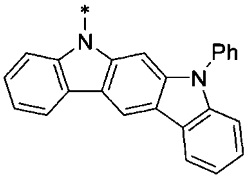
D81

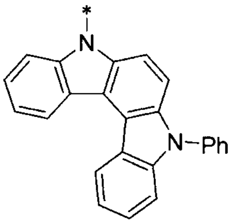
D82

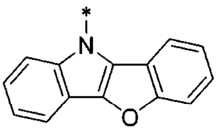
D83

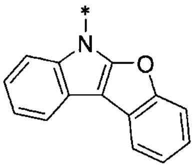
D84

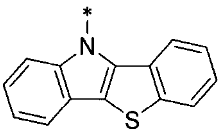
D85

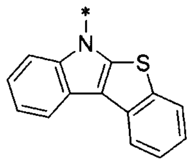
D86

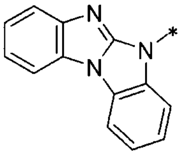
D87

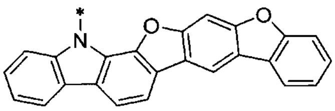
D88

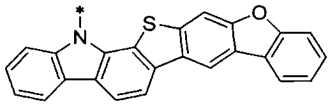
D89

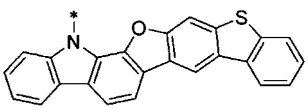
D90

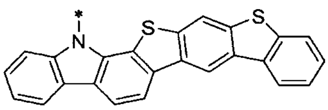
D91

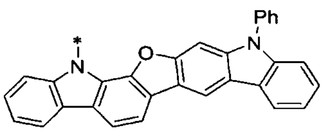
D92

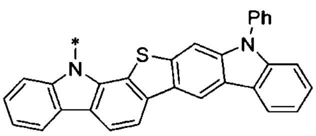
D93

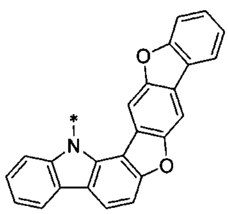
D94

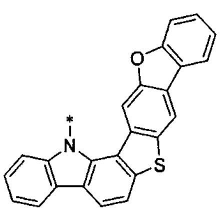
D95

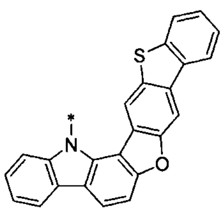
D96

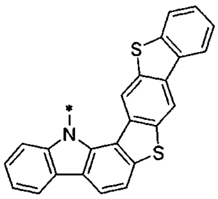
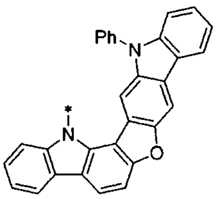
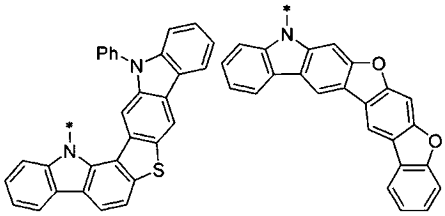
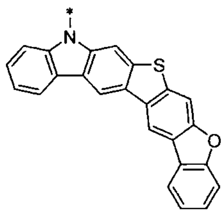

D97 D98 D99 D100 D101

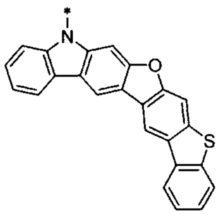
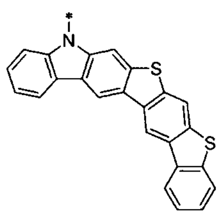
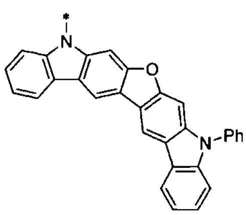
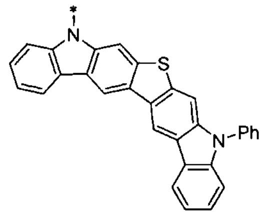

D102 D103 D104 D105

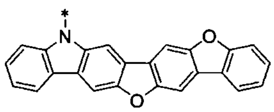
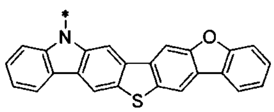
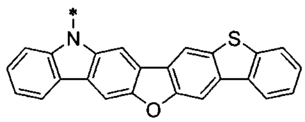

D106 D107 D108

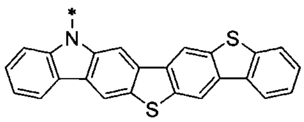
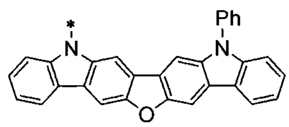
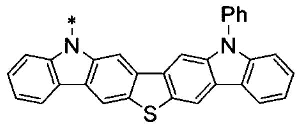

D109 D110 D111

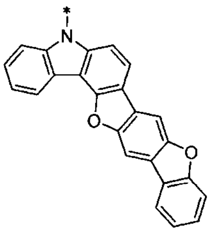
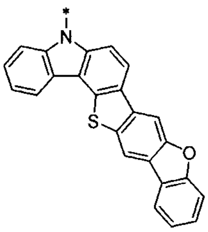
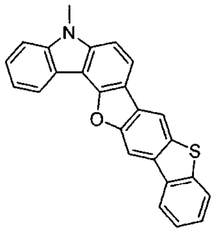
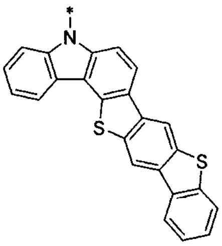
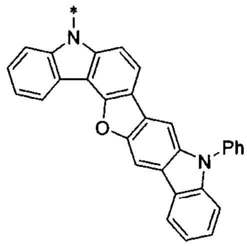

D112 D113 D114 D115 D116

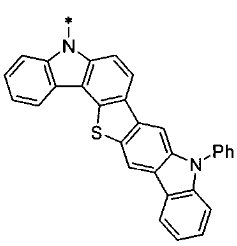
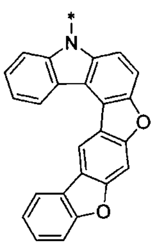
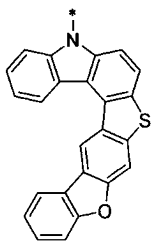
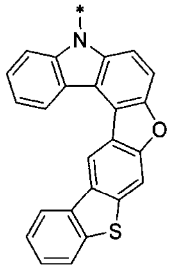
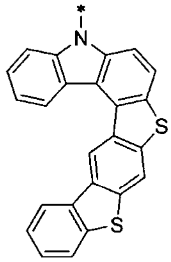
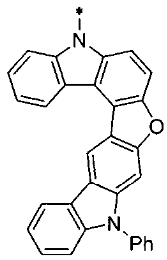

D117 D118 D119 D120 D121 D122

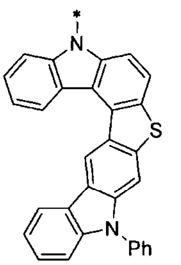

D123

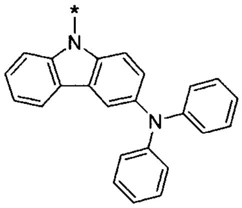

D124

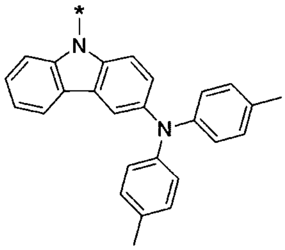

D125

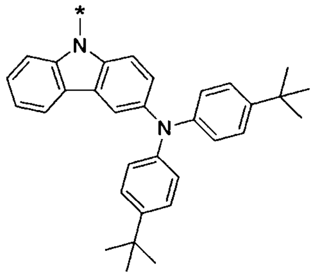

D126

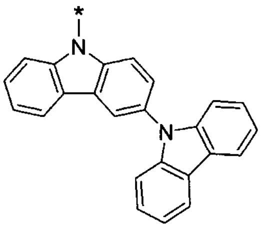

D127

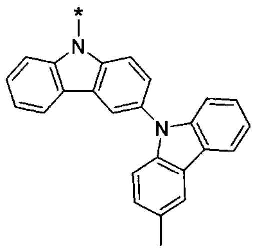

D128

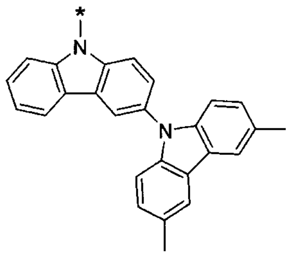

D129

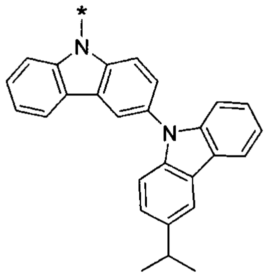

D130

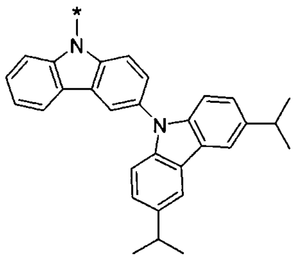

D131

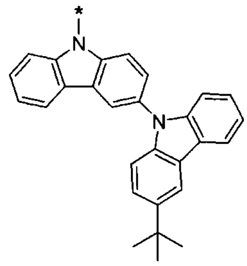

D132

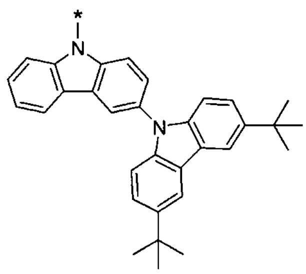

D133

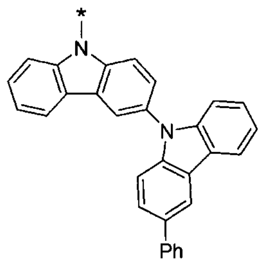

D134

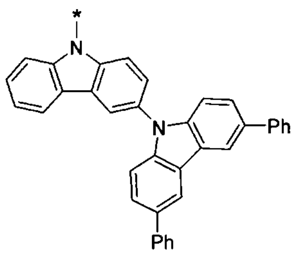

D135

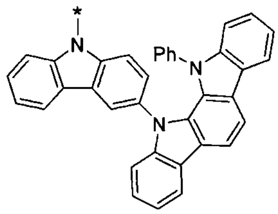

D136

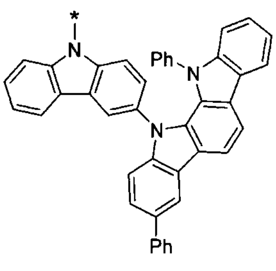

D137

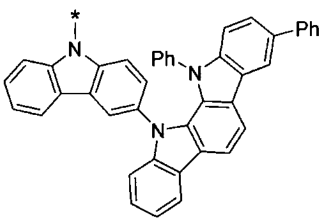

D138

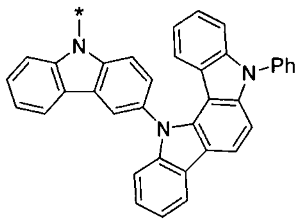

D139

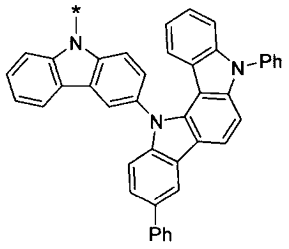

D140

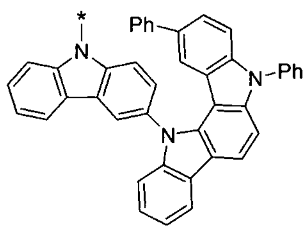

D141

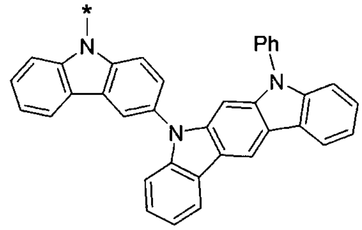

D142

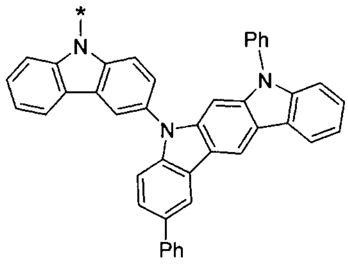

D143

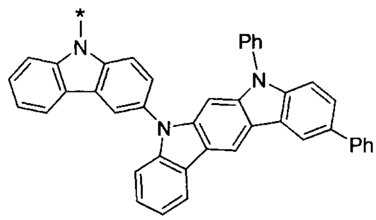

D144

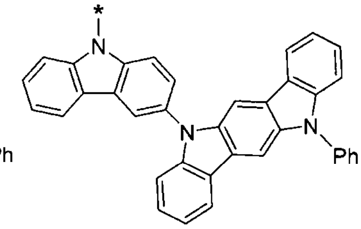

D145

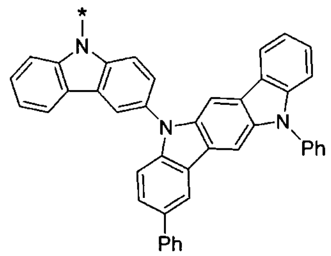

D146

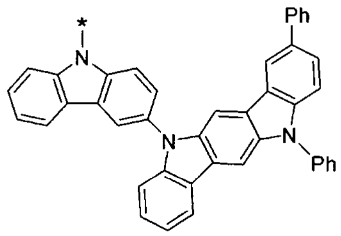

D147

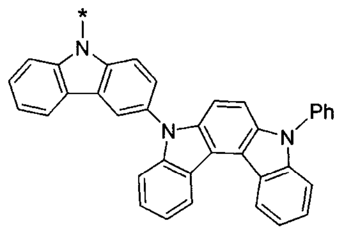

D148

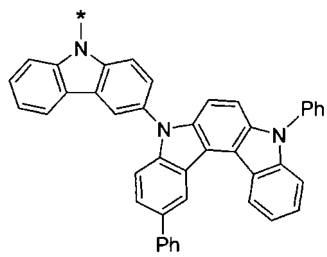

D149

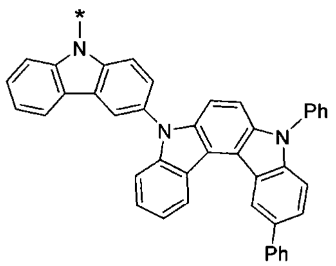

D150

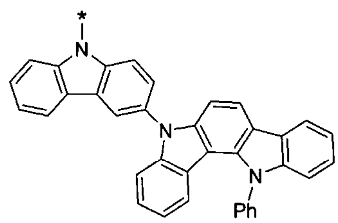

D151

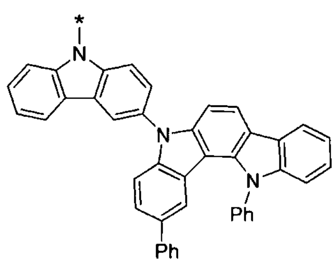

D152

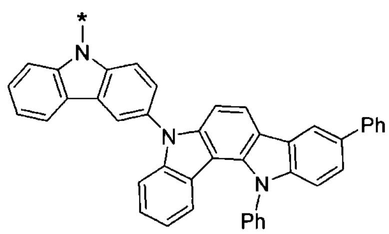

D153

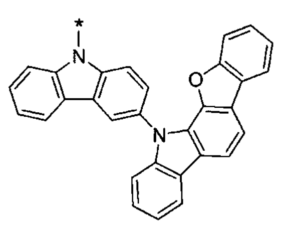

D154

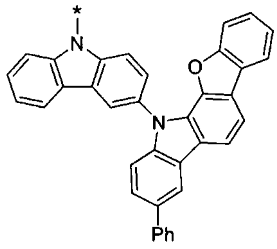

D155

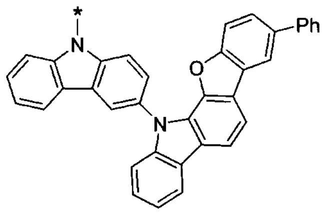

D156

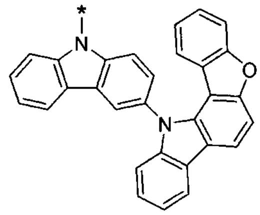

D157

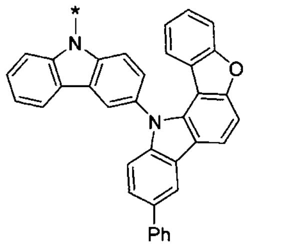

D158

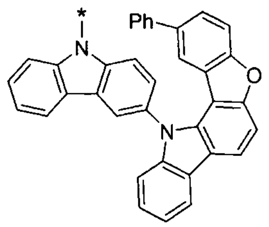

D159

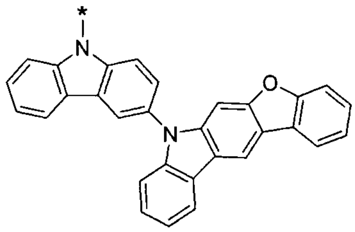

D160

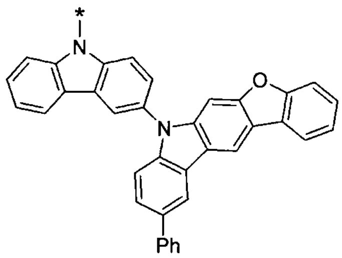

D161

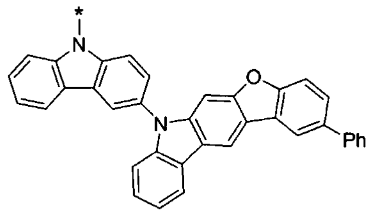

D162

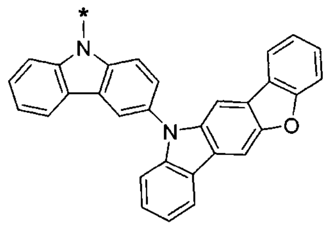

D163

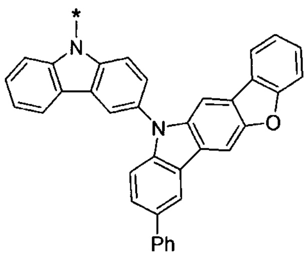

D164

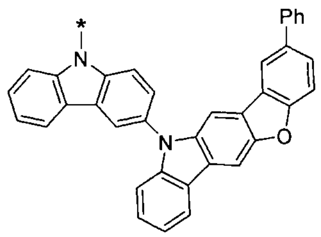

D165

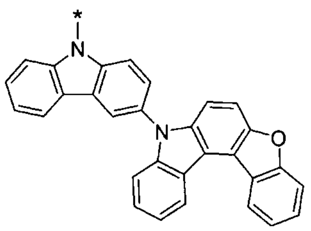

D166

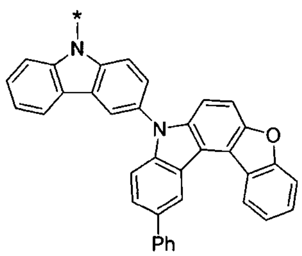

D167

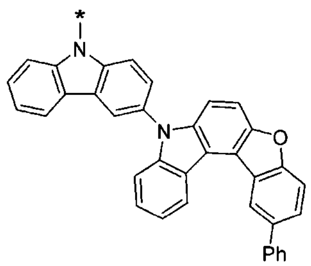

D168

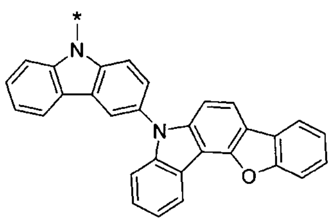

D169

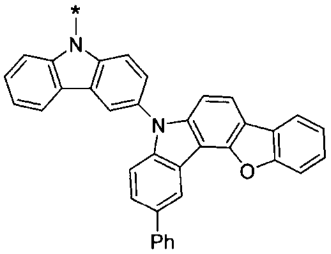 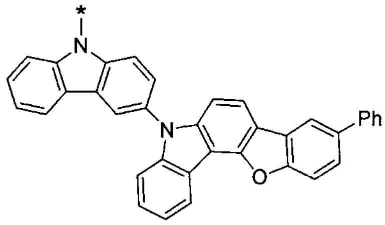 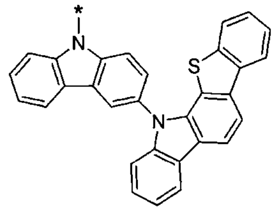

D170 D171 D172

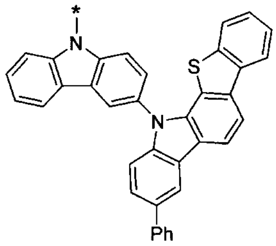 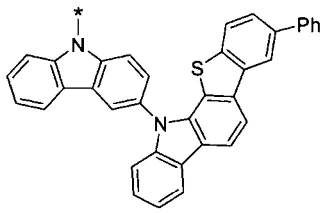 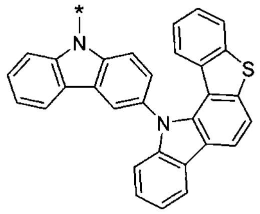

D173 D174 D175

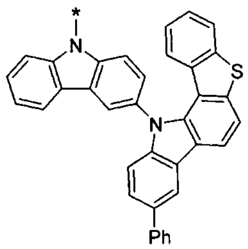 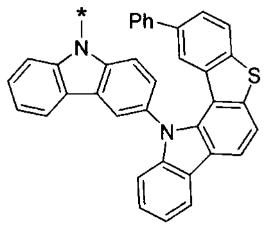 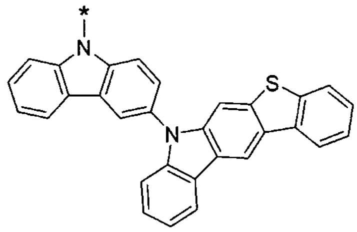

D176 D177 D178

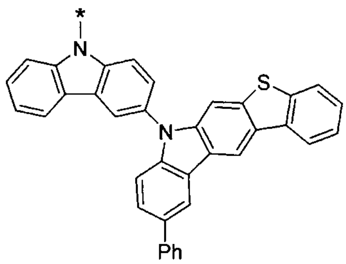 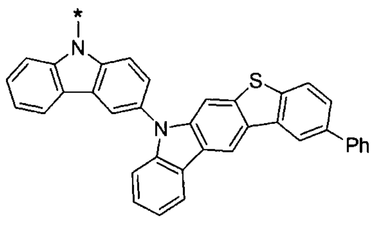 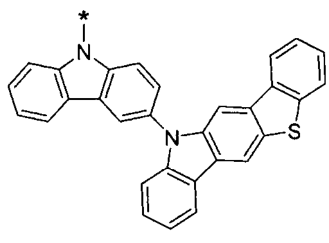

D179 D180 D181

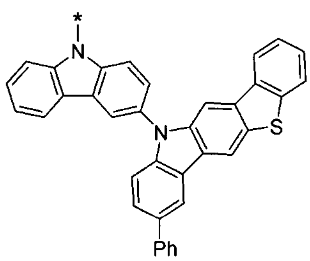 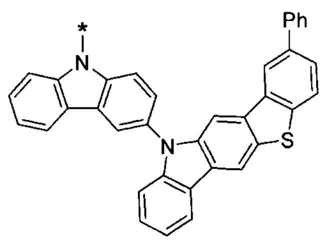 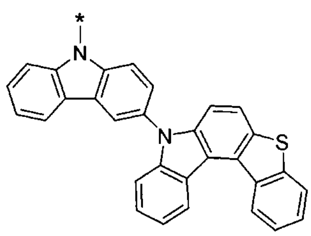

D182 D183 D184

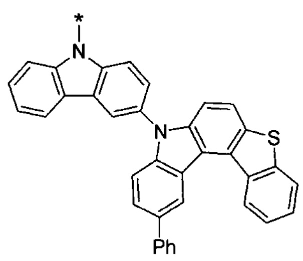
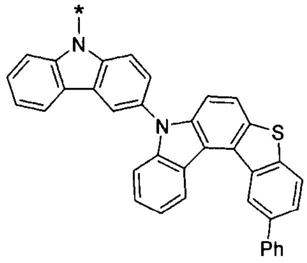
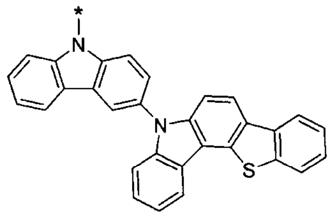

D185 D186 D187

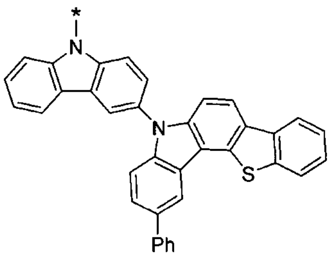
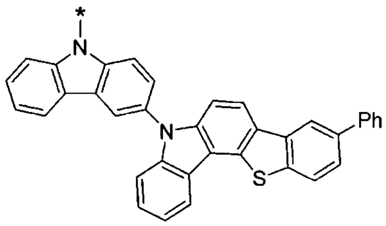
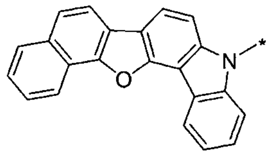

D188 D189 D190

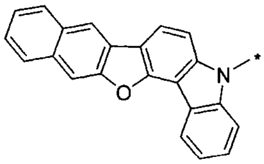
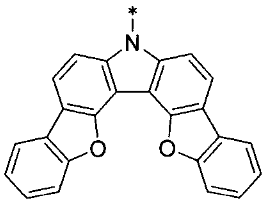
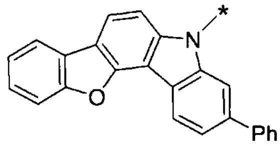
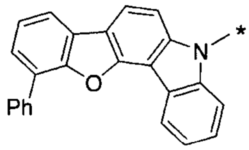

D191 D192 D193 D194

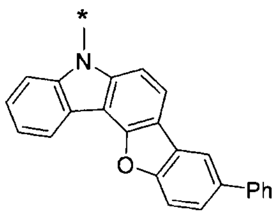
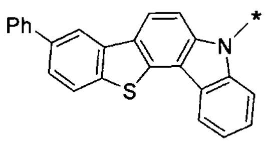
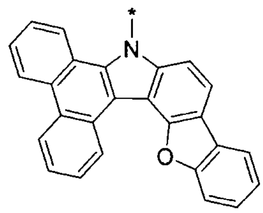
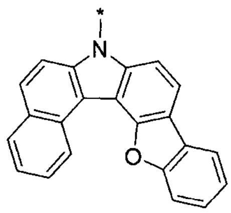

D195 D196 D197 D198

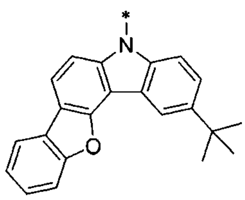
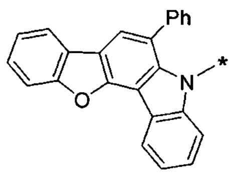
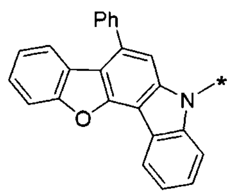
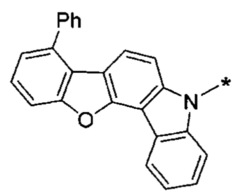

D199 D200 D201 D202

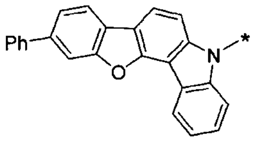
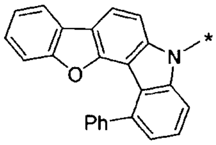
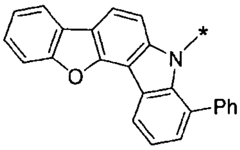
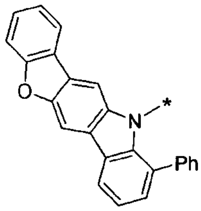
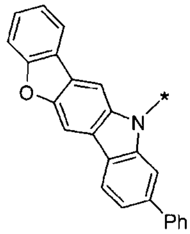

D203 D204 D205 D206 D207

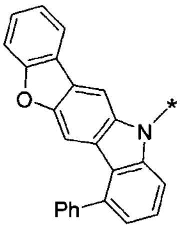
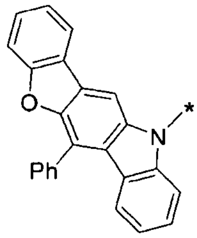
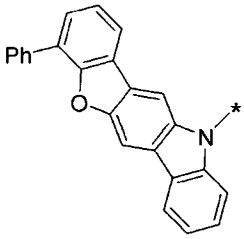
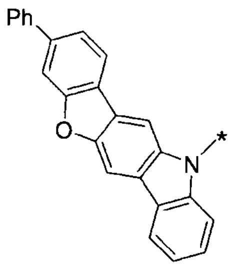
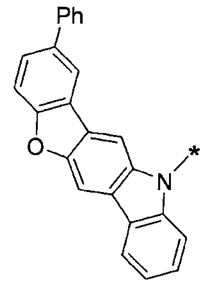

D208 D209 D210 D211 D212

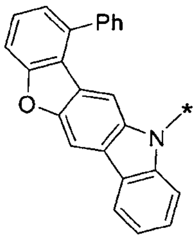
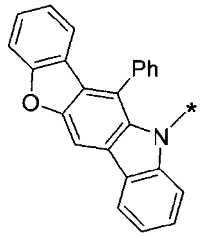
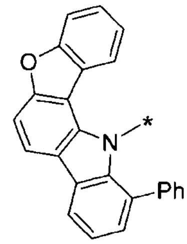
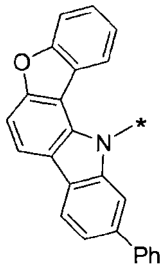

D213 D214 D215 D216

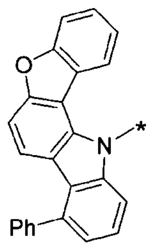
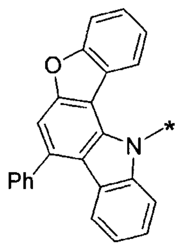
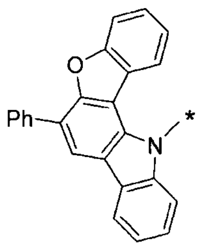
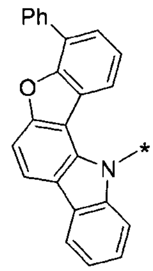

D217 D218 D219 D220

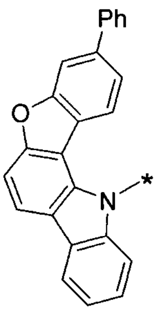
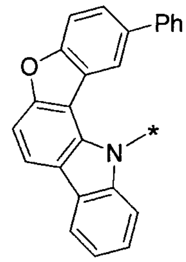
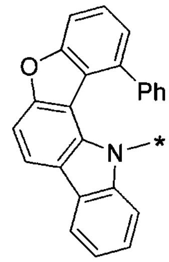
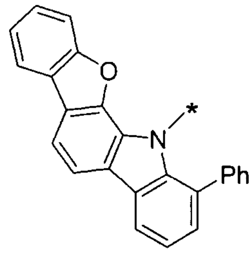

D221 D222 D223 D224

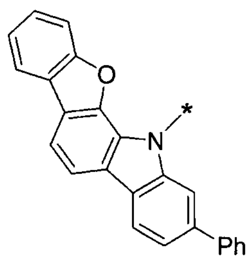
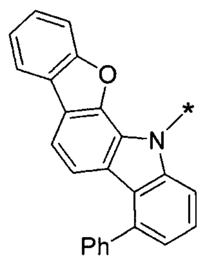
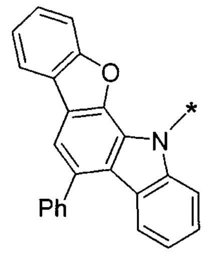
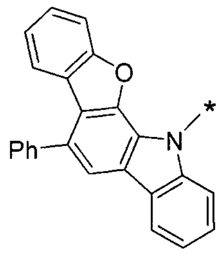

D225 D226 D227 D228

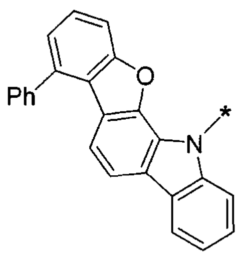
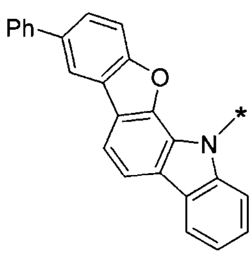
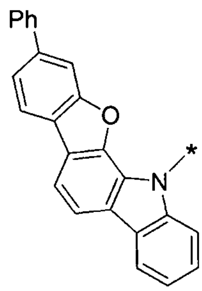
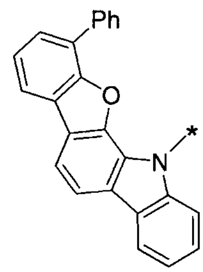

D229 D230 D231 D232

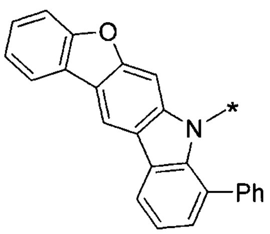
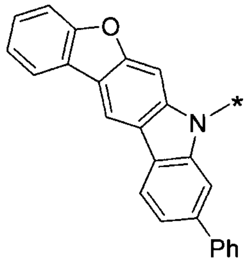
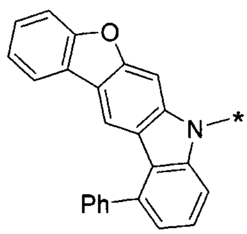
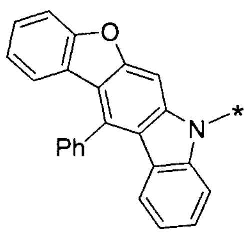

D233 D234 D235 D236

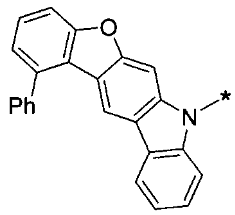 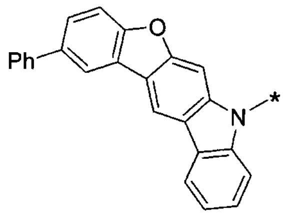 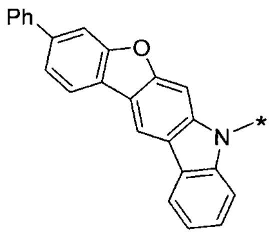 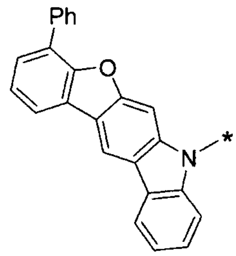

D237 D238 D239 D240

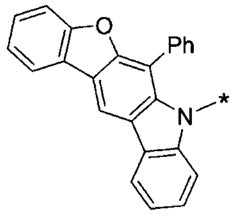 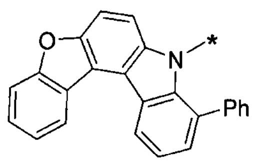 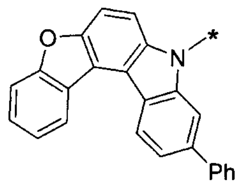 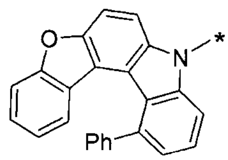

D241 D242 D243 D244

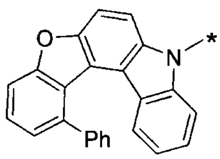 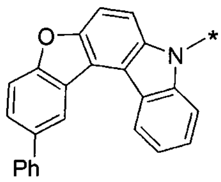 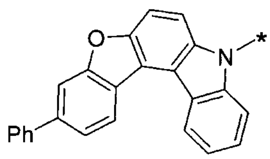 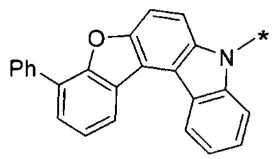

D245 D246 D247 D248

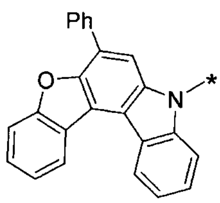 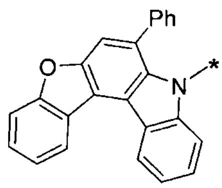 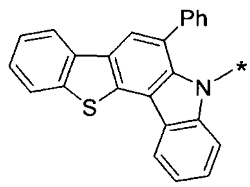 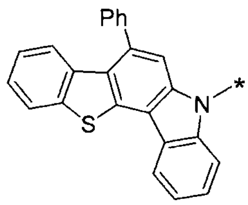

D249 D250 D251 D252

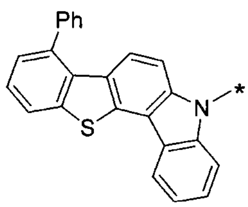 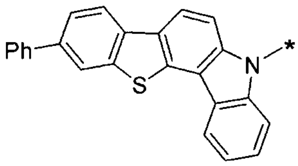 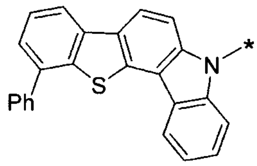 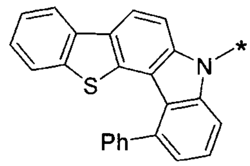

D253 D254 D255 D256

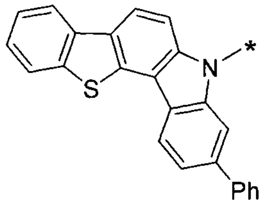
D257

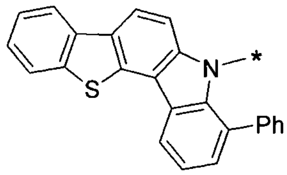
D258

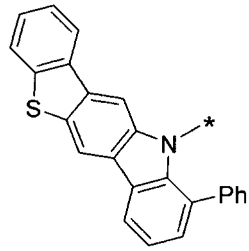
D259

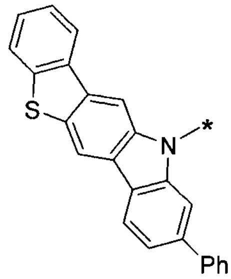
D260

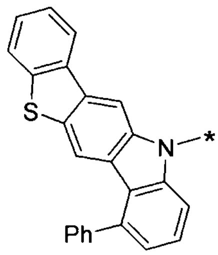
D261

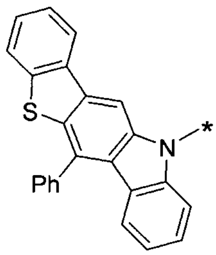
D262

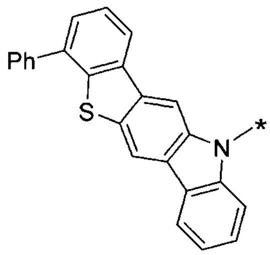
D263

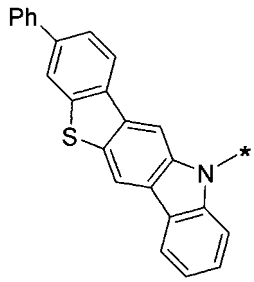
D264

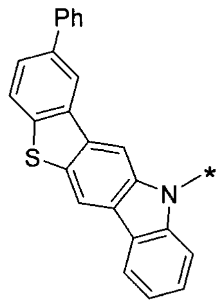
D265

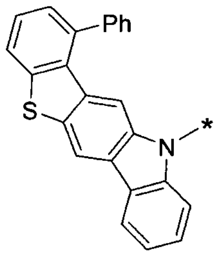
D266

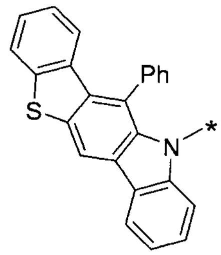
D267

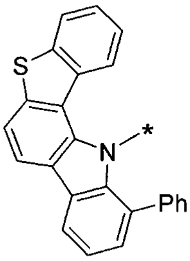
D268

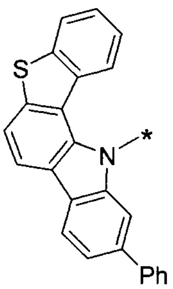
D269

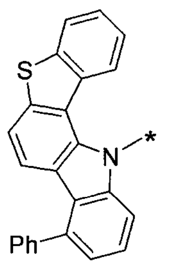
D270

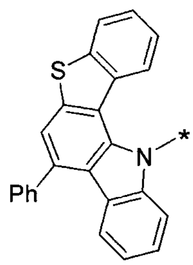
D271

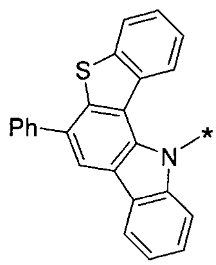
D272

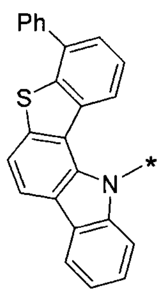

D273

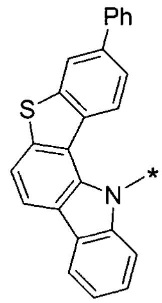

D274

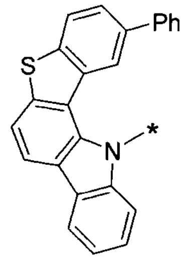

D275

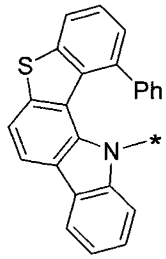

D276

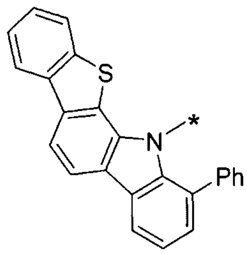

D277

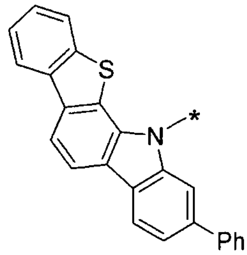

D278

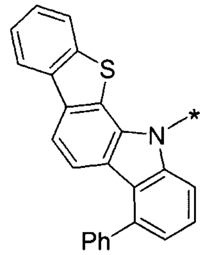

D279

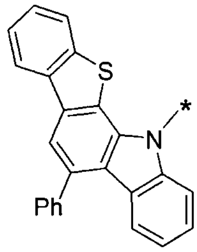

D280

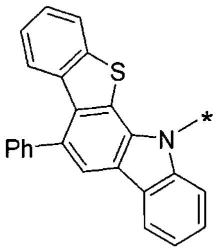

D281

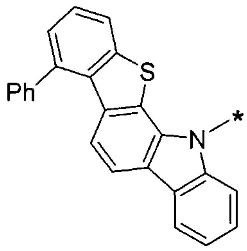

D282

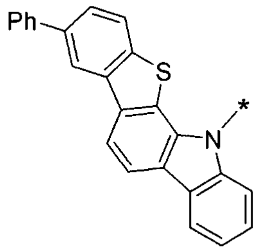

D283

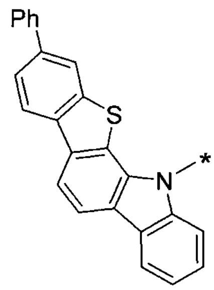

D284

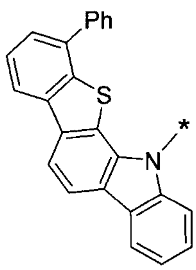

D285

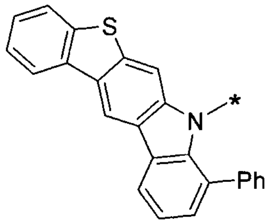

D286

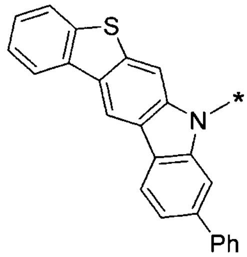

D287

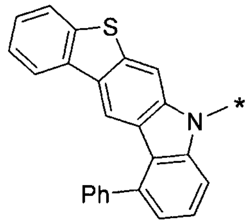

D288

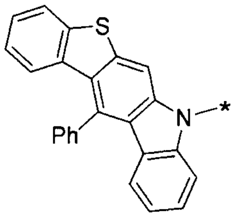

D289

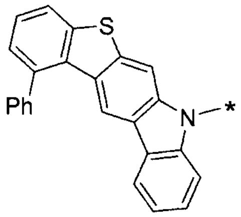

D290

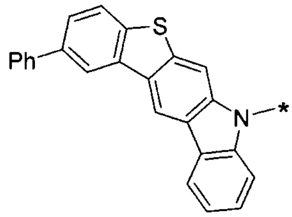

D291

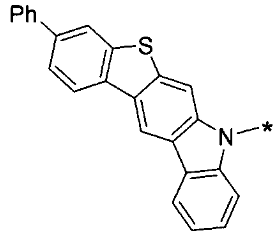

D292

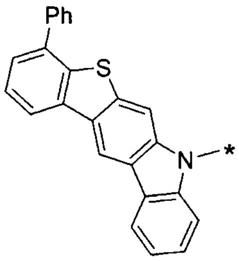

D293

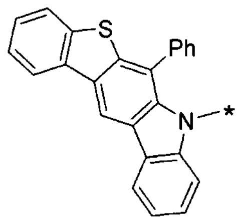

D294

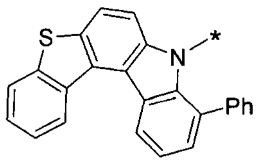

D295

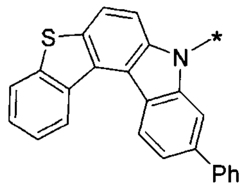

D296

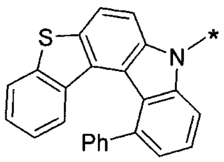

D297

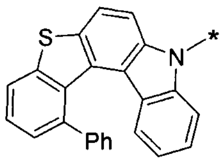

D298

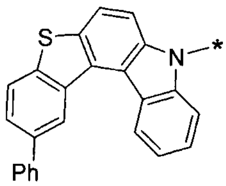

D299

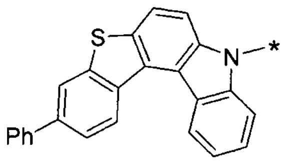

D300

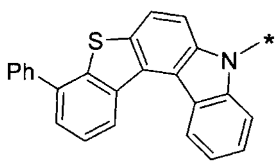

D301

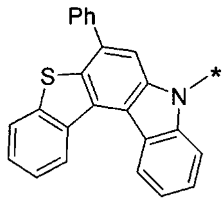

D302

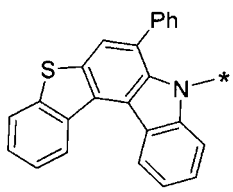

D303

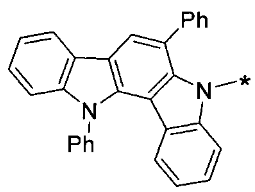

D304

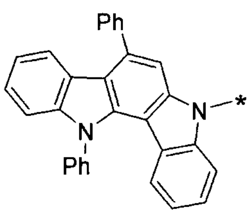

D305

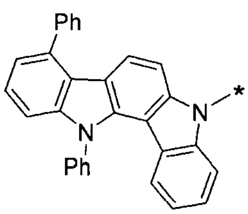

D306

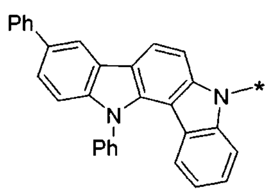

D307

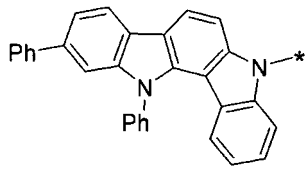

D308

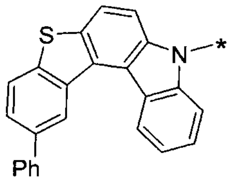
D309

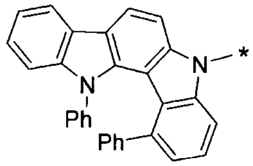
D310

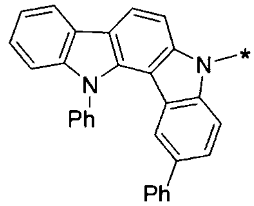
D311

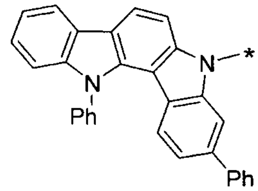
D312

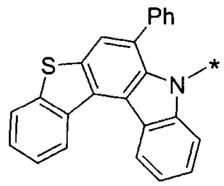
D313

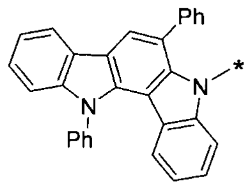
D314

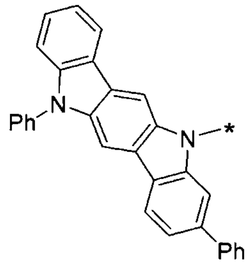
D315

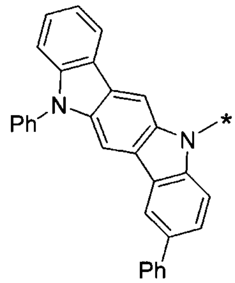
D316

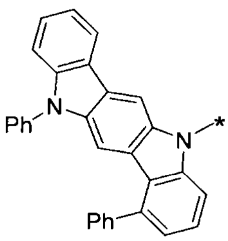
D317

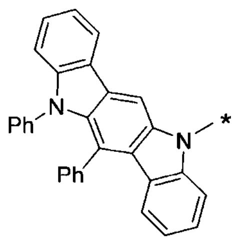
D318

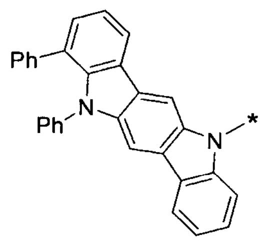
D319

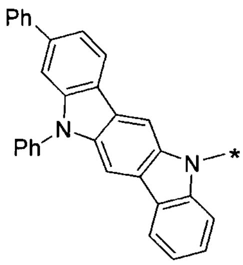
D320

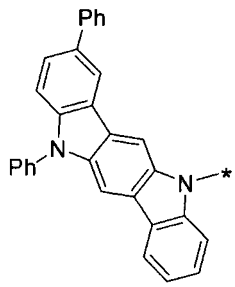
D321

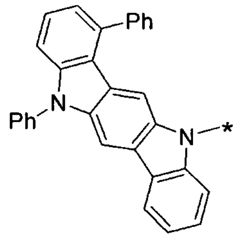
D322

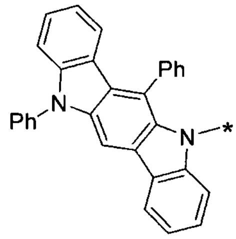
D323

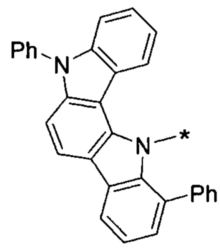
D324

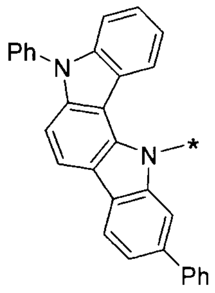

D325

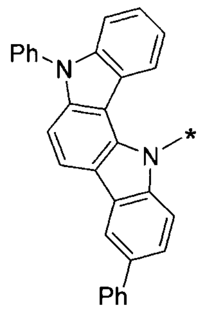

D326

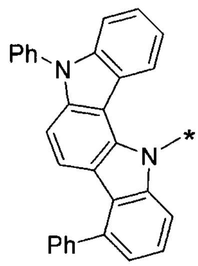

D327

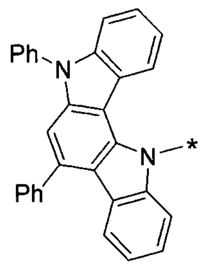

D328

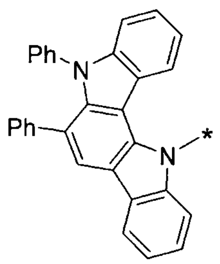

D329

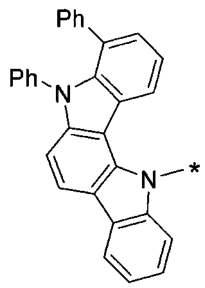

D330

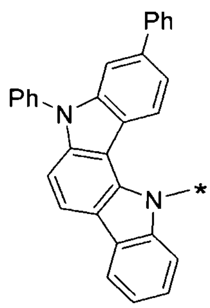

D331

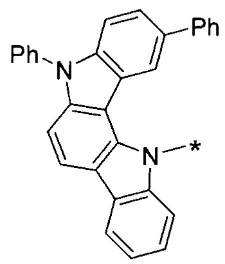

D332

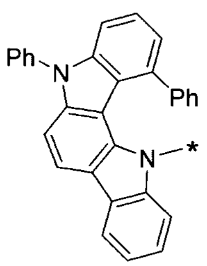

D333

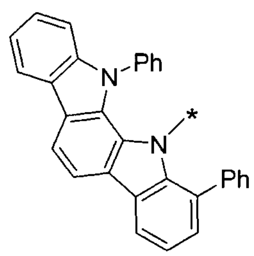

D334

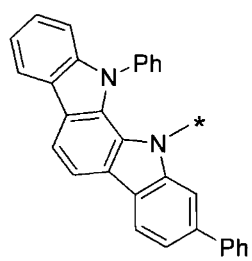

D335

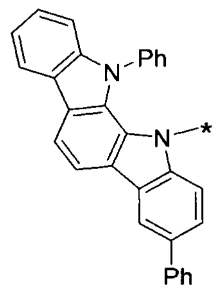

D336

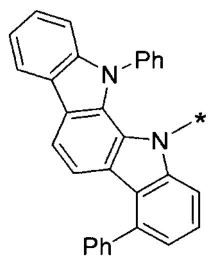

D337

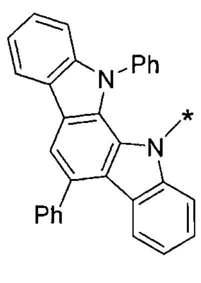

D338

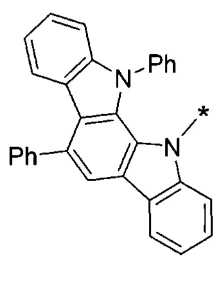

D339

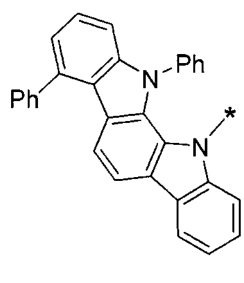

D340

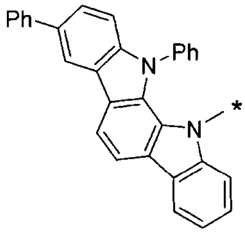
D341

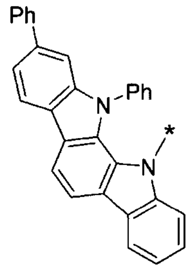
D342

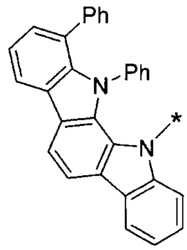
D343

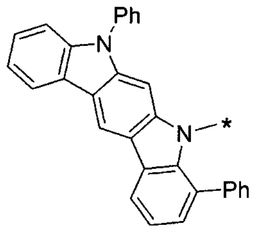
D344

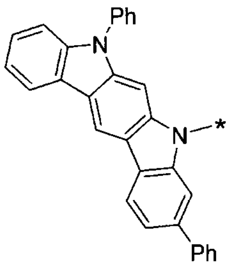
D345

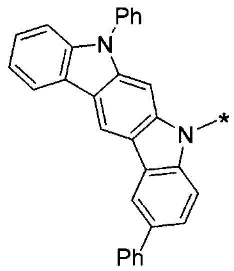
D346

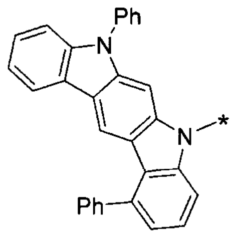
D347

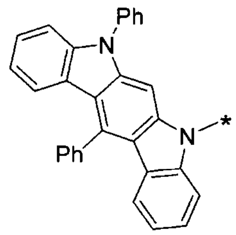
D348

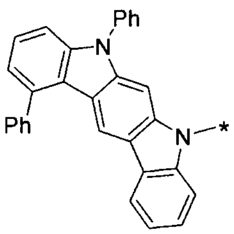
D349

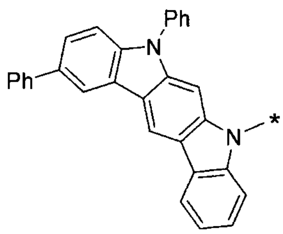
D350

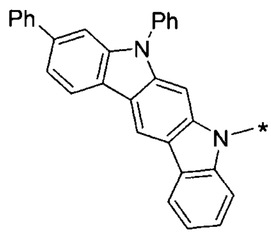
D351

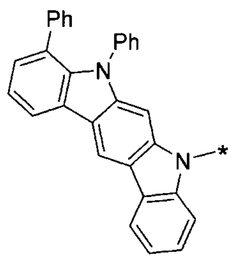
D352

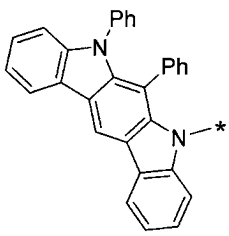
D353

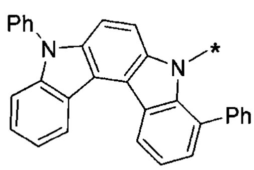
D354

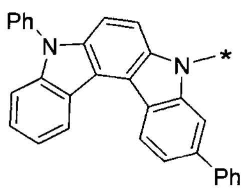
D355

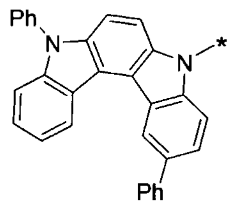
D356

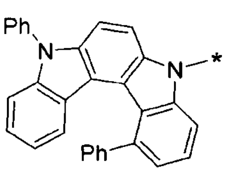
D357

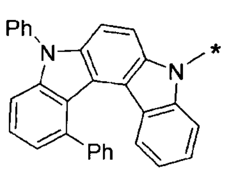
D358

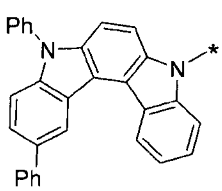
D359

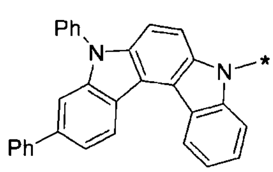
D360

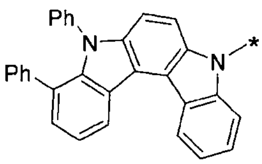

D361

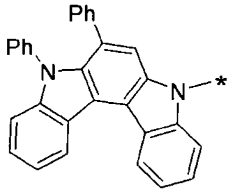

D362

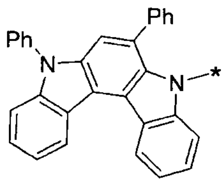

D363

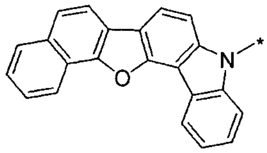

D364

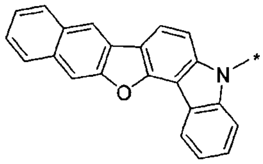

D365

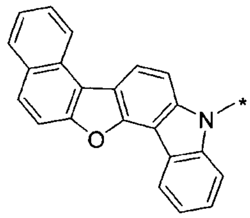

D366

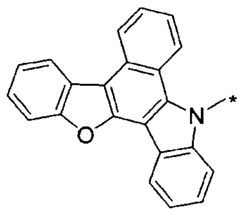

D367

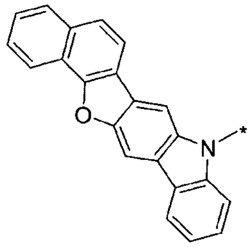

D368

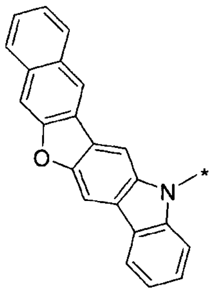

D369

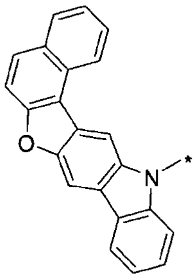

D370

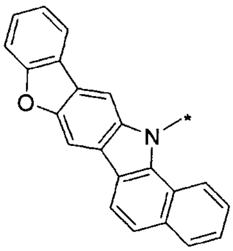

D371

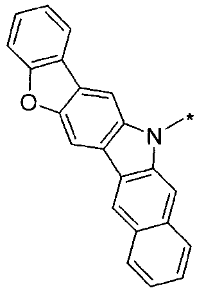

D372

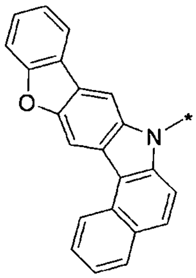

D373

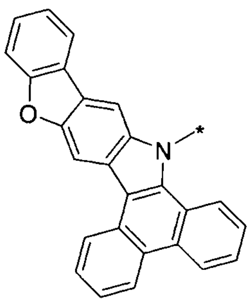

D374

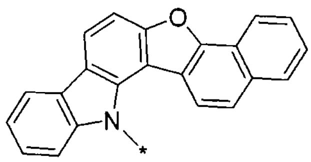

D375

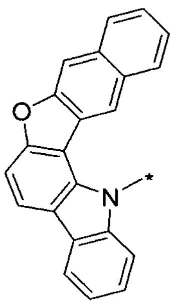

D376

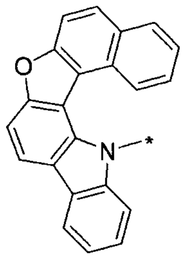

D377

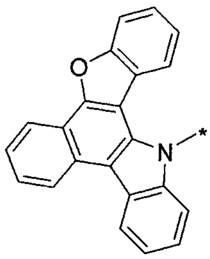

D378

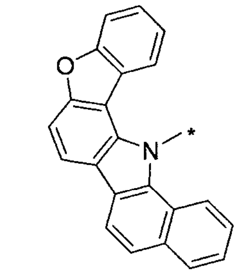

D379

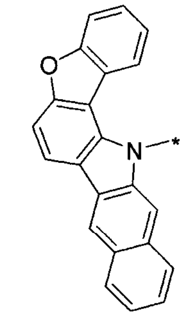

D380

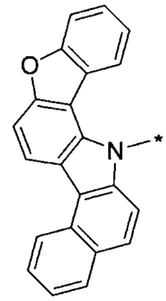

D381

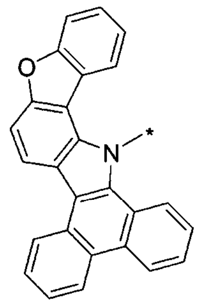

D382

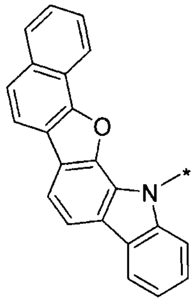

D383

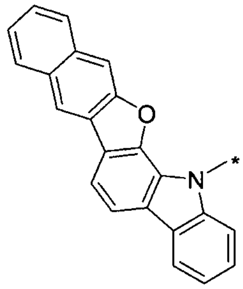

D384

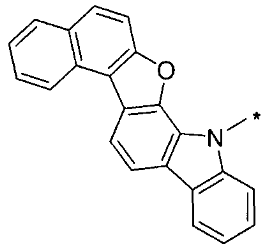

D385

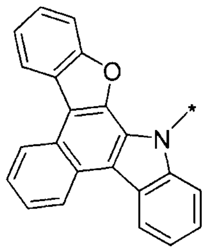

D386

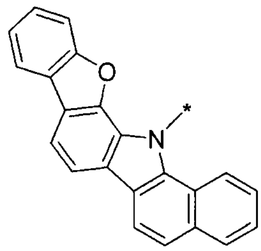

D387

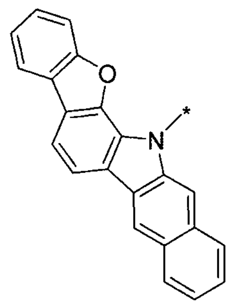

D388

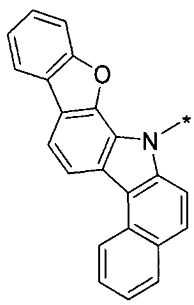

D389

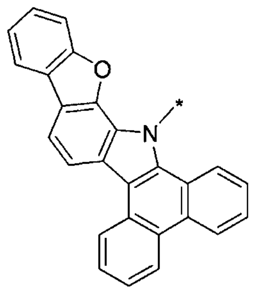

D390

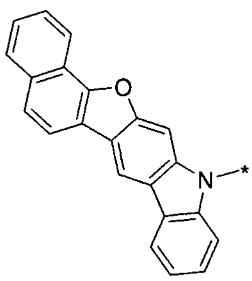

D391

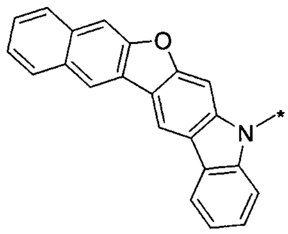

D392

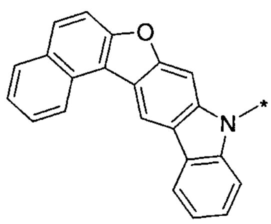

D393

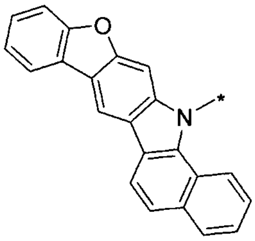 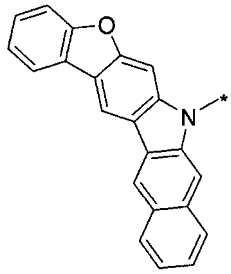 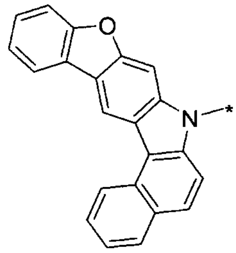 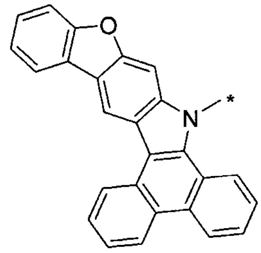

D394 D395 D396 D397

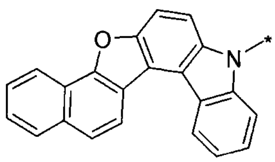 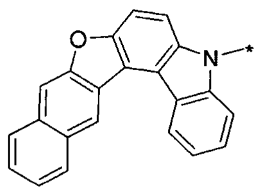 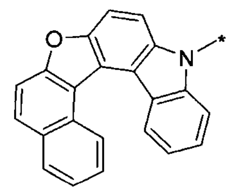 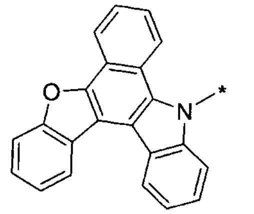

D398 D399 D400 D401

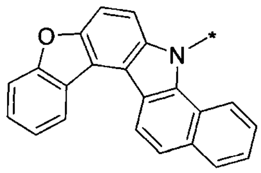 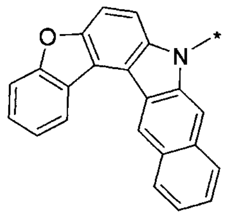 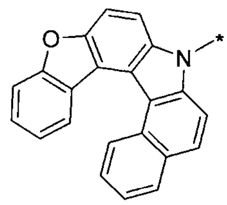 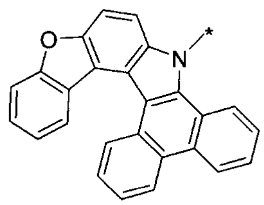

D402 D403 D404 D405

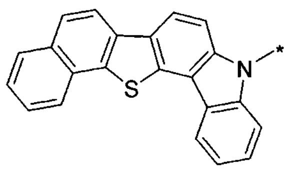 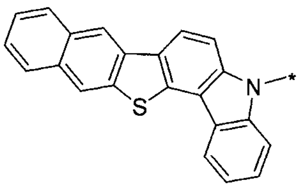 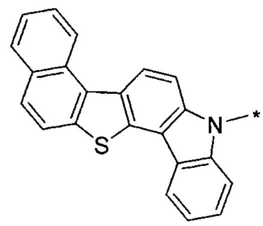 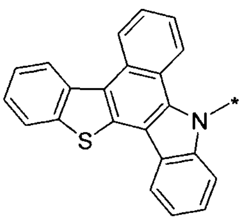

D406 D407 D408 D409

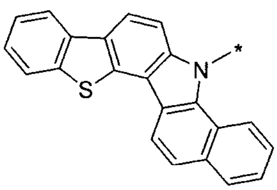 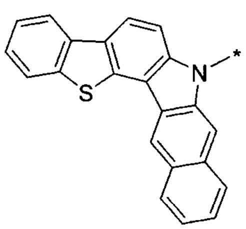 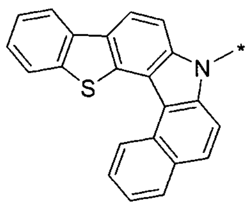 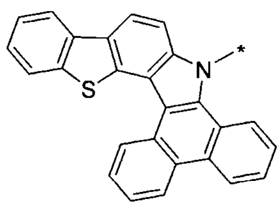

D410 D411 D412 D413

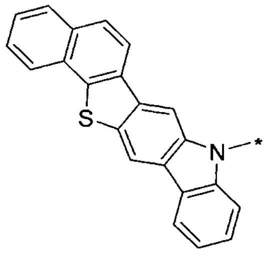

D414

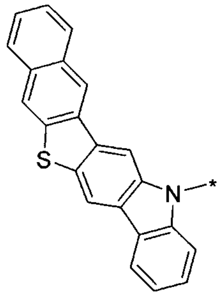

D415

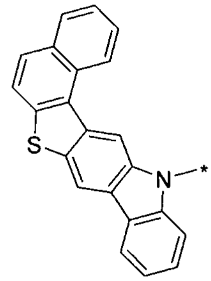

D416

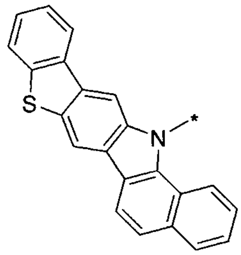

D417

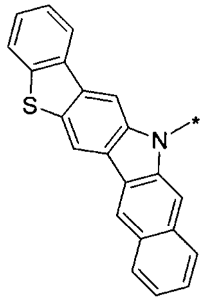

D418

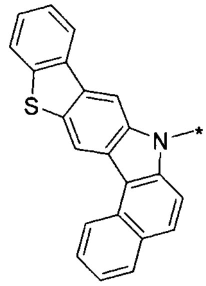

D419

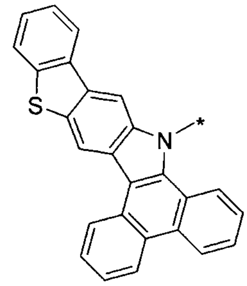

D420

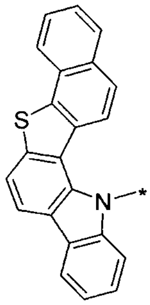

D421

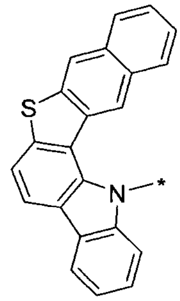

D422

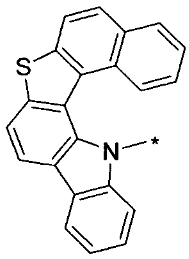

D423

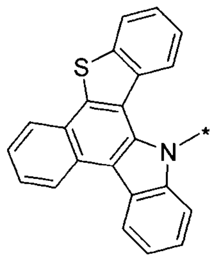

D424

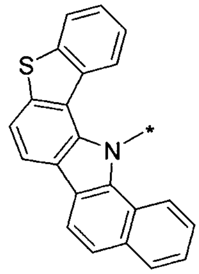

D425

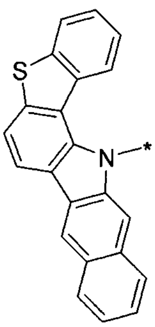

D426

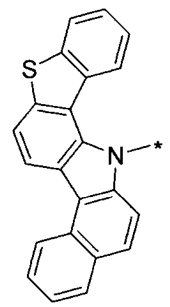

D427

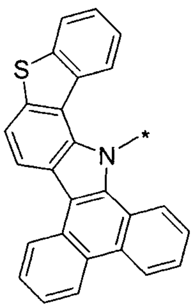

D428

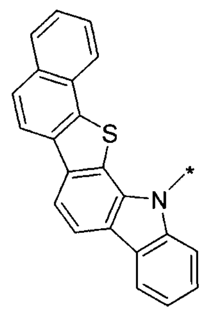

D429

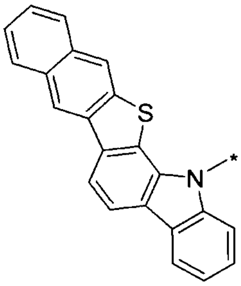 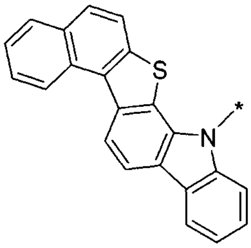 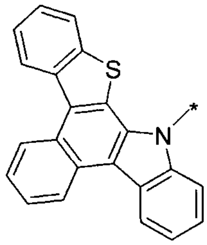 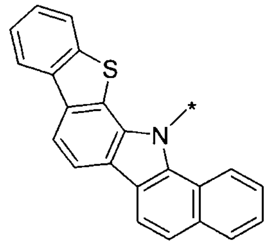

D430 D431 D432 D433

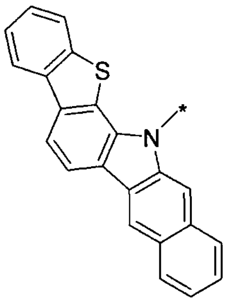 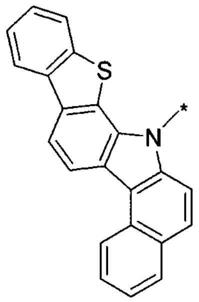 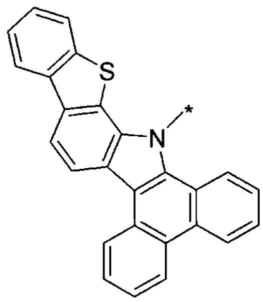 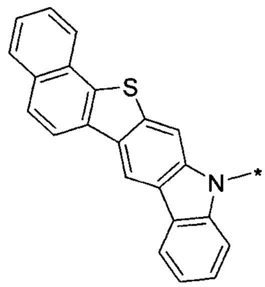

D434 D435 D436 D437

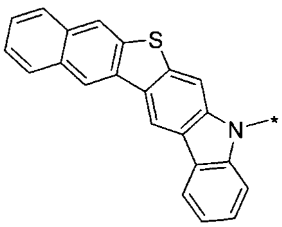 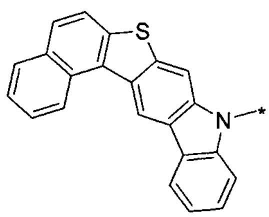 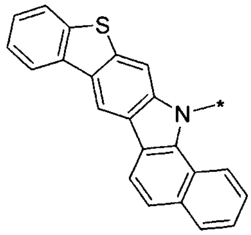 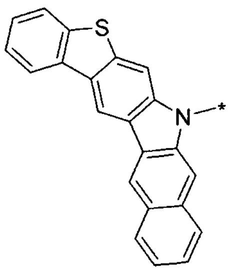

D438 D439 D440 D441

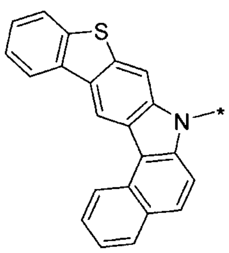 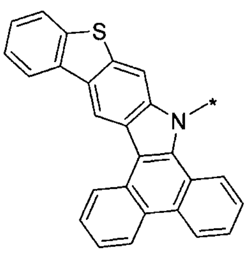 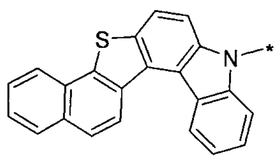 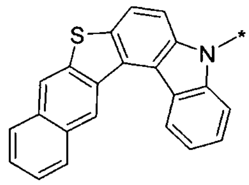

D442 D443 D444 D445

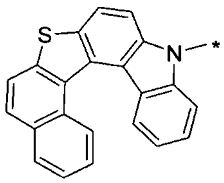

D446

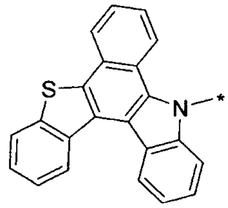

D447

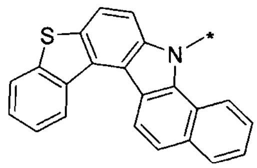

D448

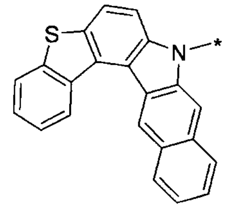

D449

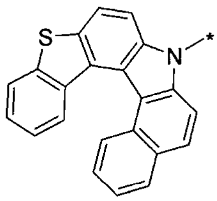

D450

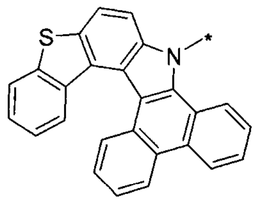

D451

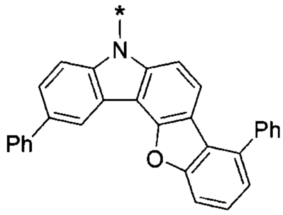

D452

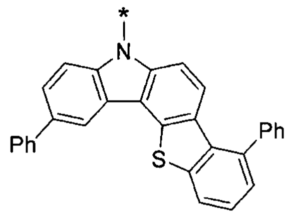

D453

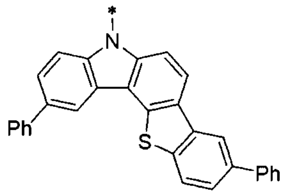

D454

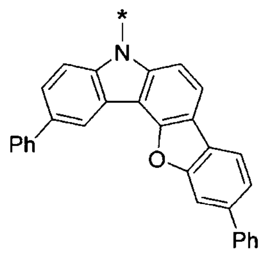

D455

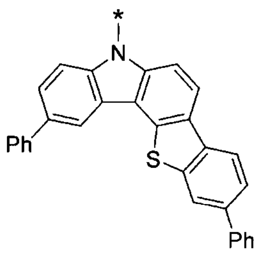

D456

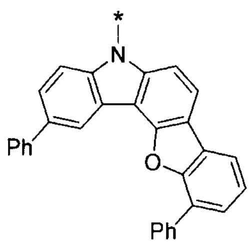

D457

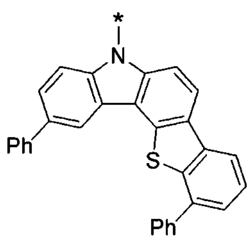

D458

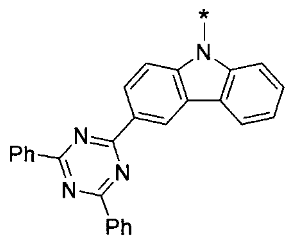

D459

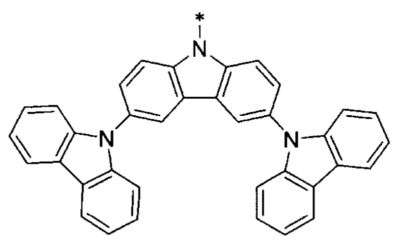

D460

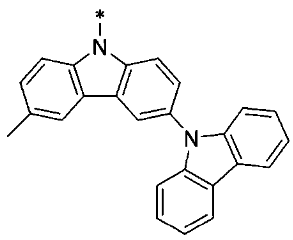

D461

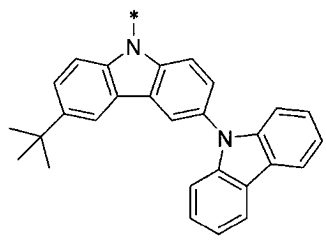

D462

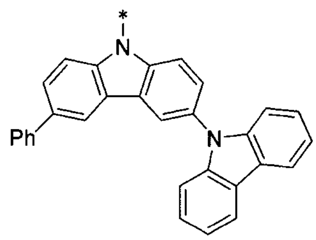

D463

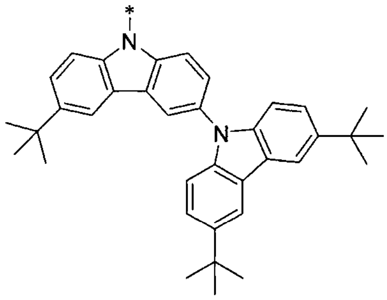 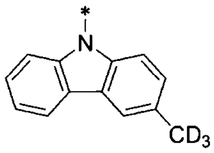 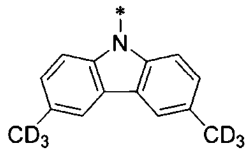

**D464** **D465** **D466**

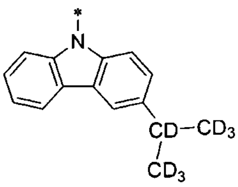 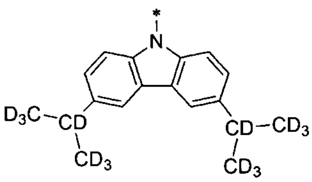 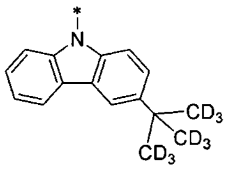 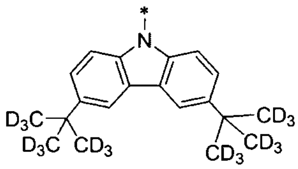

**D467** **D468** **D469** **D470**

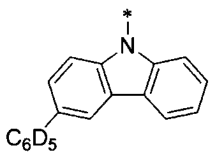 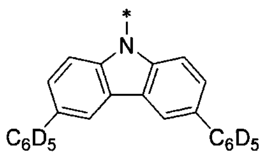 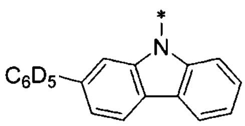 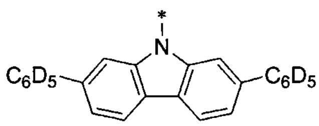

**D471** **D472** **D473** **D474**

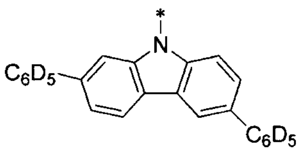 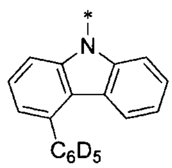 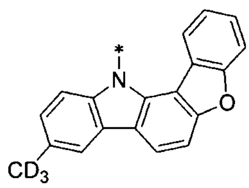 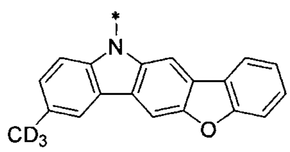

**D475** **D476** **D477** **D478**

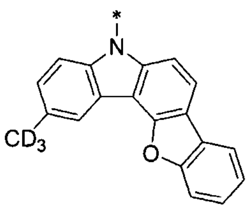 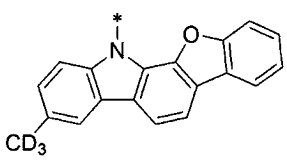 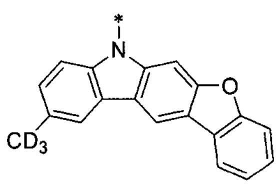 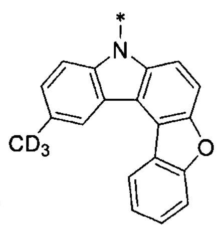

**D479** **D480** **D481** **D482**

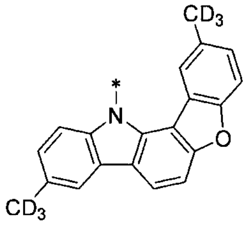

**D483**

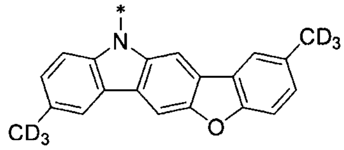

**D484**

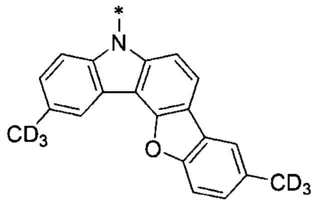

**D485**

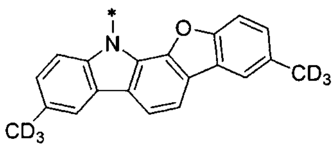

**D486**

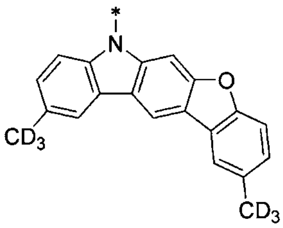

**D487**

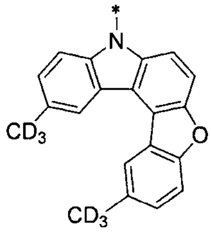

**D488**

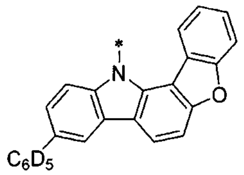

**D489**

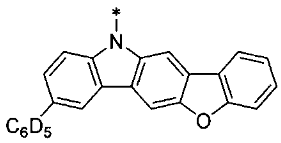

**D490**

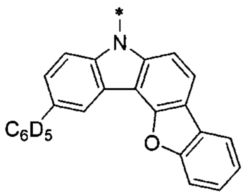

**D491**

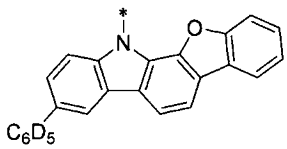

**D491**

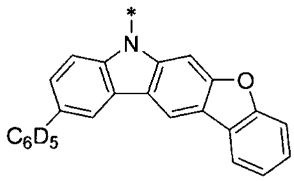

**D493**

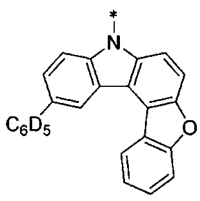

**D494**

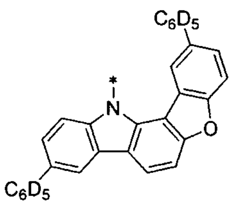

**D495**

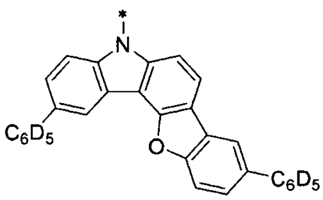

**D496**

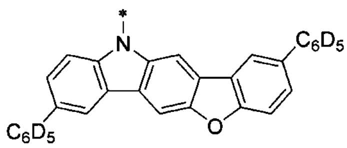

**D497**

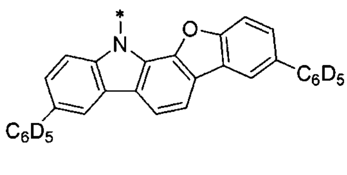

**D498**

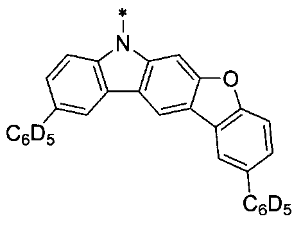

**D499**

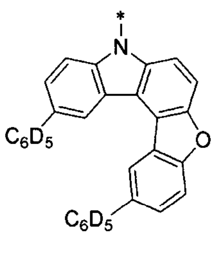

**D500**

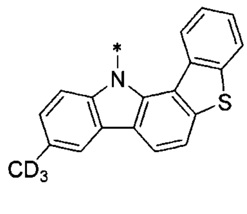

**D501**

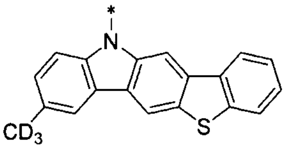
D502
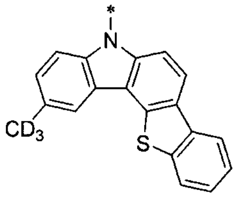
D503
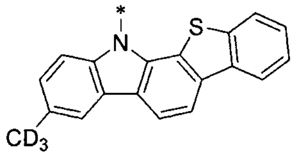
D504
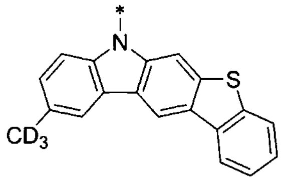
D505

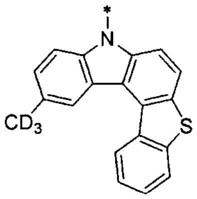
D506
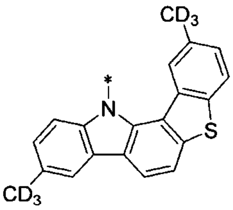
D507
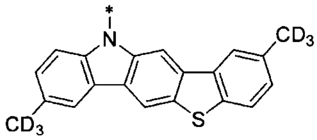
D508
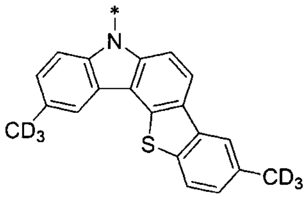
D509

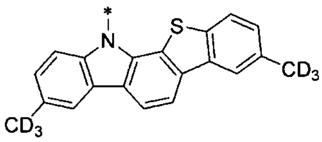
D510
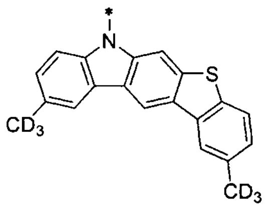
D511
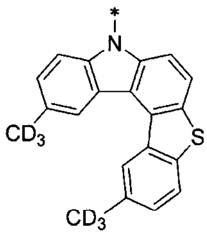
D512
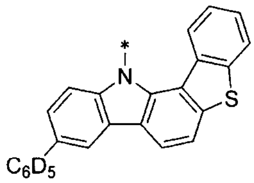
D513

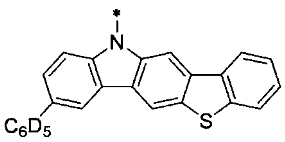
D514
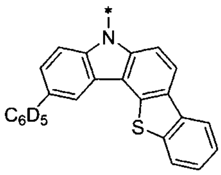
D515
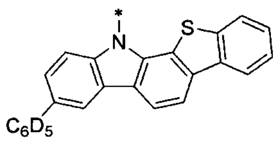
D516
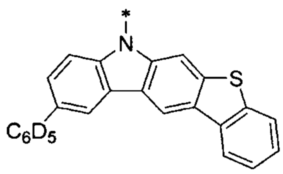
D517

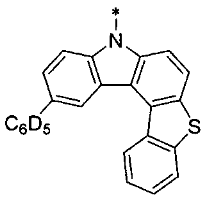
D518
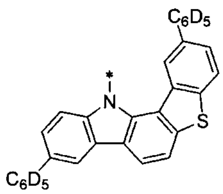
D519
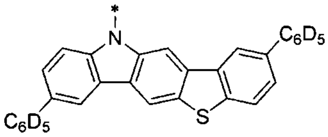
D520
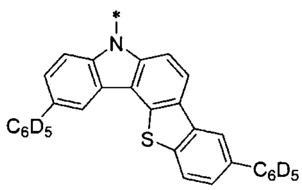
D521

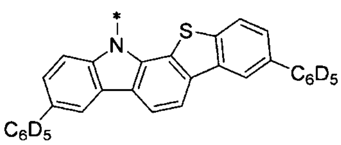
D522
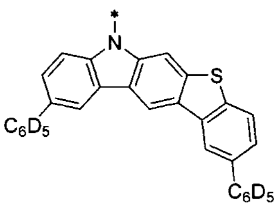
D523
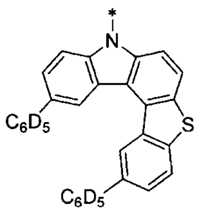
D524
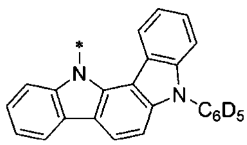
D525

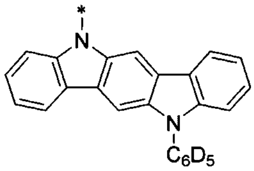

D526

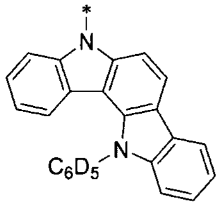

D527

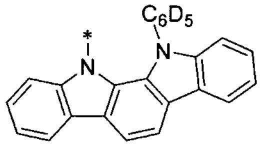

D528

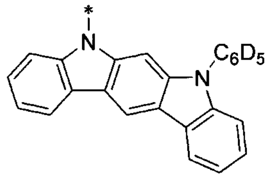

D529

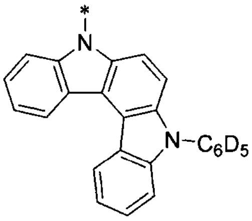

D530

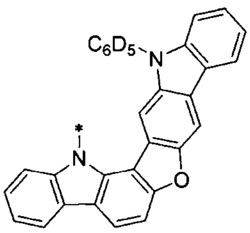

D531

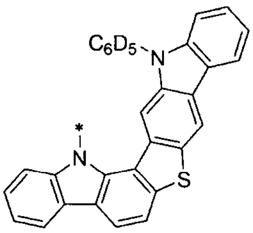

D532

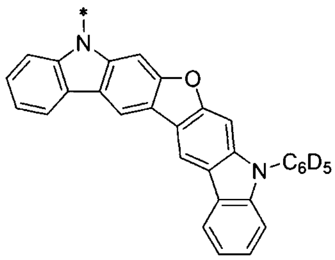

D533

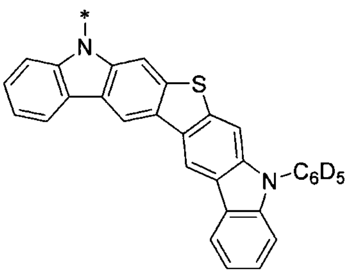

D534

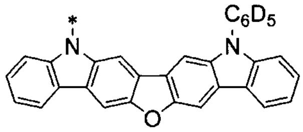

D535

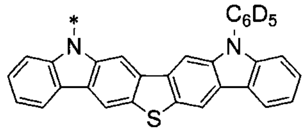

D536

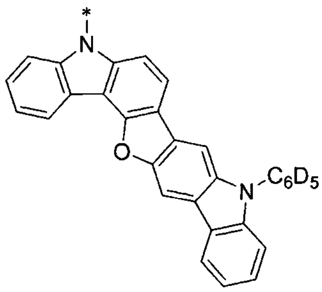

D537

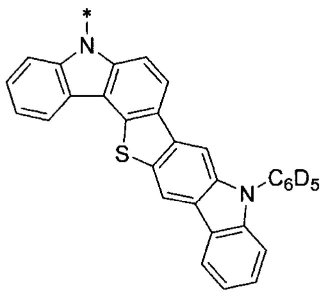

D538

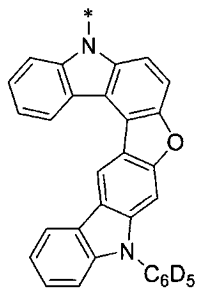

D539

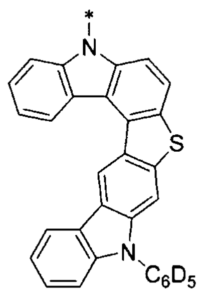

D540

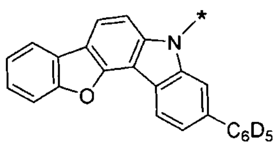

D541

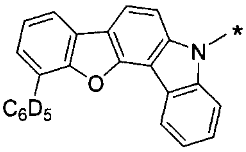

D542

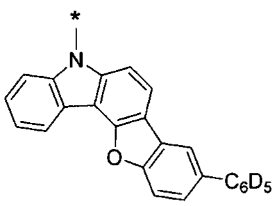

D543

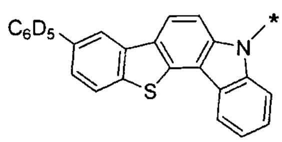

D544

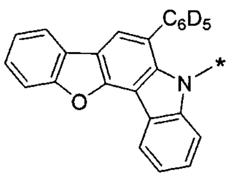
D545

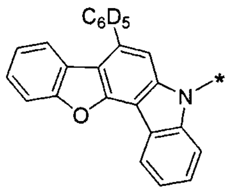
D546

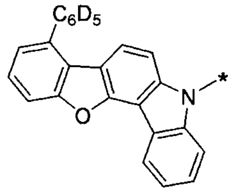
D547

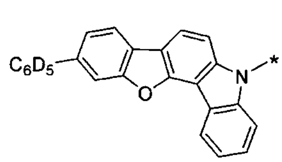
D548

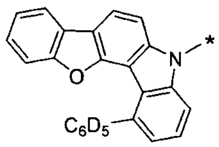
D549

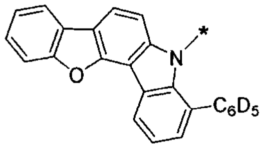
D550

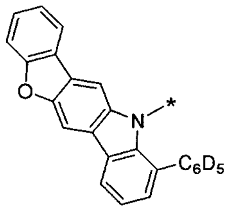
D551

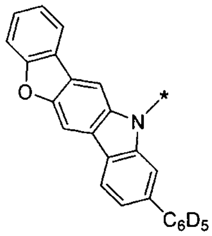
D552

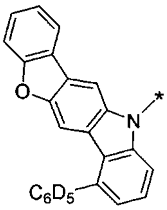
D553

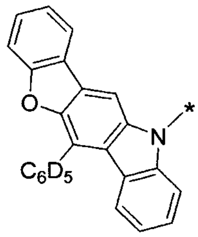
D554

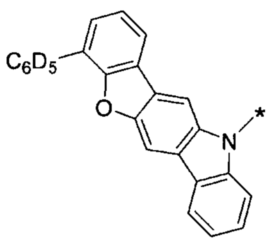
D555

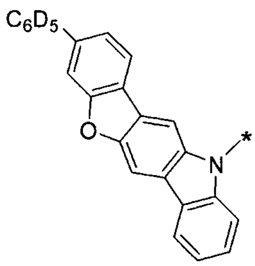
D556

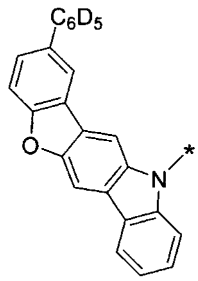
D557

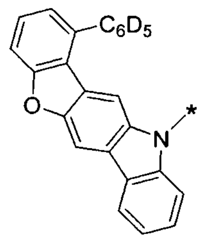
D558

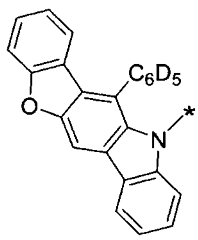
D559

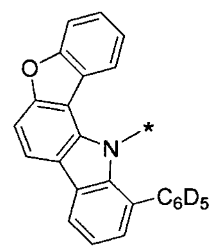
D560

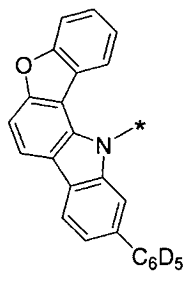
D561

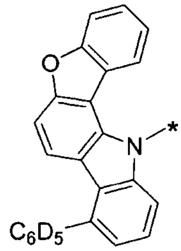
D562

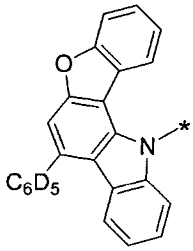
D563

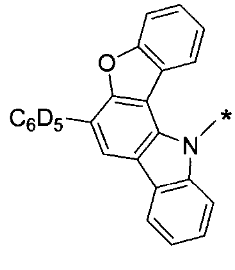
D564

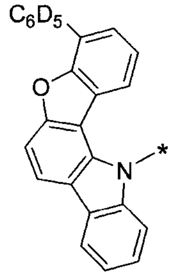
D565

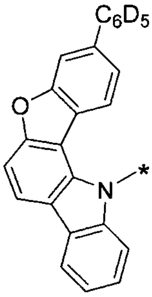

D566

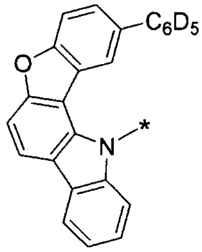

D567

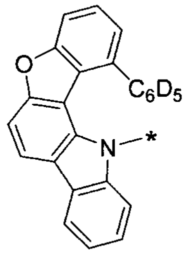

D568

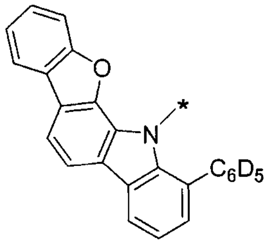

D569

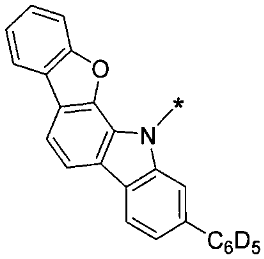

D570

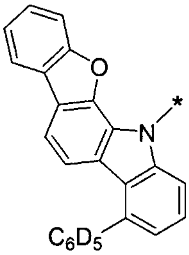

D571

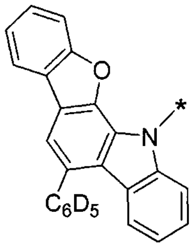

D572

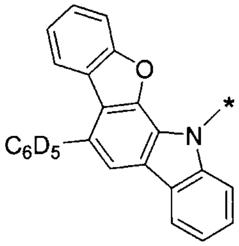

D573

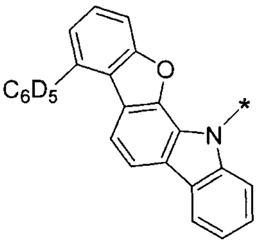

D574

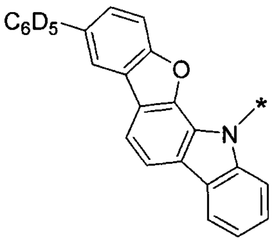

D575

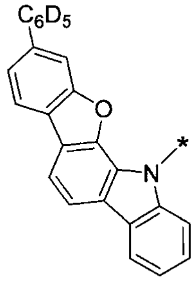

D576

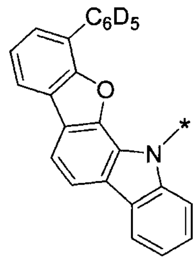

D577

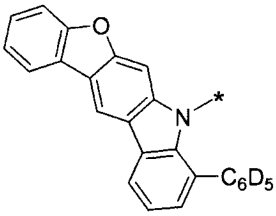

D578

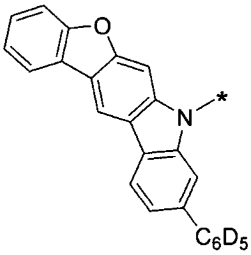

D579

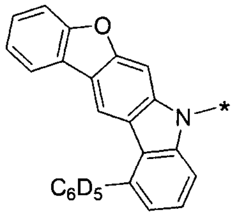

D580

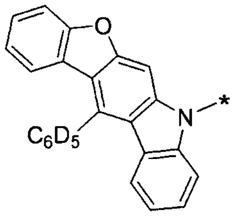

D581

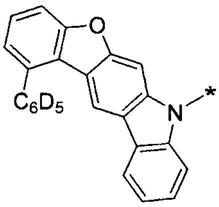

D582

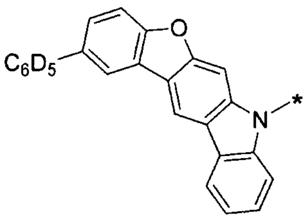

D583

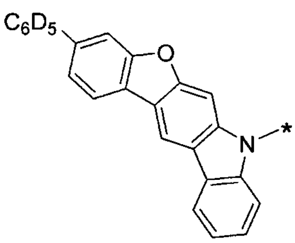

D584

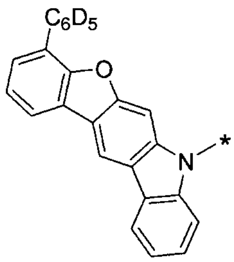

D585

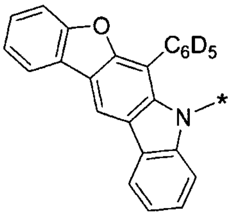
D586

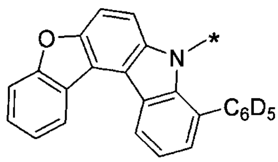
D587

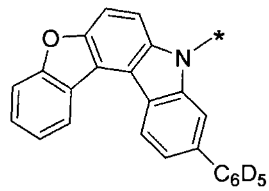
D588

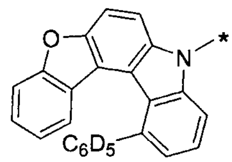
D589

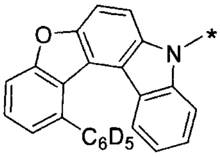
D590

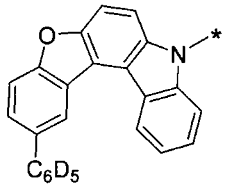
D591

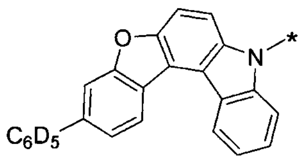
D592

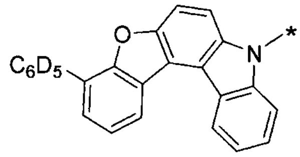
D593

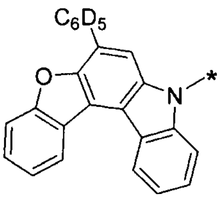
D594

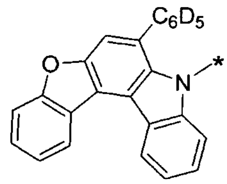
D595

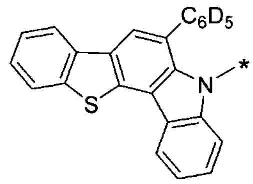
D596

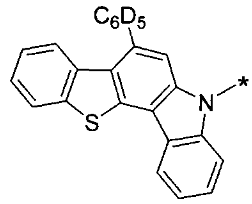
D597

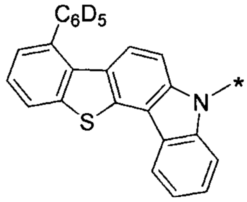
D598

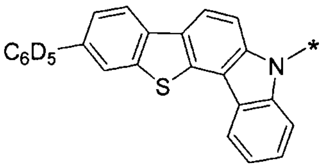
D599

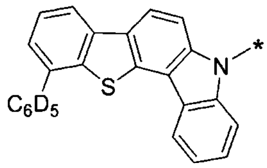
D600

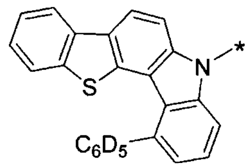
D601

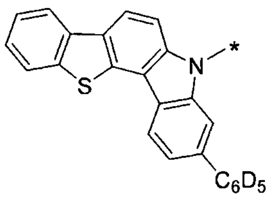
D602

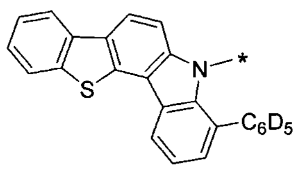
D603

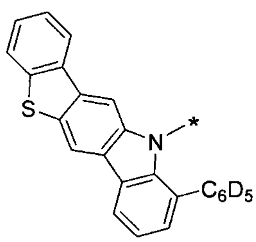
D604

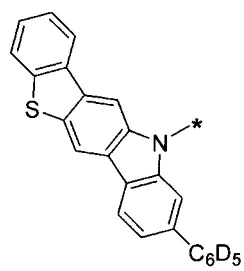
D605

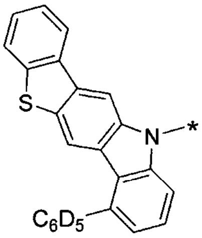 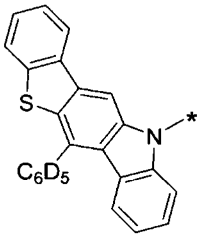 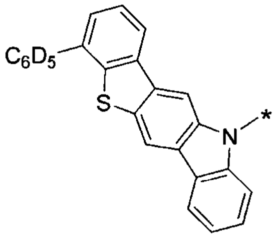 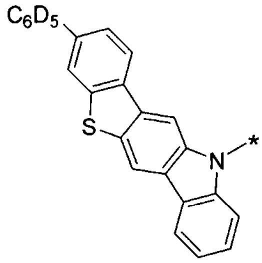

D606 D607 D608 D609

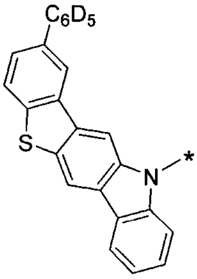 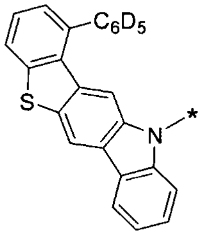 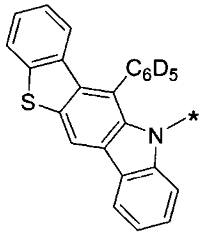 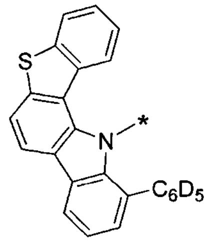

D610 D611 D612 D613

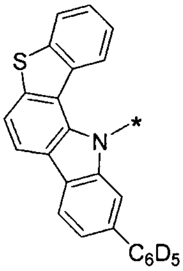 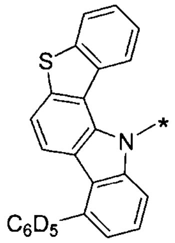 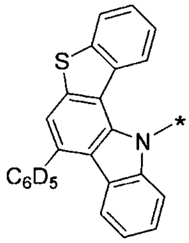 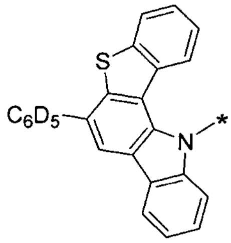

D614 D615 D616 D617

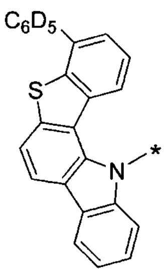 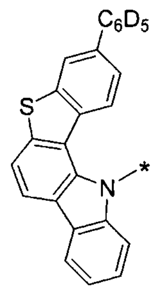 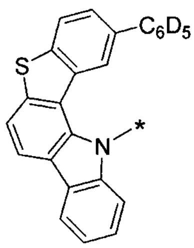 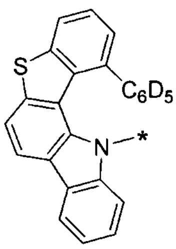

D618 D619 D620 D621

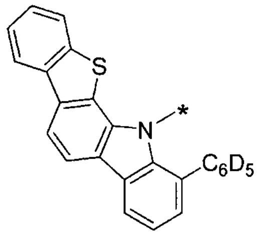 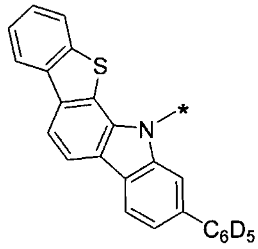 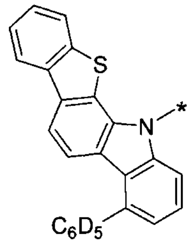 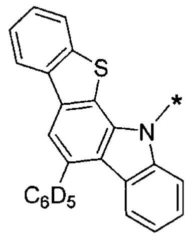

D622 D623 D624 D625

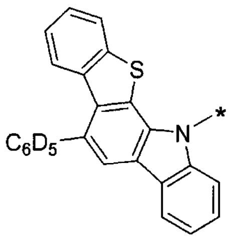 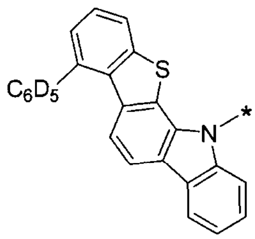 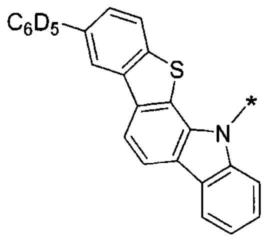 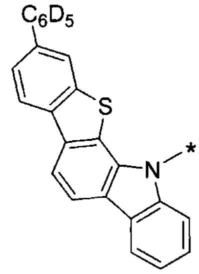

D626 D627 D628 D629

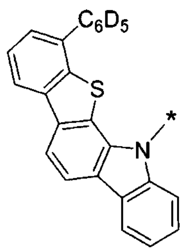 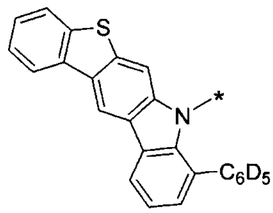 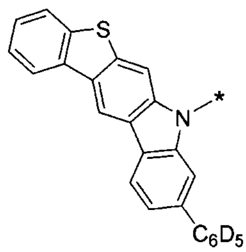 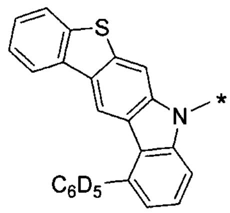

D630 D631 D632 D633

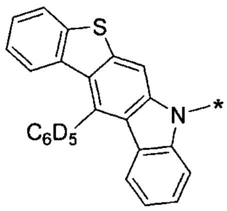 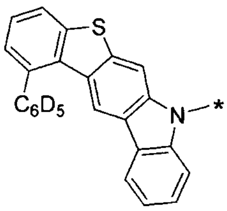 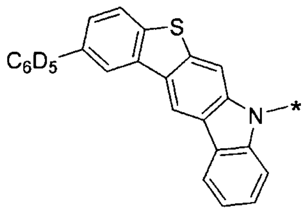 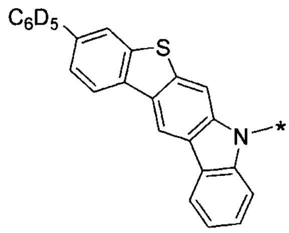

D634 D635 D636 D637

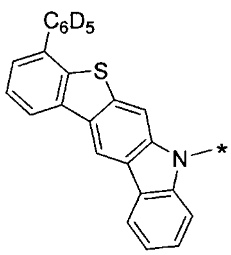 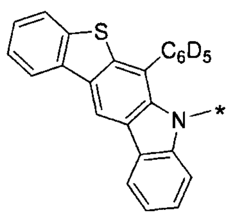 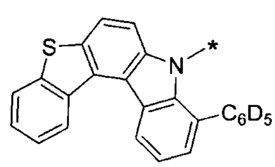 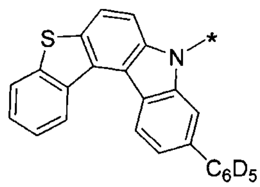

D638 D639 D640 D641

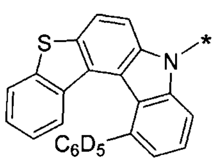 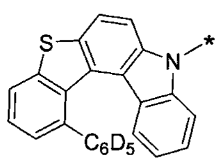 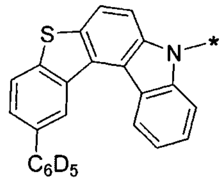 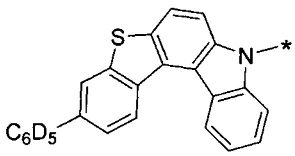

D642 D643 D644 D645

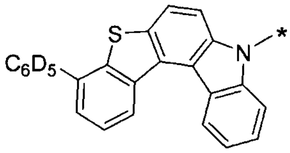
D646

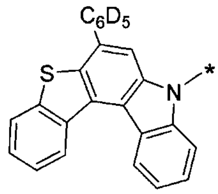
D647

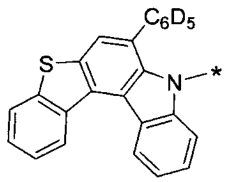
D648

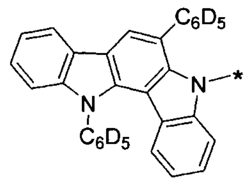
D649

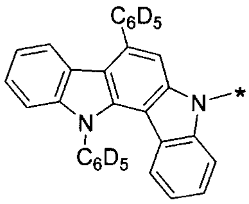 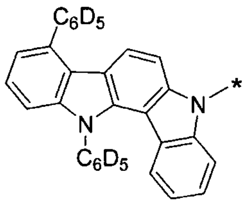 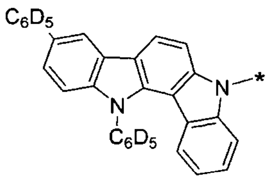 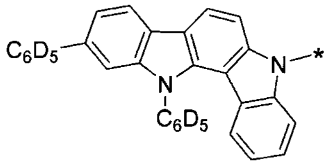

D650 D651 D652 D653

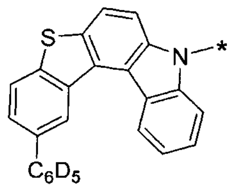 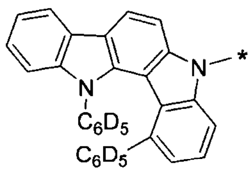 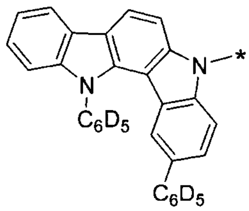 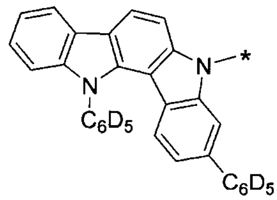

D654 D655 D656 D657

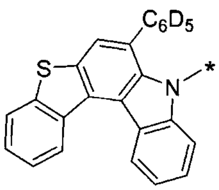
D658

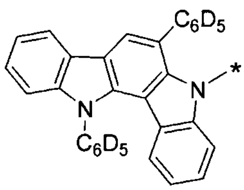
D659

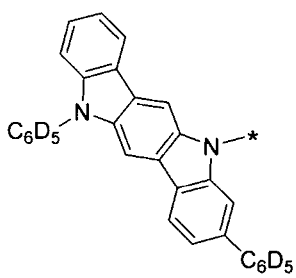
D660

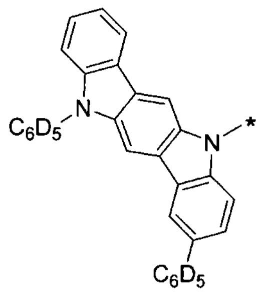
D661

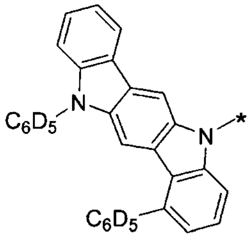

D662

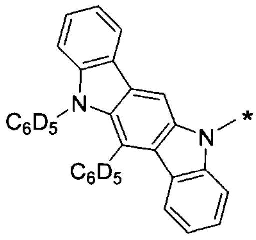

D663

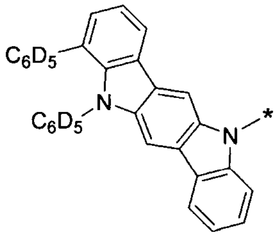

D664

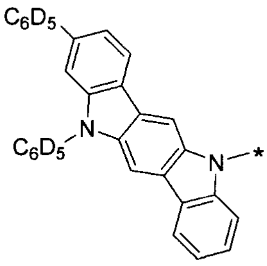

D665

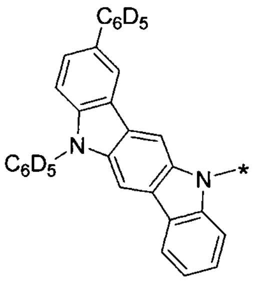

D666

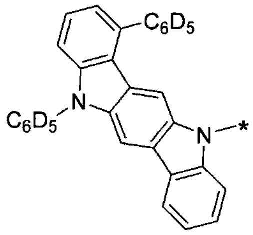

D667

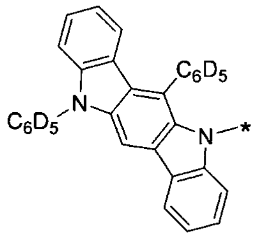

D668

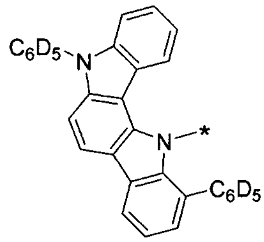

D669

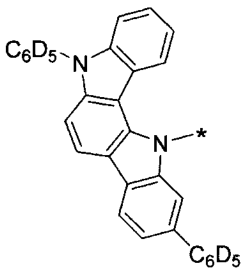

D670

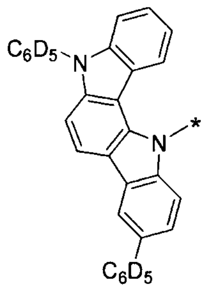

D671

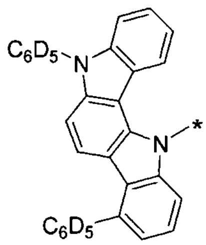

D672

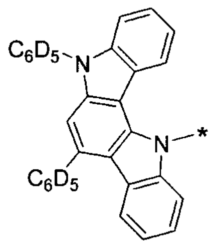

D673

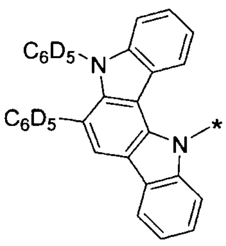

D674

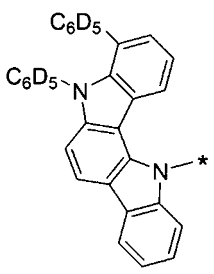

D675

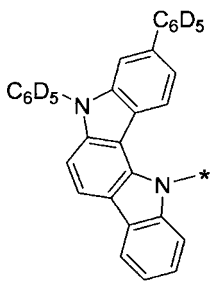

D676

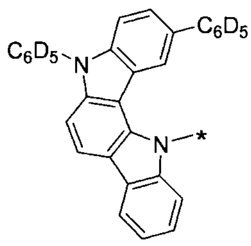

D677

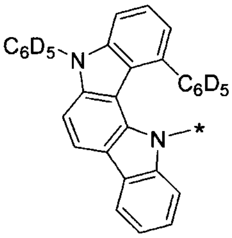

D678

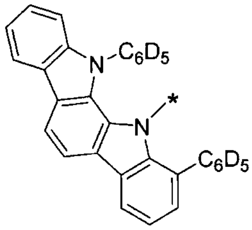

D679

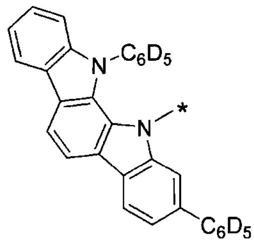

D680

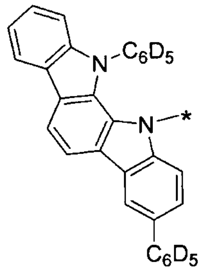

D681

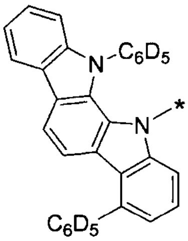

D682

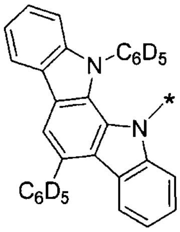

D683

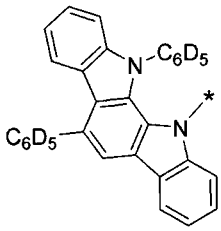

D684

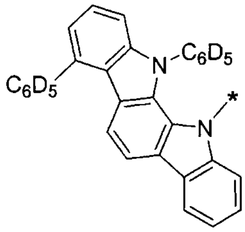

D685

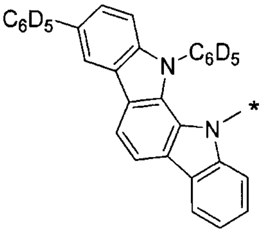

D686

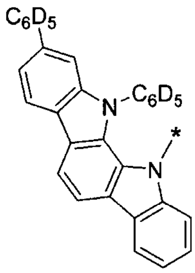

D687

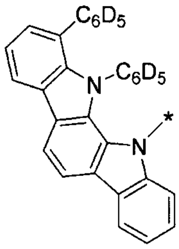

D688

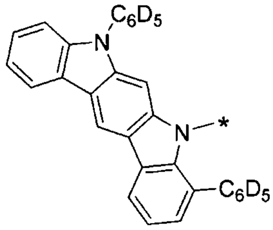

D689

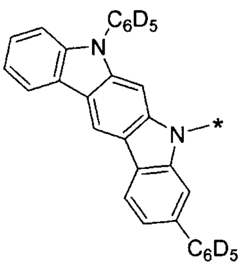

D690

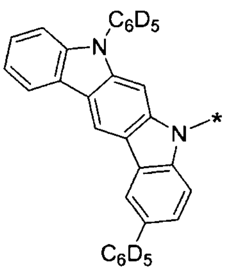

D691

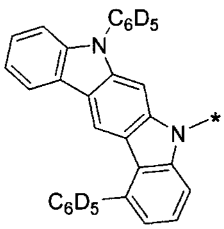

D692

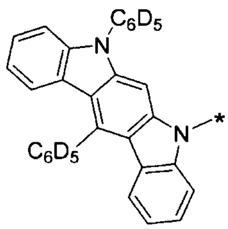

D693

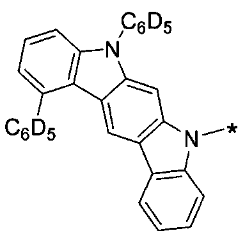
D694

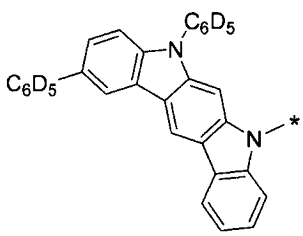
D695

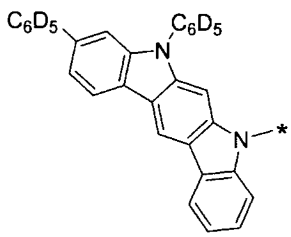
D696

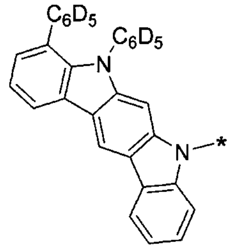
D697

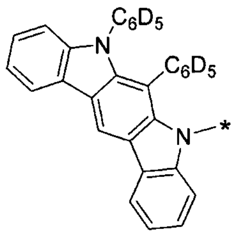
D698

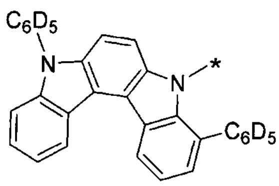
D699

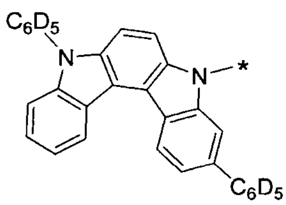
D700

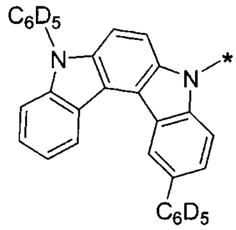
D701

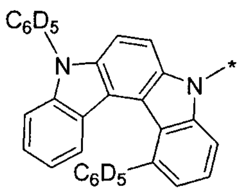
D702

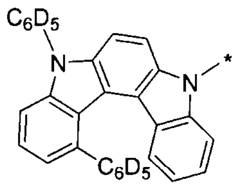
D703

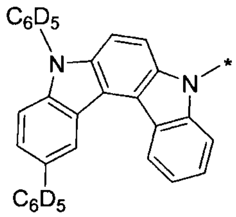
D704

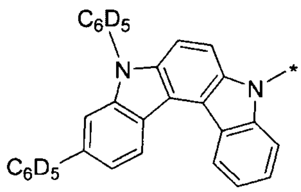
D705

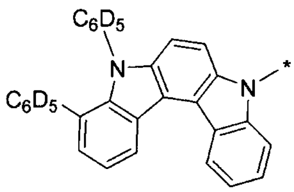
D706

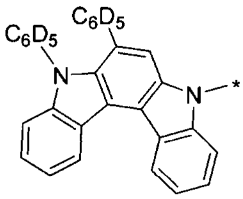
D707

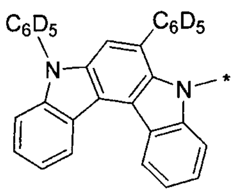
D708

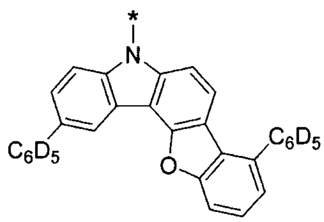
D709

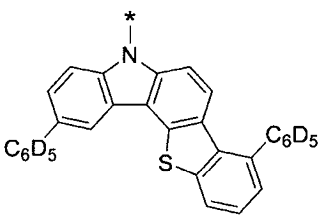
D710

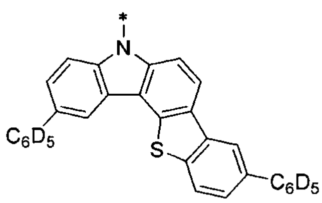
D711

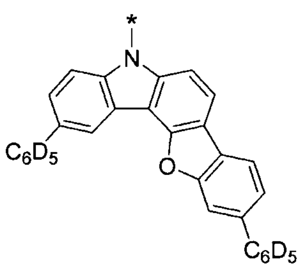
D712

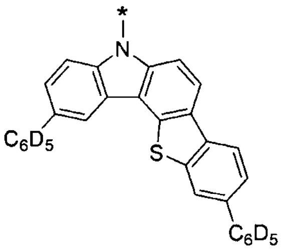

D713

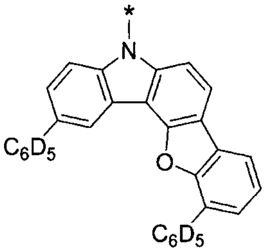

D714

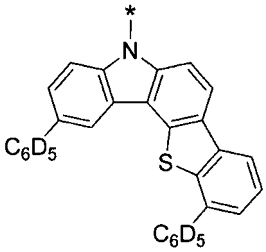

D715

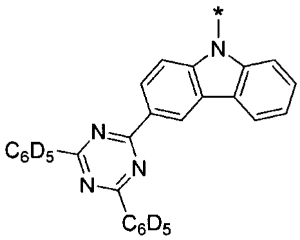

D716

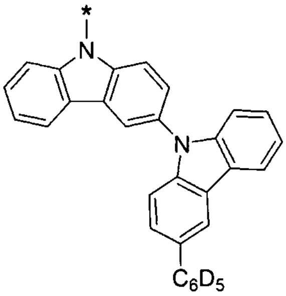

D717

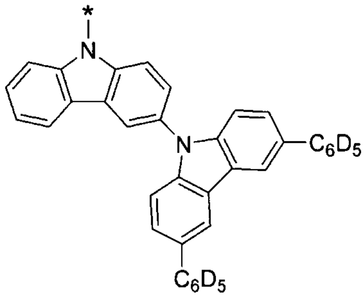

D718

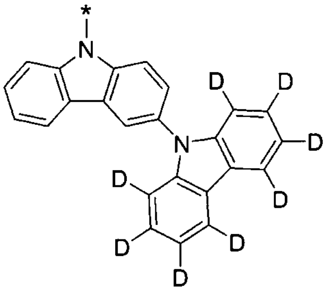

D719

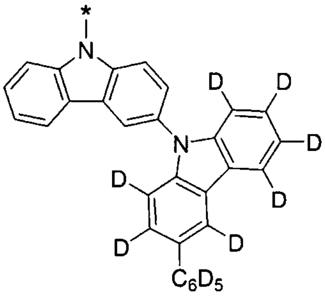

D720

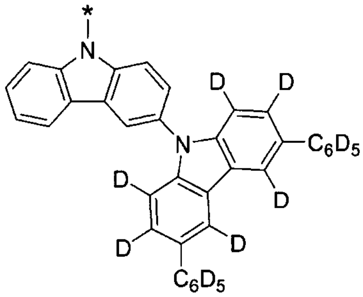

D721

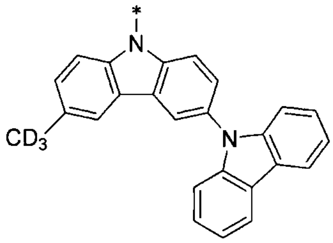

D722

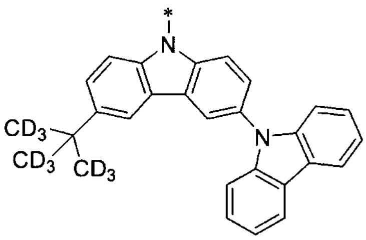

D723

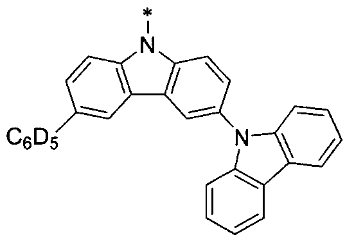

D724

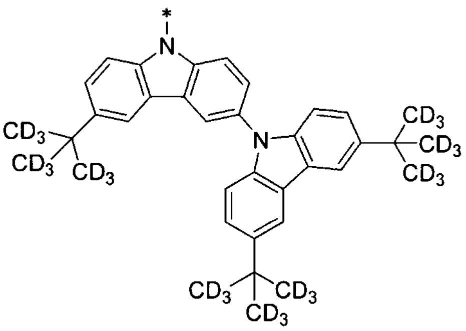

D725

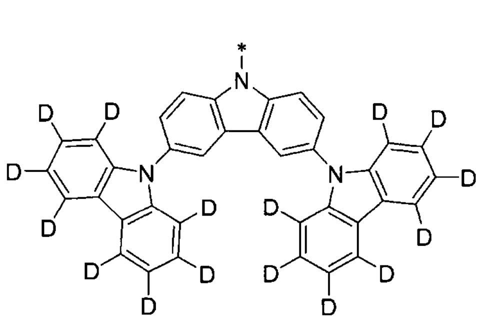

D726

Groups obtained by substituting all hydrogen atoms present in the above D1 to D464 with deuterium atoms are disclosed as D727 to D1190.

In one aspect of the present invention, the donor group that $R^1$ to $R^5$ can take is selected from the group consisting of D1 to D1190. In one aspect of the present invention, the donor group that $R^1$ to $R^5$ can take is selected from the group consisting of D1 to D16, and D465 to D476. In one aspect of the present invention, the donor group that $R^1$ to $R^5$ can take is selected from the group consisting of D17 to D76, D88 to D123, D190 to D303, D364 to D458, D477 to D524, D531 to D658, and D709 to D715. In one aspect of the present invention, the donor group that $R^1$ to $R^5$ can take is selected from the group consisting of D77 to D82, D124 to D189, D304 to D363, D460 to D464, D525 to D530, D659 to D708, and D717 to D726.

In one aspect of the present invention, the donor group that $R^1$ to $R^5$ can take is selected from the group consisting of D1 to D189, D458 to D654, and D717 to D726. In one preferred aspect of the present invention, the donor group that $R^1$ to $R^5$ can take is selected from the group consisting of D1 to D22, D35 to D38, D46 to D53, D74 to D89, D117 to D187, D458, D459, D465 to D478, D526 to D533, and D717 to D721. In one more preferred aspect of the present invention, the donor group that $R^1$ to $R^5$ can take is selected from the group consisting of D1 to D13, D17 to D22, D37, D47 to D52, D77 to D82, D124 to D136, D139, D142, D145, D151, D154, D157, D160, D163, D166, D169, D172, D175, D178, D181, D184, D187, D459, D466 to D471, D521 to D525, and D712 to D716. For example, in one more preferred aspect of the present invention, the donor group that $R^1$ to $R^5$ can take is selected from the group consisting of D1 to D13 and D466 to D471. For example, in one more preferred aspect of the present invention, the donor group that $R^1$ to $R^5$ can take is selected from the group consisting of D17 to D22, D37, and D47 to D52. For example, in one more preferred aspect of the present invention, the donor group that $R^1$ to $R^5$ can take is selected from the group consisting of D77 to D82 and D526 to D530. For example, in one more preferred aspect of the present invention, the donor group that $R^1$ to $R^5$ can take is selected from the group consisting of D124 to D136, D139, D142, D145, D151, D154, D157, D160, D163, D166, D169, D172, D175, D178, D181, D184, D187, and D712 to D716.

One or more of $R^1$ to $R^5$ in the general formula (1) represent a cyano group. In one aspect of the present invention, one or two of $R^1$ to $R^5$ represent a cyano group. In one preferred aspect of the present invention, at least $R^2$ represents a cyano group. In one aspect of the present invention, at least $R^1$ represents a cyano group. In one aspect of the present invention, at least $R^3$ represents a cyano group. In one preferred aspect of the present invention, only $R^2$ represents a cyano group. In one aspect of the present invention, only $R^1$ represents a cyano group. In one aspect of the present invention, only $R^3$ represents a cyano group. In one aspect of the present invention, only $R^2$ and $R^4$ represent a cyano group. In one aspect of the present invention, only $R^2$ and $R^5$ represent a cyano group. In one aspect of the present invention, only $R^1$ and $R^3$ represent a cyano group.

Two or more of $R^1$ to $R^5$ in the general formula (1) represent a donor group. In one aspect of the present invention, two or three of $R^1$ to $R^5$ represent a donor group. In one preferred aspect of the present invention, two of $R^1$ to $R^5$ represent a donor group. In one preferred aspect of the present invention, three of $R^1$ to $R^5$ represent a donor group. In one aspect of the present invention, at least $R^3$ represents a donor group. In one aspect of the present invention, at least $R^4$ represents a donor group. In one aspect of the present invention, at least $R^5$ represents a donor group. In one aspect of the present invention, only $R^3$ represents a donor group. In one aspect of the present invention, only $R^4$ represents a donor group. In one aspect of the present invention, only $R^5$ represents a donor group. In one aspect of the present invention, only $R^3$ and $R^5$ represent a donor group. In one aspect of the present invention, only $R^2$ and $R^5$ represent a donor group. In one aspect of the present invention, only $R^2$ and $R^4$ represent a donor group. In one aspect of the present invention, only $R^3$, $R^4$ and $R^5$ represent a donor group. In one aspect of the present invention, only $R^2$, $R^4$ and $R^5$ represent a donor group. When two or more of $R^1$ to $R^5$ represent a donor group, these can be the same or different.

The number of $R^1$ to $R^5$ that are a hydrogen atom or a deuterium atom is 0 to 2, preferably 0 or 1, and is, for example, 1, or for example, 0. For example, $R^1$ represents a hydrogen atom or a deuterium atom. These show more excellent light emission characteristics than the compounds where the number of $R^1$ to $R^5$ that are a hydrogen atom or a deuterium atom is 3. The number of $R^1$ to $R^5$ that are a substituted or unsubstituted aryl group is 0 or 1, and is preferably 1. The number can be 0. The number of $R^1$ to $R^5$ that are substituted or unsubstituted alkyl groups is 0 to 3, preferably 0 to 2, and can be 1 or can be 0.

In one preferred aspect of the present invention, one of $R^1$ to $R^5$ represents a cyano group, two represent a donor group, one represents a substituted or unsubstituted aryl group, and one represents a hydrogen atom or a deuterium atom. In one aspect of the present invention, two donor groups are the same. In one aspect of the present invention, two donor groups are different from each other. In one aspect of the present invention, $R^1$ represents a hydrogen atom or a deuterium atom, and $R^2$ or $R^3$ represents a cyano group. In one preferred aspect of the present invention, $R^1$ represents a hydrogen atom or a deuterium atom, $R^2$ represents a cyano group, $R^3$ and $R^5$ represent a donor group, and $R^4$ represents a substituted or unsubstituted aryl group. In one aspect of the present invention, $R^1$ represents a hydrogen atom or a deuterium atom, $R^2$ represents a cyano group, $R^4$ and $R^5$ represent a donor group, and $R^3$ represents a substituted or unsubstituted aryl group. In one aspect of the present invention, $R^1$ represents a hydrogen atom or a deuterium atom, $R^2$ represents a cyano group, $R^3$ and $R^4$ represent a donor group, and $R^5$ represents a substituted or unsubstituted aryl group.

In one preferred aspect of the present invention, one of $R^1$ to $R^5$ represents a cyano group, three represent a

donor group, and one represents a hydrogen atom or a deuterium atom. In one aspect of the present invention, three donor groups are the same. In one aspect of the present invention, two of three donor groups are the same, and one differs. In one aspect of the present invention, $R^1$ represents a hydrogen atom or a deuterium atom, $R^2$ represents a cyano group, and $R^3$ to $R^5$ represent a donor group. In one aspect of the present invention, $R^1$ represents a hydrogen atom or a deuterium atom, $R^3$ represents a cyano group, $R^2$, $R^4$ and $R^5$ represent a donor group.

In one aspect of the present invention, one of $R^1$ to $R^5$ represents a cyano group, two represent a donor group, and two represent a hydrogen atom or a deuterium atom. In one aspect of the present invention, $R^1$ and $R^4$ represent a hydrogen atom or a deuterium atom, $R^2$ represents a cyano group, and $R^3$ and $R^5$ represent a donor group. In one aspect of the present invention, $R^1$ and $R^5$ represent a hydrogen atom or a deuterium atom, $R^2$ represents a cyano group, and $R^3$ and $R^4$ represent a donor group. In one aspect of the present invention, $R^1$ and $R^3$ represent a hydrogen atom or a deuterium atom, $R^2$ represents a cyano group, and $R^4$ and $R^5$ represent a donor group. In one aspect of the present invention, $R^1$ and $R^4$ represent a hydrogen atom or a deuterium atom, $R^3$ represents a cyano group, and $R^2$ and $R^5$ represent a donor group.

Regarding the aryl group that $Ar^1$ and $Ar^2$ in the general formula (1) can take, reference can be made to the description and the preferred range of the aryl group that $R^1$ to $R^5$ can take.

The heteroaryl group that $Ar^1$ and $Ar^2$ in the general formula (1) can take can be a monocyclic ring or can be a fused ring of two or more kinds of rings. In the case of a fused ring, the number of fused rings is preferably 2 to 6, and can be selected from, for example, 2 to 4. Specific examples of the ring include a pyridine ring, a pyrimidine ring and a pyrrole ring, and these rings can be fused with any other ring. Specific examples of the heteroaryl group include a 2-pyridyl group, a 3-pyridyl group, a 4-pyridyl group, a carbazol-9-yl group, a carbazol-1-yl group, a carbazol-2-yl group, a carbazol-3-yl group, and a carbazol-4-yl group. The number of the ring skeleton-constituting atoms of the heteroaryl group is preferably 4 to 40, more preferably 5 to 20, and can be selected from a range of 5 to 16, or can be selected from a range of 5 to 12.

At least one of $Ar^1$ and $Ar^2$ in the general formula (1) represents a group bonding via a benzene ring that has a donor group bonding via a nitrogen atom but does not substituted with an acceptor group (hereinafter referred to as a "donor group bonding via a carbon atom"). That is, at least one of $Ar^1$ and $Ar^2$ represents a group bonding via a skeleton-constituting carbon atom of a benzene ring, and a donor group bonding via a nitrogen atom bonds to the benzene ring, but an acceptor group does not bond to. A hydrogen atom, a deuterium atom, and a donor group not bonding via a nitrogen atom may further bond to the benzene ring.

The donor group bonding via a carbon atom preferably has a structure represented by the following general formula (c):

General Formula (c)

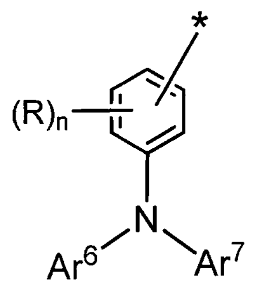

In the general formula (c), $Ar^6$ and $Ar^7$ each independently represent a substituted or unsubstituted aryl group, or a substituted or unsubstituted heteroaryl group. The heteroaryl group preferably contains a nitrogen atom as a ring skeleton-constituting atom, and examples thereof include a pyridine ring-containing heteroaryl group. Regarding details of the aryl group and the heteroaryl group, reference can be made to the description of the aryl group and heteroaryl group that $Ar^1$ and $Ar^2$ can take. $Ar^6$ and $Ar^7$ preferably represent a substituted or unsubstituted aryl group, for example, a substituted or unsubstituted phenyl group. R's each independently represent a deuterium atom or a donor group. The donor group that R can take may be a donor group bonding via a nitrogen atom or may be a donor group bonding via an atom (for example, a carbon atom) other than a nitrogen atom. The donor group bonding via a nitrogen atom may be the same as or different from $N(Ar^6)(Ar^7)$. $Ar^6$ and $Ar^7$ may bond to each other to form a cyclic structure, $Ar^6$ and R may bond to each other to form a cyclic structure, and $Ar^7$ and R may bond to each other to form a cyclic structure. In one preferred aspect of the present invention, $Ar^6$ and $Ar^7$ bond to each other to form a cyclic structure. In another preferred aspect of the present invention, $Ar^6$ and R bond to each other to form a cyclic structure. When they bond to each other, they bond via a single bond or a linking group. Preferably, they bond via a single bond or a nitrogen atom (preferably an alkyl group or an aryl group bonds to the nitrogen atom), and more preferably, they bond via a single bond. n represents an integer of 0 to 4. * indicates a bonding position.

In one preferred aspect of the present invention, the donor group bonding via a carbon atom has a structure represented by the following general formula (d), and more preferably has a structure represented by the following general

formula (e).

General Formula (d)

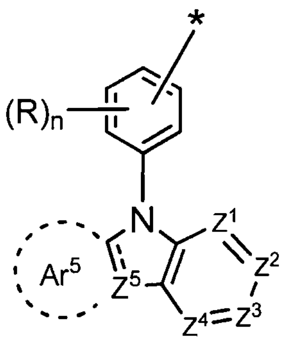

General Formula (e)

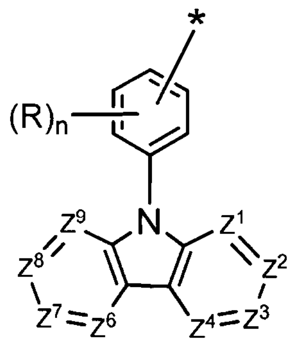

Regarding the definition and description of $Z^1$ to $Z^5$ and $Ar^5$ in the general formula (d), reference can be made to the corresponding description of the general formula (a). Regarding the definition and description of $Z^1$ to $Z^4$ and $Z^6$ to $Z^9$ in the general formula (e), reference can be made to the corresponding description of the general formula (b). In the general formula (d) and the general formula (e), R's each independently represent a deuterium atom or a donor group. The donor group that R can take may be a donor group bonding via a nitrogen atom or may be a donor group bonding via an atom (for example, a carbon atom) other than a nitrogen atom. n represents an integer of 0 to 4. * indicates a bonding position.

It is preferable that n represents 0 or n represents 4 and all four R's represent a deuterium atom. Preferably zero to two, preferably zero or one, and more preferably zero of $Z^1$ to $Z^4$ and $Z^6$ to $Z^9$ represents a nitrogen atom.

As specific examples of the group represented by the general formula (d) or the general formula (e), phenyl groups with any of the above D1 to D1190 bonding to the 3-position (that is, groups with a metaphenylene group further bonding to * of D1 to D 1190) are disclosed as D1191 to D2380. Phenyl groups with any of the above D1 to D1190 bonding to the 4-position (that is, groups with a paraphenylene group further bonding to * of D1 to D1190) are disclosed as D2381 to D3570. Perdeuterated phenyl groups with any of the above D1 to D1190 bonding to the 3-position (that is, groups with a metaphenylene group in which all hydrogen atoms are deuterated further bonding to * of D1 to D1190) are disclosed as D3571 to D4760. Perdeuterated phenyl groups with any of the above D1 to D1190 bonding to the 4-position (that is, groups with a paraphenylene group in which all hydrogen atoms are deuterated further bonding to * of D1 to D1190) are disclosed as D4761 to D5950.

In one preferred aspect of the present invention, the donor group bonding via a carbon atom has a structure represented by the following general formula (f):

General Formula (f)

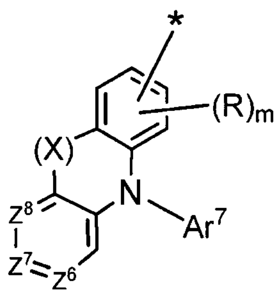

Regarding the definition and description of $Z^6$ to $Z^8$ in the general formula (f), reference can be made to the corresponding description of the general formula (a). Regarding the definition and description of $Ar^7$ in the general formula (f), reference can be made to the corresponding description of the general formula (c). X represents a single bond when absent, or represents a linking group having a linking chain of one or two atoms. Examples of the linking group having a linking chain of two atoms include a substituted or unsubstituted ethylene group. Examples of the linking group having a linking chain of one atom include an oxygen atom, a sulfur atom, or a nitrogen atom with an aryl group or alkyl group binding thereto. The linking group is preferably a nitrogen atom with an aryl group or alkyl group binding thereto. It is more preferable that X represents a single bond when absent. R represents a deuterium atom or a donor group. The donor group that R can take may be a donor group bonding via a nitrogen atom or may be a donor group bonding via an atom (for example, a carbon atom) other than a nitrogen atom. m represents an integer of 0 to 3. * indicates a bonding position.

It is preferable that m represents 0 or m represents 3 and all three R's represent a deuterium atom. Preferably

zero or one, and more preferably zero of $Z^6$ to $Z^8$ represents a nitrogen atom. In one preferred aspect of the present invention, X represents a single bond, and zero of $Z^6$ to $Z^8$ represents a nitrogen atom. In one aspect of the present invention, the donor group is a substituted or unsubstituted N-arylcarbazol-1-yl group. In one aspect of the present invention, the donor group is a substituted or unsubstituted N-arylcarbazol-2-yl group. In one preferred aspect of the present invention, the donor group is a substituted or unsubstituted N-arylcarbazol-3-yl group. In one aspect of the present invention, the donor group is a substituted or unsubstituted N-arylcarbazol-4-yl group.

Specific examples of the donor group represented by the general formula (f) are shown below. The donor group which can be employed in the present invention shall not be construed as being limited by the following specific examples. In the following specific examples, Ph indicates a phenyl group ($C_6H_5$), and * indicates a bonding position. A methyl group is not shown, and for example, Z2 has one methyl group. A deuterated methyl group is expressed as $CD_3$. $C_6D_5$ represents a phenyl group in which all hydrogen atoms are deuterated. D represents a deuterium atom.

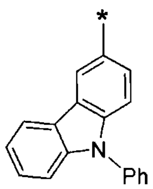
Z1

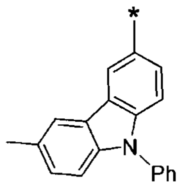
Z2

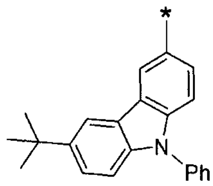
Z3

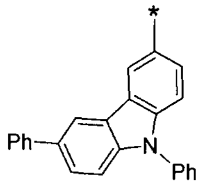
Z4

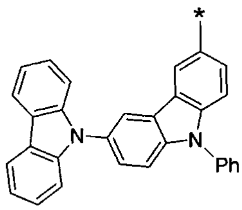
Z5

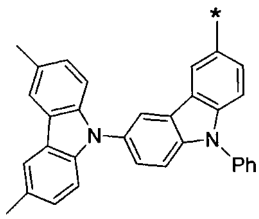
Z6

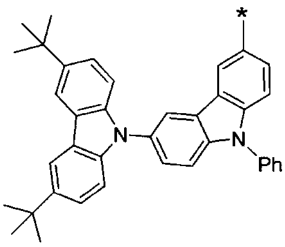
Z7

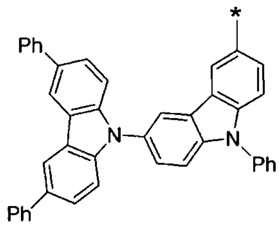
Z8

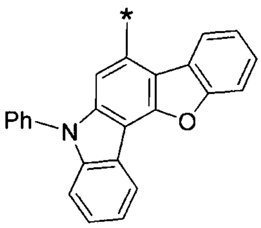
Z9

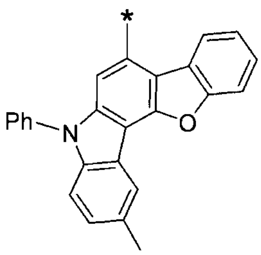
Z10

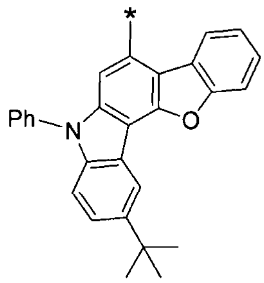
Z11

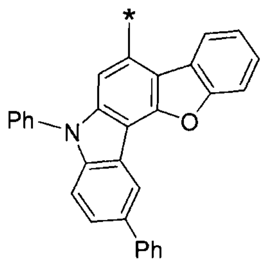
Z12

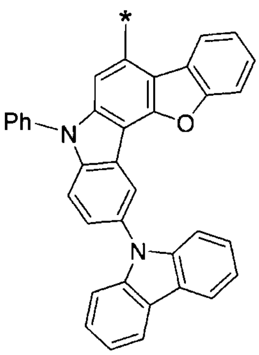
Z13

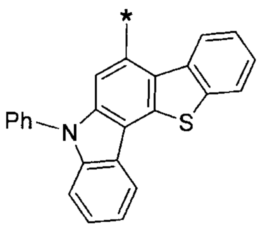
Z14

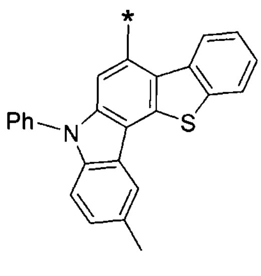
Z15

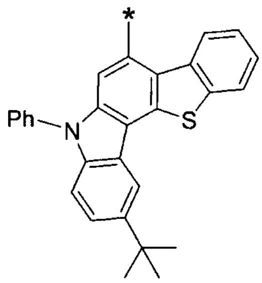
Z16

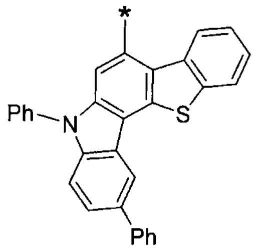
Z17

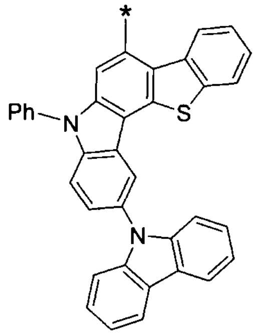
Z18

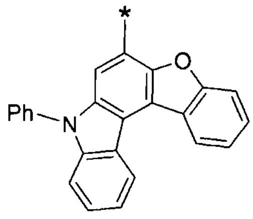
Z19

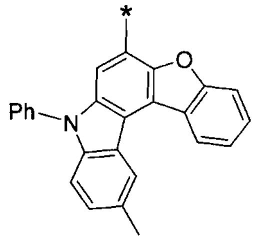
Z20

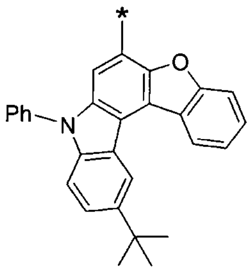
Z21

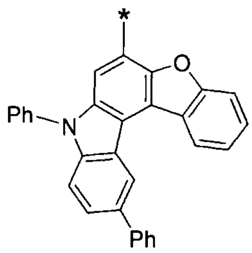
Z22

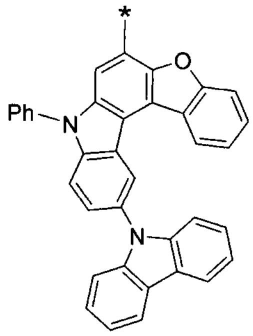
Z23

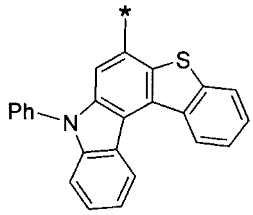
Z24

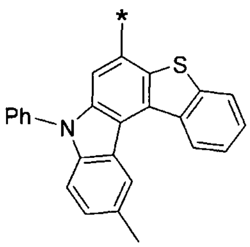
Z25

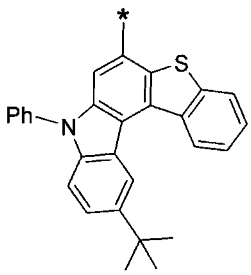
Z26

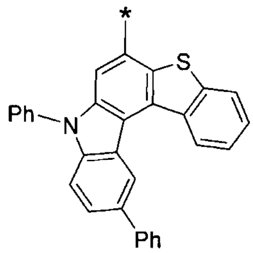
Z27

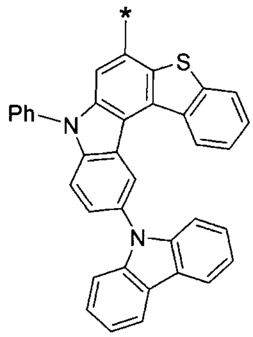
Z28

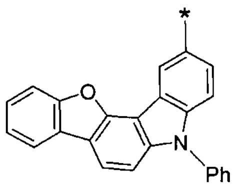
Z29

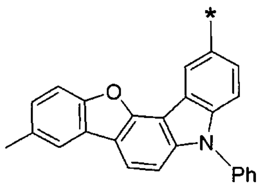
Z30

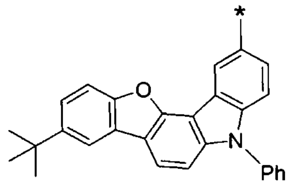
Z31

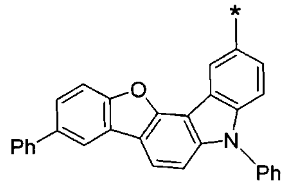
Z32

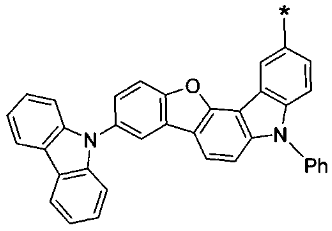
Z33

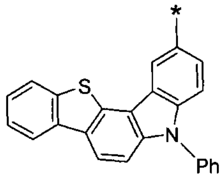
Z34

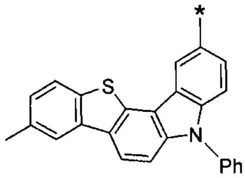
Z35

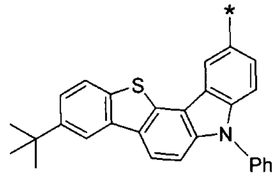
Z36

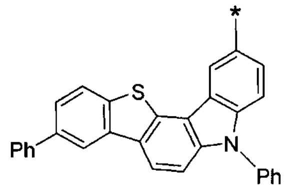
Z37

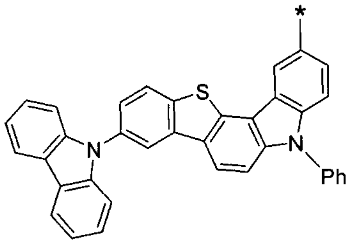
Z38

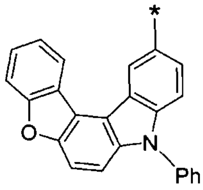
Z39

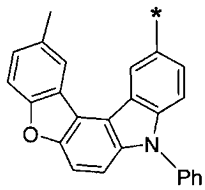
Z40

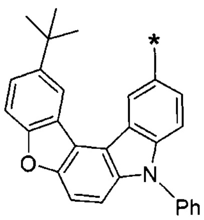
Z41

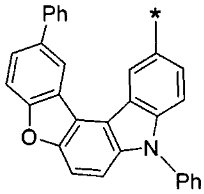
Z42

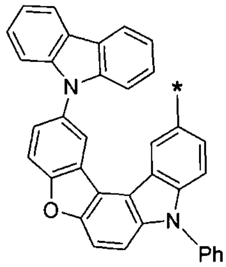
Z43

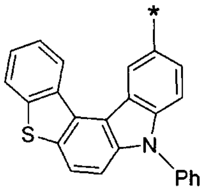
Z44

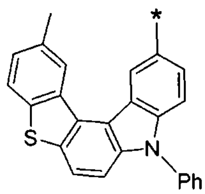
Z45

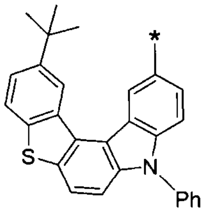
Z46

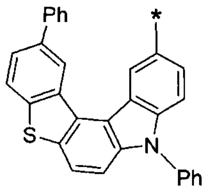
Z47

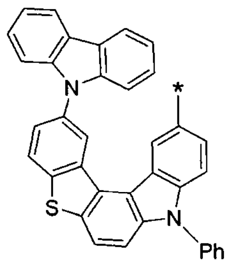
Z48

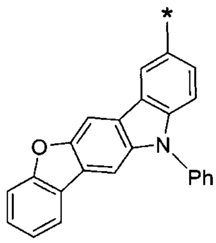
Z49

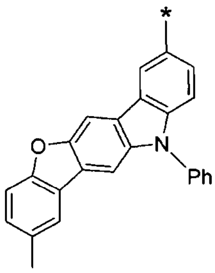
Z50

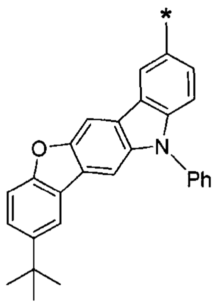
Z51

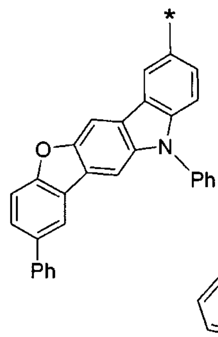
Z52

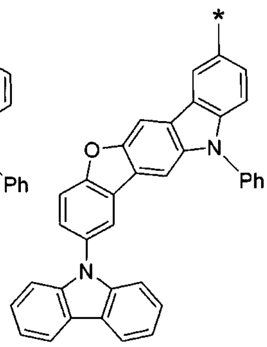
Z53

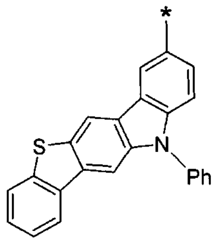
Z54

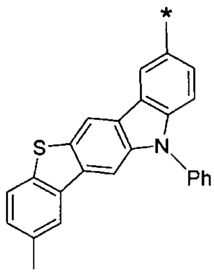
Z55

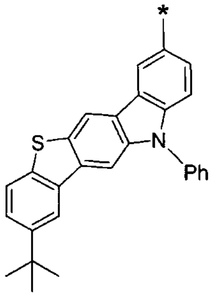
Z56

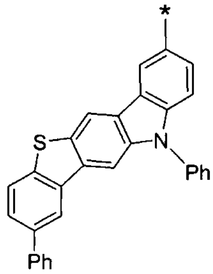
Z57

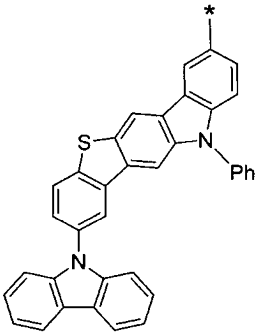
Z58

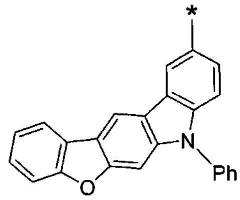
Z59

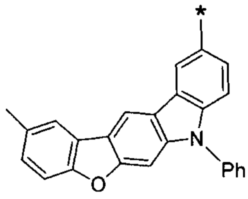

Z60

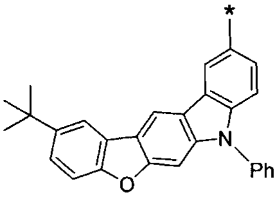

Z61

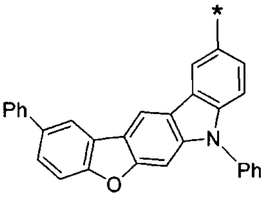

Z62

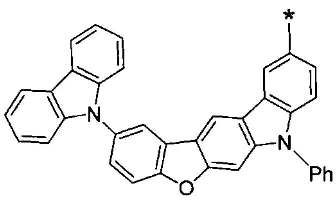

Z63

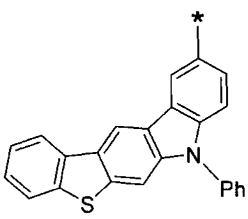

Z64

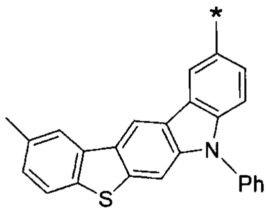

Z65

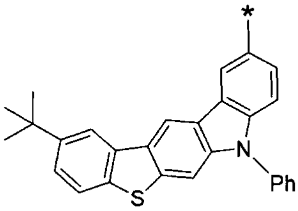

Z66

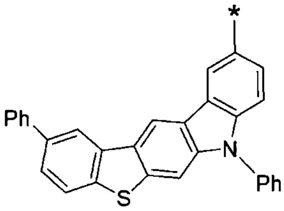

Z67

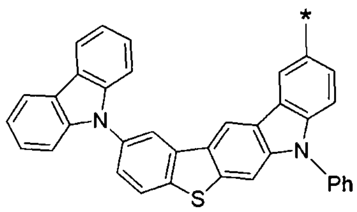

Z68

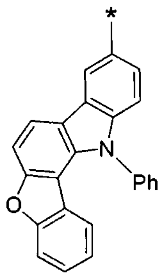

Z69

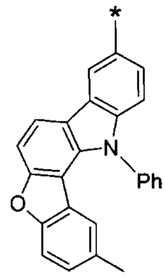

Z70

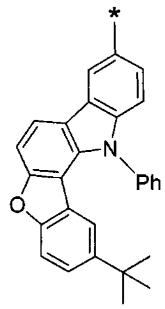

Z71

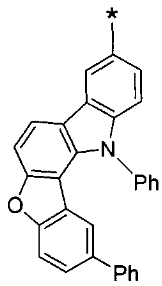

Z72

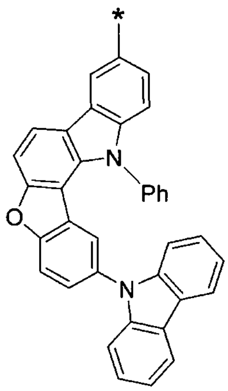

Z73

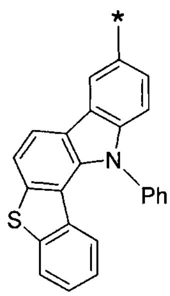

Z74

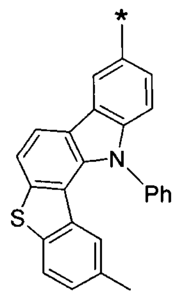

Z75

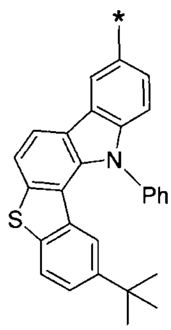

Z76

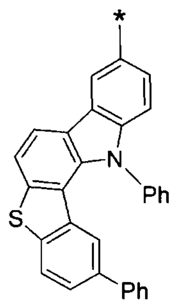

Z77

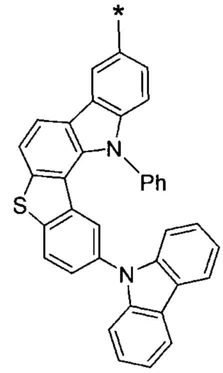
Z78

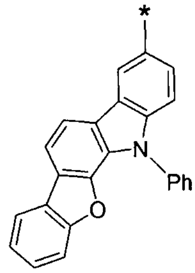
Z79

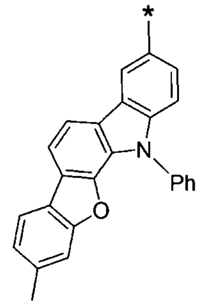
Z80

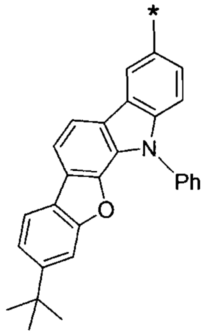
Z81

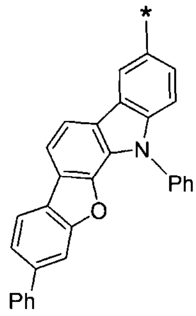
Z82

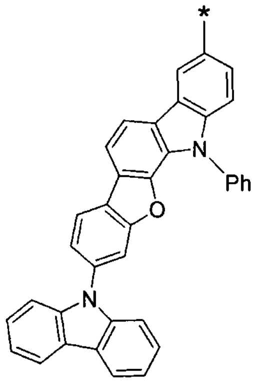
Z83

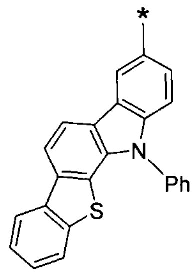
Z84

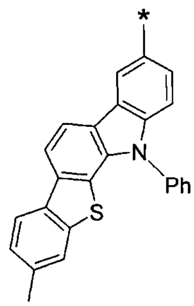
Z85

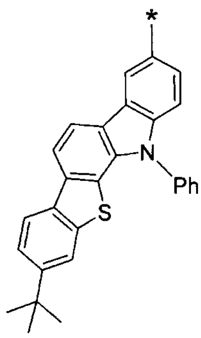
Z86

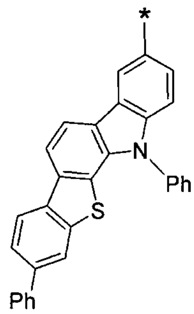
Z87

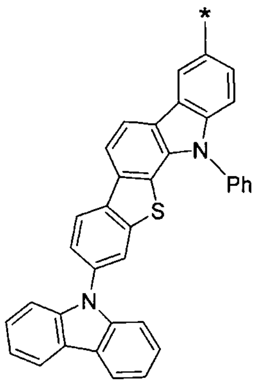
Z88

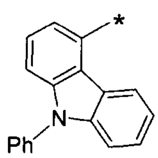
Z89

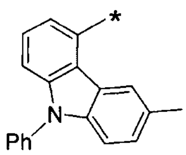
Z90

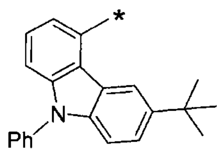
Z91

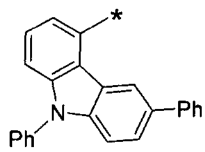
Z92

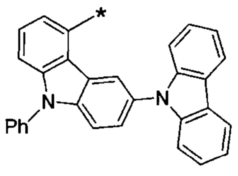
Z93

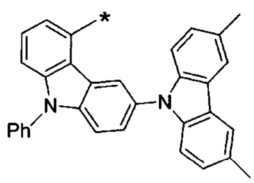
Z94

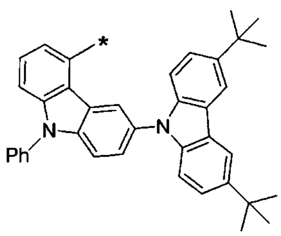
Z95

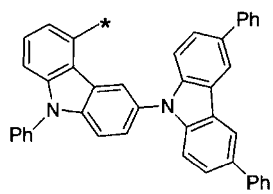
Z96

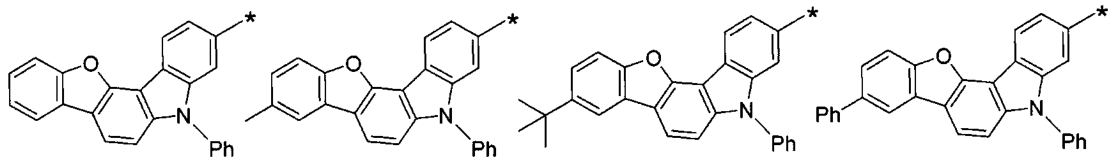

Z97 Z98 Z99 Z100

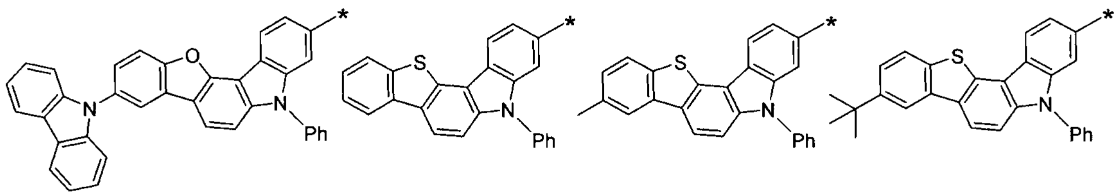

Z101 Z102 Z103 Z104

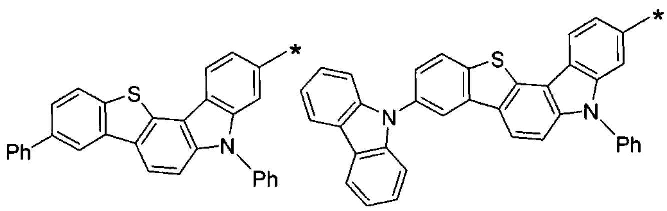

Z105 Z106

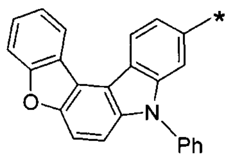

Z107

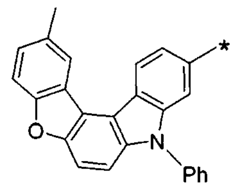

Z108

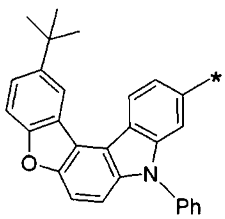

Z109

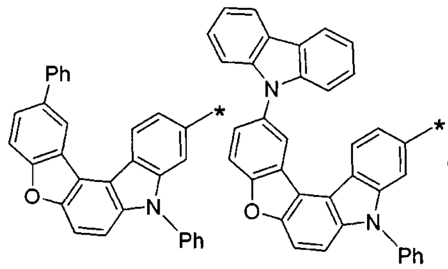

Z110 Z111

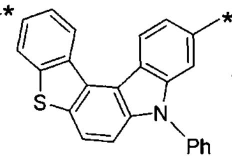

Z112

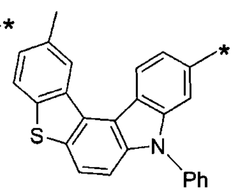

Z113

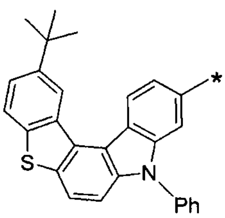

Z114

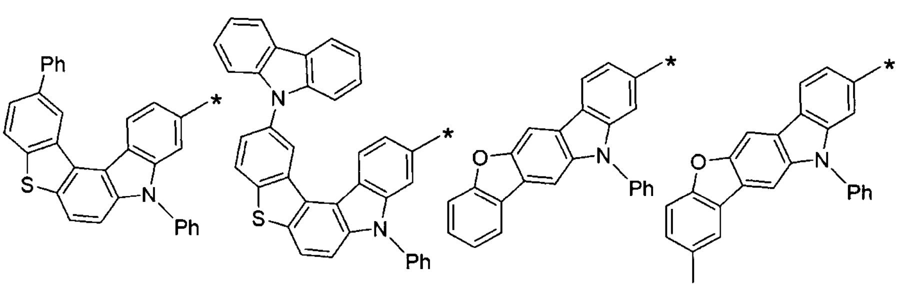

Z115 Z116 Z117 Z118

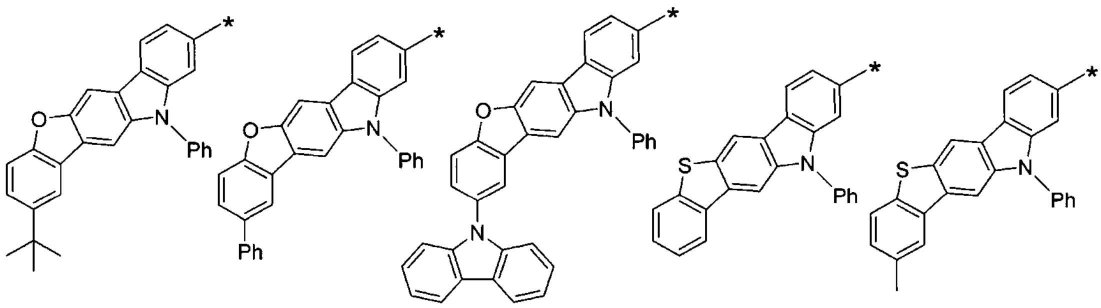

Z119 Z120 Z121 Z122 Z123

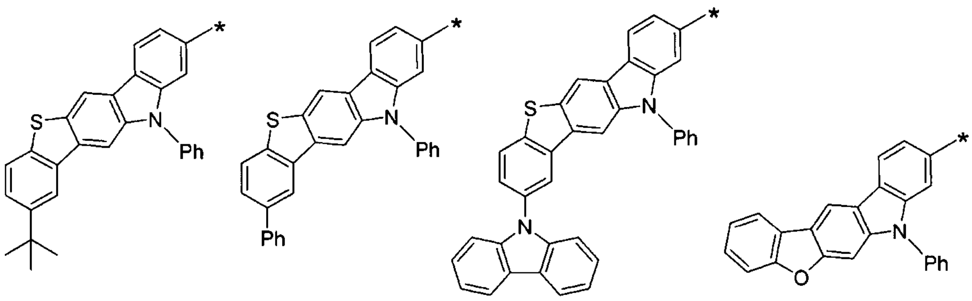

Z124 Z125 Z126 Z127

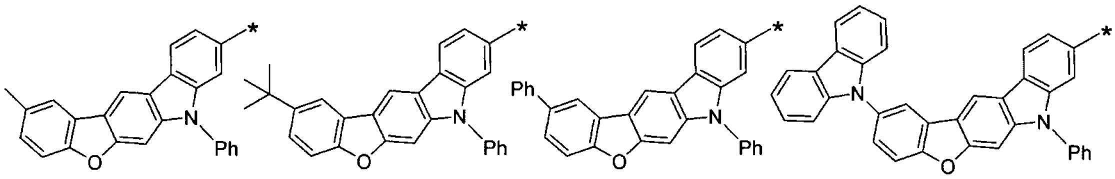

Z128 Z129 Z130 Z131

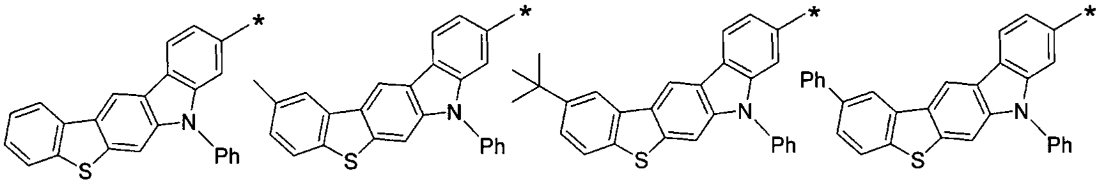

Z132 Z133 Z134 Z135

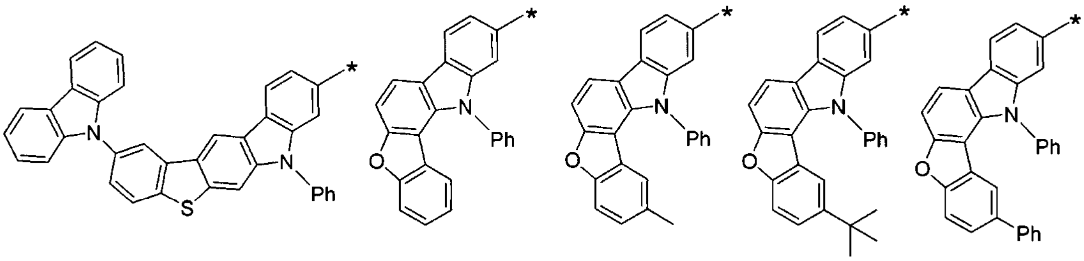

Z136 Z137 Z138 Z139 Z140

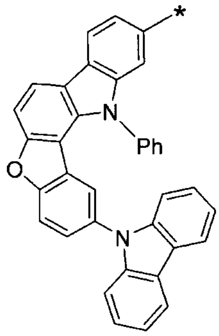
Z141

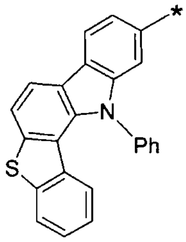
Z142

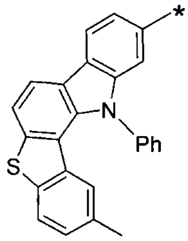
Z143

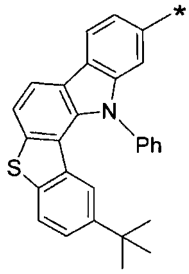
Z144

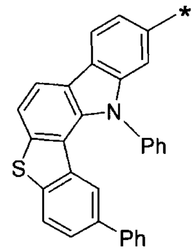
Z145

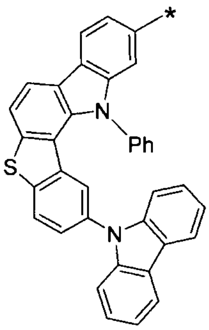
Z146

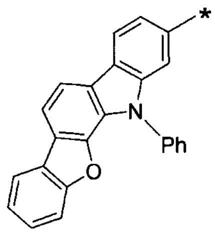
Z147

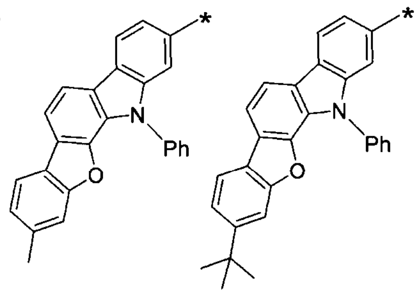
Z148

Z149

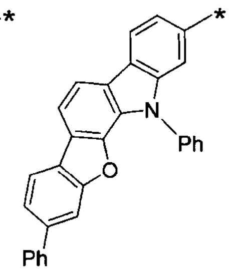
Z150

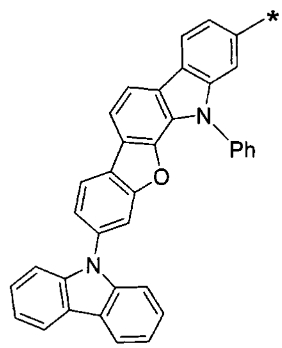
Z151

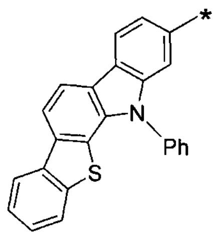
Z152

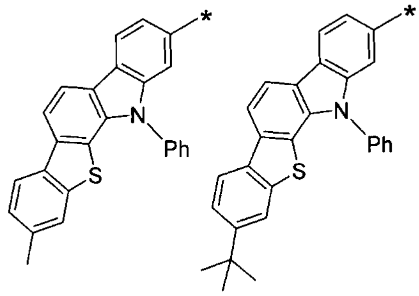
Z153

Z154

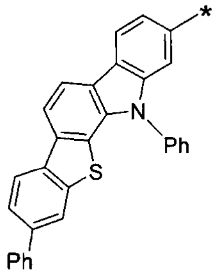
Z155

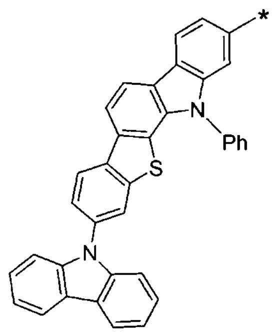
Z156

Groups obtained by substituting all hydrogen atoms present in the above Z1 to Z156 with deuterium atoms are disclosed as Z157 to Z312. Groups obtained by substituting hydrogen atoms bonding to phenyl groups and alkyl groups (methyl groups and tert-butyl groups) present in the above Z1 to Z156 with deuterium atoms are disclosed as Z313 to Z468.

In one aspect of the present invention, the donor group bonding via a carbon atom that $Ar^1$ and $Ar^2$ in the general formula (1) can take is selected from the group consisting of Z1 to Z468. In one aspect of the present invention, the donor group bonding via a carbon atom is selected from the group consisting of Z1 to Z8, Z89 to Z96, Z157 to Z164, Z245 to Z252,

Z313 to Z320, and Z401 to Z408. In one aspect of the present invention, the donor group bonding via a carbon atom is selected from the group consisting of Z9 to Z88, Z97 to Z156, Z165 to Z244, Z253 to Z312, Z321 to Z400, and Z409 to Z468. In one aspect of the present invention, the donor group bonding via a carbon atom is selected from the group consisting of Z1 to Z88, Z157 to Z244, and Z313 to Z400. In one aspect of the present invention, the donor group bonding via a carbon atom is selected from the group consisting of Z89 to Z156, Z245 to Z312, and Z401 to Z468.

In one aspect of the present invention, the donor group bonding via a carbon atom that $Ar^1$ and $Ar^2$ in the general formula (1) can take is selected from the group consisting of D1191 to D5950. In one aspect of the present invention, the donor group bonding via a carbon atom that $Ar^1$ and $Ar^2$ can take is selected from the group consisting of D1191 to D2380 and D3571 to D4760. In one aspect of the present invention, the donor group bonding via a carbon atom that $Ar^1$ and $Ar^2$ can take is selected from the group consisting of D2381 to D3570 and D4761 to D5950.

In one aspect of the present invention, the donor group bonding via a carbon atom is selected from the group consisting of D1191 to D1206, D1655 to D1666, D3571 to D3586, and D4035 to D4046. In one aspect of the present invention, the donor group bonding via a carbon atom is selected from the group consisting of D2381 to D2396, D2845 to D2856, D4761 to D4776, and D5225 to D5236. In one aspect of the present invention, the donor group bonding via a carbon atom is selected from the group consisting of D1207 to D1266, D1278 to D1313, D1380 to D1493, D1554 to D1648, D1667 to D1714, D1721 to D1848, D1899 to D1905, D3587 to D3646, D3658 to D3693, D3760 to D3873, D3934 to D4028, D4047 to D4094, D4101 to D4228, and D4279 to D4285. In one aspect of the present invention, the donor group bonding via a carbon atom is selected from the group consisting of D2397 to D2456, D2468 to D2503, D2570 to D2683, D2744 to D2838, D2857 to D2904, D2911 to D3038, D3089 to D3095, D4777 to D4836, D4848 to D4883, D4950 to D5063, D5124 to D5218, D5237 to D5284, D5291 to D5418, and D5469 to D5475. In one aspect of the present invention, the donor group bonding via a carbon atom is selected from the group consisting of D1267 to D1272, D1314 to D1379, D1494 to D1553, D1650 to D1654, D1715 to D1720, D1849 to D1989, D1907 to D1916, D3647 to D3652, D3694 to D3759, D3874 to D3933, D4030 to D4034, D4095 to D4100, D4229 to D4278, and D4287 to D4296. In one aspect of the present invention, the donor group bonding via a carbon atom is selected from the group consisting of D2457 to D2462, D2504 to D2569, D2684 to D2743, D2840 to D2844, D2905 to D2910, D3039 to D3088, D3097 to D3106, D4837 to D4842, D4884 to D4949, D5064 to D5123, D5220 to D5224, D5285 to D5290, D5419 to D5468, and D5477 to D5486. In one aspect of the present invention, the donor group bonding via a carbon atom is selected from the group consisting of D1191 to D1379, D1648 to D1844, D1907 to D1916, D3571 to D3759, D4028 to D4224, and D4287 to D4296. In one aspect of the present invention, the donor group bonding via a carbon atom is selected from the group consisting of D2381 to D2659, D2838 to D3034, D3097 to D3106, D4761 to D4949, D5218 to D5414, and D5477 to D5486.

In one aspect of the present invention, of $Ar^1$ and $Ar^2$ in the general formula (1), only $Ar^1$ represents a donor group bonding via a carbon atom. In one aspect of the present invention, both $Ar^1$ and $Ar^2$ in the general formula (1) represent the same donor group bonding via a carbon atom. In one aspect of the present invention, $Ar^1$ and $Ar^2$ in the general formula (1) represent the same donor group bonding via a carbon atom, but have different structures. In one preferred aspect of the present invention, $Ar^1$ in the general formula (1) represents a donor group bonding via a carbon atom, and $Ar^2$ represents a substituted or unsubstituted aryl group (e.g., any of Ar1 to Ar88) or a donor group bonding via a nitrogen atom (e.g., any of D1 to D1190). Regarding the description and the preferred range of the substituted or unsubstituted aryl group as referred to herein, reference can be made to the corresponding description of the substituted or unsubstituted aryl group that $R^1$ to $R^5$, $Ar^1$, and $Ar^2$ in the general formula (1) can take. Regarding the description and the preferred range of the donor group, reference can be made to the corresponding description of the donor group that $R^1$ to $R^5$ in the general formula (1) can take.

In the general formula (1), $L^1$ represents a single bond or a divalent linking group. Examples of the divalent linking group include a substituted or unsubstituted arylene group, and a substituted or unsubstituted heteroarylene group. In one preferred aspect of the present invention, $L^1$ represents a single bond. In one aspect of the present invention, $L^1$ represents a substituted or unsubstituted arylene group. In one aspect of the present invention, $L^1$ represents a substituted or unsubstituted heteroarylene group. Regarding the aryl moiety constituting the arylene group, reference can be made to the description and the preferred range of the aryl group in the description section of $R^1$ to $R^5$ mentioned above. Examples of the heteroarylene group include a linking group formed by substituting at least one ring skeleton carbon atom constituting the arylene group with a nitrogen atom.

Specific examples of $L^1$ are shown below. $L^1$ which can be employed in the present invention shall not be construed as being limited by the following specific examples. In the following specific examples, expression of a methyl group is omitted. Consequently, for example, L3 to L5 are substituted with a methyl group. * indicates a bonding position. L1 represents a single bond.

L1

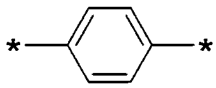
L2

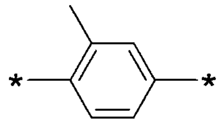
L3

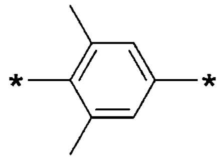
L4

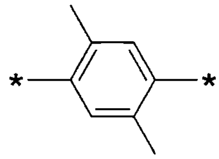
L5

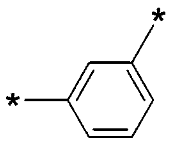
L6

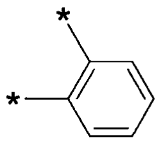
L7

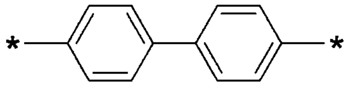
L8

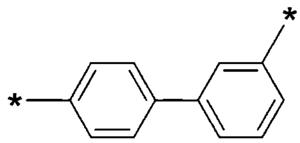
L9

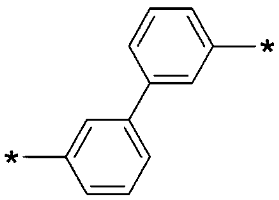
L10

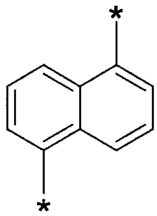
L11

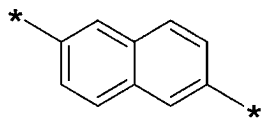
L12

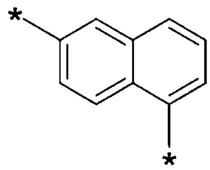
L13

Groups obtained by substituting all hydrogen atoms present in the above L2 to L13 with deuterium atoms are disclosed as L14 to L25. In one aspect of the present invention, $L^1$ is selected from the group consisting of L1 to L25. In one aspect of the present invention, $L^1$ is selected from the group consisting of L1 to L7, and L14 to L19. In one aspect of the present invention, $L^1$ is selected from the group consisting of L1, L8 to L13, and L20 to L25. In one aspect of the present invention, $L^1$ is selected from the group consisting of L2 to L25.

In the general formula (1), $X^1$ to $X^3$ each independently represent N or C(R). At least one of $X^1$ to $X^3$ represents N. R represents a hydrogen atom, a deuterium atom or a substituent. As referred to herein, the substituent can be selected from Substituent Group A, can be selected from Substituent Group B, can be selected from Substituent Group C, can be selected from Substituent Group D, or can be selected from Substituent Group E. In one preferred aspect of the present invention, $X^1$ to $X^3$ represent N. In one aspect of the present invention, $X^1$ and $X^3$ represent N, and $X^2$ represents C(R). In one aspect of the present invention, $X^1$ and $X^2$ represent N, and $X^3$ represents C(R). In one aspect of the present invention, $X^1$ represents N, and $X^2$ and $X^3$ represent C(R). In one aspect of the present invention, $X^2$ represents N, and $X^1$ and $X^3$ represent C(R). In one aspect of the present invention, R represents a hydrogen atom or a deuterium atom. In one aspect of the present invention, R represents an alkyl group optionally substituted with a deuterium atom. In one aspect of the present invention, R represents an aryl group optionally substituted with a deuterium atom, an alkyl group or an aryl group.

In one preferred aspect of the present invention, $X^1$ to $X^3$ represent N, $L^1$ represents a single bond, $Ar^1$ represents a group represented by the general formula (d), $Ar^2$ represents a substituted or unsubstituted aryl group (for example, a substituted or unsubstituted aryl group having no donor group, for example, a group represented by the general formula (d)), or a donor group bonding via a nitrogen atom, $R^2$ represents a cyano group, and two of $R^1$ and $R^3$ to $R^5$ represent a donor group (preferably a substituted or unsubstituted carbazol-9-yl group), one represents a substituted or unsubstituted aryl group, and one (preferably $R^1$) represents a hydrogen atom or a deuterium atom.

In one preferred aspect of the present invention, $X^1$ to $X^3$ represent N, $L^1$ represents a single bond, $Ar^1$ represents a group represented by the general formula (e), $Ar^2$ represents a substituted or unsubstituted aryl group (for example, a substituted or unsubstituted aryl group having no donor group, for example, a group represented by the general formula (d)), or a donor group bonding via a nitrogen atom, $R^2$ represents a cyano group, and two of $R^1$ and $R^3$ to $R^5$ represent a donor group (preferably a substituted or unsubstituted carbazol-9-yl group), one represents a substituted or unsubstituted aryl group, and one (preferably $R^1$) represents a hydrogen atom or a deuterium atom.

In one preferred aspect of the present invention, $X^1$ to $X^3$ represent N, $L^1$ represents a single bond, $Ar^1$ represents a group represented by the general formula (d), $Ar^2$ represents a substituted or unsubstituted aryl group (for example, a substituted or unsubstituted aryl group having no donor group, for example, a group represented by the general formula (d)), or a donor group bonding via a nitrogen atom, $R^2$ represents a cyano group, and three of $R^1$ and $R^3$ to $R^5$ represent a donor group (preferably a substituted or unsubstituted carbazol-9-yl group), and one (preferably $R^1$) represents a

hydrogen atom or a deuterium atom.

In one preferred aspect of the present invention, $X^1$ to $X^3$ represent N, $L^1$ represents a single bond, $Ar^1$ represents a group represented by the general formula (e), $Ar^2$ represents a substituted or unsubstituted aryl group (for example, a substituted or unsubstituted aryl group having no donor group, for example, a group represented by the general formula (d)), or a donor group bonding via a nitrogen atom, $R^2$ represents a cyano group, and three of $R^1$ and $R^3$ to $R^5$ represent a donor group (preferably a substituted or unsubstituted carbazol-9-yl group), and one (preferably $R^1$) represents a hydrogen atom or a deuterium atom.

In one preferred aspect of the present invention, $X^1$ to $X^3$ represent N, $L^1$ represents a single bond, $Ar^1$ represents a group represented by the general formula (d), $Ar^2$ represents a substituted or unsubstituted aryl group (for example, a substituted or unsubstituted aryl group having no donor group, for example, a group represented by the general formula (d)), or a donor group bonding via a nitrogen atom, $R^3$ represents a cyano group, and two or three of $R^1$, $R^2$, $R^4$ and $R^5$ represent a donor group (preferably a substituted or unsubstituted carbazol-9-yl group), one or two (preferably at least $R^1$) represent a hydrogen atom or a deuterium atom, and zero or one represents a substituted or unsubstituted aryl group.

In one preferred aspect of the present invention, $X^1$ to $X^3$ represent N, $L^1$ represents a single bond, $Ar^1$ represents a group represented by the general formula (e), $Ar^2$ represents a substituted or unsubstituted aryl group (for example, a substituted or unsubstituted aryl group having no donor group, for example, a group represented by the general formula (d)), or a donor group bonding via a nitrogen atom, $R^3$ represents a cyano group, and two or three of $R^1$, $R^2$, $R^4$ and $R^5$ represent a donor group (preferably a substituted or unsubstituted carbazol-9-yl group), one or two (preferably at least $R^1$) represent a hydrogen atom or a deuterium atom, and zero or one represents a substituted or unsubstituted aryl group.

$Ar^2$ in each of the above preferred aspects represents, for example, a substituted or unsubstituted aryl group having no donor group. $Ar^2$ in each of the above preferred aspects represents, for example, a donor group bonding via a nitrogen atom. $Ar^2$ in each of the above preferred aspects represents, for example, a group represented by the general formula (e). $Ar^2$ in each of the above preferred aspects represents, for example, a group represented by the general formula (f).

The compound represented by the general formula (1) preferably does not contain a metal atom, and can be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the general formula (1) can be a compound composed only of atoms selected from the group consisting of a carbon atom, a hydrogen atom, a deuterium atom, a nitrogen atom, and a sulfur atom. The compound represented by the general formula (1) can be a compound composed only of atoms selected from the group consisting of a carbon atom, a hydrogen atom, a deuterium atom, and a nitrogen atom. The compound represented by the general formula (1) can be a compound composed only of atoms selected from the group consisting of a carbon atom, a hydrogen atom, and a nitrogen atom. Further, the compound represented by the general formula (1) can be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), and a diarylaminoamino group (for example, having 0 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" can be selected, for example, from Substituent Group A, can be selected from Substituent Group B, can be selected from Substituent Group C, can be selected from Substituent Group D, or can be selected from

Substituent Group E.

Specific examples of the compound represented by the general formula (1) are shown in the following Tables 1 to 4. The compound represented by the general formula (1) that can be used in the present invention should not be construed as being limited by these specific examples.

In Table 1, structures of the compounds are individually shown by specifying $R^3$ to $R^5$ of the following general formula (1a) for each compound. That is, the structures where $Ar^1$ represents a 4-(perdeuterated carbazol-9-yl)phenyl group (D3107), $Ar^2$ represents a perdeuterated phenyl group (Ar45), $X^1$ to $X^3$ represent a nitrogen atom (N), $L^1$ represents a single bond (L1), $R^1$ represents a hydrogen atom, $R^2$ represents a cyano group, and $R^3$ to $R^5$ represent the groups specified in Table 1 are individually shown as structures of Compounds 1 to 240.

General Formula (1a)

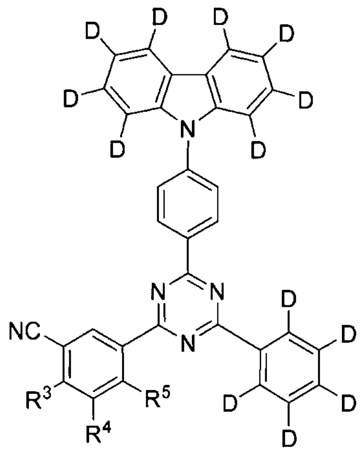

[Table 1]

| No. | $R^3$ | $R^4$ | $R^5$ |
|---|---|---|---|
| 1 | D1 | D1 | D1 |
| 2 | D2 | D2 | D2 |
| 3 | D3 | D3 | D3 |
| 4 | D4 | D4 | D4 |
| 5 | D5 | D5 | D5 |
| 6 | D6 | D6 | D6 |
| 7 | D7 | D7 | D7 |
| 8 | D8 | D8 | D8 |
| 9 | D9 | D9 | D9 |
| 10 | D10 | D10 | D10 |
| 11 | D11 | D11 | D11 |

(continued)

| No. | $R^3$ | $R^4$ | $R^5$ |
|---|---|---|---|
| 12 | D12 | D12 | D12 |
| 13 | D13 | D13 | D13 |
| 14 | D14 | D14 | D14 |
| 15 | D15 | D15 | D15 |
| 16 | D16 | D16 | D16 |
| 17 | D17 | D17 | D17 |
| 18 | D18 | D18 | D18 |
| 19 | D19 | D19 | D19 |
| 20 | D20 | D20 | D20 |
| 21 | D21 | D21 | D21 |
| 22 | D22 | D22 | D22 |
| 23 | D23 | D23 | D23 |
| 24 | D24 | D24 | D24 |
| 25 | D25 | D25 | D25 |
| 26 | D26 | D26 | D26 |
| 27 | D27 | D27 | D27 |
| 28 | D28 | D28 | D28 |
| 29 | D29 | D29 | D29 |
| 30 | D30 | D30 | D30 |
| 31 | D31 | D31 | D31 |
| 32 | D32 | D32 | D32 |
| 33 | D33 | D33 | D33 |
| 34 | D34 | D34 | D34 |
| 35 | D35 | D35 | D35 |
| 36 | D36 | D36 | D36 |
| 37 | D37 | D37 | D37 |
| 38 | D38 | D38 | D38 |
| 39 | D39 | D39 | D39 |
| 40 | D40 | D40 | D40 |
| 41 | D41 | D41 | D41 |
| 42 | D42 | D42 | D42 |
| 43 | D43 | D43 | D43 |
| 44 | D44 | D44 | D44 |
| 45 | D45 | D45 | D45 |
| 46 | D46 | D46 | D46 |
| 47 | D47 | D47 | D47 |
| 48 | D48 | D48 | D48 |
| 49 | D49 | D49 | D49 |
| 50 | D50 | D50 | D50 |

(continued)

| No. | $R^3$ | $R^4$ | $R^5$ |
|---|---|---|---|
| 51 | D51 | D51 | D51 |
| 52 | D52 | D52 | D52 |
| 53 | D53 | D53 | D53 |
| 54 | D54 | D54 | D54 |
| 55 | D55 | D55 | D55 |
| 56 | D56 | D56 | D56 |
| 57 | D57 | D57 | D57 |
| 58 | D58 | D58 | D58 |
| 59 | D59 | D59 | D59 |
| 60 | D60 | D60 | D60 |
| 61 | D61 | D61 | D61 |
| 62 | D62 | D62 | D62 |
| 63 | D63 | D63 | D63 |
| 64 | D64 | D64 | D64 |
| 65 | D65 | D65 | D65 |
| 66 | D66 | D66 | D66 |
| 67 | D67 | D67 | D67 |
| 68 | D68 | D68 | D68 |
| 69 | D69 | D69 | D69 |
| 70 | D70 | D70 | D70 |
| 71 | D71 | D71 | D71 |
| 72 | D72 | D72 | D72 |
| 73 | D73 | D73 | D73 |
| 74 | D74 | D74 | D74 |
| 75 | D75 | D75 | D75 |
| 76 | D76 | D76 | D76 |
| 77 | D77 | D77 | D77 |
| 78 | D78 | D78 | D78 |
| 79 | D79 | D79 | D79 |
| 80 | D80 | D80 | D80 |
| 81 | D81 | D81 | D81 |
| 82 | D82 | D82 | D82 |
| 83 | D83 | D83 | D83 |
| 84 | D84 | D84 | D84 |
| 85 | D85 | D85 | D85 |
| 86 | D86 | D86 | D86 |
| 87 | D87 | D87 | D87 |
| 88 | D88 | D88 | D88 |
| 89 | D89 | D89 | D89 |

(continued)

| No. | $R^3$ | $R^4$ | $R^5$ |
|---|---|---|---|
| 90 | D90 | D90 | D90 |
| 91 | D91 | D91 | D91 |
| 92 | D92 | D92 | D92 |
| 93 | D93 | D93 | D93 |
| 94 | D94 | D94 | D94 |
| 95 | D95 | D95 | D95 |
| 96 | D96 | D96 | D96 |
| 97 | D97 | D97 | D97 |
| 98 | D98 | D98 | D98 |
| 99 | D99 | D99 | D99 |
| 100 | D100 | D100 | D100 |
| 101 | D101 | D101 | D101 |
| 102 | D102 | D102 | D102 |
| 103 | D103 | D103 | D103 |
| 104 | D104 | D104 | D104 |
| 105 | D105 | D105 | D105 |
| 106 | D106 | D106 | D106 |
| 107 | D107 | D107 | D107 |
| 108 | D108 | D108 | D108 |
| 109 | D109 | D109 | D109 |
| 110 | D110 | D110 | D110 |
| 111 | D111 | D111 | D111 |
| 112 | D112 | D112 | D112 |
| 113 | D113 | D113 | D113 |
| 114 | D114 | D114 | D114 |
| 115 | D115 | D115 | D115 |
| 116 | D116 | D116 | D116 |
| 117 | D117 | D117 | D117 |
| 118 | D118 | D118 | D118 |
| 119 | D119 | D119 | D119 |
| 120 | D120 | D120 | D120 |
| 121 | D121 | D121 | D121 |
| 122 | D122 | D122 | D122 |
| 123 | D123 | D123 | D123 |
| 124 | D124 | D124 | D124 |
| 125 | D125 | D125 | D125 |
| 126 | D126 | D126 | D126 |
| 127 | D127 | D127 | D127 |
| 128 | D128 | D128 | D128 |

(continued)

| No. | $R^3$ | $R^4$ | $R^5$ |
|---|---|---|---|
| 129 | D129 | D129 | D129 |
| 130 | D130 | D130 | D130 |
| 131 | D131 | D131 | D131 |
| 132 | D132 | D132 | D132 |
| 133 | D133 | D133 | D133 |
| 134 | D134 | D134 | D134 |
| 135 | D135 | D135 | D135 |
| 136 | D136 | D136 | D136 |
| 137 | D137 | D137 | D137 |
| 138 | D138 | D138 | D138 |
| 139 | D139 | D139 | D139 |
| 140 | D140 | D140 | D140 |
| 141 | D141 | D141 | D141 |
| 142 | D142 | D142 | D142 |
| 143 | D143 | D143 | D143 |
| 144 | D144 | D144 | D144 |
| 145 | D145 | D145 | D145 |
| 146 | D146 | D146 | D146 |
| 147 | D147 | D147 | D147 |
| 148 | D148 | D148 | D148 |
| 149 | D149 | D149 | D149 |
| 150 | D150 | D150 | D150 |
| 151 | D151 | D151 | D151 |
| 152 | D152 | D152 | D152 |
| 153 | D153 | D153 | D153 |
| 154 | D154 | D154 | D154 |
| 155 | D155 | D155 | D155 |
| 156 | D156 | D156 | D156 |
| 157 | D157 | D157 | D157 |
| 158 | D158 | D158 | D158 |
| 159 | D159 | D159 | D159 |
| 160 | D160 | D160 | D160 |
| 161 | D161 | D161 | D161 |
| 162 | D162 | D162 | D162 |
| 163 | D163 | D163 | D163 |
| 164 | D164 | D164 | D164 |
| 165 | D165 | D165 | D165 |
| 166 | D166 | D166 | D166 |
| 167 | D167 | D167 | D167 |

(continued)

| No. | $R^3$ | $R^4$ | $R^5$ |
|---|---|---|---|
| 168 | D168 | D168 | D168 |
| 169 | D169 | D169 | D169 |
| 170 | D170 | D170 | D170 |
| 171 | D171 | D171 | D171 |
| 172 | D172 | D172 | D172 |
| 173 | D173 | D173 | D173 |
| 174 | D174 | D174 | D174 |
| 175 | D175 | D175 | D175 |
| 176 | D176 | D176 | D176 |
| 177 | D177 | D177 | D177 |
| 178 | D178 | D178 | D178 |
| 179 | D179 | D179 | D179 |
| 180 | D180 | D180 | D180 |
| 181 | D181 | D181 | D181 |
| 182 | D182 | D182 | D182 |
| 183 | D183 | D183 | D183 |
| 184 | D184 | D184 | D184 |
| 185 | D185 | D185 | D185 |
| 186 | D186 | D186 | D186 |
| 187 | D187 | D187 | D187 |
| 188 | D188 | D188 | D188 |
| 189 | D189 | D189 | D189 |
| 190 | D190 | D190 | D190 |
| 191 | D191 | D191 | D191 |
| 192 | D192 | D192 | D192 |
| 193 | D193 | D193 | D193 |
| 194 | D194 | D194 | D194 |
| 195 | D195 | D195 | D195 |
| 196 | D196 | D196 | D196 |
| 197 | D197 | D197 | D197 |
| 198 | D198 | D198 | D198 |
| 199 | D199 | D199 | D199 |
| 200 | D200 | D200 | D200 |
| 201 | D201 | D201 | D201 |
| 202 | D202 | D202 | D202 |
| 203 | D203 | D203 | D203 |
| 204 | D204 | D204 | D204 |
| 205 | D205 | D205 | D205 |
| 206 | D206 | D206 | D206 |

(continued)

| No. | $R^3$ | $R^4$ | $R^5$ |
|---|---|---|---|
| 207 | D207 | D207 | D207 |
| 208 | D208 | D208 | D208 |
| 209 | D209 | D209 | D209 |
| 210 | D210 | D210 | D210 |
| 211 | D211 | D211 | D211 |
| 212 | D212 | D212 | D212 |
| 213 | D213 | D213 | D213 |
| 214 | D214 | D214 | D214 |
| 215 | D215 | D215 | D215 |
| 216 | D216 | D216 | D216 |
| 217 | D217 | D217 | D217 |
| 218 | D218 | D218 | D218 |
| 219 | D219 | D219 | D219 |
| 220 | D220 | D220 | D220 |
| 221 | D221 | D221 | D221 |
| 222 | D222 | D222 | D222 |
| 223 | D223 | D223 | D223 |
| 224 | D224 | D224 | D224 |
| 225 | D225 | D225 | D225 |
| 226 | D226 | D226 | D226 |
| 227 | D227 | D227 | D227 |
| 228 | D228 | D228 | D228 |
| 229 | D229 | D229 | D229 |
| 230 | D230 | D230 | D230 |
| 231 | D231 | D231 | D231 |
| 232 | D232 | D232 | D232 |
| 233 | D233 | D233 | D233 |
| 234 | D234 | D234 | D234 |
| 235 | D235 | D235 | D235 |
| 236 | D236 | D236 | D236 |
| 237 | D237 | D237 | D237 |
| 238 | D238 | D238 | D238 |
| 239 | D239 | D239 | D239 |
| 240 | D240 | D240 | D240 |

In Table 2, structures of Compounds 1 to 1594600 are shown by collectively displaying $R^3$ to $R^5$ of a plurality of compounds in each row. For example, in the row of Compounds 1 to 240 in Table 2, compounds in which $R^3$ to $R^5$ are the same and represent D1 to D240 are referred to as Compounds 1 to 240 in that order. That is, the row of Compounds 1 to 240 in Table 2 collectively represents Compounds 1 to 240 specified in Table 1 in one row. Similarly, in the row of Compounds 241 to 5950 in Table 2, compounds in which $R^3$ to $R^5$ are the same and represent D241 to D5950 are referred to as Compounds 241 to 5950 in that order. In the row of Compounds 5951 to 11900 in Table 2, compounds in which $R^4$ is fixed to

H (hydrogen atom), $R^3$ and $R^5$ are the same and represent D1 to D5950 are referred to as Compounds 5951 to 11900 in that order. In the row of Compounds 11901 to 17850 in Table 2, compounds in which $R^4$ is fixed to Ar1, and $R^3$ and $R^5$ are the same and represent D1 to D5950 are referred to as Compounds 11901 to 17850 in that order. In the same manner, Compounds 17851 to 1594600 in Table 2 are also specified.

[Table 2]

| No. | $R^3$ | $R^4$ | $R^5$ | = |
|---|---|---|---|---|
| 1 ~ 240 | D1~D240 | D1~D240 | D1~D240 | $R^3 = R^4 = R^5$ |
| 241 ~ 5950 | D241 ~ D5950 | D241 ~ D5950 | D241 ~ D5950 | |
| 5951 ~ 11900 | D1 ~ D5950 | H | D1-D5950 | $R^3 = R^5$ |
| 11901 ~ 17850 | D1 ~ D5950 | Ar1 | D1 ~ D5950 | |
| 17851 ~ 23800 | D1 ~ D5950 | Ar2 | D1 ~ D5950 | |
| 23801 ~ 29750 | D1 ~ D5950 | Ar3 | D1 ~ D5950 | |
| 29751 ~ 35700 | D1 ~ D5950 | Ar4 | D1 ~ D5950 | |
| 35701 ~ 41650 | D1 ~ D5950 | Ar5 | D1 ~ D5950 | |
| 41651 ~ 47600 | D1 ~ D5950 | Ar6 | D1 ~ D5950 | |
| 47601 ~ 53550 | D1 ~ D5950 | Ar7 | D1 ~ D5950 | |
| 53551 ~ 59500 | D1 ~ D5950 | Ar8 | D1 ~ D5950 | |
| 59501 ~ 65450 | D1 ~ D5950 | Ar9 | D1 ~ D5950 | |
| 65451 ~ 71400 | D1 ~ D5950 | Ar10 | D1 ~ D5950 | |
| 71401 ~ 77350 | D1 ~ D5950 | Ar11 | D1 ~ D5950 | |
| 77351 ~ 83300 | D1 ~ D5950 | Ar12 | D1 ~ D5950 | |
| 83301 ~ 89250 | D1~D5950 | Ar13 | D1 ~ D5950 | |
| 89251 ~ 95200 | D1~D5950 | Ar14 | D1 ~ D5950 | |
| 95201 ~ 101150 | D1 ~ D5950 | Ar15 | D1-D5950 | |
| 101151 ~ 107100 | D1-D5950 | Ar16 | D1~D5950 | |
| 107101 ~ 113050 | D1 ~ D5950 | Ar17 | D1~D5950 | |
| 113051 ~ 119000 | D1~D5950 | Ar18 | D1 ~ D5950 | |
| 119001 ~ 124950 | D1-D5950 | Ar19 | D1~D5950 | |
| 124951 ~ 130900 | D1 ~ D5950 | Ar20 | D1~D5950 | |
| 130901 ~ 136850 | D1 ~ D5950 | Ar21 | D1 ~ D5950 | |
| 136851 ~ 142800 | D1 ~ D5950 | Ar22 | D1 ~ D5950 | |
| 142801 ~ 148750 | D1 ~ D5950 | Ar23 | D1~D5950 | |
| 148751 ~ 154700 | D1 ~ D5950 | Ar24 | D1~D5950 | |
| 154701 ~ 160650 | D1 ~ D5950 | Ar25 | D1 ~ D5950 | |
| 160651 ~ 166600 | D1 ~ D5950 | Ar26 | D1 ~ D5950 | |
| 166601 ~ 172550 | D1 ~ D5950 | Ar27 | D1 ~ D5950 | |
| 172551 ~ 178500 | D1 ~ D5950 | Ar28 | D1 ~ D5950 | |
| 178501 ~ 184450 | D1 ~ D5950 | Ar29 | D1 ~ D5950 | |
| 184451 ~ 190400 | D1 ~ D5950 | Ar30 | D1 ~ D5950 | |
| 190401 ~ 196350 | D1-D5950 | Ar31 | D1 ~ D5950 | |
| 196351 ~ 202300 | D1 ~ D5950 | Ar32 | D1 ~ D5950 | |
| 202301 ~ 208250 | D1 ~ D5950 | Ar33 | D1 ~ D5950 | |

(continued)

| No. | $R^3$ | $R^4$ | $R^5$ | = |
|---|---|---|---|---|
| 208251 ~ 214200 | D1 ~ D5950 | Ar34 | D1 ~ D5950 | |
| 214201 ~ 220150 | D1 ~ D5950 | Ar35 | D1-D5950 | |
| 220151 ~ 226100 | D1 ~ D5950 | Ar36 | D1 ~ D5950 | |
| 226101 ~ 232050 | D1-D5950 | Ar37 | D1 ~ D5950 | |
| 232051 ~ 238000 | D1 ~ D5950 | Ar38 | D1~D5950 | |
| 238001 ~ 243950 | D1~D5950 | Ar39 | D1~D5950 | |
| 243951 ~ 249900 | D1 ~ D5950 | Ar40 | D1 ~ D5950 | |
| 249901 ~ 255850 | D1 ~ D5950 | Ar41 | D1 ~ D5950 | |
| 255851 ~ 261800 | D1 ~ D5950 | Ar42 | D1 ~ D5950 | |
| 261801 ~ 267750 | D1 ~ D5950 | Ar43 | D1~D5950 | |
| 267751 ~ 273700 | D1 ~ D5950 | Ar44 | D1~D5950 | |
| 273701 ~ 279650 | D1 ~ D5950 | Ar45 | D1~D5950 | |
| 279651 ~ 285600 | D1 ~ D5950 | Ar46 | D1 ~ D5950 | |
| 285601 ~ 291550 | D1 ~ D5950 | Ar47 | D1 ~ D5950 | |
| 291551 ~ 297500 | D1 ~ D5950 | Ar48 | D1~D5950 | |
| 297501 ~ 303450 | D1 ~ D5950 | Ar49 | D1-D5950 | |
| 303451 ~ 309400 | D1 ~ D5950 | Ar50 | D1~D5950 | |
| 309401 ~ 315350 | D1 ~ D5950 | Ar51 | D1 ~ D5950 | |
| 315351 ~ 321300 | D1 ~ D5950 | Ar52 | D1-D5950 | |
| 321301 ~ 327250 | D1 ~ D5950 | Ar53 | D1 ~ D5950 | |
| 327251 ~ 333200 | D1 ~ D5950 | Ar54 | D1~D5950 | |
| 333201 ~ 339150 | D1 ~ D5950 | Ar55 | D1 ~ D5950 | |
| 339151 ~ 345100 | D1 ~ D5950 | Ar56 | D1~D5950 | |
| 345101 ~ 351050 | D1 ~ D5950 | Ar57 | D1~D5950 | |
| 351051 ~ 357000 | D1 ~ D5950 | Ar58 | D1 ~ D5950 | |
| 357001 ~ 362950 | D1 ~ D5950 | Ar59 | D1-D5950 | |
| 362951 ~ 368900 | D1 ~ D5950 | Ar60 | D1 ~ D5950 | |
| 368901 ~ 374850 | D1 ~ D5950 | Ar61 | D1 ~ D5950 | |
| 374851 ~ 380800 | D1 ~ D5950 | Ar62 | D1 ~ D5950 | |
| 380801 ~ 386750 | D1 ~ D5950 | Ar63 | D1~D5950 | |
| 386751 ~ 392700 | D1-D5950 | Ar64 | D1~D5950 | |
| 392701 ~ 398650 | D1 ~ D5950 | Ar65 | D1 ~ D5950 | |
| 398651 ~ 404600 | D1 ~ D5950 | Ar66 | D1 ~ D5950 | |
| 404601 ~ 410550 | D1 ~ D5950 | Ar67 | D1 ~ D5950 | |
| 410551 ~ 416500 | D1 ~ D5950 | Ar68 | D1 ~ D5950 | |
| 416501 ~ 422450 | D1 ~ D5950 | Ar69 | D1 ~ D5950 | |
| 422451 ~ 428400 | D1 ~ D5950 | Ar70 | D1~D5950 | |
| 428401 ~ 434350 | D1 ~ D5950 | Ar71 | D1~D5950 | |
| 434351 ~ 440300 | D1 ~ D5950 | Ar72 | D1~D5950 | |

(continued)

| No. | $R^3$ | $R^4$ | $R^5$ | = |
|---|---|---|---|---|
| 440301 ~ 446250 | D1 ~ D5950 | Ar73 | D1 ~ D5950 | $R^3 = R^5$ |
| 446251 ~ 452200 | D1-D5950 | Ar74 | D1~D5950 | |
| 452201 ~ 458150 | D1 ~ D5950 | Ar75 | D1 ~ D5950 | |
| 458151 ~ 464100 | D1 ~ D5950 | Ar76 | D1 ~ D5950 | |
| 464101 ~ 470050 | D1 ~ D5950 | Ar77 | D1 ~ D5950 | |
| 470051 ~ 476000 | D1 ~ D5950 | Ar78 | D1 ~ D5950 | |
| 476001 ~ 481950 | D1 ~ D5950 | Ar79 | D1-D5950 | |
| 481951 ~ 487900 | D1 ~ D5950 | Ar80 | D1 ~ D5950 | |
| 487901 ~ 493850 | D1 ~ D5950 | Ar81 | D1 ~ D5950 | |
| 493851 ~ 499800 | D1 ~ D5950 | Ar82 | D1~D5950 | |
| 499801 ~ 505750 | D1 ~ D5950 | Ar83 | D1 ~ D5950 | |
| 505751 ~ 511700 | D1-D5950 | Ar84 | D1-D5950 | |
| 511701 ~ 517650 | D1 ~ D5950 | Ar85 | D1-D5950 | |
| 517651 ~ 523600 | D1 ~ D5950 | Ar86 | D1~D5950 | |
| 523601 ~ 529550 | D1 ~ D5950 | Ar87 | D1 ~ D5950 | |
| 529551 ~ 535500 | D1 ~ D5950 | Ar88 | D1 ~ D5950 | |
| 535501 ~ 541450 | D1 ~ D5950 | D1 ~ D5950 | H | $R^3 = R^4$ |
| 541451 ~ 547400 | D1 ~ D5950 | D1 ~ D5950 | Ar1 | |
| 547401 ~ 553350 | D1 ~ D5950 | D1 ~ D5950 | Ar2 | |
| 553351 ~ 559300 | D1 ~ D5950 | D1 ~ D5950 | Ar3 | |
| 559301 ~ 565250 | D1 ~ D5950 | D1 ~ D5950 | Ar4 | |
| 565251 ~ 571200 | D1~D5950 | D1 ~ D5950 | Ar5 | |
| 571201 ~ 577150 | D1 ~ D5950 | D1 ~ D5950 | Ar6 | |
| 577151 ~ 583100 | D1 ~ D5950 | D1 ~ D5950 | Ar7 | |
| 583101 ~ 589050 | D1 ~ D5950 | D1 ~ D5950 | Ar8 | |
| 589051 ~ 595000 | D1 ~ D5950 | D1 ~ D5950 | Ar9 | |
| 595001 ~ 600950 | D1 ~ D5950 | D1 ~ D5950 | Ar10 | |
| 600951 ~ 606900 | D1 ~ D5950 | D1 ~ D5950 | Ar11 | |
| 606901 ~ 612850 | D1 ~ D5950 | D1 ~ D5950 | Ar12 | |
| 612851 ~ 618800 | D1 ~ D5950 | D1 ~ D5950 | Ar13 | |
| 618801 ~ 624750 | D1 ~ D5950 | D1 ~ D5950 | Ar14 | |
| 624751 ~ 630700 | D1 ~ D5950 | D1 ~ D5950 | Ar15 | |
| 630701 ~ 636650 | D1 ~ D5950 | D1 ~ D5950 | Ar16 | |
| 636651 ~ 642600 | D1 ~ D5950 | D1 ~ D5950 | Ar17 | |
| 642601 ~ 648550 | D1 ~ D5950 | D1 ~ D5950 | Ar18 | |
| 648551 ~ 654500 | D1 ~ D5950 | D1 ~ D5950 | Ar19 | |
| 654501 ~ 660450 | D1 ~ D5950 | D1 ~ D5950 | Ar20 | |
| 660451 ~ 666400 | D1 ~ D5950 | D1 ~ D5950 | Ar21 | |
| 666401 ~ 672350 | D1 ~ D5950 | D1 ~ D5950 | Ar22 | |

(continued)

| No. | $R^3$ | $R^4$ | $R^5$ | = |
|---|---|---|---|---|
| 672351 ~ 678300 | D1 ~ D5950 | D1 ~ D5950 | Ar23 | |
| 678301 ~ 684250 | D1 ~ D5950 | D1 ~ D5950 | Ar24 | |
| 684251 ~ 690200 | D1 ~ D5950 | D1 ~ D5950 | Ar25 | |
| 690201 ~ 696150 | D1 ~ D5950 | D1 ~ D5950 | Ar26 | |
| 696151 ~ 702100 | D1 ~ D5950 | D1 ~ D5950 | Ar27 | |
| 702101 ~ 708050 | D1 ~ D5950 | D1 ~ D5950 | Ar28 | |
| 708051 ~ 714000 | D1~D5950 | D1~D5950 | Ar29 | $R^3 = R^4$ |
| 714001 ~ 719950 | D1~D5950 | D1 ~D5950 | Ar30 | |
| 719951 ~ 725900 | D1~D5950 | D1~D5950 | Ar31 | |
| 725901 ~ 731850 | D1~D5950 | D1~D5950 | Ar32 | |
| 731851 ~ 737800 | D1~D5950 | D1~D5950 | Ar33 | |
| 737801 ~ 743750 | D1~D5950 | D1~D5950 | Ar34 | |
| 743751 ~ 749700 | D1~D5950 | D1~D5950 | Ar35 | |
| 749701 ~ 755650 | D1~D5950 | D1~D5950 | Ar36 | |
| 755651 ~ 761600 | D1~D5950 | D1~D5950 | Ar37 | |
| 761601 ~ 767550 | D1~D5950 | D1~D5950 | Ar38 | |
| 767551 ~ 773500 | D1~D5950 | D1~D5950 | Ar39 | |
| 773501 ~ 779450 | D1~D5950 | D1~D5950 | Ar40 | |
| 779451 ~ 785400 | D1~D5950 | D1~D5950 | Ar41 | |
| 785401 ~ 791350 | D1~D5950 | D1~D5950 | Ar42 | |
| 791351 ~ 797300 | D1~D5950 | D1~D5950 | Ar43 | |
| 797301 ~ 803250 | D1~D5950 | D1~D5950 | Ar44 | |
| 803251 ~ 809200 | D1~D5950 | D1~D5950 | Ar45 | |
| 809201 ~ 815150 | D1~D5950 | D1~D5950 | Ar46 | |
| 815151 ~ 821100 | D1~D5950 | D1~D5950 | Ar47 | |
| 821101 ~ 827050 | D1~D5950 | D1~D5950 | Ar48 | |
| 827051 ~ 833000 | D1~D5950 | D1~D5950 | Ar49 | |
| 833001 ~ 838950 | D1~D5950 | D1~D5950 | Ar50 | |
| 838951 ~ 844900 | D1~D5950 | D1~D5950 | Ar51 | |
| 844901 ~ 850850 | D1~D5950 | D1~D5950 | Ar52 | |
| 850851 ~ 856800 | D1~D5950 | D1~D5950 | Ar53 | |
| 856801 ~ 862750 | D1~D5950 | D1~D5950 | Ar54 | |
| 862751 ~ 868700 | D1~D5950 | D1~D5950 | Ar55 | |
| 868701 ~ 874650 | D1~D5950 | D1~D5950 | Ar56 | |
| 874651 ~ 880600 | D1~D5950 | D1~D5950 | Ar57 | |
| 880601 ~ 886550 | D1~D5950 | D1~D5950 | Ar58 | |
| 886551 ~ 892500 | D1~D5950 | D1~D5950 | Ar59 | |
| 892501 ~ 898450 | D1~D5950 | D1~D5950 | Ar60 | |
| 898451 ~ 904400 | D1~D5950 | D1~D5950 | Ar61 | |

(continued)

| No. | $R^3$ | $R^4$ | $R^5$ | = |
|---|---|---|---|---|
| 904401 ~ 910350 | D1~D5950 | D1~D5950 | Ar62 | |
| 910351 ~ 916300 | D1~D5950 | D1~D5950 | Ar63 | |
| 916301 ~ 922250 | D1~D5950 | D1~D5950 | Ar64 | |
| 922251 ~ 928200 | D1~D5950 | D1~D5950 | Ar65 | |
| 928201 ~ 934150 | D1~D5950 | D1~D5950 | Ar66 | |
| 934151 ~ 940100 | D1~D5950 | D1~D5950 | Ar67 | |
| 940101 ~ 946050 | D1~D5950 | D1~D5950 | Ar68 | |
| 946051 ~ 952000 | D1~D5950 | D1~D5950 | Ar69 | |
| 952001 ~ 957950 | D1~D5950 | D1~D5950 | Ar70 | |
| 957951 ~ 963900 | D1~D5950 | D1~D5950 | Ar71 | |
| 963901 ~ 969850 | D1~D5950 | D1~D5950 | Ar72 | |
| 969851 ~ 975800 | D1~D5950 | D1~D5950 | Ar73 | |
| 975801 ~ 981750 | D1~D5950 | D1~D5950 | Ar74 | |
| 981751 ~ 987700 | D1~D5950 | D1~D5950 | Ar75 | |
| 987701 ~ 993650 | D1~D5950 | D1~D5950 | Ar76 | |
| 993651 ~ 999600 | D1~D5950 | D1~D5950 | Ar77 | |
| 999601 ~ 1005550 | D1~D5950 | D1~D5950 | Ar78 | |
| 1005551 ~ 1011500 | D1~D5950 | D1~D5950 | Ar79 | |
| 1011501 ~ 1017450 | D1~D5950 | D1~D5950 | Ar80 | |
| 1017451 ~ 1023400 | D1~D5950 | D1~D5950 | Ar81 | |
| 1023401 ~ 1029350 | D1~D5950 | D1~D5950 | Ar82 | |
| 1029351 ~ 1035300 | D1~D5950 | D1~D5950 | Ar83 | |
| 1035301 ~ 1041250 | D1~D5950 | D1~D5950 | Ar84 | |
| 1041251 ~ 1047200 | D1~D5950 | D1~D5950 | Ar85 | |
| 1047201 ~ 1053150 | D1~D5950 | D1~D5950 | Ar86 | |
| 1053151 ~ 1059100 | D1~D5950 | D1~D5950 | Ar87 | |
| 1059101 ~ 1065050 | D1~D5950 | D1~D5950 | Ar88 | |
| 1065051 ~ 1071000 | H | D1~D5950 | D1~D5950 | |
| 1071001 ~ 1076950 | Ar1 | D1~D5950 | D1~D5950 | |
| 1076951 ~ 1082900 | Ar2 | D1~D5950 | D1~D5950 | |
| 1082901 ~ 1088850 | Ar3 | D1~D5950 | D1~D5950 | |
| 1088851 ~ 1094800 | Ar4 | D1~D5950 | D1~D5950 | |
| 1094801 ~ 1100750 | Ar5 | D1~D5950 | D1~D5950 | |
| 1100751 ~ 1106700 | Ar6 | D1~D5950 | D1~D5950 | |
| 1106701 ~ 1112650 | Ar7 | D1~D5950 | D1~D5950 | |
| 1112651 ~ 1118600 | Ar8 | D1~D5950 | D1~D5950 | |
| 1118601 ~ 1124550 | Ar9 | D1~D5950 | D1~D5950 | |
| 1124551 ~ 1130500 | Ar10 | D1~D5950 | D1~D5950 | |
| 1130501 ~ 1136450 | Ar11 | D1~D5950 | D1~D5950 | |

(continued)

| No. | $R^3$ | $R^4$ | $R^5$ | = |
|---|---|---|---|---|
| 1136451 ~ 1142400 | Ar12 | D1~D5950 | D1~D5950 | $R^4 = R^5$ |
| 1142401 ~ 1148350 | Ar13 | D1~D5950 | D1~D5950 | |
| 1148351 ~ 1154300 | Ar14 | D1~D5950 | D1~D5950 | |
| 1154301 ~ 1160250 | Ar15 | D1~D5950 | D1~D5950 | |
| 1160251 ~ 1166200 | Ar16 | D1~D5950 | D1~D5950 | |
| 1166201 ~ 1172150 | Ar17 | D1~D5950 | D1~D5950 | |
| 1172151 ~ 1178100 | Ar18 | D1~D5950 | D1~D5950 | |
| 1178101 ~ 1184050 | Ar19 | D1~D5950 | D1~D5950 | |
| 1184051 ~ 1190000 | Ar20 | D1~D5950 | D1~D5950 | |
| 1190001 ~ 1195950 | Ar21 | D1~D5950 | D1~D5950 | |
| 1195951 ~ 1201900 | Ar22 | D1~D5950 | D1~D5950 | |
| 1201901 ~ 1207850 | Ar23 | D1~D5950 | D1~D5950 | |
| 1207851 ~ 1213800 | Ar24 | D1~D5950 | D1~D5950 | |
| 1213801 ~ 1219750 | Ar25 | D1~D5950 | D1~D5950 | |
| 1219751 ~ 1225700 | Ar26 | D1~D5950 | D1~D5950 | |
| 1225701 ~ 1231650 | Ar27 | D1~D5950 | D1~D5950 | |
| 1231651 ~ 1237600 | Ar28 | D1~D5950 | D1~D5950 | |
| 1237601 ~ 1243550 | Ar29 | D1~D5950 | D1~D5950 | |
| 1243551 ~ 1249500 | Ar30 | D1~D5950 | D1~D5950 | |
| 1249501 ~ 1255450 | Ar31 | D1~D5950 | D1~D5950 | |
| 1255451 ~ 1261400 | Ar32 | D1~D5950 | D1~D5950 | |
| 1261401 ~ 1267350 | Ar33 | D1~D5950 | D1~D5950 | |
| 1267351 ~ 1273300 | Ar34 | D1~D5950 | D1~D5950 | |
| 1273301 ~ 1279250 | Ar35 | D1~D5950 | D1~D5950 | |
| 1279251 ~ 1285200 | Ar36 | D1~D5950 | D1~D5950 | |
| 1285201 ~ 1291150 | Ar37 | D1~D5950 | D1~D5950 | |
| 1291151 ~ 1297100 | Ar38 | D1~D5950 | D1~D5950 | |
| 1297101 ~ 1303050 | Ar39 | D1~D5950 | D1~D5950 | |
| 1303051 ~ 1309000 | Ar40 | D1~D5950 | D1~D5950 | |
| 1309001 ~ 1314950 | Ar41 | D1~D5950 | D1~D5950 | |
| 1314951 ~ 1320900 | Ar42 | D1~D5950 | D1~D5950 | |
| 1320901 ~ 1326850 | Ar43 | D1~D5950 | D1~D5950 | |
| 1326851 ~ 1332800 | Ar44 | D1~D5950 | D1~D5950 | |
| 1332801 ~ 1338750 | Ar45 | D1~D5950 | D1~D5950 | |
| 1338751 ~ 1344700 | Ar46 | D1~D5950 | D1~D5950 | |
| 1344701 ~ 1350650 | Ar47 | D1~D5950 | D1~D5950 | |
| 1350651 ~ 1356600 | Ar48 | D1~D5950 | D1~D5950 | |
| 1356601 ~ 1362550 | Ar49 | D1~D5950 | D1~D5950 | |
| 1362551 ~ 1368500 | Ar50 | D1~D5950 | D1~D5950 | |

(continued)

| No. | $R^3$ | $R^4$ | $R^5$ | = |
|---|---|---|---|---|
| 1368501 ~ 1374450 | Ar51 | D1~D5950 | D1~D5950 | |
| 1374451 ~ 1380400 | Ar52 | D1~D5950 | D1~D5950 | |
| 1380401 ~ 1386350 | Ar53 | D1~D5950 | D1~D5950 | |
| 1386351 ~ 1392300 | Ar54 | D1~D5950 | D1~D5950 | |
| 1392301 ~ 1398250 | Ar55 | D1~D5950 | D1~D5950 | |
| 1398251 ~ 1404200 | Ar56 | D1~D5950 | D1~D5950 | |
| 1404201 ~ 1410150 | Ar57 | D1~D5950 | D1~D5950 | |
| 1410151 ~ 1416100 | Ar58 | D1~D5950 | D1~D5950 | |
| 1416101 ~ 1422050 | Ar59 | D1~D5950 | D1~D5950 | |
| 1422051 ~ 1428000 | Ar60 | D1~D5950 | D1~D5950 | |
| 1428001 ~ 1433950 | Ar61 | D1~D5950 | D1~D5950 | |
| 1433951 ~ 1439900 | Ar62 | D1~D5950 | D1~D5950 | |
| 1439901 ~ 1445850 | Ar63 | D1~D5950 | D1~D5950 | |
| 1445851 ~ 1451800 | Ar64 | D1~D5950 | D1~D5950 | |
| 1451801 ~ 1457750 | Ar65 | D1~D5950 | D1~D5950 | |
| 1457751 ~ 1463700 | Ar66 | D1~D5950 | D1~D5950 | |
| 1463701 ~ 1469650 | Ar67 | D1~D5950 | D1~D5950 | |
| 1469651 ~ 1475600 | Ar68 | D1~D5950 | D1~D5950 | |
| 1475601 ~ 1481550 | Ar69 | D1~D5950 | D1~D5950 | |
| 1481551 ~ 1487500 | Ar70 | D1~D5950 | D1~D5950 | |
| 1487501 ~ 1493450 | Ar71 | D1~D5950 | D1~D5950 | |
| 1493451 ~ 1499400 | Ar72 | D1~D5950 | D1~D5950 | |
| 1499401 ~ 1505350 | Ar73 | D1~D5950 | D1~D5950 | |
| 1505351 ~ 1511300 | Ar74 | D1~D5950 | D1~D5950 | $R^4 = R^5$ |
| 1511301 ~ 1517250 | Ar75 | D1~D5950 | D1~D5950 | |
| 1517251 ~ 1523200 | Ar76 | D1~D5950 | D1~D5950 | |
| 1523201 ~ 1529150 | Ar77 | D1~D5950 | D1~D5950 | |
| 1529151 ~ 1535100 | Ar78 | D1~D5950 | D1~D5950 | |
| 1535101 ~ 1541050 | Ar79 | D1~D5950 | D1~D5950 | |
| 1541051 ~ 1547000 | Ar80 | D1~D5950 | D1~D5950 | |
| 1547001 ~ 1552950 | Ar81 | D1~D5950 | D1~D5950 | |
| 1552951 ~ 1558900 | Ar82 | D1~D5950 | D1~D5950 | |
| 1558901 ~ 1564850 | Ar83 | D1~D5950 | D1~D5950 | |
| 1564851 ~ 1570800 | Ar84 | D1~D5950 | D1~D5950 | |
| 1570801 ~ 1576750 | Ar85 | D1~D5950 | D1~D5950 | |
| 1576751 ~ 1582700 | Ar86 | D1~D5950 | D1~D5950 | |
| 1582701 ~ 1588650 | Ar87 | D1~D5950 | D1~D5950 | |
| 1588651 ~ 1594600 | Ar88 | D1~D5950 | D1~D5950 | |

Next, specific examples of the compound having a structure represented by the following general formula (1b)

are shown in Table 3. In Table 3, structures of the compounds are shown in the same manner as in Table 2.

General Formula (1b)

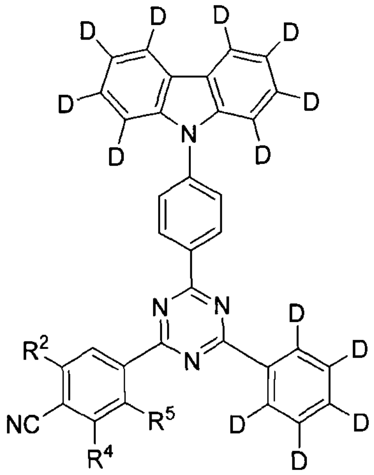

[Table 3]

| No. | $R^2$ | $R^4$ | $R^5$ | = |
|---|---|---|---|---|
| 1594601 ~ 1594840 | D1~D240 | D1~D240 | D1~D240 | $R^2 = R^4 = R^5$ |
| 1594841 ~ 1600550 | D241~D5950 | D241~D5950 | D241~D5950 | |
| 1600551 ~ 1606500 | D1~D5950 | H | D1~D5950 | |
| 1606501 ~ 1612450 | D1~D5950 | Ar1 | D1~D5950 | |
| 1612451 ~ 1618400 | D1~D5950 | Ar2 | D1~D5950 | |
| 1618401 ~ 1624350 | D1~D5950 | Ar3 | D1~D5950 | |
| 1624351 ~ 1630300 | D1~D5950 | Ar4 | D1~D5950 | |
| 1630301 ~ 1636250 | D1~D5950 | Ar5 | D1~D5950 | |
| 1636251 ~ 1642200 | D1~D5950 | Ar6 | D1~D5950 | |
| 1642201 ~ 1648150 | D1~D5950 | Ar7 | D1~D5950 | |
| 1648151 ~ 1654100 | D1~D5950 | Ar8 | D1~D5950 | |
| 1654101 ~ 1660050 | D1~D5950 | Ar9 | D1~D5950 | |
| 1660051 ~ 1666000 | D1~D5950 | Ar10 | D1~D5950 | |
| 1666001 ~ 1671950 | D1~D5950 | Ar11 | D1~D5950 | |
| 1671951 ~ 1677900 | D1-D5950 | Ar12 | D1~D5950 | |
| 1677901 ~ 1683850 | D1~D5950 | Ar13 | D1~D5950 | |
| 1683851 ~ 1689800 | D1~D5950 | Ar14 | D1~D5950 | |
| 1689801 ~ 1695750 | D1~D5950 | Ar15 | D1~D5950 | |
| 1695751 ~ 1701700 | D1~D5950 | Ar16 | D1~D5950 | |
| 1701701 ~ 1707650 | D1~D5950 | Ar17 | D1~D5950 | |
| 1707651 ~ 1713600 | D1~D5950 | Ar18 | D1~D5950 | |
| 1713601 ~ 1719550 | D1~D5950 | Ar19 | D1~D5950 | |
| 1719551 ~ 1725500 | D1~D5950 | Ar20 | D1~D5950 | |
| 1725501 ~ 1731450 | D1~D5950 | Ar21 | D1~D5950 | |

(continued)

| No. | $R^2$ | $R^4$ | $R^5$ | = |
|---|---|---|---|---|
| 1731451 ~ 1737400 | D1-D5950 | Ar22 | D1~D5950 | $R^2 = R^5$ |
| 1737401 ~ 1743350 | D1~D5950 | Ar23 | D1~D5950 | |
| 1743351 ~ 1749300 | D1~D5950 | Ar24 | D1~D5950 | |
| 1749301 ~ 1755250 | D1~D5950 | Ar25 | D1~D5950 | |
| 1755251 ~ 1761200 | D1~D5950 | Ar26 | D1~D5950 | |
| 1761201 ~ 1767150 | D1~D5950 | Ar27 | D1~D5950 | |
| 1767151 ~ 1773100 | D1~D5950 | Ar28 | D1~D5950 | |
| 1773101 ~ 1779050 | D1~D5950 | Ar29 | D1~D5950 | |
| 1779051 ~ 1785000 | D1~D5950 | Ar30 | D1~D5950 | |
| 1785001 ~ 1790950 | D1~D5950 | Ar31 | D1~D5950 | |
| 1790951 ~ 1796900 | D1~D5950 | Ar32 | D1~D5950 | |
| 1796901 ~ 1802850 | D1~D5950 | Ar33 | D1~D5950 | |
| 1802851 ~ 1808800 | D1~D5950 | Ar34 | D1~D5950 | |
| 1808801 ~ 1814750 | D1~D5950 | Ar35 | D1~D5950 | |
| 1814751 ~ 1820700 | D1~D5950 | Ar36 | D1~D5950 | |
| 1820701 ~ 1826650 | D1~D5950 | Ar37 | D1~D5950 | |
| 1826651 ~ 1832600 | D1~D5950 | Ar38 | D1~D5950 | |
| 1832601 ~ 1838550 | D1~D5950 | Ar39 | D1~D5950 | |
| 1838551 ~ 1844500 | D1~D5950 | Ar40 | D1~D5950 | |
| 1844501 ~ 1850450 | D1~D5950 | Ar41 | D1~D5950 | |
| 1850451 ~ 1856400 | D1~D5950 | Ar42 | D1~D5950 | |
| 1856401 ~ 1862350 | D1~D5950 | Ar43 | D1~D5950 | |
| 1862351 ~ 1868300 | D1~D5950 | Ar44 | D1~D5950 | |
| 1868301 ~ 1874250 | D1~D5950 | Ar45 | D1~D5950 | |
| 1874251 ~ 1880200 | D1~D5950 | Ar46 | D1~D5950 | |
| 1880201 ~ 1886150 | D1~D5950 | Ar47 | D1~D5950 | |
| 1886151 ~ 1892100 | D1~D5950 | Ar48 | D1~D5950 | |
| 1892101 ~ 1898050 | D1~D5950 | Ar49 | D1~D5950 | |
| 1898051 ~ 1904000 | D1~D5950 | Ar50 | D1~D5950 | |
| 1904001 ~ 1909950 | D1~D5950 | Ar51 | D1~D5950 | |
| 1909951 ~ 1915900 | D1~D5950 | Ar52 | D1~D5950 | |
| 1915901 ~ 1921850 | D1~D5950 | Ar53 | D1~D5950 | |
| 1921851 ~ 1927800 | D1~D5950 | Ar54 | D1~D5950 | |
| 1927801 ~ 1933750 | D1~D5950 | Ar55 | D1~D5950 | |
| 1933751 ~ 1939700 | D1~D5950 | Ar56 | D1~D5950 | |
| 1939701 ~ 1945650 | D1~D5950 | Ar57 | D1~D5950 | |
| 1945651 ~ 1951600 | D1~D5950 | Ar58 | D1~D5950 | |
| 1951601 ~ 1957550 | D1~D5950 | Ar59 | D1~D5950 | |
| 1957551 ~ 1963500 | D1~D5950 | Ar60 | D1~D5950 | |

(continued)

| No. | $R^2$ | $R^4$ | $R^5$ | = |
|---|---|---|---|---|
| 1963501 ~ 1969450 | D1~D5950 | Ar61 | D1~D5950 | $R^2 = R^5$ |
| 1969451 ~ 1975400 | D1~D5950 | Ar62 | D1~D5950 | |
| 1975401 ~ 1981350 | D1~D5950 | Ar63 | D1~D5950 | |
| 1981351 ~ 1987300 | D1~D5950 | Ar64 | D1~D5950 | |
| 1987301 ~ 1993250 | D1~D5950 | Ar65 | D1~D5950 | |
| 1993251 ~ 1999200 | D1~D5950 | Ar66 | D1~D5950 | |
| 1999201 ~ 2005150 | D1~D5950 | Ar67 | D1~D5950 | |
| 2005151 ~ 2011100 | D1~D5950 | Ar68 | D1~D5950 | |
| 2011101 ~ 2017050 | D1~D5950 | Ar69 | D1~D5950 | |
| 2017051 ~ 2023000 | D1~D5950 | Ar70 | D1~D5950 | |
| 2023001 ~ 2028950 | D1~D5950 | Ar71 | D1~D5950 | |
| 2028951 ~ 2034900 | D1~D5950 | Ar72 | D1~D5950 | |
| 2034901 ~ 2040850 | D1~D5950 | Ar73 | D1~D5950 | |
| 2040851 ~ 2046800 | D1~D5950 | Ar74 | D1~D5950 | |
| 2046801 ~ 2052750 | D1~D5950 | Ar75 | D1~D5950 | |
| 2052751 ~ 2058700 | D1~D5950 | Ar76 | D1~D5950 | |
| 2058701 ~ 2064650 | D1~D5950 | Ar77 | D1~D5950 | |
| 2064651 ~ 2070600 | D1~D5950 | Ar78 | D1~D5950 | |
| 2070601 ~ 2076550 | D1~D5950 | Ar79 | D1~D5950 | |
| 2076551 ~ 2082500 | D1~D5950 | Ar80 | D1~D5950 | |
| 2082501 ~ 2088450 | D1~D5950 | Ar81 | D1~D5950 | |
| 2088451 ~ 2094400 | D1~D5950 | Ar82 | D1~D5950 | |
| 2094401 ~ 2100350 | D1~D5950 | Ar83 | D1~D5950 | |
| 2100351 ~ 2106300 | D1~D5950 | Ar84 | D1~D5950 | |
| 2106301 ~ 2112250 | D1~D5950 | Ar85 | D1~D5950 | |
| 2112251 ~ 2118200 | D1~D5950 | Ar86 | D1~D5950 | |
| 2118201 ~ 2124150 | D1~D5950 | Ar87 | D1~D5950 | |
| 2124151 ~ 2130100 | D1~D5950 | Ar88 | D1~D5950 | |
| 2130101 ~ 2136050 | D1~D5950 | D1~D5950 | H | |
| 2136051 ~ 2142000 | D1~D5950 | D1~D5950 | Ar1 | |
| 2142001 ~ 2147950 | D1~D5950 | D1~D5950 | Ar2 | |
| 2147951 ~ 2153900 | D1~D5950 | D1~D5950 | Ar3 | |
| 2153901 ~ 2159850 | D1~D5950 | D1~D5950 | Ar4 | |
| 2159851 ~ 2165800 | D1~D5950 | D1~D5950 | Ar5 | |
| 2165801 ~ 2171750 | D1~D5950 | D1~D5950 | Ar6 | |
| 2171751 ~ 2177700 | D1~D5950 | D1~D5950 | Ar7 | |
| 2177701 ~ 2183650 | D1~D5950 | D1~D5950 | Ar8 | |
| 2183651 ~ 2189600 | D1~D5950 | D1~ D5950 | Ar9 | |
| 2189601 ~ 2195550 | D1~D5950 | D1~D5950 | Ar10 | |

(continued)

| No. | $R^2$ | $R^4$ | $R^5$ | = |
|---|---|---|---|---|
| 2195551 ~ 2201500 | D1~D5950 | D1~D5950 | Ar11 | $R^2 = R^4$ |
| 2201501 ~ 2207450 | D1~D5950 | D1~D5950 | Ar12 | |
| 2207451 ~ 2213400 | D1~D5950 | D1~D5950 | Ar13 | |
| 2213401 ~ 2219350 | D1~D5950 | D1~D5950 | Ar14 | |
| 2219351 ~ 2225300 | D1~D5950 | D1~D5950 | Ar15 | |
| 2225301 ~ 2231250 | D1~D5950 | D1~D5950 | Ar16 | |
| 2231251 ~ 2237200 | D1~D5950 | D1~D5950 | Ar17 | |
| 2237201 ~ 2243150 | D1~D5950 | D1~D5950 | Ar18 | |
| 2243151 ~ 2249100 | D1~D5950 | D1~D5950 | Ar19 | |
| 2249101 ~ 2255050 | D1~D5950 | D1~D5950 | Ar20 | |
| 2255051 ~ 2261000 | D1~D5950 | D1~D5950 | Ar21 | |
| 2261001 ~ 2266950 | D1~D5950 | D1~D5950 | Ar22 | |
| 2266951 ~ 2272900 | D1~D5950 | D1~D5950 | Ar23 | |
| 2272901 ~ 2278850 | D1~D5950 | D1~D5950 | Ar24 | |
| 2278851 ~ 2284800 | D1~D5950 | D1~D5950 | Ar25 | |
| 2284801 ~ 2290750 | D1~D5950 | D1~D5950 | Ar26 | |
| 2290751 ~ 2296700 | D1~D5950 | D1~D5950 | Ar27 | |
| 2296701 ~ 2302650 | D1~D5950 | D1~D5950 | Ar28 | |
| 2302651 ~ 2308600 | D1~D5950 | D1~D5950 | Ar29 | |
| 2308601 ~ 2314550 | D1~D5950 | D1~D5950 | Ar30 | |
| 2314551 ~ 2320500 | D1~D5950 | D1~D5950 | Ar31 | |
| 2320501 ~ 2326450 | D1~D5950 | D1~D5950 | Ar32 | |
| 2326451 ~ 2332400 | D1~D5950 | D1~D5950 | Ar33 | |
| 2332401 ~ 2338350 | D1~D5950 | D1~D5950 | Ar34 | |
| 2338351 ~ 2344300 | D1~D5950 | D1~D5950 | Ar35 | |
| 2344301 ~ 2350250 | D1~D5950 | D1~D5950 | Ar36 | |
| 2350251 ~ 2356200 | D1~D5950 | D1~D5950 | Ar37 | |
| 2356201 ~ 2362150 | D1~D5950 | D1~D5950 | Ar38 | |
| 2362151 ~ 2368100 | D1~D5950 | D1~D5950 | Ar39 | |
| 2368101 ~ 2374050 | D1~D5950 | D1~D5950 | Ar40 | |
| 2374051 ~ 2380000 | D1~D5950 | D1~D5950 | Ar41 | |
| 2380001 ~ 2385950 | D1~D5950 | D1~D5950 | Ar42 | |
| 2385951 ~ 2391900 | D1-D5950 | D1~ D5950 | Ar43 | |
| 2391901 ~ 2397850 | D1~D5950 | D1~D5950 | Ar44 | |
| 2397851 ~ 2403800 | D1~D5950 | D1~D5950 | Ar45 | |
| 2403801 ~ 2409750 | D1~D5950 | D1~D5950 | Ar46 | |
| 2409751 ~ 2415700 | D1~D5950 | D1~D5950 | Ar47 | |
| 2415701 ~ 2421650 | D1~D5950 | D1~D5950 | Ar48 | |
| 2421651 ~ 2427600 | D1~D5950 | D1~D5950 | Ar49 | |

(continued)

| No. | $R^2$ | $R^4$ | $R^5$ | = |
|---|---|---|---|---|
| 2427601 ~ 2433550 | D1~D5950 | D1~D5950 | Ar50 | $R^2 = R^4$ |
| 2433551 ~ 2439500 | D1~D5950 | D1~D5950 | Ar51 | |
| 2439501 ~ 2445450 | D1~ D5950 | D1~D5950 | Ar52 | |
| 2445451 ~ 2451400 | D1~D5950 | D1~D5950 | Ar53 | |
| 2451401 ~ 2457350 | D1~D5950 | D1~D5950 | Ar54 | |
| 2457351 ~ 2463300 | D1~D5950 | D1~D5950 | Ar55 | |
| 2463301 ~ 2469250 | D1~D5950 | D1~D5950 | Ar56 | |
| 2469251 ~ 2475200 | D1~D5950 | D1~D5950 | Ar57 | |
| 2475201 ~ 2481150 | D1~D5950 | D1~D5950 | Ar58 | |
| 2481151 ~ 2487100 | D1~D5950 | D1~D5950 | Ar59 | |
| 2487101 ~ 2493050 | D1~D5950 | D1~D5950 | Ar60 | |
| 2493051 ~ 2499000 | D1~D5950 | D1~D5950 | Ar61 | |
| 2499001 ~ 2504950 | D1~D5950 | D1~D5950 | Ar62 | |
| 2504951 ~ 2510900 | D1~D5950 | D1~D5950 | Ar63 | |
| 2510901 ~ 2516850 | D1~D5950 | D1~D5950 | Ar64 | |
| 2516851 ~ 2522800 | D1~D5950 | D1~ D5950 | Ar65 | |
| 2522801 ~ 2528750 | D1~D5950 | D1~D5950 | Ar66 | |
| 2528751 ~ 2534700 | D1~D5950 | D1~D5950 | Ar67 | |
| 2534701 ~ 2540650 | D1~D5950 | D1~D5950 | Ar68 | |
| 2540651 ~ 2546600 | D1~D5950 | D1~D5950 | Ar69 | |
| 2546601 ~ 2552550 | D1~D5950 | D1~D5950 | Ar70 | |
| 2552551 ~ 2558500 | D1~D5950 | D1~D5950 | Ar71 | |
| 2558501 ~ 2564450 | D1~D5950 | D1~D5950 | Ar72 | |
| 2564451 ~ 2570400 | D1~D5950 | D1~D5950 | Ar73 | |
| 2570401 ~ 2576350 | D1~D5950 | D1~D5950 | Ar74 | |
| 2576351 ~ 2582300 | D1~D5950 | D1~D5950 | Ar75 | |
| 2582301 ~ 2588250 | D1~D5950 | D1~D5950 | Ar76 | |
| 2588251 ~ 2594200 | D1~D5950 | D1~D5950 | Ar77 | |
| 2594201 ~ 2600150 | D1~D5950 | D1~D5950 | Ar78 | |
| 2600151 ~ 2606100 | D1~D5950 | D1~D5950 | Ar79 | |
| 2606101 ~ 2612050 | D1~D5950 | D1~D5950 | Ar80 | |
| 2612051 ~ 2618000 | D1~D5950 | D1~D5950 | Ar81 | |
| 2618001 ~ 2623950 | D1~D5950 | D1~D5950 | Ar82 | |
| 2623951 ~ 2629900 | D1~D5950 | D1~D5950 | Ar83 | |
| 2629901 ~ 2635850 | D1~D5950 | D1~D5950 | Ar84 | |
| 2635851 ~ 2641800 | D1~D5950 | D1~D5950 | Ar85 | |
| 2641801 ~ 2647750 | D1~D5950 | **D1~D5950** | Ar86 | |
| 2647751 ~ 2653700 | D1~D5950 | D1~D5950 | Ar87 | |
| 2653701 ~ 2659650 | D1~D5950 | D1~D5950 | Ar88 | |

(continued)

| No. | $R^2$ | $R^4$ | $R^5$ | = |
|---|---|---|---|---|
| 2659651 ~ 2665600 | H | D1 ~ D5950 | D1 ~ D5950 | $R^4 = R^5$ |
| 2665601 ~ 2671550 | Ar1 | D1 ~ D5950 | D1~D5950 | |
| 2671551 ~ 2677500 | Ar2 | D1 ~ D5950 | D1 ~ D5950 | |
| 2677501 ~ 2683450 | Ar3 | D1 ~ D5950 | D1 ~ D5950 | |
| 2683451 ~ 2689400 | Ar4 | D1 ~ D5950 | D1 ~ D5950 | |
| 2689401 ~ 2695350 | Ar5 | D1 ~ D5950 | D1 ~ D5950 | |
| 2695351 ~ 2701300 | Ar6 | D1 ~ D5950 | D1~D5950 | |
| 2701301 ~ 2707250 | Ar7 | D1 ~ D5950 | D1 ~ D5950 | |
| 2707251 ~ 2713200 | Ar8 | D1 ~ D5950 | D1 ~ D5950 | |
| 2713201 ~ 2719150 | Ar9 | D1 ~ D5950 | D1 ~ D5950 | |
| 2719151 ~ 2725100 | Ar10 | D1 ~ D5950 | D1 ~ D5950 | |
| 2725101 ~ 2731050 | Ar11 | D1 ~ D5950 | D1~D5950 | |
| 2731051 ~ 2737000 | Ar12 | D1 ~ D5950 | D1 ~ D5950 | |
| 2737001 ~ 2742950 | Ar13 | D1 ~ D5950 | D1~D5950 | |
| 2742951 ~ 2748900 | Ar14 | D1 ~ D5950 | D1~D5950 | |
| 2748901 ~ 2754850 | Ar15 | D1 ~ D5950 | D1~D5950 | |
| 2754851 ~ 2760800 | Ar16 | D1 ~ D5950 | D1 ~ D5950 | |
| 2760801 ~ 2766750 | Ar17 | D1 ~ D5950 | D1~D5950 | |
| 2766751 ~ 2772700 | Ar18 | D1 ~ D5950 | D1 ~ D5950 | |
| 2772701 ~ 2778650 | Ar19 | D1 ~ D5950 | D1 ~ D5950 | |
| 2778651 ~ 2784600 | Ar20 | D1 ~ D5950 | D1~D5950 | |
| 2784601 ~ 2790550 | Ar21 | D1 ~ D5950 | D1 ~ D5950 | |
| 2790551 ~ 2796500 | Ar22 | D1 ~ D5950 | D1 ~ D5950 | |
| 2796501 ~ 2802450 | Ar23 | D1 ~ D5950 | D1 ~ D5950 | |
| 2802451 ~ 2808400 | Ar24 | D1 ~ D5950 | D1 ~ D5950 | |
| 2808401 ~ 2814350 | Ar25 | D1 ~ D5950 | D1~D5950 | |
| 2814351 ~ 2820300 | Ar26 | D1 ~ D5950 | D1 ~ D5950 | |
| 2820301 ~ 2826250 | Ar27 | D1 ~ D5950 | D1 ~ D5950 | |
| 2826251 ~ 2832200 | Ar28 | D1 ~ D5950 | D1 ~ D5950 | |
| 2832201 ~ 2838150 | Ar29 | D1 ~ D5950 | D1~D5950 | |
| 2838151 ~ 2844100 | Ar30 | D1~D5950 | D1 ~ D5950 | |
| 2844101 ~ 2850050 | Ar31 | D1~D5950 | D1~D5950 | |
| 2850051 ~ 2856000 | Ar32 | D1 ~ D5950 | D1~D5950 | |
| 2856001 ~ 2861950 | Ar33 | D1~D5950 | D1~D5950 | |
| 2861951 ~ 2867900 | Ar34 | D1~D5950 | D1~D5950 | |
| 2867901 ~ 2873850 | Ar35 | D1 ~ D5950 | D1 ~ D5950 | |
| 2873851 ~ 2879800 | Ar36 | D1 ~ D5950 | D1~D5950 | |
| 2879801 ~ 2885750 | Ar37 | D1 ~ D5950 | D1~D5950 | |
| 2885751 ~ 2891700 | Ar38 | D1 ~ D5950 | D1~D5950 | |

(continued)

| No. | $R^2$ | $R^4$ | $R^5$ | = |
|---|---|---|---|---|
| 2891701 ~ 2897650 | Ar39 | D1 ~ D5950 | D1~D5950 | |
| 2897651 ~ 2903600 | Ar40 | D1 ~ D5950 | D1 ~ D5950 | |
| 2903601 ~ 2909550 | Ar41 | D1~D5950 | D1~D5950 | |
| 2909551 ~ 2915500 | Ar42 | D1~D5950 | D1~D5950 | |
| 2915501 ~ 2921450 | Ar43 | D1 ~ D5950 | D1~D5950 | |
| 2921451 ~ 2927400 | Ar44 | D1 ~ D5950 | D1~D5950 | |
| 2927401 ~ 2933350 | Ar45 | D1 ~ D5950 | D1 ~ D5950 | |
| 2933351 ~ 2939300 | Ar46 | D1 ~ D5950 | D1~D5950 | |
| 2939301 ~ 2945250 | Ar47 | D1 ~ D5950 | D1~D5950 | |
| 2945251 ~ 2951200 | Ar48 | D1 ~ D5950 | D1 ~ D5950 | |
| 2951201 ~ 2957150 | Ar49 | D1 ~ D5950 | D1~D5950 | |
| 2957151 ~ 2963100 | Ar50 | D1 ~ D5950 | D1 ~ D5950 | |
| 2963101 ~ 2969050 | Ar51 | D1 ~ D5950 | D1~D5950 | |
| 2969051 ~ 2975000 | Ar52 | D1 ~ D5950 | D1~D5950 | |
| 2975001 ~ 2980950 | Ar53 | D1 ~ D5950 | D1~D5950 | |
| 2980951 ~ 2986900 | Ar54 | D1 ~ D5950 | D1~D5950 | |
| 2986901 ~ 2992850 | Ar55 | D1 ~ D5950 | D1~D5950 | |
| 2992851 ~ 2998800 | Ar56 | D1 ~ D5950 | D1~D5950 | |
| 2998801 ~ 3004750 | Ar57 | D1 ~ D5950 | D1 ~ D5950 | |
| 3004751 ~ 3010700 | Ar58 | D1~D5950 | D1~D5950 | |
| 3010701 ~ 3016650 | Ar59 | D1 ~ D5950 | D1 ~ D5950 | |
| 3016651 ~ 3022600 | Ar60 | D1 ~ D5950 | D1 ~ D5950 | |
| 3022601 ~ 3028550 | Ar61 | D1 ~ D5950 | D1 ~ D5950 | |
| 3028551 ~ 3034500 | Ar62 | D1 ~ D5950 | D1~D5950 | |
| 3034501 ~ 3040450 | Ar63 | D1~D5950 | D1 ~ D5950 | |
| 3040451 ~ 3046400 | Ar64 | D1 ~ D5950 | D1~D5950 | |
| 3046401 ~ 3052350 | Ar65 | D1~D5950 | D1~D5950 | |
| 3052351 ~ 3058300 | Ar66 | D1 ~ D5950 | D1~D5950 | |
| 3058301 ~ 3064250 | Ar67 | D1~D5950 | D1~D5950 | |
| 3064251 ~ 3070200 | Ar68 | D1~D5950 | D1 ~ D5950 | |
| 3070201 ~ 3076150 | Ar69 | D1 ~ D5950 | D1 ~ D5950 | |
| 3076151 ~ 3082100 | Ar70 | D1 ~ D5950 | D1 ~ D5950 | |
| 3082101 ~ 3088050 | Ar71 | D1 ~ D5950 | D1 ~ D5950 | |
| 3088051 ~ 3094000 | Ar72 | D1 ~ D5950 | D1~D5950 | |
| 3094001 ~ 3099950 | Ar73 | D1 ~ D5950 | D1~D5950 | |
| 3099951 ~ 3105900 | Ar74 | D1~D5950 | D1 ~ D5950 | $R^4 = R^5$ |
| 3105901 ~ 3111850 | Ar75 | D1~D5950 | D1~D5950 | |
| 3111851 ~ 3117800 | Ar76 | D1~D5950 | D1~D5950 | |
| 3117801 ~ 3123750 | Ar77 | D1~D5950 | D1 ~ D5950 | |

(continued)

| No. | $R^2$ | $R^4$ | $R^5$ | = |
|---|---|---|---|---|
| 3123751 ~ 3129700 | Ar78 | D1 ~ D5950 | D1~D5950 | |
| 3129701 ~ 3135650 | Ar79 | D1 ~ D5950 | D1~D5950 | |
| 3135651 ~ 3141600 | Ar80 | D1 ~ D5950 | D1~D5950 | |
| 3141601 ~ 3147550 | Ar81 | D1~D5950 | D1~D5950 | |
| 3147551 ~ 3153500 | Ar82 | D1 ~ D5950 | D1 ~ D5950 | |
| 3153501 ~ 3159450 | Ar83 | D1 ~ D5950 | D1 ~ D5950 | |
| 3159451 ~ 3165400 | Ar84 | D1 ~ D5950 | D1 ~ D5950 | |
| 3165401 ~3171350 | Ar85 | D1 ~ D5950 | D1 ~ D5950 | |
| 3171351 ~ 3177300 | Ar86 | D1 ~ D5950 | D1 ~ D5950 | |
| 3177301 ~ 3183250 | Ar87 | D1~D5950 | D1 ~ D5950 | |
| 3183251 ~ 3189200 | Ar88 | D1~D5950 | D1 ~ D5950 | |

In Tables 1 to 3, the structures where $Ar^1$ in the general formula (1) represents 4-(perdeuterated carbazol-9-yl) phenyl group (D3107) and $Ar^2$ represents a perdeuterated phenyl group (Ar45) was specified as structures of Compounds 1 to 3189200. In Table 4, $Ar^1$ and $Ar^2$ in Compounds 1 to 3189200 are changed as in Table 4, and the resultant compounds are sequentially displayed as in the table format. In Table 4, Compounds 1 to 3189200 are also shown in the first row for clarifying the correspondence relationship. In the second row in Table 4, for example, Compound 1(1) shows a compound having a structure in which $Ar^1$ of Compound 1 is substituted with D1191. Compound 2(1) indicates a compound having a structure in which $Ar^1$ of Compound 2 is substituted with D1191. Compound 3189200(1) indicates a compound having a structure in which $Ar^1$ of Compound 3189200 is substituted with D1191. Compounds 1(2) to 3189200(2) in the third row in Table 4 and the subsequent compounds are also specified in the same manner. $X^1$ to $X^3$ of the compounds specified in Table 4 all represent a nitrogen atom (N), $L^1$ represents a single bond (L1), and $R^1$ represents a hydrogen atom.

[Table 4]

| No. | $Ar^1$ | $Ar^2$ |
|---|---|---|
| 1 ~ 3189200 | D3107 | Ar45 |
| 1(1) ~ 3189200(1) | D1191 | Ar45 |
| 1(2) ~ 3189200(2) | D1193 | Ar45 |
| 1(3) ~ 3189200(3) | D1199 | Ar45 |
| 1(4) ~ 3189200(4) | D1209 | Ar45 |
| 1(5) ~ 3189200(5) | D1239 | Ar45 |
| 1(6) ~ 3189200(6) | D1650 | Ar45 |
| 1(7) ~ 3189200(7) | D1656 | Ar45 |
| 1(8) ~ 3189200(8) | D1662 | Ar45 |
| 1(9) ~ 3189200(9) | D1916 | Ar45 |
| 1(10) ~ 3189200(10) | D1917 | Ar45 |
| 1(11) ~ 3189200(11) | D2381 | Ar45 |
| 1(12) ~ 3189200(12) | D2383 | Ar45 |
| 1(13) ~ 3189200(13) | D2389 | Ar45 |
| 1(14) ~ 3189200(14) | D2399 | Ar45 |
| 1(15) ~ 3189200(15) | D2429 | Ar45 |
| 1(16) ~ 3189200(16) | D2840 | Ar45 |
| 1(17) ~ 3189200(17) | D2846 | Ar45 |

(continued)

| No. | $Ar^1$ | $Ar^2$ |
|---|---|---|
| 1(18) ~ 3189200(18) | D2852 | Ar45 |
| 1(19) ~ 3189200(19) | D3106 | Ar45 |
| 1(20) ~ 3189200(20) | Z1 | Ar45 |
| 1(21) ~ 3189200(21) | Z2 | Ar45 |
| 1(22) ~ 3189200(22) | Z4 | Ar45 |
| 1(23) ~ 3189200(23) | Z5 | Ar45 |
| 1(24) ~ 3189200(24) | Z9 | Ar45 |
| 1(25) ~ 3189200(25) | Z14 | Ar45 |
| 1(26) ~ 3189200(26) | Z29 | Ar45 |
| 1(27) ~ 3189200(27) | Z34 | Ar45 |
| 1(28) ~ 3189200(28) | Z313 | Ar45 |
| 1(29) ~ 3189200(29) | Z314 | Ar45 |
| 1(30) ~ 3189200(30) | Z316 | Ar45 |
| 1(31) ~ 3189200(31) | Z317 | Ar45 |
| 1(32) ~ 3189200(32) | D3107 | Ar1 |
| 1(33) ~ 3189200(33) | D1191 | Ar1 |
| 1(34) ~ 3189200(34) | D1193 | Ar1 |
| 1(35) ~ 3189200(35) | D1199 | Ar1 |
| 1(36) ~ 3189200(36) | D1209 | Ar1 |
| 1(37) ~ 3189200(37) | D1239 | Ar1 |
| 1(38) ~ 3189200(38) | D1650 | Ar1 |
| 1(39) ~ 3189200(39) | D1656 | Ar1 |
| 1(40) ~ 3189200(40) | D1662 | Ar1 |
| 1(41) ~ 3189200(41) | D1916 | Ar1 |
| 1(42) ~ 3189200(42) | D1917 | Ar1 |
| 1(43) ~ 3189200(43) | D2381 | Ar1 |
| 1(44) ~ 3189200(44) | D2383 | Ar1 |
| 1(45) ~ 3189200(45) | D2389 | Ar1 |
| 1(46) ~ 3189200(46) | D2399 | Ar1 |
| 1(47) ~ 3189200(47) | D2429 | Ar1 |
| 1(48) ~ 3189200(48) | D2840 | Ar1 |
| 1(49) ~ 3189200(49) | D2846 | Ar1 |
| 1(50) ~ 3189200(50) | D2852 | Ar1 |
| 1(51) ~ 3189200(51) | D3106 | Ar1 |
| 1(52) ~ 3189200(52) | Z1 | Ar1 |
| 1(53) ~ 3189200(53) | Z2 | Ar1 |
| 1(54) ~ 3189200(54) | Z4 | Ar1 |
| 1(55) ~ 3189200(55) | Z5 | Ar1 |
| 1(56) ~ 3189200(56) | Z9 | Ar1 |

(continued)

| No. | $Ar^1$ | $Ar^2$ |
|---|---|---|
| 1(57) ~ 3189200(57) | Z14 | Ar1 |
| 1(58) ~ 3189200(58) | Z29 | Ar1 |
| 1(59) ~ 3189200(59) | Z34 | Ar1 |
| 1(60) ~ 3189200(60) | Z313 | Ar1 |
| 1(61) ~ 3189200(61) | Z314 | Ar1 |
| 1(62) ~ 3189200(62) | Z316 | Ar1 |
| 1(63) ~ 3189200(63) | Z317 | Ar1 |
| 1(64) ~ 3189200(64) | D3107 | Ar14 |
| 1(65) ~ 3189200(65) | D1191 | Ar14 |
| 1(66) ~ 3189200(66) | D1193 | Ar14 |
| 1(67) ~ 3189200(67) | D1199 | Ar14 |
| 1(68) ~ 3189200(68) | D1209 | Ar14 |
| 1(69) ~ 3189200(69) | D1239 | Ar14 |
| 1(70) ~ 3189200(70) | D1650 | Ar14 |
| 1(71) ~ 3189200(71) | D1656 | Ar14 |
| 1(72) ~ 3189200(72) | D1662 | Ar14 |
| 1(73) ~ 3189200(73) | D1916 | Ar14 |
| 1(74) ~ 3189200(74) | D1917 | Ar14 |
| 1(75) ~ 3189200(75) | D2381 | Ar14 |
| 1(76) ~ 3189200(76) | D2383 | Ar14 |
| 1(77) ~ 3189200(77) | D2389 | Ar14 |
| 1(78) ~ 3189200(78) | D2399 | Ar14 |
| 1(79) ~ 3189200(79) | D2429 | Ar14 |
| 1(80) ~ 3189200(80) | D2840 | Ar14 |
| 1(81) ~ 3189200(81) | D2846 | Ar14 |
| 1(82) ~ 3189200(82) | D2852 | Ar14 |
| 1(83) ~ 3189200(83) | D3106 | Ar14 |
| 1(84) ~ 3189200(84) | Z1 | Ar14 |
| 1(85) ~ 3189200(85) | Z2 | Ar14 |
| 1(86) ~ 3189200(86) | Z4 | Ar14 |
| 1(87) ~ 3189200(87) | Z5 | Ar14 |
| 1(88) ~ 3189200(88) | Z9 | Ar14 |
| 1(89) ~ 3189200(89) | Z14 | Ar14 |
| 1(90) ~ 3189200(90) | Z29 | Ar14 |
| 1(91) ~ 3189200(91) | Z34 | Ar14 |
| 1(92) ~ 3189200(92) | Z313 | Ar14 |
| 1(93) ~ 3189200(93) | Z314 | Ar14 |
| 1(94) ~ 3189200(94) | Z316 | Ar14 |
| 1(95) ~ 3189200(95) | Z317 | Ar14 |

(continued)

| No. | $Ar^1$ | $Ar^2$ |
|---|---|---|
| 1(96) ~ 3189200(96) | D3107 | D1 |
| 1(97) ~ 3189200(97) | D1191 | D1 |
| 1(98) ~ 3189200(98) | D1193 | D1 |
| 1(99) ~ 3189200(99) | D1199 | D1 |
| 1(100) ~ 3189200(100) | D1209 | D1 |
| 1(101) ~ 3189200(101) | D1239 | D1 |
| 1(102) ~ 3189200(102) | D1650 | D1 |
| 1(103) ~ 3189200(103) | D1656 | D1 |
| 1(104) ~ 3189200(104) | D1662 | D1 |
| 1(105) ~ 3189200(105) | D1916 | D1 |
| 1(106) ~ 3189200(106) | D1917 | D1 |
| 1(107) ~ 3189200(107) | D2381 | D1 |
| 1(108) ~ 3189200(108) | D2383 | D1 |
| 1(109) ~ 3189200(109) | D2389 | D1 |
| 1(110) ~ 3189200(110) | D2399 | D1 |
| 1(111) ~ 3189200(111) | D2429 | D1 |
| 1(112) ~ 3189200(112) | D2840 | D1 |
| 1(113) ~ 3189200(113) | D2846 | D1 |
| 1(114) ~ 3189200(114) | D2852 | D1 |
| 1(115) ~ 3189200(115) | D3106 | D1 |
| 1(116) ~ 3189200(116) | Z1 | D1 |
| 1(117) ~ 3189200(117) | Z2 | D1 |
| 1(118) ~ 3189200(118) | Z4 | D1 |
| 1(119) ~ 3189200(119) | Z5 | D1 |
| 1(120) ~ 3189200(120) | Z9 | D1 |
| 1(121) ~ 3189200(121) | Z14 | D1 |
| 1(122) ~ 3189200(122) | Z29 | D1 |
| 1(123) ~ 3189200(123) | Z34 | D1 |
| 1(124) ~ 3189200(124) | Z313 | D1 |
| 1(125) ~ 3189200(125) | Z314 | D1 |
| 1(126) ~ 3189200(126) | Z316 | D1 |
| 1(127) ~ 3189200(127) | Z317 | D1 |
| 1(128) ~ 3189200(128) | D3107 | D19 |
| 1(129) ~ 3189200(129) | D1191 | D19 |
| 1(130) ~ 3189200(130) | D1193 | D19 |
| 1(131) ~ 3189200(131) | D1199 | D19 |
| 1(132) ~ 3189200(132) | D1209 | D19 |
| 1(133) ~ 3189200(133) | D1239 | D19 |
| 1(134) ~ 3189200(134) | D1650 | D19 |

(continued)

| No. | $Ar^1$ | $Ar^2$ |
|---|---|---|
| 1(135) ~ 3189200(135) | D1656 | D19 |
| 1(136) ~ 3189200(136) | D1662 | D19 |
| 1(137) ~ 3189200(137) | D1916 | D19 |
| 1(138) ~ 3189200(138) | D1917 | D19 |
| 1(139) ~ 3189200(139) | D2381 | D19 |
| 1(140) ~ 3189200(140) | D2383 | D19 |
| 1(141) ~ 3189200(141) | D2389 | D19 |
| 1(142) ~ 3189200(142) | D2399 | D19 |
| 1(143) ~ 3189200(143) | D2429 | D19 |
| 1(144) ~ 3189200(144) | D2840 | D19 |
| 1(145) ~ 3189200(145) | D2846 | D19 |
| 1(146) ~ 3189200(146) | D2852 | D19 |
| 1(147) ~ 3189200(147) | D3106 | D19 |
| 1(148) ~ 3189200(148) | Z1 | D19 |
| 1(149) ~ 3189200(149) | Z2 | D19 |
| 1(150) ~ 3189200(150) | Z4 | D19 |
| 1(151) ~ 3189200(151) | Z5 | D19 |
| 1(152) ~ 3189200(152) | Z9 | D19 |
| 1(153) ~ 3189200(153) | Z14 | D19 |
| 1(154) ~ 3189200(154) | Z29 | D19 |
| 1(155) ~ 3189200(155) | Z34 | D19 |
| 1(156) ~ 3189200(156) | Z313 | D19 |
| 1(157) ~ 3189200(157) | Z314 | D19 |
| 1(158) ~ 3189200(158) | Z316 | D19 |
| 1(159) ~ 3189200(159) | Z317 | D19 |
| 1(160) ~ 3189200(160) | D3107 | Ar38 |
| 1(161) ~ 3189200(161) | D1191 | Ar38 |
| 1(162) ~ 3189200(162) | D1193 | Ar38 |
| 1(163) ~ 3189200(163) | D1199 | Ar38 |
| 1(164) ~ 3189200(164) | D1209 | Ar38 |
| 1(165) ~ 3189200(165) | D1239 | Ar38 |
| 1(166) ~ 3189200(166) | D1650 | Ar38 |
| 1(167) ~ 3189200(167) | D1656 | Ar38 |
| 1(168) ~ 3189200(168) | D1662 | Ar38 |
| 1(169) ~ 3189200(169) | D1916 | Ar38 |
| 1(170) ~ 3189200(170) | D1917 | Ar38 |
| 1(171) ~ 3189200(171) | D2381 | Ar38 |
| 1(172) ~ 3189200(172) | D2383 | Ar38 |
| 1(173) ~ 3189200(173) | D2389 | Ar38 |

(continued)

| No. | $Ar^1$ | $Ar^2$ |
|---|---|---|
| 1(174) ~ 3189200(174) | D2399 | Ar38 |
| 1(175) ~ 3189200(175) | D2429 | Ar38 |
| 1(176) ~ 3189200(176) | D2840 | Ar38 |
| 1(177) ~ 3189200(177) | D2846 | Ar38 |
| 1(178) ~ 3189200(178) | D2852 | Ar38 |
| 1(179) ~ 3189200(179) | D3106 | Ar38 |
| 1(180) ~ 3189200(180) | Z1 | Ar38 |
| 1(181) ~ 3189200(181) | Z2 | Ar38 |
| 1(182) ~ 3189200(182) | Z4 | Ar38 |
| 1(183) ~ 3189200(183) | Z5 | Ar38 |
| 1(184) ~ 3189200(184) | Z9 | Ar38 |
| 1(185) ~ 3189200(185) | Z14 | Ar38 |
| 1(186) ~ 3189200(186) | Z29 | Ar38 |
| 1(187) ~ 3189200(187) | Z34 | Ar38 |
| 1(188) ~ 3189200(188) | Z313 | Ar38 |
| 1(189) ~ 3189200(189) | Z314 | Ar38 |
| 1(190) ~ 3189200(190) | Z316 | Ar38 |
| 1(191) ~ 3189200(191) | Z317 | Ar38 |
| 1(192) ~ 3189200(192) | D3107 | Ar58 |
| 1(193) ~ 3189200(193) | D1191 | Ar58 |
| 1(194) ~ 3189200(194) | D1193 | Ar58 |
| 1(195) ~ 3189200(195) | D1199 | Ar58 |
| 1(196) ~ 3189200(196) | D1209 | Ar58 |
| 1(197) ~ 3189200(197) | D1239 | Ar58 |
| 1(198) ~ 3189200(198) | D1650 | Ar58 |
| 1(199) ~ 3189200(199) | D1656 | Ar58 |
| 1(200) ~ 3189200(200) | D1662 | Ar58 |
| 1(201) ~ 3189200(201) | D1916 | Ar58 |
| 1(202) ~ 3189200(202) | D1917 | Ar58 |
| 1(203) ~ 3189200(203) | D2381 | Ar58 |
| 1(204) ~ 3189200(204) | D2383 | Ar58 |
| 1(205) ~ 3189200(205) | D2389 | Ar58 |
| 1(206) ~ 3189200(206) | D2399 | Ar58 |
| 1(207) ~ 3189200(207) | D2429 | Ar58 |
| 1(208) ~ 3189200(208) | D2840 | Ar58 |
| 1(209) ~ 3189200(209) | D2846 | Ar58 |
| 1(210) ~ 3189200(210) | D2852 | Ar58 |
| 1(211) ~ 3189200(211) | D3106 | Ar58 |
| 1(212) ~ 3189200(212) | Z1 | Ar58 |

(continued)

| No. | $Ar^1$ | $Ar^2$ |
|---|---|---|
| 1(213) ~ 3189200(213) | Z2 | Ar58 |
| 1(214) ~ 3189200(214) | Z4 | Ar58 |
| 1(215) ~ 3189200(215) | Z5 | Ar58 |
| 1(216) ~ 3189200(216) | Z9 | Ar58 |
| 1(217) ~ 3189200(217) | Z14 | Ar58 |
| 1(218) ~ 3189200(218) | Z29 | Ar58 |
| 1(219) ~ 3189200(219) | Z34 | Ar58 |
| 1(220) ~ 3189200(220) | Z313 | Ar58 |
| 1(221) ~ 3189200(221) | Z314 | Ar58 |
| 1(222) ~ 3189200(222) | Z316 | Ar58 |
| 1(223) ~ 3189200(223) | Z317 | Ar58 |
| 1(224) ~ 3189200(224) | D3107 | D727 |
| 1(225) ~ 3189200(225) | D1191 | D727 |
| 1(226) ~ 3189200(226) | D1193 | D727 |
| 1(227) ~ 3189200(227) | D1199 | D727 |
| 1(228) ~ 3189200(228) | D1209 | D727 |
| 1(229) ~ 3189200(229) | D1239 | D727 |
| 1(230) ~ 3189200(230) | D1650 | D727 |
| 1(231) ~ 3189200(231) | D1656 | D727 |
| 1(232) ~ 3189200(232) | D1662 | Ar38 |
| 1(233) ~ 3189200(233) | D1916 | Ar38 |
| 1(234) ~ 3189200(234) | D1917 | Ar38 |
| 1(235) ~ 3189200(235) | D2381 | Ar38 |
| 1(236) ~ 3189200(236) | D2383 | Ar38 |
| 1(237) ~ 3189200(237) | D2389 | D727 |
| 1(238) ~ 3189200(238) | D2399 | D727 |
| 1(239) ~ 3189200(239) | D2429 | D727 |
| 1(240) ~ 3189200(240) | D2840 | D727 |
| 1(241) ~ 3189200(241) | D2846 | D727 |
| 1(242) ~ 3189200(242) | D2852 | D727 |
| 1(243) ~ 3189200(243), | D3106 | D727 |
| 1(244) ~ 3189200(244) | Z1 | D727 |
| 1(245) ~ 3189200(245) | Z2 | D727 |
| 1(246) ~ 3189200(246) | Z4 | D727 |
| 1(247) ~ 3189200(247) | Z5 | D727 |
| 1(248) ~ 3189200(248) | Z9 | D727 |
| 1(249) ~ 3189200(249) | Z14 | D727 |
| 1(250) ~ 3189200(250) | Z29 | D727 |
| 1(251) ~ 3189200(251) | Z34 | D727 |

(continued)

| No. | $Ar^1$ | $Ar^2$ |
|---|---|---|
| 1(252) ~ 3189200(252) | Z313 | D727 |
| 1(253) ~ 3189200(253) | Z314 | D727 |
| 1(254) ~ 3189200(254) | Z316 | D727 |
| 1(255) ~ 3189200(255) | Z317 | D727 |

Compounds in which $Ar^1$ and $Ar^2$ of Compounds 1 to 3189200 both represent D1191 are referred to as Compounds 1(1191) to 3189200(1191) in that order. Compounds in which $Ar^1$ and $Ar^2$ of Compounds 1 to 3189200 both represent D1192 are referred to as Compounds 1(1192) to 3189200(1192) in that order. In this manner, compounds in which $Ar^1$ and $Ar^2$ of Compounds 1 to 3189200 both represent Dx (x = 1191 to 5950) are referred to as Compounds 1(x) to 3189200(x). Finally, compounds in which $Ar^1$ and $Ar^2$ of Compounds 1 to 3189200 both represent D5950 are referred to as Compounds 1(5950) to 3189200(5950) in that order.

Compounds in which $Ar^1$ and $Ar^2$ of Compounds 1 to 3189200 both represent Z1 are referred to as Compounds 1(Z1) to 3189200(Z1) in that order. Compounds in which $Ar^1$ and $Ar^2$ of Compounds 1 to 3189200 both represent Z2 are referred to as Compounds 1(Z2) to 3189200(Z2) in that order. In this manner, compounds in which $Ar^1$ and $Ar^2$ of Compounds 1 to 3189200 both represent Zx (x = 1 to 468) are referred to as Compounds 1(x) to 3189200(x). Finally, compounds in which $Ar^1$ and $Ar^2$ of Compounds 1 to 3189200 both represent Z468 are referred to as Compounds 1(Z468) to 3189200(Z468) in that order.

The compounds specified by the above numbers are all individually disclosed. In addition, among the specific examples of the compounds, in the case where a rotamer is present, a mixture of rotamers and each separated rotamer are also disclosed in the description herein.

In one aspect of the present invention, compounds are selected from Compound Group consisting of Compounds 1 to 3189200 and Compounds 1(n) to 3189200(n), where n is 1 to 255, 1191 to 5950, and Z1 to Z468.

A molecular weight of the compound represented by the general formula (1) is preferably 1500 or less, more preferably 1200 or less, still more preferably 1000 or less, and even more preferably 900 or less, for example, in the case where an organic layer containing the compound represented by the general formula (1) is intended to be film-formed and used by a vapor deposition method. A lower limit of the molecular weight is the molecular weight of the minimum compound represented by the general formula (1).

The compound represented by the general formula (1) can be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable to a coating method and is easily purified to increase its purity.

It is also conceivable to use a compound containing a plurality of structures represented by the general formula (1) in a molecule as a light-emitting material by applying the present invention.

For example, it is conceivable that a polymer obtained by allowing a polymerizable group to be present in the structure represented by the general formula (1) in advance and polymerizing the polymerizable group is used as the light-emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light-emitting material. Alternatively, it is also conceivable to obtain a dimer or a trimer by coupling compounds having a structure represented by the general formula (1) to each other and to use the dimer or the trimer as a light-emitting material.

Examples of the polymer having a repeating unit containing a structure represented by the general formula (1) include polymers containing a structure represented by any one of the following two general formulae.

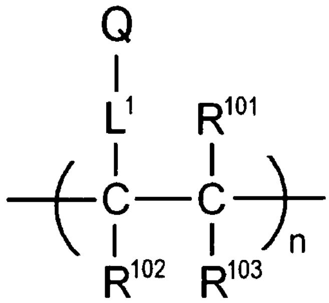

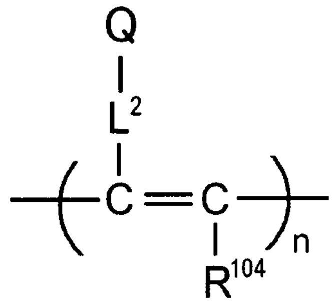

In the above general formulae, Q represents a group containing the structure represented by the general formula (1), $L^1$ and $L^2$ each represent a linking group. The linking group preferably has 0 to 20 carbon atoms, more preferably 1 to 15 carbon atoms, and still more preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or unsubstituted phenylene group.

In the above general formulae, $R^{101}$, $R^{102}$, $R^{103}$ and $R^{104}$ each independently represent a substituent. The substituent is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and still more preferably an unsubstituted alkyl group having 1 to 3 carbon atoms or an unsubstituted alkoxy group having 1 to 3 carbon atoms.

The linking group represented by $L^1$ and $L^2$ can bond to any site of the general formula (1) constituting Q. Two or more linking groups can be linked to one Q to form a cross-linked structure or a network structure.

Specific structural examples of the repeating unit include structures represented by the following formulae.

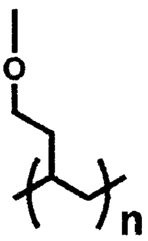 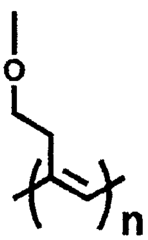 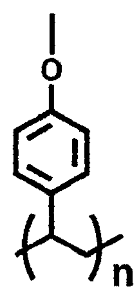 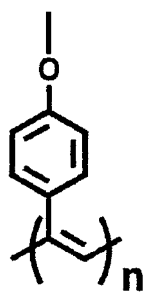

The polymer having a repeating unit including these formulae can be synthesized by introducing a hydroxyl group into any site of the general formula (1), reacting the following compound using the hydroxy group as a linker to introduce a polymerizable group, and polymerizing the polymerizable group.

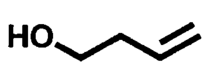

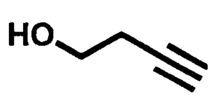

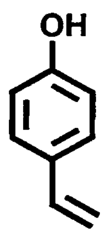

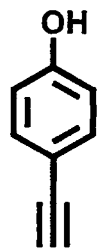

The polymer having a structure represented by the general formula (1) in the molecule can be a polymer having only a repeating unit that has the structure represented by the general formula (1), or can be a polymer containing a repeating unit that has any other structure. The repeating unit having the structure represented by the general formula (1) to be contained in the polymer can be a single kind or two or more kinds. The repeating unit not having the structure represented by the general formula (1) includes those derived from monomers used in general copolymerization. For example, the repeating unit includes repeating units derived from monomers having an ethylenically unsaturated bond,

such as ethylene or styrene.

In some embodiments, the compound represented by the general formula (1) is a light-emitting material.

In some embodiments, the compound represented by the general formula (1) is a compound capable of emitting delayed fluorescence.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm) or emit light in a near IR region.

In some embodiments of the present disclosure, the compound represented by the general formula (1) is, when excited thermally or by an electronic means, able to emit light of red or orange in a visible spectral region (e.g., about 620 nm to about 780 nm, about 650 nm).

In some embodiments of the present disclosure, the compound represented by the general formula (1) is, when excited thermally or by an electronic means, able to emit light of orange or yellow in a visible spectral region (e.g., about 570 nm to about 620 nm, about 590 nm, about 570 nm).

In some embodiments of the present disclosure, the compound represented by the general formula (1) is, when excited thermally or by an electronic means, able to emit light of green in a visible spectral region (e.g., about 490 nm to about 575 nm, about 510 nm).

In some embodiments of the present disclosure, the compound represented by the general formula (1) is, when excited thermally or by an electronic means, able to emit light of blue in a visible spectral region (e.g., about 400 nm to about 490 nm, about 475 nm).

In some embodiments of the present disclosure, the compound represented by the general formula (1) is, when excited thermally or by an electronic means, able to emit light in a UV spectral region (e.g., about 280 nm to 400 nm).

In some embodiments of the present disclosure, the compound represented by the general formula (1) is, when excited thermally or by an electronic means, able to emit light in an IR spectral region (e.g., about 780 nm to 2 $\mu$m).

In some embodiments of the present disclosure, an organic semiconductor device using the compound represented by the general formula (1) can be produced. The organic semiconductor device referred to herein can be an organic optical device in which light is interposed or an organic device in which light is not interposed. The organic optical device can be an organic light-emitting device in which the device emits light, an organic light receiving device in which the device receives light, or a device in which energy transfer by light occurs in the device. In some embodiments of the present disclosure, an organic optical device such as an organic electroluminescent device or a solid-state imaging device (for example, a CMOS image sensor) can be produced by using the compound represented by the general formula (1). In some embodiments of the present disclosure, a complementary metal-oxide film semiconductor (CMOS) or the like using the compound represented by the general formula (1) can be produced.

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFT/B3LYP/6-31G*) is analyzed and molecular fractions (parts) having HOMO not lower than a specific threshold value and LUMO not higher than a specific threshold value can be screened. With that, for example, in the presence of a HOMO energy (for example, ionizing potential) of -6.5 eV or more, a donor part ("D") can be selected. Also, for example, in the presence of a LUMO energy (for example, electron affinity) of -0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the $\pi$-conjugated system. In some embodiments, a compound library is screened using one or more of the following characteristics.

1. Light emission around a specific wavelength
2. A triplet state over a calculated specific energy level
3. $\Delta E_{ST}$ value lower than a specific value
4. Quantum yield more than a specific value
5. HOMO level
6. LUMO level

In some embodiments, the difference ($\Delta E_{ST}$) between the lowest singlet excited state and the lowest triplet excited state at 77 K is less than about 0.5 eV, less than about 0.4 eV, less than about 0.3 eV, less than about 0.2 eV, or less than about 0.1 eV. In some embodiments, $\Delta E_{ST}$ value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV.

In some embodiments, the compound represented by the general formula (1) shows a quantum yield of more

than 25%, for example, about 30%, about 35%, about 40%, about 45%, about 50%, about 55%, about 60%, about 65%, about 70%, about 75%, about 80%, about 85%, about 90%, about 95% or more.

[Method for Synthesizing Compound Represented by General Formula (1)]

The compound represented by the general formula (1) includes a novel compound.

The compound represented by the general formula (1) can be synthesized by combining known reactions. In the compound represented by the general formula (1), two or more of $R^1$ to $R^5$ represent a donor group, and the compound of the general formula (1) in which a substituted or unsubstituted carbazol-9-yl group is a donor group can be synthesized by, for example, reacting substituted or unsubstituted carbazole with a precursor in which a donor group site is a fluorine atom. For details of the reaction conditions, Synthesis Examples described later can be referred to.

[Structure Using Compound Represented by General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

[Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In the case where a vacuum deposition method is employed, compounds to constitute a film can be co-deposited from individual vapor deposition sources, or can be co-deposited from a single vapor deposition source formed by mixing the compounds. In the case where a single vapor deposition source is used, a mixed powder prepared by mixing compound powders can be used, or a compression molded body prepared by compression-molding the mixed powder can be used, or a mixture prepared by heating and melting the compounds and cooling the resulting melt can be used. In some embodiments, by co-deposition under the condition where the vapor deposition rate (weight reduction rate) of the plural compounds contained in a single vapor deposition source is the same or is nearly the same, a film having a compositional ratio corresponding to the compositional ratio of the plural compounds contained in the vapor deposition source can be formed. When plural compounds are mixed in the same compositional ratio as the compositional ratio of the film to be formed to prepare a vapor deposition source, a film having a desired compositional ratio can be formed in a simplified manner. In some embodiments, the temperature at which the compounds to be co-deposited have the same weight reduction ratio is specifically defined, and the temperature can be employed as the temperature of co-deposition.

[Use Examples of Compound Represented by General Formula (1)]

The compound represented by the general formula (1) is useful as a material for an organic light-emitting device. In particular, the compound is preferably used for an organic light-emitting diode or the like.

Organic Light-Emitting Diode:

One embodiment of the present invention relates to use of the compound represented by the general formula (1) of the present invention as a light-emitting material for organic light-emitting devices. In some embodiments, the compound represented by the general formula (1) of the present invention can be effectively used as a light-emitting material in a light-emitting layer in an organic light-emitting device. In some embodiments, the compound represented by the general formula (1) includes a delayed fluorescent substance that emits delayed fluorescence. In some embodiments, the present invention provides a delayed fluorescent substance having a structure represented by the general formula (1). In some embodiments, the present invention relates to use of the compound represented by the general formula (1) as a delayed fluorescent substance. In some embodiments, the compound represented by the general formula (1) of the present invention can be used as a host material, and can be used along with one or more light-emitting materials, and the light-emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light-emitting device containing the compound as a light-emitting material emits delayed fluorescence and shows a high light emission efficiency.

In some embodiments, the light-emitting layer contains the compound represented by the general formula (1), and the compound represented by the general formula (1) is aligned in parallel to the substrate. In some embodiments, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light-emitting layer can be improved.

One aspect of the present invention relates to an organic light-emitting device. In some embodiments, the organic light-emitting device includes a light-emitting layer. In some embodiments, the light-emitting layer contains, as a light-emitting material, the compound represented by the general formula (1). In some embodiments, the organic light-emitting device is an organic photoluminescent device (organic PL device). In some embodiments, the organic light-emitting device is an organic electroluminescent device (organic EL device). In some embodiments, the compound represented by the general formula (1) assists light irradiation from the other light-emitting materials contained in the light-emitting layer (as a so-called assist dopant). In some embodiments, the compound represented by the general formula (1) contained in the light-emitting layer is in a lowest excited singlet energy level, and is contained between the lowest excited single energy level of the host material contained in the light-emitting layer and the lowest excited singlet energy level of the other light-emitting materials contained in the light-emitting layer.

In some embodiments, the organic photoluminescent device includes at least one light-emitting layer. In some embodiments, the organic electroluminescent device includes at least an anode, a cathode, and an organic layer between the anode and the cathode. In some embodiments, the organic layer includes at least a light-emitting layer. In some embodiments, the organic layer includes only a light-emitting layer. In some embodiments, the organic layer includes one or more organic layers in addition to the light-emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer can be a hole injection and transporting layer having a hole injection function, and the electron transporting layer can be an electron injection and transporting layer having an electron injection function.

Light-Emitting Layer:

In some embodiments, the light-emitting layer is a layer where holes and electrons injected from the anode and the cathode, respectively, are recombined to form excitons. In some embodiments, the layer emits light.

In some embodiments, only a light-emitting material is used as the light-emitting layer. In some embodiments, the light-emitting layer contains a light-emitting material and a host material. In some embodiments, the light-emitting material is one or more compounds represented by the general formula (1). In some embodiments, for improving light emission efficiencies of an organic electroluminescent device and an organic photoluminescent device, the singlet exciton and the triplet exciton generated in a light-emitting material are confined inside the light-emitting material. In some embodiments, a host material is used in the light-emitting layer in addition to a light-emitting material. In some embodiments, the host material is an organic compound. In some embodiments, the organic compound has an excited singlet energy and an excited triplet energy, and at least one of them is higher than those in the light-emitting material of the present invention. In some embodiments, the singlet exciton and the triplet exciton generated in the light-emitting material of the present invention are confined in the molecules of the light-emitting material of the present invention. In some embodiments, the

singlet and triplet excitons are fully confined for improving light emission efficiency. In some embodiments, although high luminous radiation efficiency is still attained, singlet excitons and triplet excitons are not fully confined, that is, a host material capable of attaining high light emission efficiency can be used in the present invention with no specific limitation. In some embodiments, in the light-emitting material in the light-emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant and has a lower excited singlet energy than the host material in the light-emitting layer and a higher excited singlet energy than the light-emitting material in the light-emitting layer.

In the case where the compound represented by the general formula (1) is used as an assist dopant, various compounds can be employed as a light-emitting material (preferably a fluorescent material). As such light-emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a derivative having a metal (Al, Zn). These exemplified skeletons can have a substituent, or may not have a substituent. These exemplified skeletons can be combined.

Light-emitting materials that can be used in combination with the assist dopant having a structure represented by the general formula (1) are shown below.

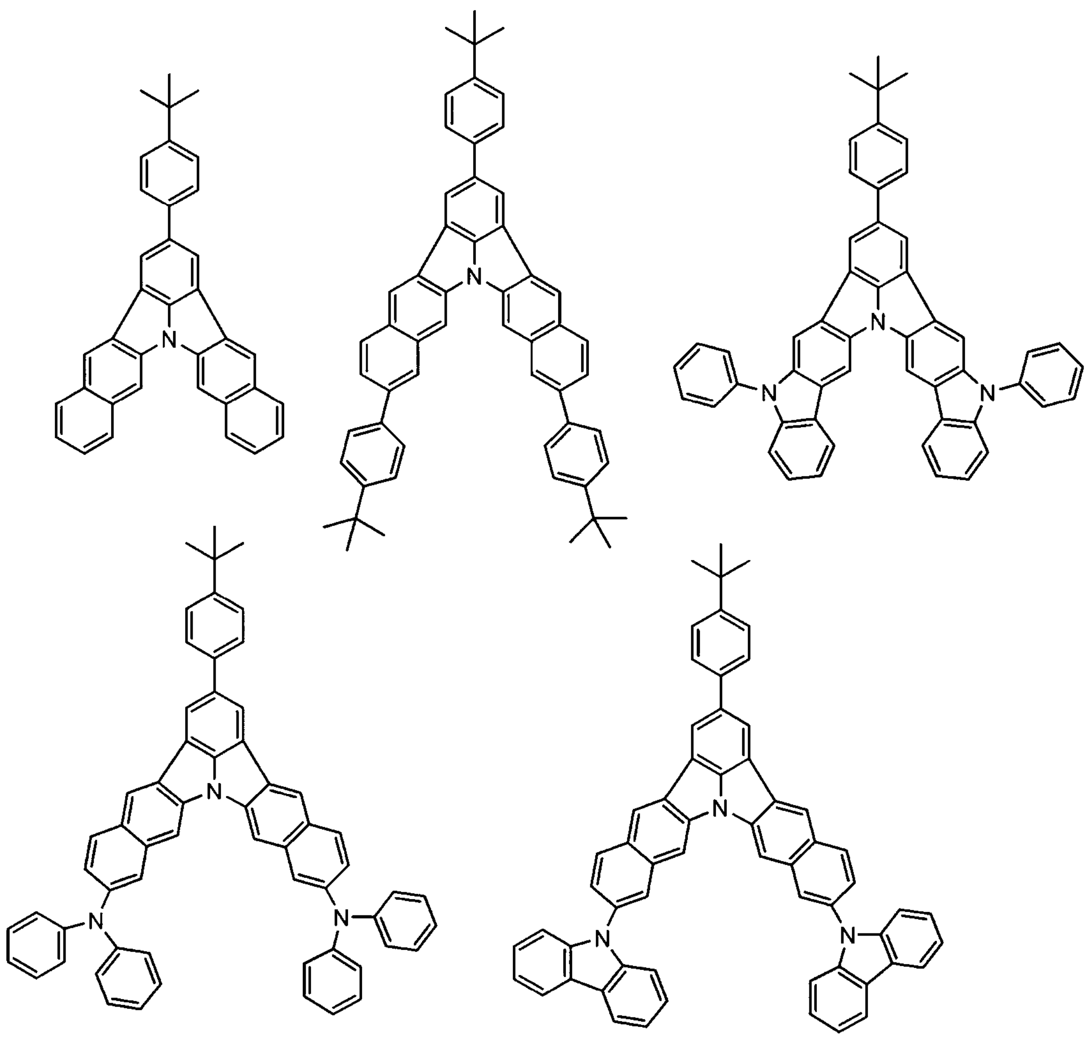

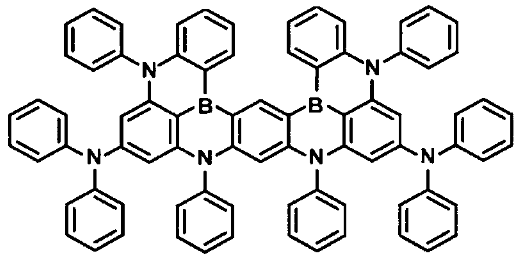
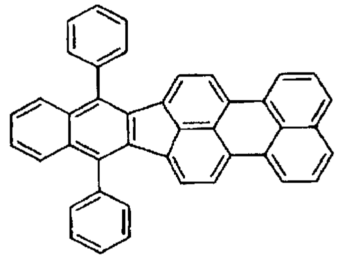

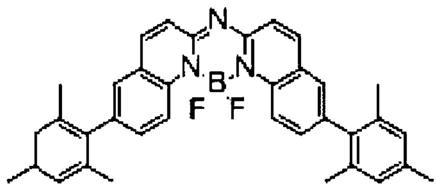

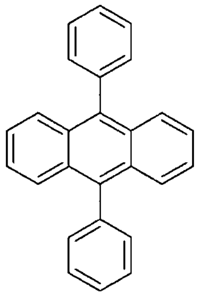
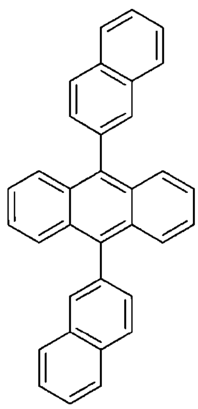
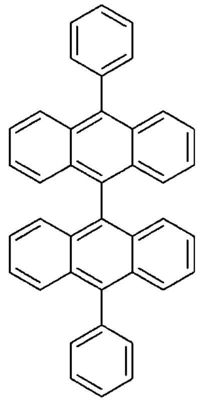
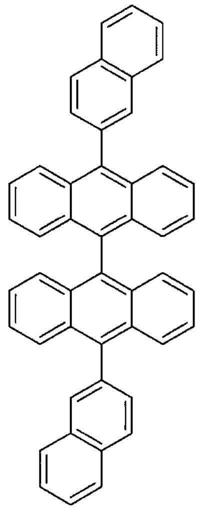

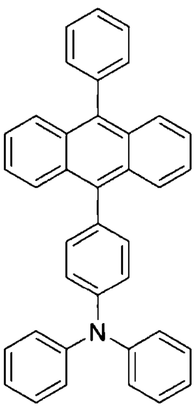
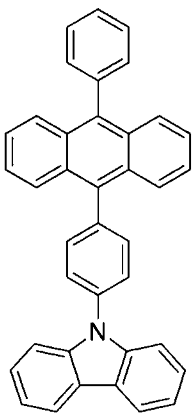
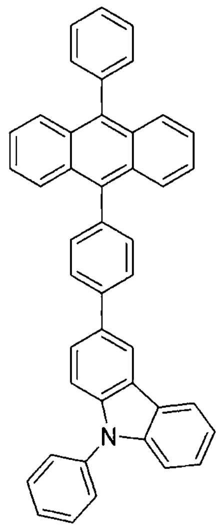
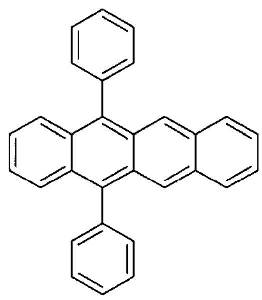

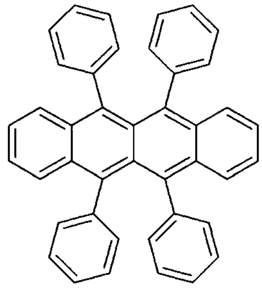

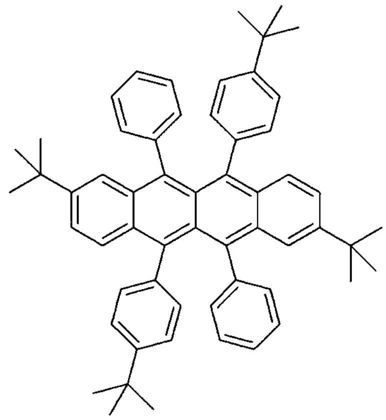

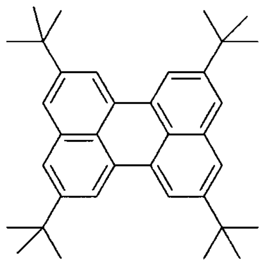

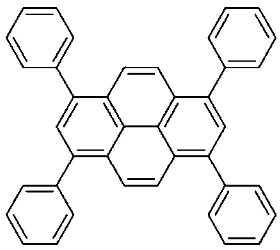

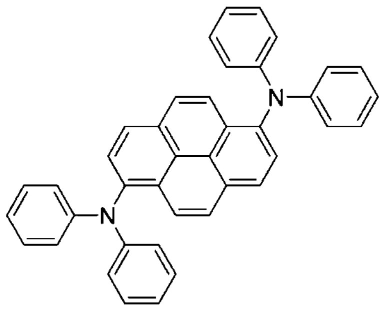

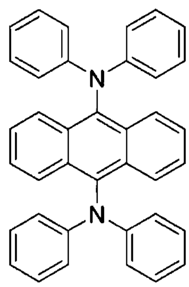

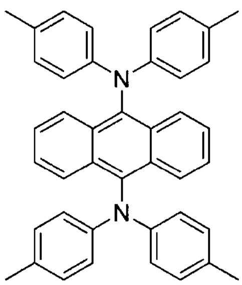

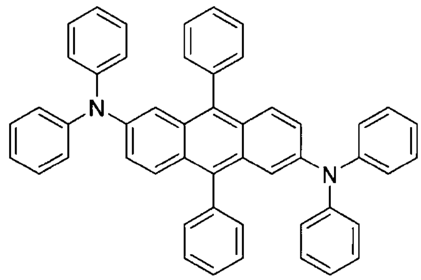

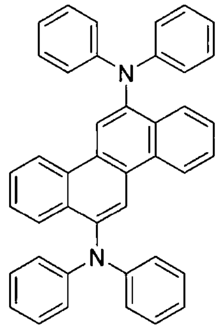

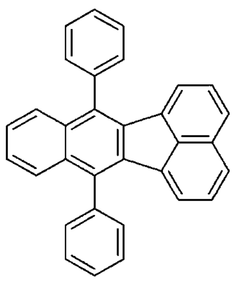

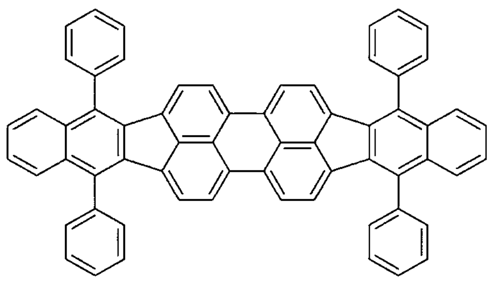

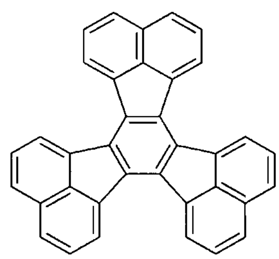

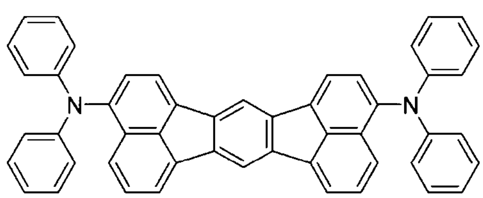

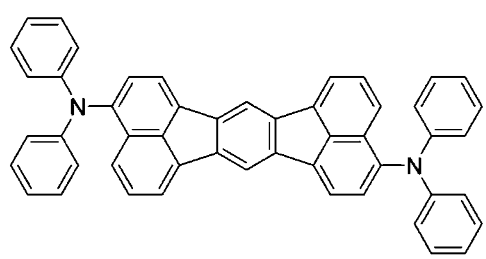

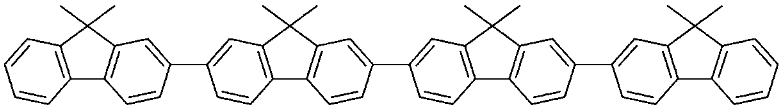

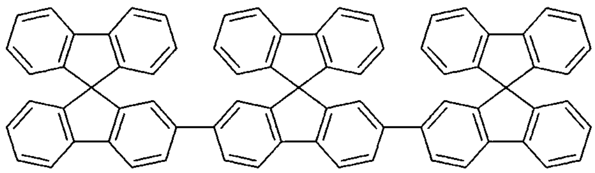

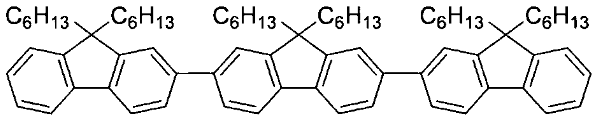

In addition, the compounds described in WO2015/022974, paragraphs 0220 to 0239 are also especially favorably employable as a light-emitting material for use along with the assist dopant having a structure represented by the general formula (1).

Compounds represented by the following general formula (2) are further preferred light-emitting materials.

General Formula (2)

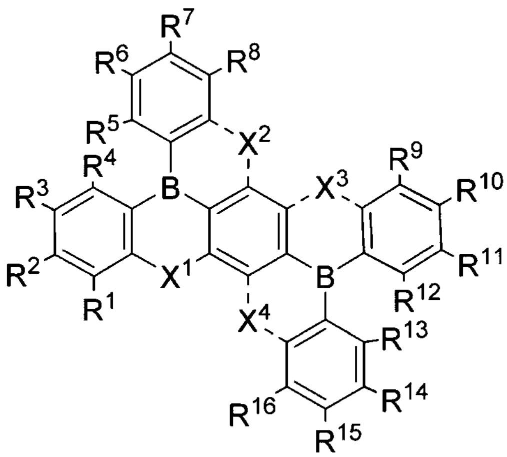

In the general formula (2), $R^1$ and $R^3$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group. $R^3$ and $R^4$, $R^4$ and $R^5$, $R^5$ and $R^6$, $R^6$ and $R^7$, $R^7$ and $R^8$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ represents O or NR, and the remainder can be O or NR, or unlinked. When not linked, both ends each independently represent a hydrogen atom, a deuterium atom or a substituent. In the general formula (2), C-$R^1$, C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, when $X^2$ represents O or NR, $R^7$ represents an acceptor group, $R^6$ and $R^7$ bond to each other to form an acceptor group, or $R^7$ and $R^8$ bond to each other to form an acceptor group. In one aspect of the present invention, when $X^3$ represents O or NR, $R^{10}$ represents an acceptor group, $R^9$ and $R^{10}$ bond to each other to form an acceptor group, or $R^{10}$ and $R^{11}$ bond to each other to form an acceptor group. In one aspect of the present invention, when $X^4$ represents O or NR, $R^{15}$ represents an acceptor group, $R^{14}$ and $R^{15}$ bond to each other to form an acceptor group, or $R^{15}$ and $R^{16}$ bond to each other to form an acceptor group. In one aspect of the present invention, when $X^2$ represents NR and when R represents a substituted or unsubstituted phenyl group and forms a carbazole ring by directly bonding to the carbon atom to which $R^8$ bonds, at least one of the 3-position and the 6-position of the carbazole ring is substituted with an acceptor group. In one aspect of the present invention, when $X^3$ represents NR and when R represents a substituted or unsubstituted phenyl group and forms a carbazole ring by directly bonding to the carbon atom to which $R^9$ bonds, at least one of the 3-position and the 6-position of the carbazole ring is substituted with an acceptor

group. In one aspect of the present invention, when $X^4$ represents NR and when R represents a substituted or unsubstituted phenyl group and forms a carbazole ring by directly bonding to the carbon atom to which $R^{16}$ bonds, at least one of the 3-position and the 6-position of the carbazole ring is substituted with an acceptor group. In one aspect of the present invention, when $X^1$ represents NR and when R represents a substituted or unsubstituted phenyl group and forms a carbazole ring by directly bonding to the carbon atom to which $R^1$ bonds, the 3-position of the carbazole ring is substituted with an acceptor group (here, the 3-position is on the phenyl group). One aspect of the present invention is a compound represented by the following general formula (2a).

General Formula (2a)

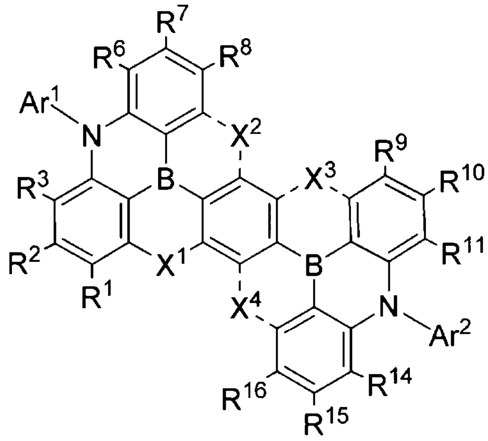

In the general formula (2a), $R^1$, $R^3$, $R^6$ to $R^{11}$, and $R^{14}$ to $R^{16}$ each independently represent a hydrogen atom, a deuterium atom, or a substituent. $R^2$ represents an acceptor group, or $R^1$ and $R^2$ bond to each other to form an acceptor group, or $R^2$ and $R^3$ bond to each other to form an acceptor group.

$R^6$ and $R^7$, $R^7$ and $R^8$, $R^9$ and $R^{10}$, $R^{10}$ and $R^{11}$, $R^{14}$ and $R^{15}$, and $R^{15}$ and $R^{16}$ each can bond to each other to form a cyclic structure. $X^1$ represents O or NR, and R represents a substituent. Of $X^2$ to $X^4$, at least one of $X^3$ and $X^4$ represents O or NR, and the remainder can be O or NR, or unlinked. When not linked, both ends each independently represent a hydrogen atom, a deuterium atom or a substituent. $Ar^1$ and $Ar^2$ each independently represent a substituted or unsubstituted aryl group, or a substituted or unsubstituted heteroaryl group. In the general formula (2a), C-$R^1$, C-$R^3$, C-$R^6$, C-$R^7$, C-$R^8$, C-$R^9$, C-$R^{10}$, C-$R^{11}$, C-$R^{14}$, C-$R^{15}$, and C-$R^{16}$ can be substituted with N.

Compounds represented by the following general formula (3) are further preferred light-emitting materials.

General Formula (3)

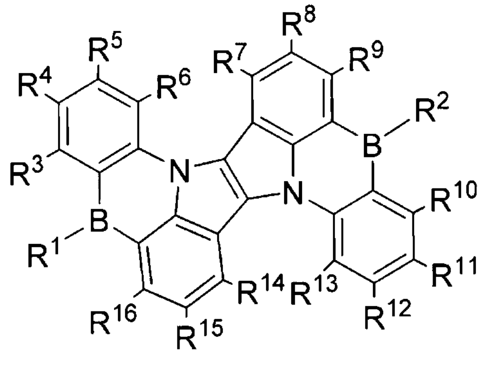

In the general formula (3), $R^1$ and $R^2$ each independently represent a substituted or unsubstituted alkyl group, a substituted or unsubstituted aryl group, or a substituted or unsubstituted heteroaryl group, and $R^3$ to $R^{16}$ each independently represent a hydrogen atom, a deuterium atom or a substituent. $R^1$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^2$, $R^2$ and $R^{10}$, $R^{10}$ and $R^{11}$, $R^{11}$ and $R^{12}$, $R^{12}$ and $R^{13}$, $R^{13}$ and $R^{14}$, $R^{14}$ and $R^{15}$, $R^{15}$ and $R^{16}$, and $R^{16}$ and $R^1$ each can bond to each other to form a cyclic structure. In the general formula (3), C-$R^3$, C-$R^4$, C-$R^5$, C-$R^6$, C-$R^7$, C-$R^8$, C-$R^9$, C-$R^{10}$, C-$R^{11}$, C-$R^{12}$, C-$R^{13}$, C-$R^{14}$, C-$R^{15}$, and C-$R^{16}$ can be substituted with N.

In one aspect of the present invention, $R^1$ and $R^2$ each independently represent a substituted or unsubstituted phenyl group optionally fused with any other ring. In one aspect of the present invention, $R^3$ and $R^{10}$ each independently represent a substituted amino group. In one aspect of the present invention, at least one pair of $R^1$ and $R^3$, and $R^2$ and $R^{10}$

bonds to each other to form a cyclic structure. In one aspect of the present invention, the cyclic structure includes a benzazaborine ring.

Compounds represented by the following general formula (4) are further preferred light-emitting materials.

General Formula (4)

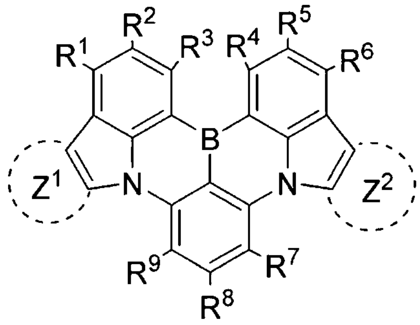

In the general formula (4), $Z^1$ and $Z^2$ each independently represent a substituted or unsubstituted aromatic ring, or a substituted or unsubstituted heteroaromatic ring, $R^1$ to $R^9$ each independently represent a hydrogen atom, a deuterium atom or a substituent. $R^1$ and $R^2$, $R^2$ and $R^3$, $R^3$ and $R^4$, $R^4$ and $R^5$, $R^5$ and $R^6$, $R^7$ and $R^8$, and $R^8$ and $R^9$ each can bond to each other to form a cyclic structure. At least one of the ring formed by $Z^1$, $Z^2$, $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring of a substituted or unsubstituted benzofuran, a thiophene ring of a substituted or unsubstituted benzothiophene, or a pyrrole ring of a substituted or unsubstituted indole, and at least one of $R^1$ to $R^9$ is a substituted or unsubstituted aryl group or an acceptor group, or at least one of $Z^1$ and $Z^2$ is a ring having an aryl group or an acceptor group as a substituent. Of the benzene ring skeleton-constituting carbon atoms to constitute the benzofuran ring, the benzothiophene ring, and the indole ring, a substitutable carbon atom can be substituted with a nitrogen atom. In the general formula (4), C-$R^1$, C-$R^2$, C-$R^3$, C-$R^4$, C-$R^5$, C-$R^6$, C-$R^7$, C-$R^8$, and C-$R^9$ can be substituted with N.

In one aspect of the present invention, $Z^1$ and $Z^2$ each independently represent a substituted or unsubstituted non-fused benzene ring, a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^1$ to $R^9$ each independently represent a substituted or unsubstituted aryl group or an acceptor group, or at least one ring selected from the group consisting of the ring formed by $R^1$ and $R^2$ bonding to each other, the ring formed by $R^2$ and $R^3$ bonding to each other, the ring formed by $R^4$ and $R^5$ bonding to each other, and the ring formed by $R^5$ and $R^6$ bonding to each other is a furan ring fused with a substituted or unsubstituted benzene ring, a thiophene ring fused with a substituted or unsubstituted benzene ring, or a pyrrole ring fused with a substituted or unsubstituted benzene ring. In one aspect of the present invention, $R^8$ represents a substituted or unsubstituted aryl group, or an acceptor group. One aspect of the present invention contains two or more rings selected from the group consisting of the benzofuran ring, the benzothiophene ring, and the indole ring.

Further more preferred light-emitting materials include compounds having a ring-fused structure A, in which the carbon-carbon bond a in the following structure $\alpha$ is fused with a furan ring constituting a substituted or unsubstituted benzofuran ring, a thiophene ring constituting a substituted or unsubstituted benzothiophene ring, or a pyrrole ring constituting a substituted or unsubstituted indole ring, or the carbon-carbon bond b is fused with a benzene ring constituting a substituted or unsubstituted dibenzofuran ring, a benzene ring constituting a substituted or unsubstituted dibenzothiophene ring, a benzene ring constituting a substituted or unsubstituted carbazole ring, or a benzene ring constituting a substituted or unsubstituted dibenzodioxane ring (the hydrogen atom in the structure can be substituted with a deuterium atom or a substituent).

Structure α

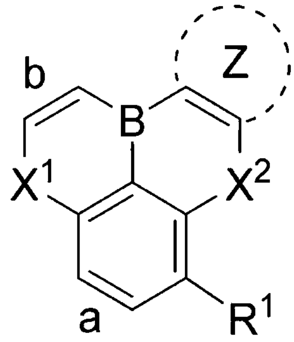

In the structure α, X1 and X2 each independently represent a nitrogen atom to which a substituted or unsubstituted aryl group bonds or an oxygen atom to which a substituted or unsubstituted aryl group bonds, Z represents a substituted or unsubstituted aromatic ring, or a substituted or unsubstituted heteroaromatic ring, R1 represents a hydrogen atom, a deuterium atom or a substituent, and Z and X2 can bond to each other to form a cyclic structure.

In the ring-fused structure A, the structure fused to b and $X^1$, the structure fused to b and Z, and Z and $X^2$ each can bond to each other to form a cyclic structure.

Compounds represented by the following general formula (5) are further preferred light-emitting materials.

General Formula (5)

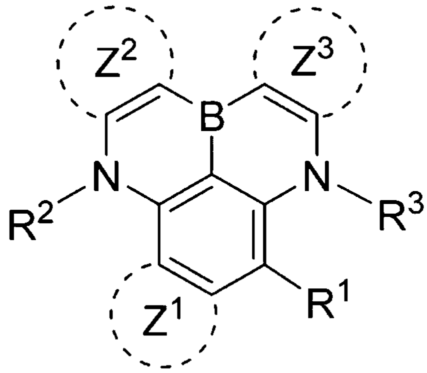

In the general formula (5), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ and $Z^3$ each independently represent a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^2$, $Z^2$ and $Z^3$, and $Z^3$ and $R^3$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (6) are further preferred light-emitting materials.

General Formula (6)

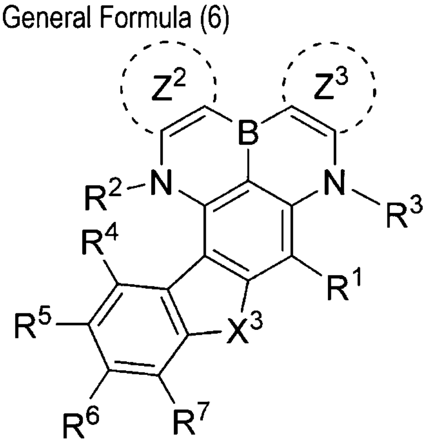

In the general formula (6), $X^3$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^4$ to $R^7$ each represent a hydrogen atom, a deuterium atom or a substituent, $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^4$ and $R^5$, $R^5$ and $R^6$, and $R^6$ and $R^7$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (7) are further preferred light-emitting materials.

General Formula (7)

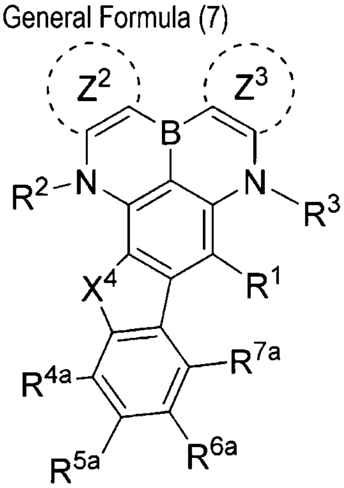

In the general formula (7), $X^4$ represents an oxygen atom or a sulfur atom, $Z^2$ and $Z^3$ each independently represent a substituted or unsubstituted aromatic ring, or a substituted or unsubstituted heteroaromatic ring, $R^1$ and $R^{4a}$ to $R^{7a}$ each represent a hydrogen atom, a deuterium atom or a substituent, and $R^2$ and $R^3$ each independently represent a substituted or unsubstituted aryl group, or a substituted or unsubstituted heteroaryl group. $R^2$ and $Z^2$, $Z^2$ and $Z^3$, $Z^3$ and $R^3$, $R^{4a}$ and $R^{5a}$, $R^{5a}$ and $R^{6a}$, $R^{6a}$ and $R^{7a}$, and $R^{7a}$ and $R^1$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (8) are further preferred light-emitting materials.

General Formula (8)

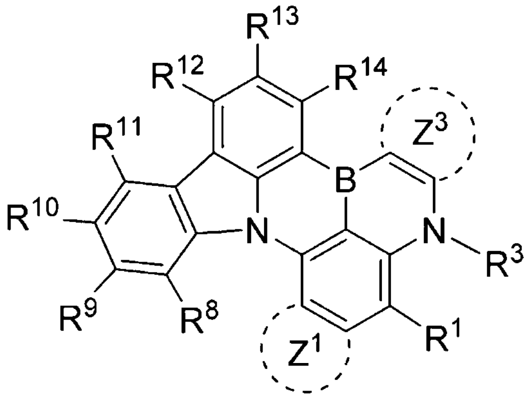

In the general formula (8), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^8$ to $R^{14}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $R^8$ and $R^9$, $R^9$ and $R^{10}$, $R^{10}$ and $R^{11}$, $R^{11}$ and $R^{12}$, $R^{12}$ and $R^{13}$, $R^{13}$ and $R^{14}$, $R^{14}$ and $Z^3$, and $Z^3$ and $R^3$ each can bond to each other to form a cyclic structure.

Compounds represented by the following general formula (9) are further preferred light-emitting materials.

General Formula (9)

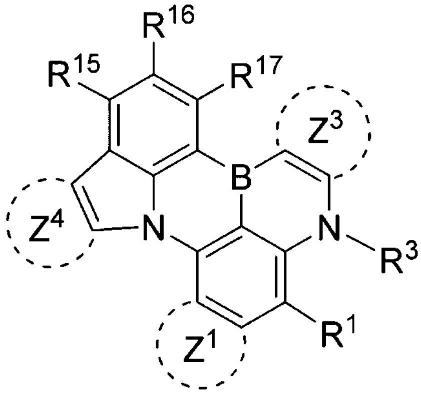

In the general formula (9), $Z^1$ and $Z^4$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ and $R^{15}$ to $R^{17}$ each independently represent a hydrogen atom, a deuterium atom, or a substituent, and $R^3$ represents a substituted or unsubstituted aryl group, or a substituted or unsubstituted heteroaryl group. $Z^1$ and $R^1$, $Z^4$ and $R^{15}$, $R^{15}$ and $R^{16}$, $R^{16}$ and $R^{17}$, $R^{17}$ and $Z^3$, and $Z^3$ and $R^3$ each can bond to each other to form a cyclic structure.

Compounds represented by the following general formula (10) are further preferred light-emitting materials.

General Formula (10)

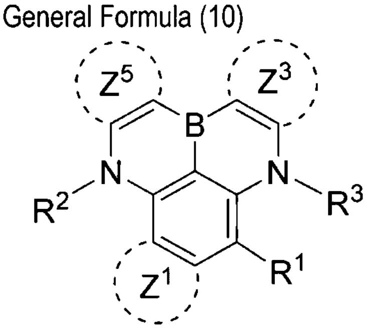

In the general formula (10), $Z^1$ and $Z^5$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^3$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $Z^1$ and $R^1$, $R^2$ and $Z^5$, $Z^5$ and $Z^3$, $Z^3$ and $R^3$ each can bond to each other to form a cyclic structure. At least one pair of $R^2$ and $Z^2$, $Z^2$ and $Z^3$, and $Z^3$ and $R^3$ bonds to each other to form a cyclic structure.

Compounds represented by the following general formula (11) are further preferred light-emitting materials.

General Formula (11)

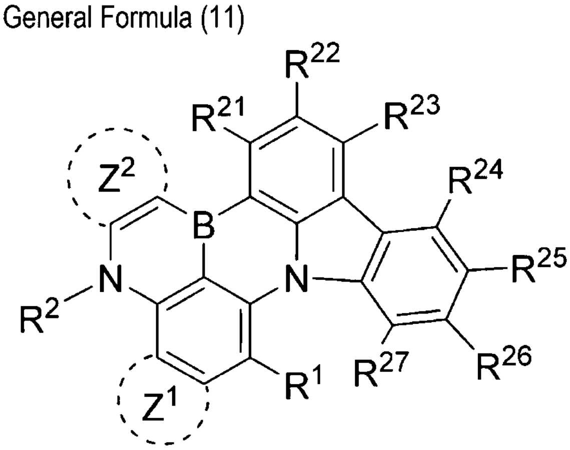

In the general formula (11), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{21}$ to $R^{27}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{21}$, $R^{21}$ and $R^{22}$, $R^{22}$ and $R^{23}$, $R^{23}$ and $R^{24}$, $R^{24}$ and $R^{25}$, $R^{25}$ and $R^{26}$, and $R^{26}$ and $R^{27}$ each can bond to each other to form a cyclic structure.

Compounds represented by the following general formula (12) are further preferred light-emitting materials.

General Formula (12)

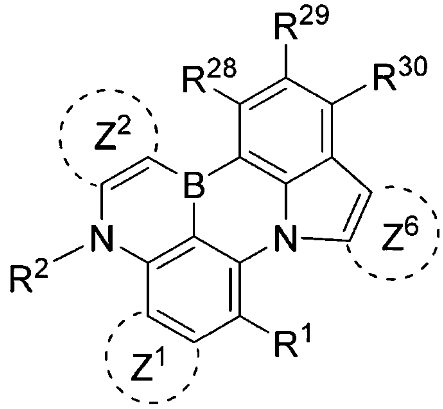

In the general formula (12), $Z^1$ and $Z^6$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ and $R^{28}$ to $R^{30}$ each independently represent a hydrogen atom, a deuterium atom, or a substituent, and $R^2$ represents a substituted or unsubstituted aryl group, or a substituted or unsubstituted heteroaryl group. $R^1$ and $Z^1$, $R^2$ and $Z^2$, $Z^2$ and $R^{28}$, $R^{28}$ and $R^{29}$, $R^{29}$ and $R^{30}$, and $R^{30}$ and $Z^6$ each can bond to each other to form a cyclic structure.

Compounds represented by the following general formula (13) are further preferred light-emitting materials.

General Formula (13)

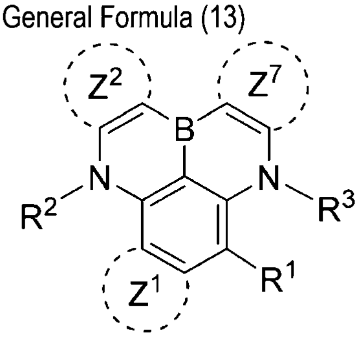

In the general formula (13), $Z^1$ and $Z^7$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $Z^2$ represents a substituted or unsubstituted aromatic ring, or a substituted or unsubstituted heteroaromatic ring, $R^1$ represents a hydrogen atom, a deuterium atom, or a substituent, and $R^2$ and $R^3$ each independently represent a substituted or unsubstituted aryl group, or a substituted or unsubstituted heteroaryl group. $R^1$ and $Z^1$, $R^2$ and $Z^2$, $Z^2$ and $Z^7$, and $Z^7$ and $R^3$ each can bond to each other to form a cyclic structure. At least one pair of $R^2$ and $Z^2$, $Z^2$ and $Z^7$, and $Z^7$ and $R^3$ bonds to each other to form a cyclic structure.

Compounds represented by the following general formula (14) are further preferred light-emitting materials.

General Formula (14)

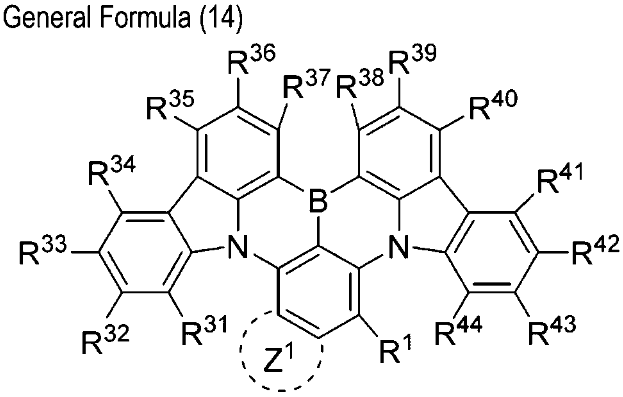

In the general formula (14), $Z^1$ represents a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{31}$ to $R^{44}$ each independently represent a substituted or unsubstituted aryl group, or a substituted or unsubstituted heteroaryl group. $R^1$ and $Z^1$, $R^{31}$ and $R^{32}$ , $R^{32}$ and $R^{33}$, $R^{33}$ and $R^{34}$, $R^{34}$ and $R^{35}$, $R^{35}$ and $R^{36}$, $R^{36}$ and $R^{37}$, $R^{37}$ and $R^{38}$, $R^{38}$ and $R^{39}$, $R^{39}$ and $R^{40}$, $R^{40}$ and $R^{41}$, $R^{41}$ and $R^{42}$, $R^{42}$ and $R^{43}$, and $R^{43}$ and $R^{44}$ each can bond to each other to form a cyclic structure.

Compounds represented by the following general formula (15) are further preferred light-emitting materials.

General Formula (15)

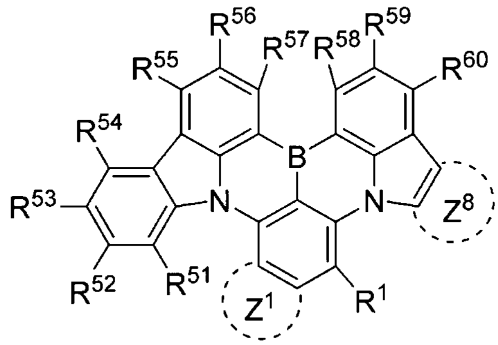

In the general formula (15), $Z^1$ and $Z^8$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{51}$ to $R^{60}$ each independently represent a hydrogen atom, a deuterium atom, or a substituent. $R^1$ and $Z^1$, $R^{51}$ and $R^{52}$, $R^{52}$ and $R^{33}$, $R^{53}$ and $R^{34}$, $R^{54}$ and $R^{55}$, $R^{55}$ and $R^{56}$, $R^{56}$ and $R^{57}$, $R^{57}$ and $R^{58}$, $R^{58}$ and $R^{59}$, $R^{59}$ and $R^{60}$, and $R^{60}$ and $Z^8$ each can bond to each other to form a cyclic structure.

Compounds represented by the following general formula (16) are further preferred light-emitting materials.

General Formula (16)

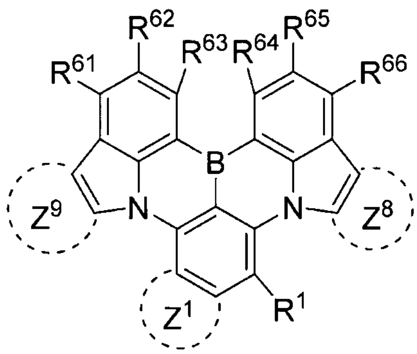

In the general formula (16), $Z^1$, $Z^8$ and $Z^9$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, and $R^1$ and $R^{61}$ to $R^{66}$ each independently represent a hydrogen atom, a deuterium atom, or a substituent. $R^1$ and $Z^1$, $Z^9$ and $R^{61}$, $R^{61}$ and $R^{62}$, $R^{62}$ and $R^{63}$, $R^{63}$ and $R^{64}$, $R^{64}$ and $R^{65}$, $R^{65}$ and $R^{66}$, and $R^{66}$ and $Z^8$ each can bond to each other to form a cyclic structure.

Compounds represented by the following general formula (17) are further preferred light-emitting materials.

General Formula (17)

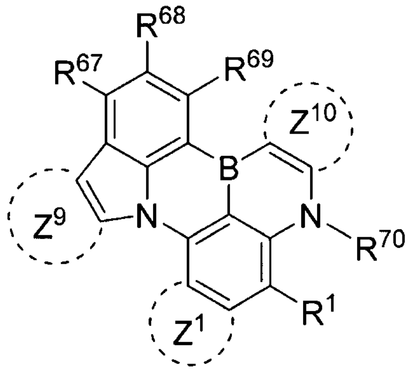

In the general formula (17), $Z^1$, $Z^9$ and $Z^{10}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{67}$ to $R^{69}$ each independently represent a hydrogen atom, a deuterium atom, or a substituent, and $R^{70}$ represents a substituted or unsubstituted aryl group, or a substituted or unsubstituted heteroaryl group. $R^1$ and $Z^1$, $Z^9$ and $R^{67}$, $R^{67}$ and $R^{68}$, $R^{68}$ and $R^{69}$, $R^{69}$ and $Z^{10}$, and $Z^{10}$ and $R^{70}$ each can bond to each other to form a cyclic structure.

Compounds represented by the following general formula (18) are further preferred light-emitting materials.

General Formula (18)

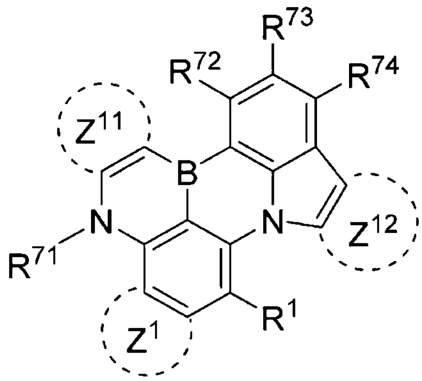

In the general formula (18), $Z^1$, $Z^{11}$ and $Z^{12}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{72}$ to $R^{74}$ each independently represent a hydrogen atom, a deuterium atom, or a substituent, and $R^{71}$ represents a substituted or unsubstituted aryl group, or a substituted or unsubstituted heteroaryl group. $R^1$ and $Z^1$, $R^{71}$ and $Z^{11}$, $Z^{11}$ and $R^{72}$, $R^{72}$ and $R^{73}$, $R^{73}$ and $Z^{74}$, and $R^{74}$ and $Z^{12}$ each can bond to each other to form a cyclic structure.

Compounds represented by the following general formula (19) are further preferred light-emitting materials.

General Formula (19)

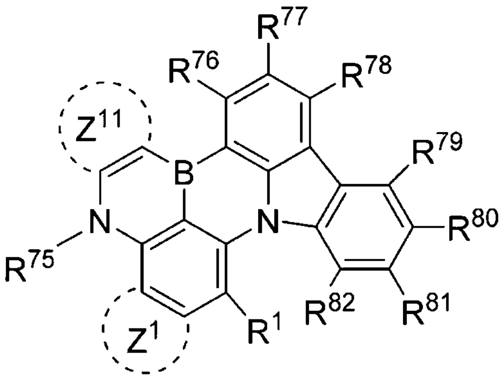

In the general formula (19), $Z^1$ and $Z^{11}$ each independently represent a furan ring fused with a substituted or unsubstituted benzene ring, a thiophene ring fused with a substituted or unsubstituted benzene ring, or an N-substituted pyrrole ring fused with a substituted or unsubstituted benzene ring, $R^1$ and $R^{76}$ to $R^{82}$ each independently represent a substituted or unsubstituted aryl group, or a substituted or unsubstituted heteroaryl group, and $R^{75}$ represents a substituted or unsubstituted aryl group, or a substituted or unsubstituted heteroaryl group. $R^1$ and $Z^1$, $R^{75}$ and $Z^{11}$, $Z^{11}$ and $R^{76}$, $R^{76}$ and $R^{77}$, $R^{77}$ and $R^{78}$, $R^{78}$ and $R^{71}$, $R^{79}$ and $R^{80}$, $R^{80}$ and $R^{81}$, and $R^{81}$ and $R^{82}$ each can bond to each other to form a cyclic structure.

Compounds represented by the following general formula (20) are further preferred light-emitting materials.

General Formula (20)

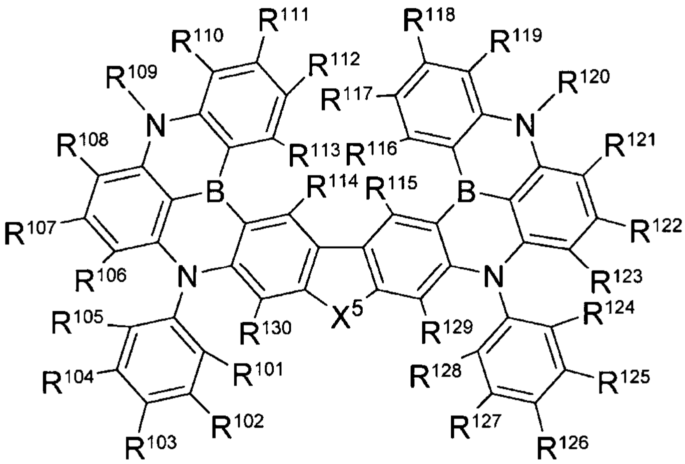

In the general formula (20), $X^5$ represents an oxygen atom, a sulfur atom, or a nitrogen atom to which a substituted or unsubstituted aryl group or a substituted or unsubstituted heteroaryl group bonds, $R^{101}$ to $R^{130}$ each independently represent a hydrogen atom, a deuterium atom or a substituent, and $R^{101}$ and $R^{102}$, $R^{102}$ and $R^{103}$, $R^{103}$ and $R^{104}$, $R^{104}$ and $R^{105}$, $R^{105}$ and $R^{106}$, $R^{106}$ and $R^{107}$, $R^{107}$ and $R^{108}$, $R^{108}$ and $R^{109}$, $R^{109}$ and $R^{110}$, $R^{110}$ and $R^{111}$, $R^{111}$ and $R^{112}$, $R^{112}$ and $R^{113}$, $R^{113}$ and $R^{114}$, $R^{114}$ and $R^{115}$, $R^{115}$ and $R^{116}$, $R^{116}$ and $R^{117}$, $R^{117}$ and $R^{118}$, $R^{118}$ and $R^{119}$, $R^{119}$ and $R^{120}$, $R^{120}$ and $R^{121}$, $R^{121}$ and $R^{122}$, $R^{122}$ and $R^{123}$, $R^{123}$ and $R^{124}$, $R^{124}$ and $R^{125}$, $R^{125}$ and $R^{126}$, $R^{126}$ and $R^{127}$, $R^{127}$ and $R^{128}$, $R^{128}$ and $R^{129}$, $R^{129}$ and $R^{130}$, and $R^{130}$ and $R^{101}$ each can bond to each other to form a cyclic structure.

Compounds represented by the following general formula (21) are further preferred light-emitting materials.

General Formula (21)

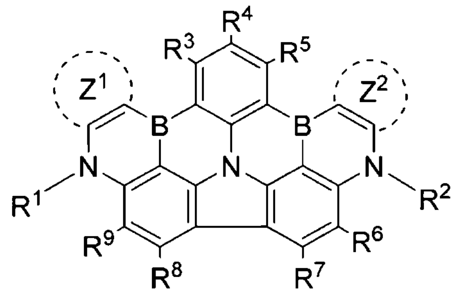

In the general formula (21), $R^1$ and $R^2$ each independently represent a substituted or unsubstituted alkyl group, a substituted or unsubstituted aryl group, or a substituted or unsubstituted heteroaryl group, $Z^1$ and $Z^2$ each independently represent a substituted or unsubstituted aromatic ring, or a substituted or unsubstituted heteroaromatic ring, and $R^3$ to $R^9$ each independently represent a hydrogen atom, a deuterium atom or a substituent. At least one of $R^1$, $R^2$, $Z^1$ and $Z^2$ includes a substituted or unsubstituted benzofuran ring, a substituted or unsubstituted benzothiophene ring, or a substituted or unsubstituted indole ring. $R^1$ and $Z^1$, $Z^1$ and $R^3$, $R^3$ and $R^4$, $R^4$ and $R^5$, $R^5$ and $Z^2$, $Z^2$ and $R^2$, $R^2$ and $R^6$, $R^6$ and $R^7$, $R^7$ and $R^8$, $R^8$ and $R^9$, and $R^9$ and $R^1$ each can bond to each other to form a cyclic structure. Of the benzene ring skeleton-constituting carbon atoms to constitute the benzofuran ring, the benzothiophene ring, and the indole ring, a substitutable carbon atom can be substituted with a nitrogen atom. In the general formula (21), C-$R^3$, C-$R^4$, C-$R^5$, C-$R^6$, C-$R^7$, C-$R^8$, and C-$R^9$ can be substituted with N.

In one aspect of the present invention, $R^1$ and $R^2$ each independently represent a substituted or unsubstituted alkyl group, a substituted or unsubstituted phenyl group, or a group containing one or more ring structures selected from the group consisting of a substituted or unsubstituted benzofuran ring, a substituted or unsubstituted benzothiophene ring and a substituted or unsubstituted indole ring. In one aspect of the present invention, $Z^1$ and $Z^2$ each independently represent a substituted or unsubstituted non-fused benzene ring, a furan ring fused with a substituted or unsubstituted benzene ring, a thiophene ring fused with a substituted or unsubstituted benzene ring, a pyrrole ring fused with a substituted or unsubstituted benzene ring, a benzene ring fused with a substituted or unsubstituted benzofuran ring, a benzene ring fused with a substituted or unsubstituted benzothiophene ring, or a benzene ring fused with a substituted or unsubstituted indole ring. In one aspect of the present invention, $R^1$ and $Z^1$ bond to each other to form a cyclic structure. In one aspect of the present invention, $R^1$ and $Z^1$ bond to each other to form a pyrrole ring.

Compounds represented by the following general formula (22) are further preferred light-emitting materials.

General Formula (22)

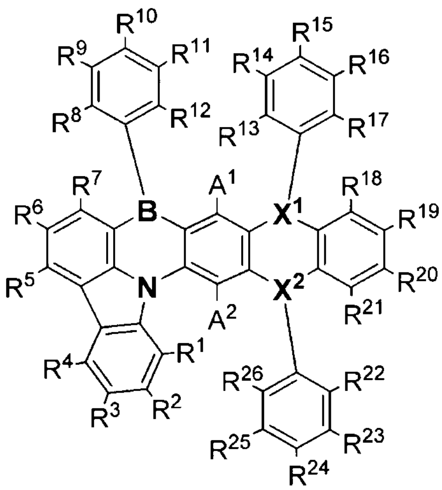

In the general formula (22), one of $X^1$ and $X^2$ represents a nitrogen atom, and the other represents a boron atom. $R^1$ to $R^{26}$, $A^1$ and $A^2$ each independently represent a hydrogen atom, a deuterium atom, or a substituent. $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ each can bond to each other to form a cyclic structure. When $X^1$ represents a

nitrogen atom, $R^{17}$ and $R^{18}$ bond to each other to be a single bond to form a pyrrole ring, and when $X^2$ represents a nitrogen atom, $R^{21}$ and $R^{22}$ bond to each other to be a single bond to form a pyrrole ring. In the case where $X^1$ represents a nitrogen atom, and where $R^7$ and $R^8$ and $R^{21}$ and $R^{22}$ each bond to each other via a nitrogen atom to form a 6-membered ring, and $R^{17}$ and $R^{18}$ bond to each other to form a single bond, at least one of $R^1$ to $R^6$ represents a substituted or unsubstituted aryl group, or any of $R^1$ and $R^2$, $R^2$ and $R^3$, $R^3$ and $R^4$, $R^4$ and $R^5$, and $R^5$ and $R^6$ bond to each other to form an aromatic ring or a heteroaromatic ring.

In one aspect of the present invention, at least one of $R^3$ and $R^6$ represents a substituent. In one aspect of the present invention, both $R^3$ and $R^6$ represent a substituent. In one aspect of the present invention, the substituent represented by $R^3$ and $R^6$ is one group selected from the group consisting of an alkyl group and an aryl group, or a group obtained by combining two or more of the groups. In one aspect of the present invention, both $R^8$ and $R^{12}$ represent a substituent. In one aspect of the present invention, the compounds are represented by the following general formula (1a).

General Formula (22a)

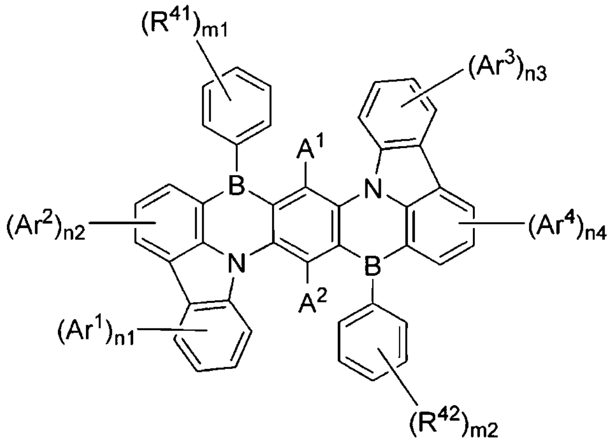

In the general formula (22a), $Ar^1$ to $Ar^4$ each independently represent a substituted or unsubstituted aryl group, or a substituted or unsubstituted heteroaryl group. $R^{41}$ and $R^{42}$ each independently represent a substituted or unsubstituted alkyl group. m1 and m2 each independently represent an integer of 0 to 5, n1 and n3 each independently represent an integer of 0 to 4, and n2 and n4 each independently represent an integer of 0 to 3. $A^1$ and $A^2$ each independently represent a hydrogen atom, a deuterium atom, or a substituent.

In one aspect of the present invention, $A^1$ and $A^2$ each independently represent a group having a Hammett' $\sigma p$ value of more than 0.2. In one aspect of the present invention, both $A^1$ and $A^2$ represent a cyano group. In one aspect of the present invention, both $A^1$ and $A^2$ represent a halogen atom. One aspect of the present invention has a rotationally symmetrical structure.

Preferred specific examples of compounds having the above-mentioned ring-fused structure A, and compounds represented by any of the general formulae (5) to (22) are shown below.

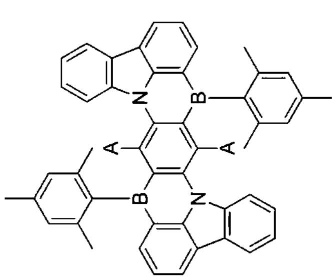

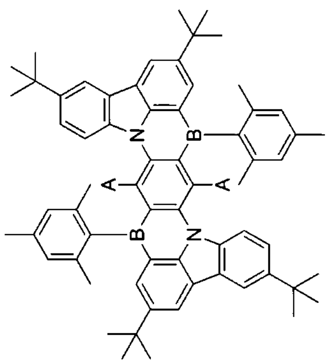

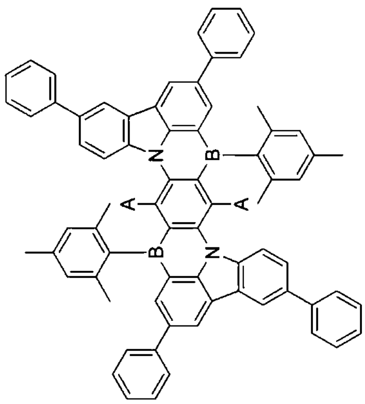

1a:A=A1
1b:A=A2
1c:A=A3
1d:A=A4
1e:A=A7
1f:A=A10
1g:A=A11
1h:A=A16
1i:A=A35
1j:A=A39
1k:A=A40
1l:A=A41
1m:A=A42

2a:A=A1
2b:A=A2
2c:A=A3
2d:A=A4
2e:A=A7
2f:A=A1
2g:A=A11
2h:A=A16
2i:A=A35
2j:A=A39
2k:A=A40
2l:A=A41
2m:A=A42

3a:A=A1
3b:A=A2
3c:A=A3
3d:A=A4
3e:A=A7
3f:A=A10
3g:A=A11
3h:A=A16
3i:A=35
3j:A=A39
3k:A=A40
3l:A=A41
3m:A=A42

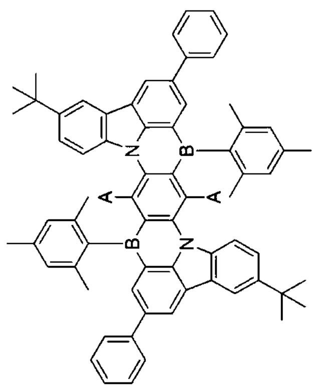

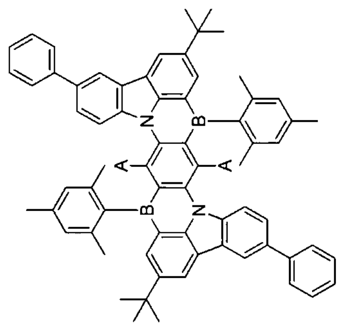

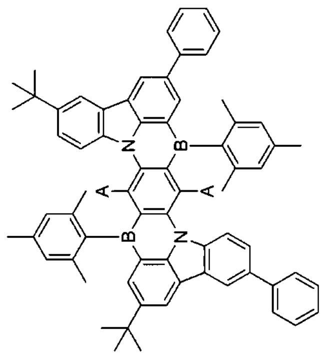

4a:A=A1

5a:A=A1

6a:A=A1

(continued)

| | | |
|---|---|---|
| 4b:A=A2<br>4c:A=A3<br>4d:A=A4<br>4e:A=A7<br>4f:A=A10<br>4g:A=A11<br>4h:A=A16<br>4i:A=A35<br>4j:A=A39<br>4k:A=A40<br>4l:A=A41<br>4m:A=A42 | 5b:A=A2<br>5c:A=A3<br>5d:A=A4<br>5e:A=A7<br>5f:A=A10<br>5g:A=A11<br>5h:A=A16<br>5i:A=A35<br>5j:A=A39<br>5k:A=A40<br>5l:A=A41<br>5m:A=A42 | 6b:A=A2<br>6c:A=A3<br>6d:A=A4<br>6e:A=A7<br>6f:A=A10<br>6g:A=A11<br>6h:A=A16<br>6i:A=A35<br>6j:A=A39<br>6k:A=A40<br>6l:A=A41<br>6m:A=A42 |
| 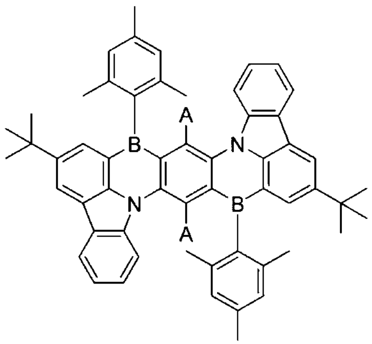 | 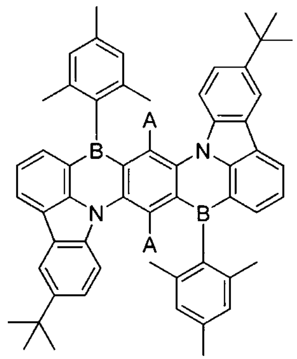 | 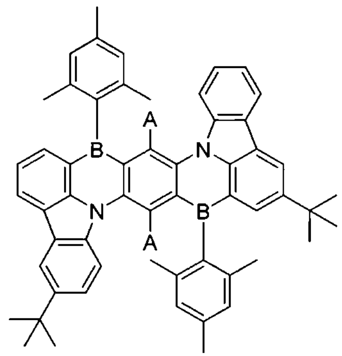 |
| 7a:A=A1<br>7b:A=A2<br>7c:A=A3<br>7d:A=A4<br>7e:A=A7<br>7f:A=A10<br>7g:A=A11<br>7h:A=A16<br>7i:A=A35<br>7j:A=A39<br>7k:A=A40<br>7l:A=A41 | 8a:A=A1<br>8b:A=A2<br>8c:A=A3<br>8d:A=A4<br>8e:A=A7<br>8f:A=A10<br>8g:11<br>8h:A=A16<br>8i:A=A35<br>8j:A=A39<br>8k:A=A40<br>8l:A=A41 | 9a:A=A1<br>9b:A=A2<br>9c:A=A3<br>9d:A=A4<br>9e:A=A7<br>9f:A=A10<br>9g:A=A11<br>9h:A=A16<br>9i:A=A35<br>9j:A=A39<br>9k:A=A40<br>9l:A=A41 |

(continued)

| | | |
|---|---|---|
| 7m:A=A42 | 8m:A=A42 | 9m:A=A42 |
| 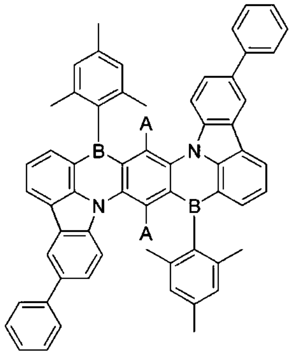 | 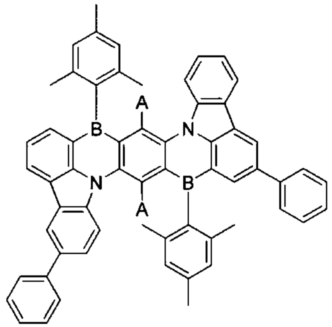 | 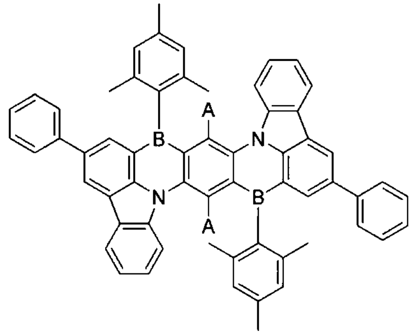 |
| 10a:A=A1<br>10b:A=A2<br>10c:A=A3<br>10d:A=A4<br>10e:A=A7<br>10f:A=A10<br>10g:A=A11<br>10h:A=A16<br>10i:A=A35<br>10j:A=A39<br>10k:A=A40<br>10l:A=A41<br>10m:A=A42 | 11a:A=A1<br>11b:A=A2<br>11c:A=A3<br>11d:A=A4<br>11e:A=A7<br>11f:A=A10<br>11g:A=A11<br>11h:A=A16<br>11i:A=A35<br>11j:A=A39<br>11k:A=A40<br>11l:A=A41<br>11m:A=A42 | 12a:A=A1<br>12b:A=A2<br>12c:A=A3<br>12d:A=A4<br>12e:A=A7<br>12f:A=A10<br>12g:A=A11<br>12h:A=A16<br>12i:A=A35<br>12j:A=A39<br>12k:A=A40<br>12l:A=A41<br>12m:A=A42 |

(continued)

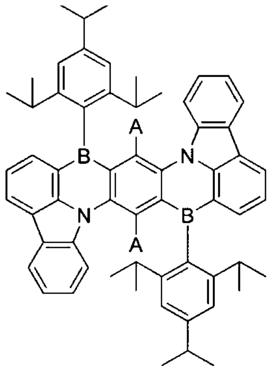

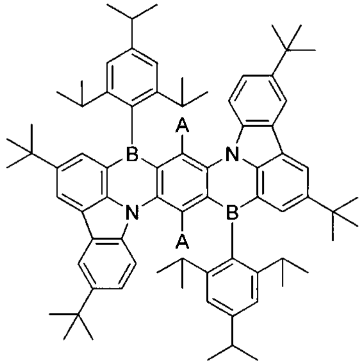

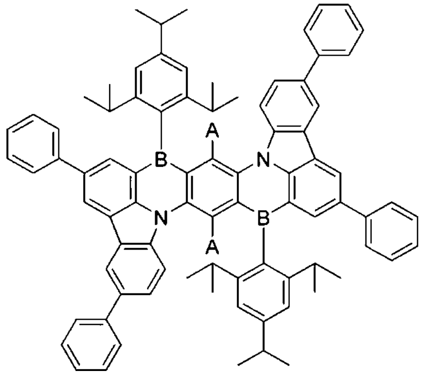

13a : A=A1
13b : A=A2
13c : A=A3
13d : A=A4
13e : A=A7
13f : A=A10
13g : A=A11
13h : A=A16
13i : A=A35
13j : A=A39
13k : A=A40
13l : A=A41
13m : A=A42

14a : A=A1
14b : A=A2
14c : A=A3
14d : A=A4
14e : A=A7
14f : A=A10
14g : A=A11
14h : A=A16
14i : A=A35
14j : A=A39
14k : A=A40
14l : A=A41
14m : A=A42

15a : A=A1
15b : A=A2
15c : A=A3
15d : A=A4
15e : A=A7
15f : A=A10
15g : A=A11
15h : A=A16
15i : A=A35
15j : A=A39
15k : A=A40
15l : A=A41
15m : A=A42

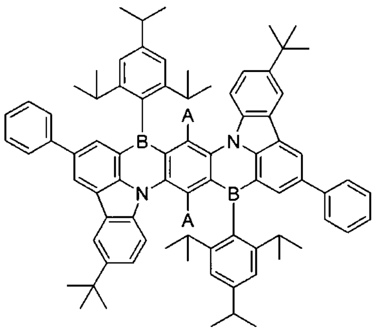

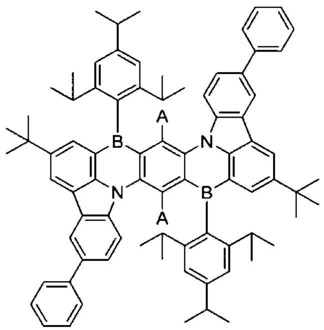

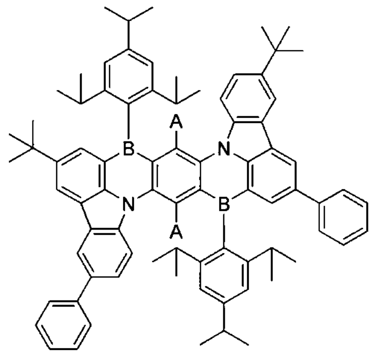

(continued)

16a : A=A1
16b : A=A2
16c : A=A3
16d : A=A4
16e : A=A7
16f : A=A10
16g : A=A11
16h : A=A16
16i : A=A35
16j : A=A39
16k : A=A40
16l : A=A41
16m : A=A42

17a : A=A1
17b : A=A2
17c : A=A3
17d : A=A4
17e : A=A7
17f : A=A10
17g : A=A11
17h : A=A16
17i : A=A35
17j : A=A39
17k : A=A40
17l : A=A41
17m : A=A42

18a : A=A1
18b : A=A2
18c : A=A3
18d : A=A4
18e : A=A7
18f : A=A10
18g : A=A11
18h : A=A16
18i : A=A35
18j : A=A39
18k : A=A40
18l : A=A41
18m : A=A42

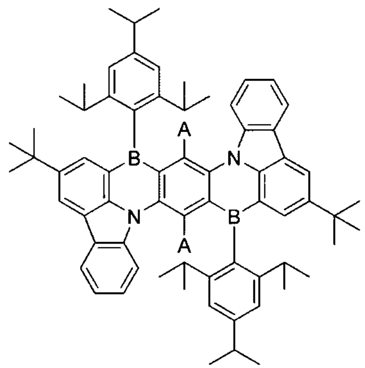

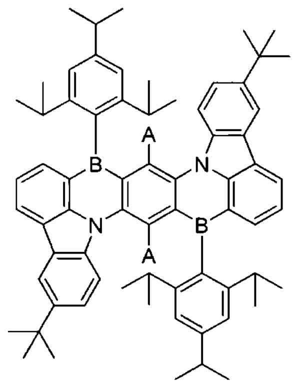

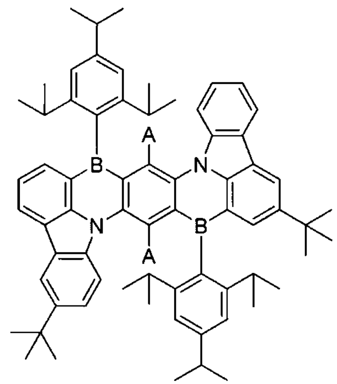

19a : A=A1
19b : A=A2
19c : A=A3
19d : A=A4
19e : A=A7
19f : A=A10
19g : A=A11
19h : A=A16
19i : A=A35
19j : A=A39

20a : A=A1
20b : A=A2
20c : A=A3
20d : A=A4
20e : A=A7
20f : A=A10
20g : A=A11
20h : A=A16
20i : A=A35
20j : A=A39

21a : A=A1
21b : A=A2
21c : A=A3
21d : A=A4
21e : A=A7
21f : A=A10
21g : A=A11
21h : A=A16
21i : A=A35
21j : A=A39

(continued)

19k : A=A40
19l : A=A41
19m : A=A42

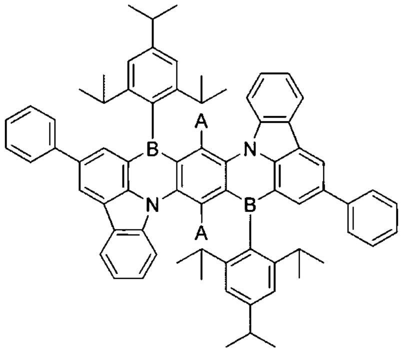

22a : A=A1
22b : A=A2
22c : A=A3
22d : A=A4
22e : A=A7
22f : A=A10
22g : A=A11
22h : A=A16
22i : A=A35
22j : A=A39
22k : A=A40
22l :A=A41
22m : A=A42

20k : A=A40
20l : A=A41
20m : A=A42

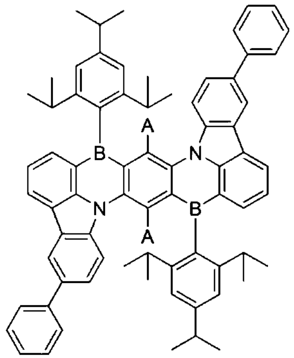

23a : A=A1
23b : A=A2
23c : A=A3
23d : A=A4
23e : A=A7
23f : A=A10
23g : A=A11
23h : A=A16
23i : A=A35
23j : A=A39
23k : A=A40
23l : A=A41
23m : A=A42

21k : A=A40
21l : A=A41
21m : A=A42

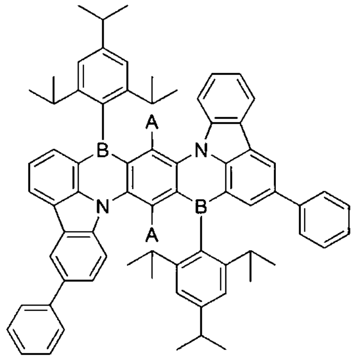

24a : A=A1
24b : A=A2
24c : A=A3
24d : A=A4
24e : A=A7
24f : A=A10
24g : A=A11
24h : A=A16
24i : A=A35
24j : A=A39
24k : A=A40
24l : A=A41
24m : A=A42

(continued)

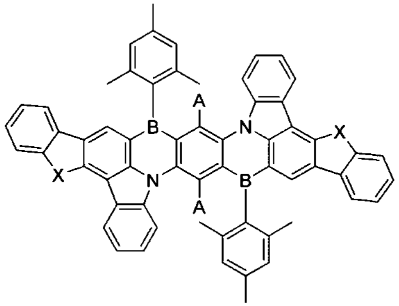

25a : A=A1
25b : A=A2
25c : A=A3
25d : A=A4
25e : A=A7
25f : A=A10
25g : A=A11
25h : A=A16
25i : A=A35
25j : A=A39
25k : A=A40
25l : A=A41
25m : A=A42

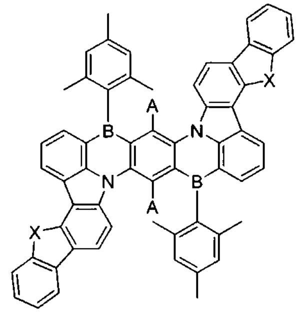

26a : A=A1
26b : A=A2
26c : A=A3
26d : A=A4
26e : A=A7
26f : A=A10
26g : A=A11
26h : A=A16
26i : A=A35
26j : A=A39
26k : A=A40
26l : A=A41
26m : A=A42

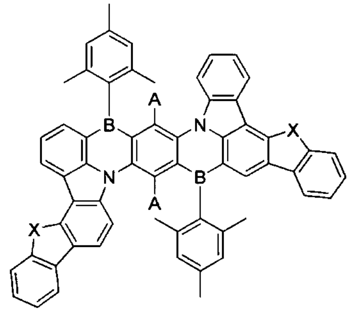

27a : A=A1
27b : A=A2
27c : A=A3
27d : A=A4
27e : A=A7
27f : A=A10
27g : A=A11
27h : A=A16
27i : A=A35
27j : A=A39
27k : A=A40
27l : A=A41
27m : A=A42

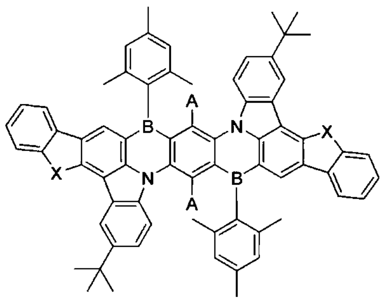

28a : A=A1
28b : A=A2

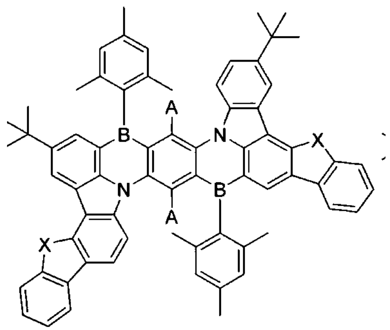

29a : A=A1
29b : A=A2

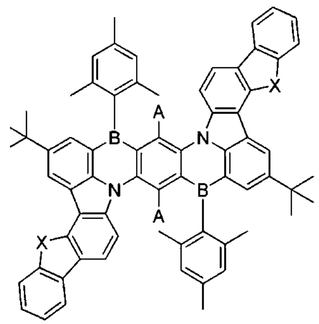

30a : A=A1
30b : A=A2

(continued)

28c : A=A3
28d : A=A4
28e : A=A7
28f : A=A10
28g : A=A11
28h : A=A16
28i : A=A35
28j : A=A39
28k : A=A40
28l : A=A41
28m : A=A42

29c : A=A3
29d : A=A4
29e : A=A7
29f : A=A10
29g : A=A11 29h : A=A16
29i : A=A35
29j : A=A39 29k : A=A40
29l : A=A41
29m : A=A42

30c : A=A3
30d : A=A4
30e : A=A7
30f : A=A10
30g : A=A11
30h : A=A16
30i : A=A35
30j : A=A39
30k : A=A40
30l : A=A41
30m : A=A42

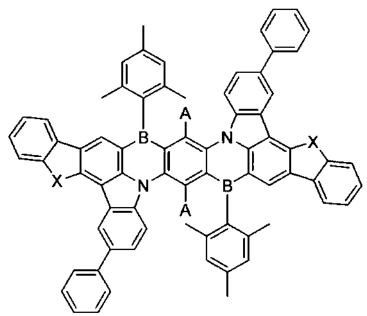

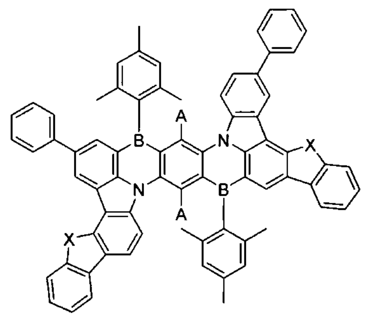

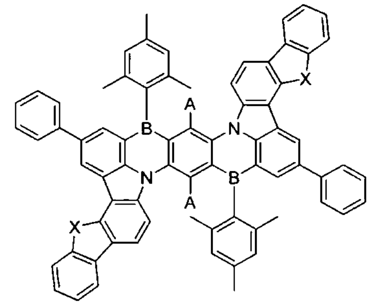

31a : A=A1
31b : A=A2
31c : A=A3
31d : A=A4
31e : A=A7
31f : A=A10
31g : A=A11
31h : A=A16
31i : A=A35
31j : A=A39
31k : A=A40
31l : A=A41
31m : A=A42

32a : A=A1
32b : A=A2
32c : A=A3
32d : A=A4
32e : A=A7
32f : A=A10
32g : A=A11
32h : A=A16
32i : A=A35
32j : A=A39
32k : A=A40
32l : A=A41
32m : A=A42

33a : A=A1
33b : A=A2
33c : A=A3
33d : A=A4
33e : A=A7
33f : A=A10
33g : A=A11
33h : A=A16
33i : A=A35
33j : A=A39
33k : A=A40
33l : A=A41
33m : A=A42

(continued)

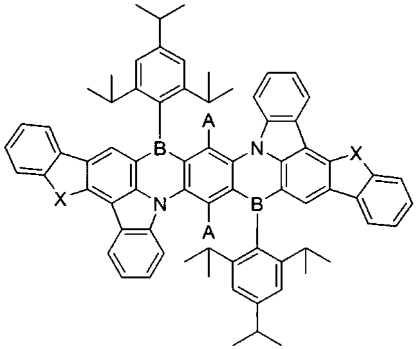

34a : A=A1
34b : A=A2
34c : A=A3
34d : A=A4
34e : A=A7
34f : A=A10
34g : A=A11
34h : A=A16
34i : A=A35
34j : A=A39
34k : A=A40
34l : A=A41
34m : A=A42

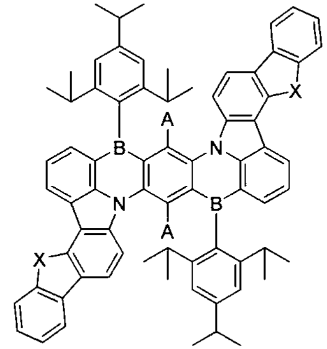

35a : A=A1
35b : A=A2
35c : A=A3
35d : A=A4
35e : A=A7
35f : A=A10
35g : A=A11
35h : A=A16
35i : A=A35
35j : A=A39
35k : A=A40
35l : A=A41
35m : A=A42

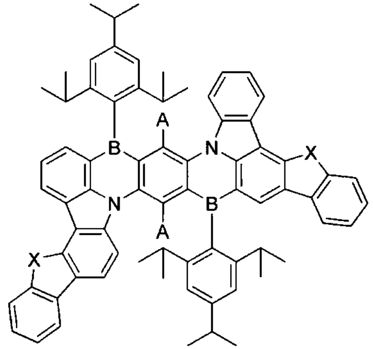

36a : A=A1
36b : A=A2
36c : A=A3
36d : A=A4
36e : A=A7
36f : A=A10
36g : A=A11
36h : A=A16
36i : A=A35
36j : A=A39
36k : A=A40
36l : A=A41
36m : A=A42

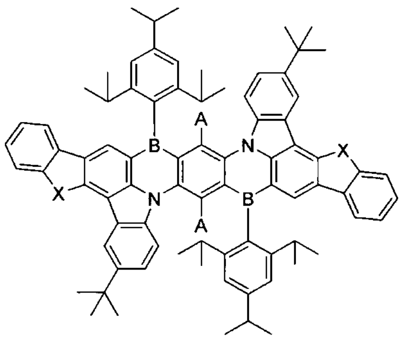

37a : A=A1

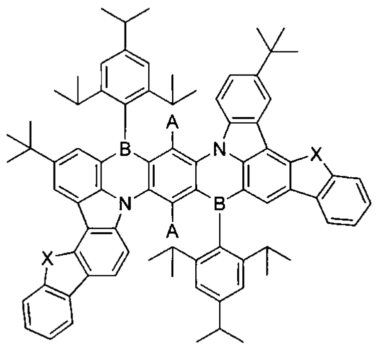

38a : A=A1

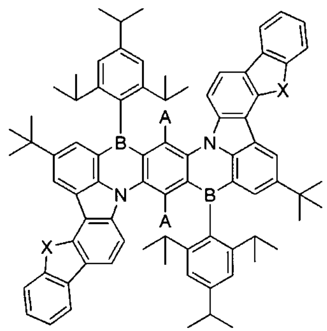

39a : A=A1

(continued)

| | | |
|---|---|---|
| 37b : A=A2<br>37c : A=A3<br>37d : A=A4<br>37e : A=A7<br>37f : A=A10<br>37g : A=A11<br>37h : A=A16<br>37i : A=35<br>37j : A=A39<br>37k : A=A40<br>37l : A=A41<br>37m : A=A42 | 38b:A=A2<br>38c : A=A3<br>38d : A=A4<br>38e : A=A7<br>38f : A=A10<br>38g : A=A11<br>38h : A=A16<br>38i : A=A35<br>38j : A=A39<br>38k : A=A40<br>38l : A=A41<br>38m : A=A42 | 39b : A=A2<br>39c : A=A3<br>39d : A=A4<br>39e : A=A7<br>39f : A=A10<br>39g : A=A11<br>39h : A=A16<br>39i : A=A35<br>39j : A=A39<br>39k : A=A40<br>39l : A=A41<br>39m : A=A42 |
| 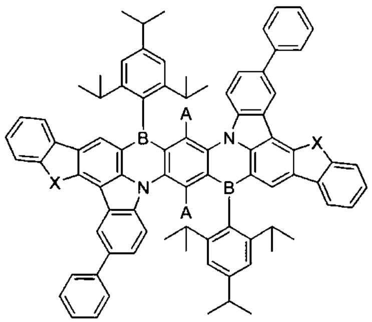 | 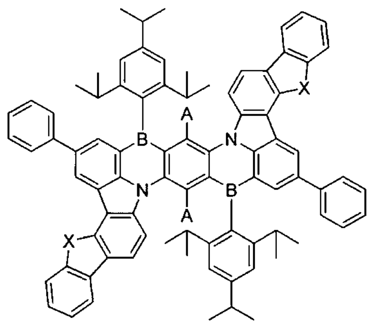 | 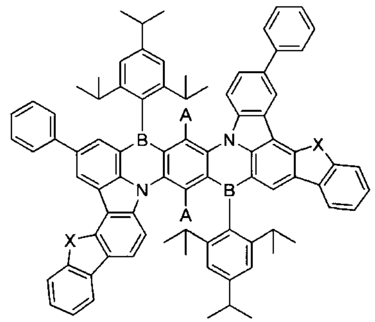 |
| 40a : A=A1<br>40b : A=A2<br>40c : A=A3<br>40d : A=A4<br>40e : A=A7<br>40f : A=A10<br>40g : A=A11<br>40h : A=A16<br>40i A=A35<br>40j : A=A39<br>40k : A=A40<br>40l : A=A41<br>40m : A=A42 | 41a : A=A1<br>41b : A=A2<br>41c : A=A3<br>41d : A=A4<br>41e : A=A7<br>41f : A=A10<br>41g :A=A11<br>41h :A=A16<br>41i : A=A35<br>41j : A=A39<br>41k : A=A40<br>41l : A=A41<br>41m : A=A42 | 42a : A=A1<br>42b : A=A2<br>42c : A=A3<br>42d : A=A4<br>42e : A=A7<br>42f : A=A10<br>42g : A=A11<br>42h : A=A16<br>42i : A=A35<br>42j : A=A39<br>42k : A=A40<br>42l : A=A41<br>42m : A=A42 |

(continued)

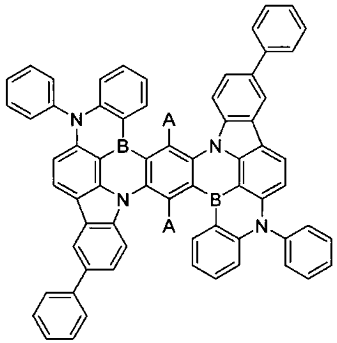

43a : A=A1
43b : A=A2
43c : A=A3
43d : A=A4
43e : A=A7
43f : A=A10
43g : A=A11
43h : A=A16
43i : A=A35
43j : A=A39
43k : A=A40
43l : A=A41
43m : A=A42

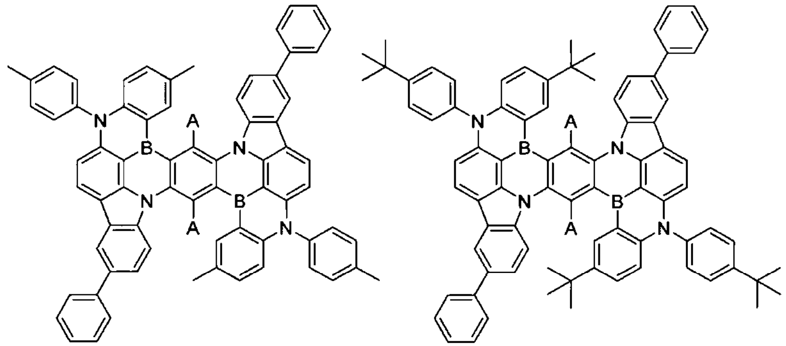

44a : A=A1
44b : A=A2
44c : A=A3
44d : A=A4
44e : A=A7
44f : A=A10
44g : A=A11
44h : A=A16
44i : A=A35
44j : A=A39
44k : A=A40
44l : A=A41
44m : A=A42

45a : A=A1
45b : A=A2
45c : A=A3
45d : A=A4
45e : A=A7
45f : A=A10
45g : A=A11
45h : A=A16
45i : A=A35
45j : A=A39
45k : A=A40
45l : A=A41
45m : A=A42

(continued)

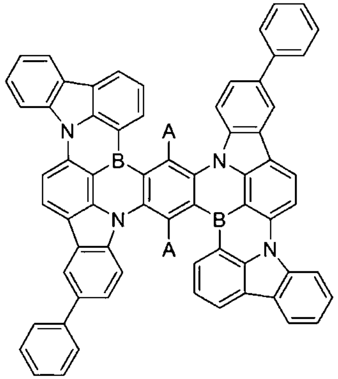

46a : A=A1
46b : A=A2
46c : A=A3
46d : A=A4
46e : A=A7
46f : A=A10
46g:A=A11
46h:A=A16
46i : A=A35
46 j : A=A39
46k : A=A40
46l : A=A41
46m : A=A42

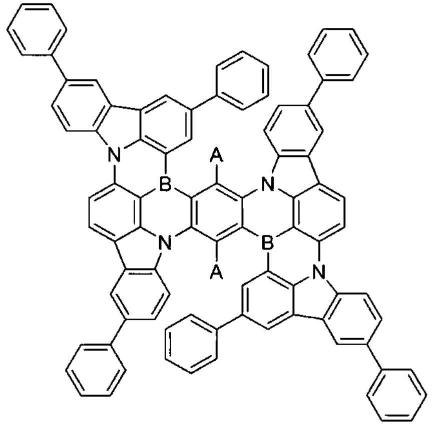

47a : A=A1
47b : A=A2
47c : A=A3
47d : A=A4
47e : A=A7
47f : A=A10
47g : A=A1 1
47h : A=A16
47i : A=A35
47j : A=A39
47k : A=A40
47l : A=A41
47m : A=A42

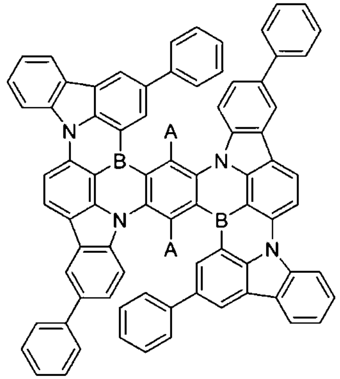

48a : A=A1
48b : A=A2
48c : A=A3
48d : A=A4
48e : A=A7
48f : A=A10
48g : A=A11
48h : A=A16
48i : A=A35
48j : A=A39
48k : A=A40
48l : A=A41
48m : A=A42

(continued)

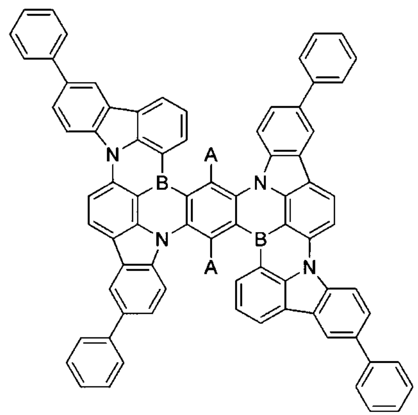

49a : A=A1
49b : A=A2
49c : A=A3
49d : A=A4
49e : A=A7
49f : A = A10
49g : A=A11
49h : A=A16
49i : A=A35
49j :A=A39
49k : A=A40
49l : A=A41
49m : A=A42

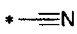 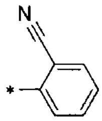 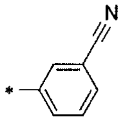 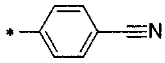 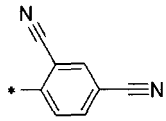 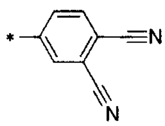

A1 A2 A3 A4 A5 A6

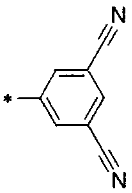 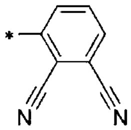 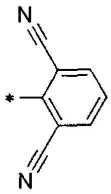 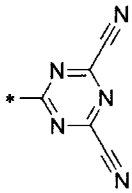 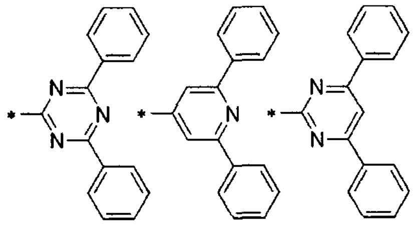

A7 A8 A9 A10 A11 A12 A13

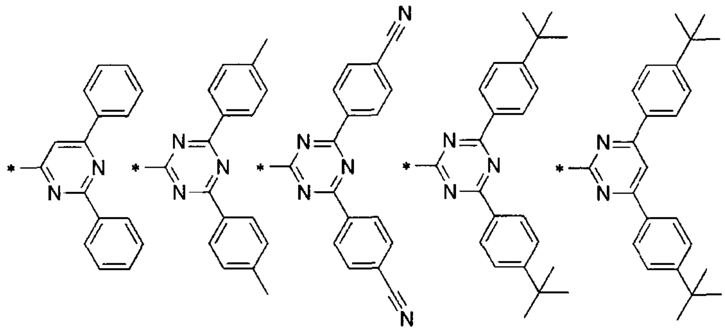

A14 A15 A16 A17 A18

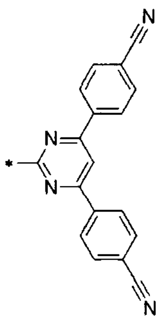 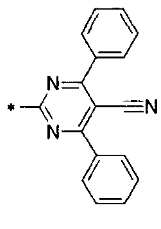 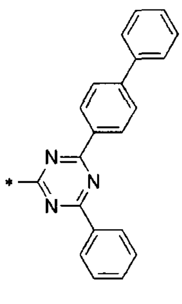 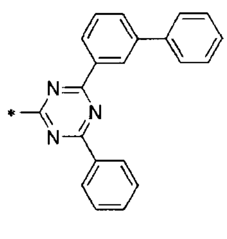

A19 A20 A21 A22

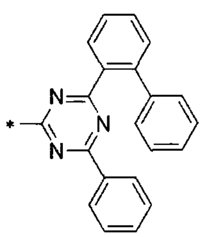 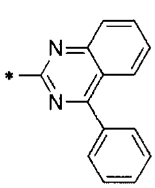 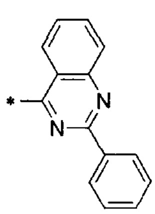

A23 A24 A25

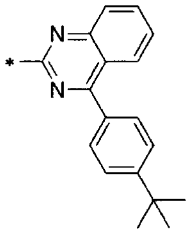 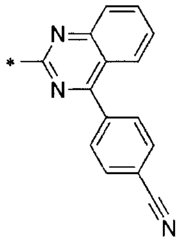 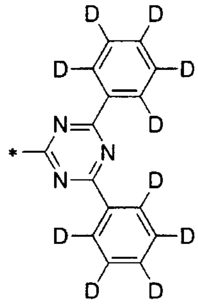 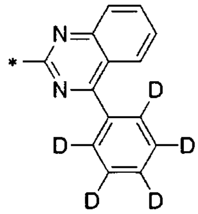

A26 A27 A28 A29

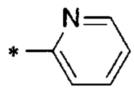 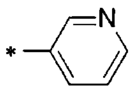 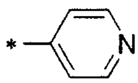 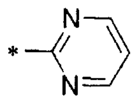 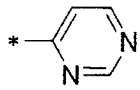

A30 A31 A32 A33 A34

*— $CF_3$ *— $C_2F_5$ 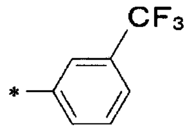 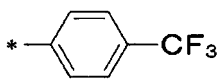

A35 A36 A37 A38

*-F A39

*-Cl A40

*-Br A41

*- I A42

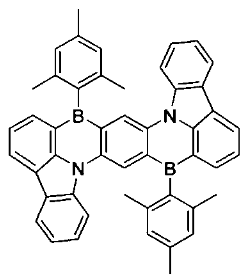 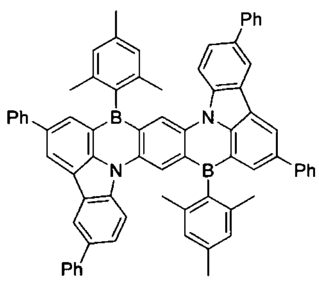 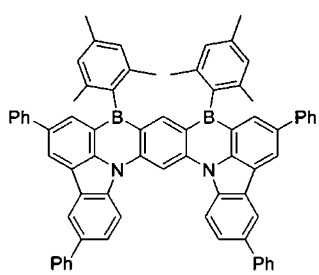

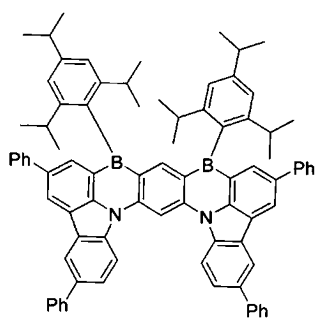 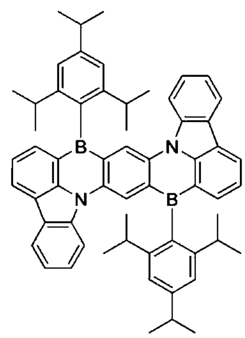 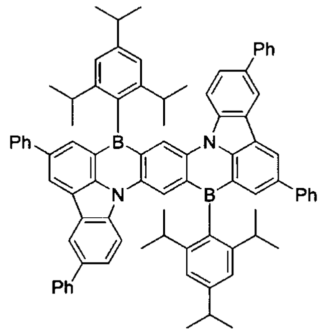

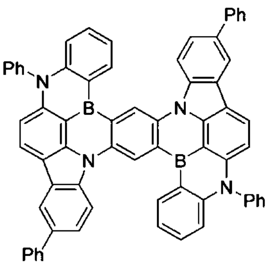
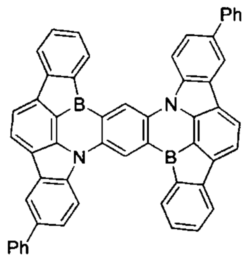
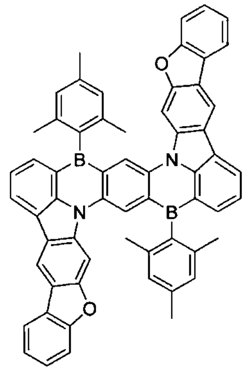
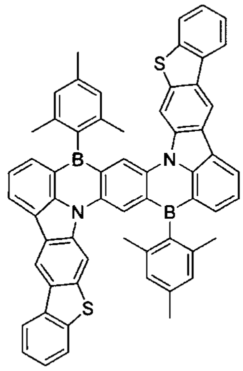

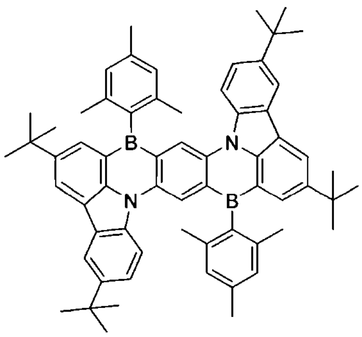
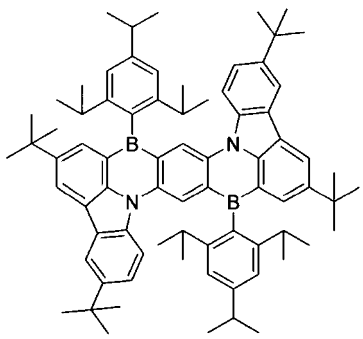
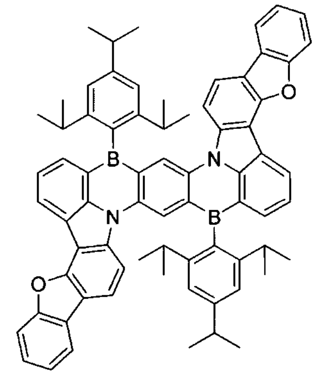

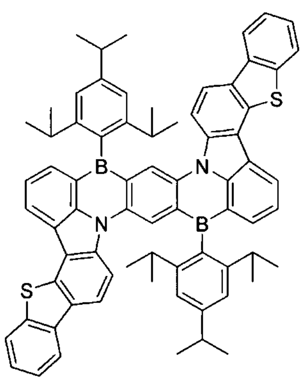
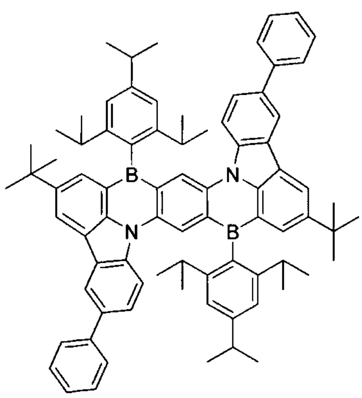
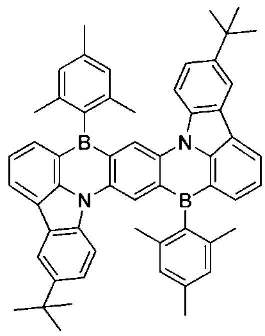

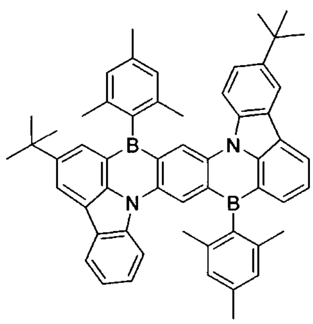
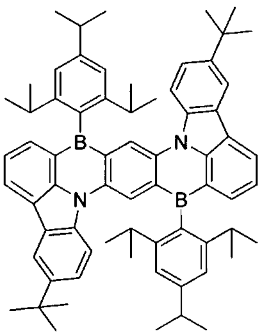
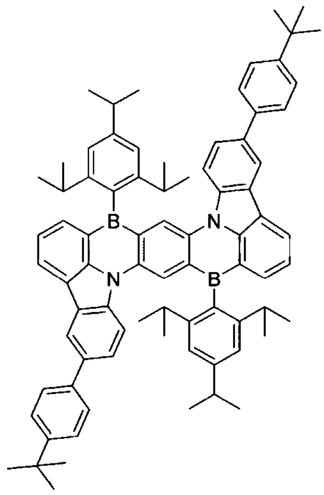

In some embodiments where a host material is used, the amount of the compound of the present invention contained in a light-emitting layer as a light-emitting material is 0.1% by weight or more. In some embodiments where a host material is used, the amount of the compound of the present invention contained in a light-emitting layer as a light-emitting material is 1% by weight or more. In some embodiments where a host material is used, the amount of the

compound of the present invention contained in a light-emitting layer as a light-emitting material is 50% by weight or less. In some embodiments where a host material is used, the amount of the compound of the present invention contained in a light-emitting layer as a light-emitting material is 20% by weight or less. In some embodiments where a host material is used, the amount of the compound of the present invention contained in a light-emitting layer as a light-emitting material is 10% by weight or less.

In some embodiments, the host material in a light-emitting layer is an organic compound having a hole transporting capability and an electron transporting capability. In some embodiments, the host material in a light-emitting layer is an organic compound that prevents increase in the wavelength of emitted light. In some embodiments, the host material in a light-emitting layer is an organic compound having a high glass transition temperature.

In some embodiments, the host material is selected from the group consisting of the followings:

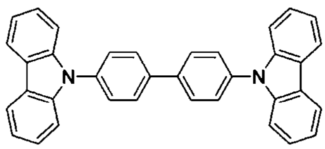
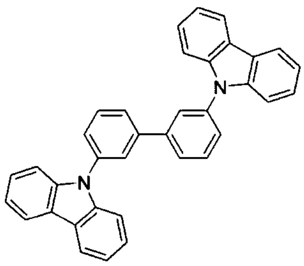
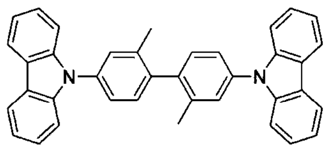
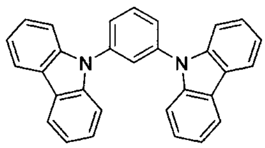
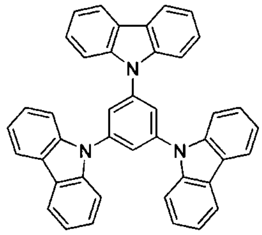
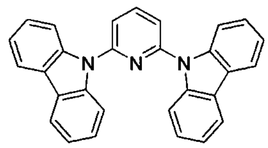
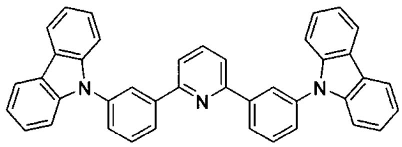
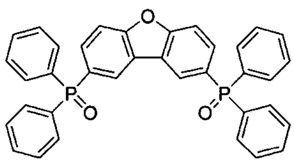
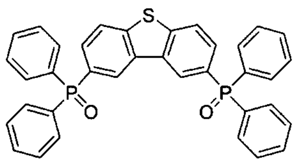
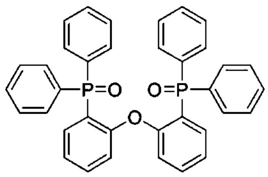
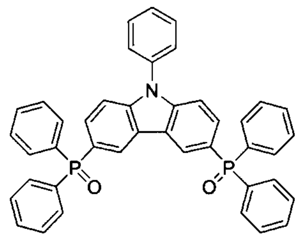
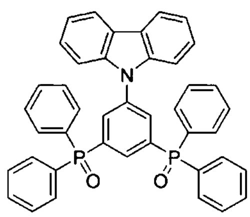
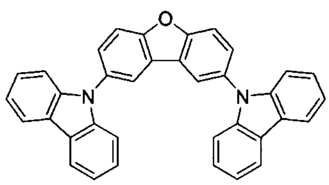
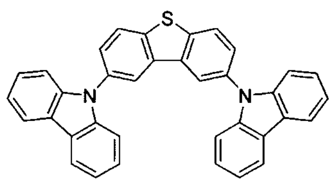
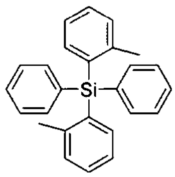
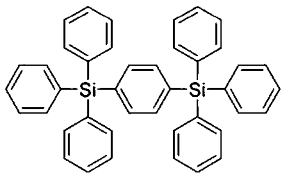
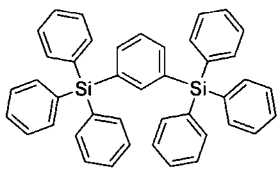
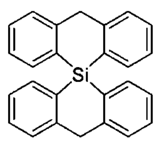

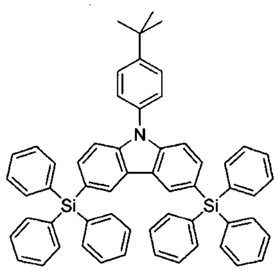
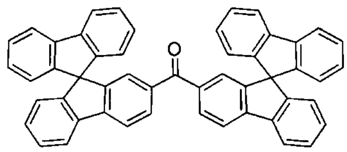
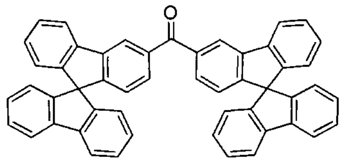

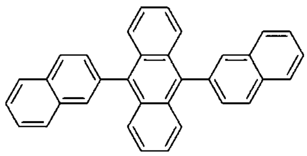
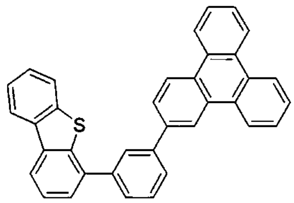
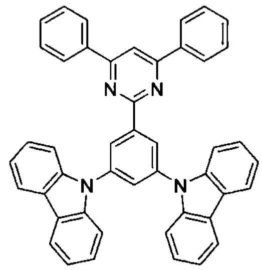

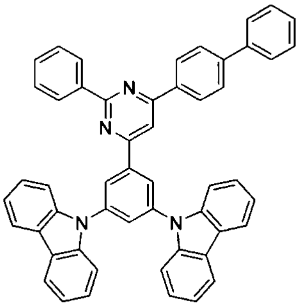
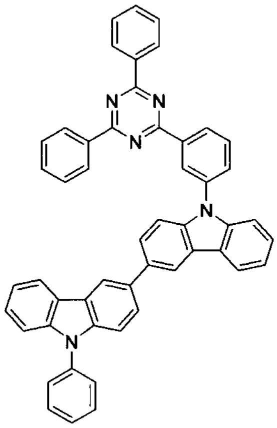
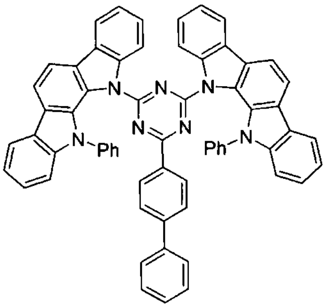

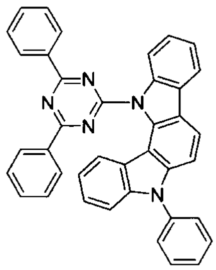
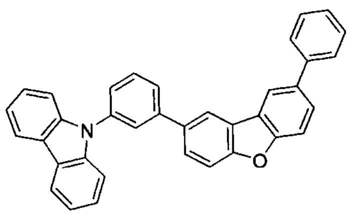
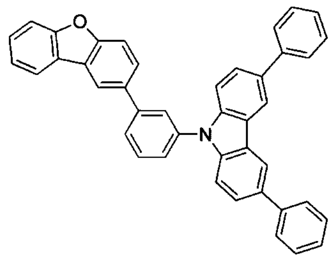

In some embodiments, the light-emitting layer contains two or more kinds of TADF molecules differing in the structure. For example, the light-emitting layer can contain three kinds of materials of a host material, a first TADF molecule and a second TADF molecule whose excited singlet energy level is higher in that order. In that case, both the first TADF molecule and the second TADF molecule are preferably such that the difference $\Delta E_{ST}$ between the lowest excited singlet energy level and the lowest excited triplet energy level at 77 K is 0.3 eV or less, more preferably 0.25 eV or less, still more preferably 0.2 eV or less, even more preferably 0.15 eV or less, further preferably 0.1 eV or less, further more preferably 0.07 eV or less, further more preferably 0.05 eV or less, further more preferably 0.03 eV or less, and particularly preferably 0.01 eV or less. The content of the first TADF molecule in the light-emitting layer is preferably larger than the content of the second TADF molecule therein. The content of the host material in the light-emitting layer is preferably larger than the content of the second TADF molecule therein. The content of the first TADF molecule in the light-emitting layer can be larger than or can be smaller than or can be the same as the content of the host material therein. In some embodiments, the composition in the light-emitting layer can be 10% by weight to 70% by weight of a host material, 10% by weight to 80% by weight of a first TADF molecule, and 0.1% by weight to 30% by weighty of a second TADF molecule. In some embodiments,

the composition in the light-emitting layer can be 20% by weight to 45% by weight of a host material, 50% by weight to 75% by weight of a first TADF molecule, and 5% by weight to 20% by weighty of a second TADF molecule. In some embodiments, the photoluminescence quantum yield φPL1(A) by photo-excitation of a co-deposited film of a first TADF molecule and a host material (the content of the first TADF molecule in the co-deposited film = A% by weight) and the photoluminescence quantum yield φPL2(A) by photo-excitation of a co-deposited film of a second TADF molecule and a host material (the content of the second TADF molecule in the co-deposited film = A% by weight) satisfy a relational formula φPL1(A) > φPL2(A). In some embodiments, the photoluminescence quantum yield φPL2(B) by photo-excitation of a co-deposited film of a second TADF molecule and a host material (the content of the second TADF molecule in the co-deposited film = B% by weight) and the photoluminescence quantum yield φPL2(100) by photo-excitation of a single film of a second TADF molecule satisfy a relational formula φPL2(B) > φPL2(100). In some embodiments, the light-emitting layer can contain three kinds of TADF molecules differing in the structure. The compound of the present invention can be any of the plural TADF compounds contained in the light-emitting layer.

In some embodiments, the light-emitting layer can be composed of materials selected from the group consisting of a host material, an assist dopant and a light-emitting material. In some embodiments, the light-emitting layer does not contain a metal element. In some embodiments, the light-emitting layer can be formed of a material composed of atoms alone selected from the group consisting of a carbon atom, a hydrogen atom, a deuterium atom, a nitrogen atom, an oxygen atom and a sulfur atom. Alternatively, the light-emitting layer can be formed of a material composed of atoms alone selected from the group consisting of a carbon atom, a hydrogen atom, a deuterium atom, a nitrogen atom and an oxygen atom. Alternatively, the light-emitting layer can be formed of a material composed of atoms alone selected from the group consisting of a carbon atom, a hydrogen atom, a nitrogen atom and an oxygen atom.

In the case where the light-emitting layer contains any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light-emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541. These patent publications described in this paragraph are hereby incorporated as a part of this description by reference.

In the following, the constituent members and the other layers than the light-emitting layer of the organic electroluminescent device are described.

Substrate:

In some embodiments, the organic electroluminescent device of the present invention is supported by a substrate, in which the substrate is not particularly limited and can be any of those that have been commonly used in an organic electroluminescent device, for example those formed of glass, transparent plastics, quartz and silicon.

Anode:

In some embodiments, the anode of the organic electroluminescent device is made of a metal, an alloy, a conductive compound, or a combination thereof. In some embodiments, the metal, alloy, or conductive compound has a large work function (4 eV or more). In some embodiments, the metal is Au. In some embodiments, the conductive transparent material is selected from CuI, indium tin oxide (ITO), $SnO_2$, and ZnO. In some embodiments, an amorphous material capable of forming a transparent conductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, where the pattern may not require high accuracy (for example, approximately 100 μm or more), the pattern can be formed with a mask having a desired shape on

vapor deposition or sputtering of the electrode material. In some embodiments, when a material can be applied as a coating, such as an organic conductive compound, a wet film forming method, such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is 10 nm to 1,000 nm. In some embodiments, the thickness of the anode is 10 nm to 200 nm. In some embodiments, the thickness of the anode varies depending on the material used.

Cathode:

In some embodiments, the cathode is made of an electrode material such as a metal having a small work function (4 eV or less) (referred to as an electron injection metal), an alloy, a conductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a work function larger than that of the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode is 10 nm to 5 μm. In some embodiments, the thickness of the cathode is 50 nm to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with a conductive transparent material, as described for the anode, to form a transparent or translucent cathode. In some embodiments, a device includes an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light-emitting layer or the hole transporting layer, and between the cathode and the light-emitting layer or the electron transporting layer. In some embodiments, an injection layer is present. In some embodiments, no injection layer is present.

Preferred compound examples for use as a hole injection material are shown below.

$MoO_3$,

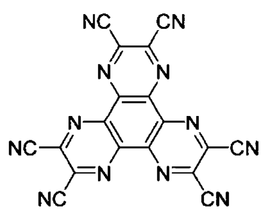
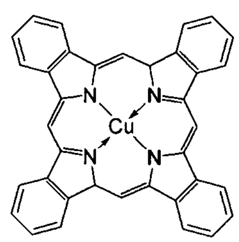
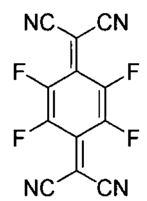
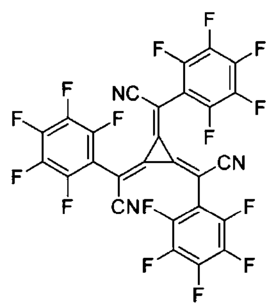

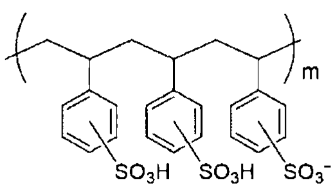
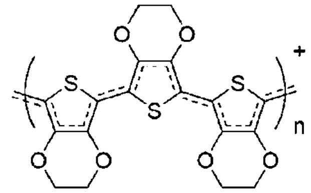

Next, preferred compound examples for use as an electron injection material are shown below.
LiF, CsF,

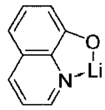

Barrier Layer:

A barrier layer is a layer capable of inhibiting charges (electrons or holes) and/or excitons present in the light-emitting layer from being diffused outside the light-emitting layer. In some embodiments, the electron barrier layer is between the light-emitting layer and the hole transporting layer, and inhibits electrons from passing through the light-emitting layer toward the hole transporting layer. In some embodiments, the hole barrier layer is between the light-emitting layer and the electron transporting layer, and inhibits holes from passing through the light-emitting layer toward the electron transporting layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has both the function of an electron barrier layer and the function of an exciton barrier layer.

Hole Barrier Layer:

A hole barrier layer functions as an electron transporting layer. In some embodiments, the hole barrier layer inhibits holes from reaching the electron transporting layer while transporting electrons. In some embodiments, the hole barrier layer enhances the recombination probability of electrons and holes in the light-emitting layer. The material used for the hole barrier layer can be the same materials as the ones described for the electron transporting layer.

Preferred compound examples for use for the hole barrier layer are shown below.

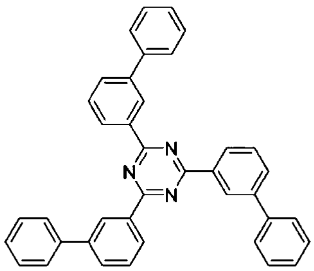 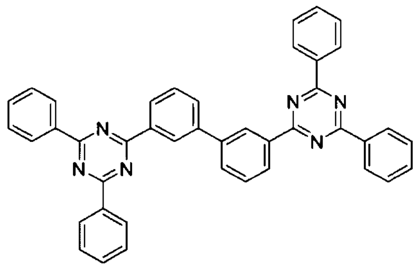 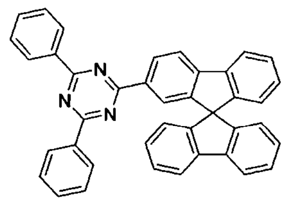

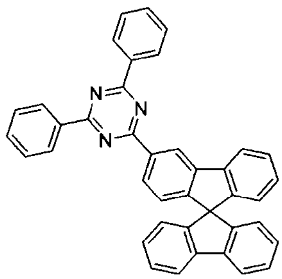 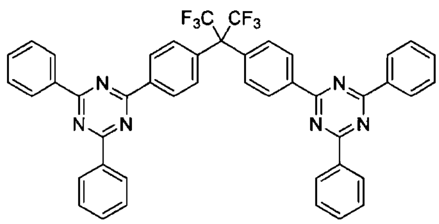 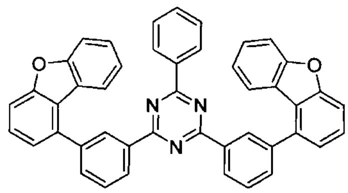

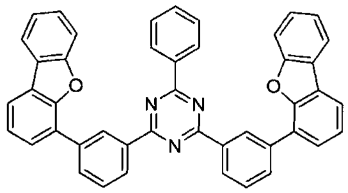 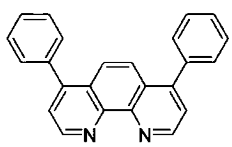 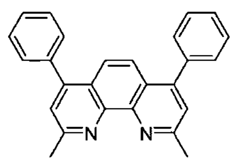

Electron Barrier Layer:

An electron barrier layer transports holes. In some embodiments, the electron barrier layer inhibits electrons from reaching the hole transporting layer while transporting holes. In some embodiments, the electron barrier layer enhances

the recombination probability of electrons and holes in the light-emitting layer. The material used for the electron barrier layer can be the same material as the ones described above for the hole transporting layer.

Specific examples of preferred compounds for use as the electron barrier material are shown below.

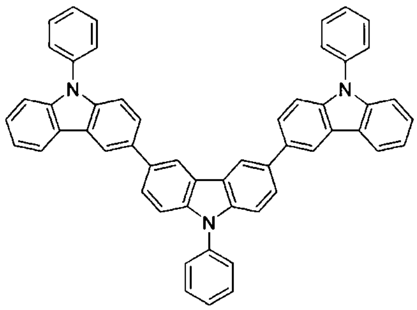
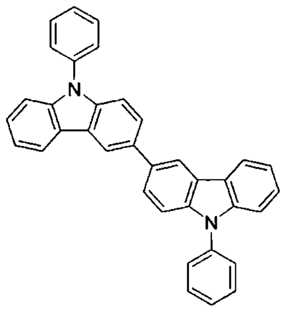
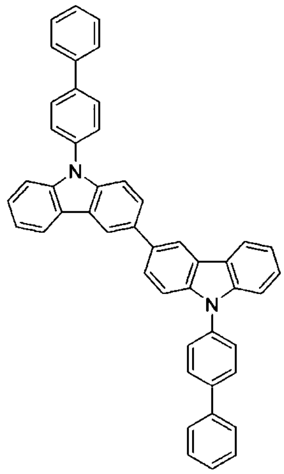
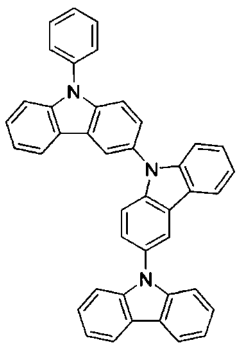
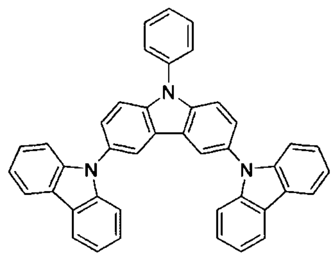
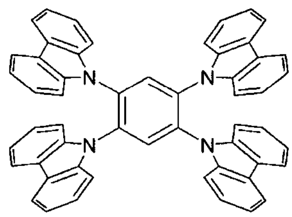
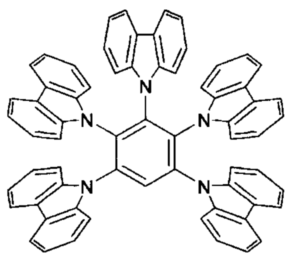
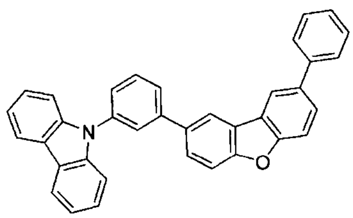
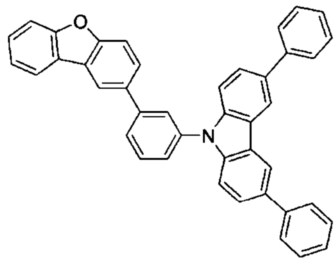
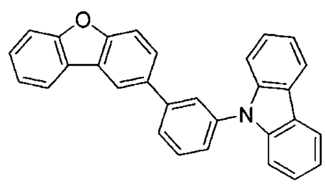

Exciton Barrier Layer:

An exciton barrier layer inhibits excitons generated through recombination of holes and electrons in the light-emitting layer from being diffused to the charge transporting layer. In some embodiments, the exciton barrier layer enables effective confinement of excitons in the light-emitting layer. In some embodiments, the light emission efficiency of the device is enhanced. In some embodiments, the exciton barrier layer is adjacent to the light-emitting layer on any of the side of the anode and the side of the cathode, and on both the sides. In some embodiments, where the exciton barrier layer is on

the side of the anode, the layer can be between the hole transporting layer and the light-emitting layer and adjacent to the light-emitting layer. In some embodiments, where the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer contains excited singlet energy and excited triplet energy, at least one of which is higher than the excited singlet energy and the excited triplet energy of the light-emitting material, respectively.

Hole Transporting Layer:

The hole transporting layer includes a hole transporting material. In some embodiments, the hole transporting layer is a single layer. In some embodiments, the hole transporting layer includes a plurality of layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that can be used in the present invention include, but are not limited to, a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a stilbene derivative, a silazane derivative, an aniline copolymer and a conductive polymer oligomer (particularly, a thiophene oligomer), or a combination thereof. In some embodiments, the hole transporting material is selected from a porphyrin compound, an aromatic tertiary amine compound, and a styrylamine compound. In some embodiments, the hole transporting material is an aromatic tertiary amine compound. Specific examples of preferred compounds for use as the hole transporting material are shown below.

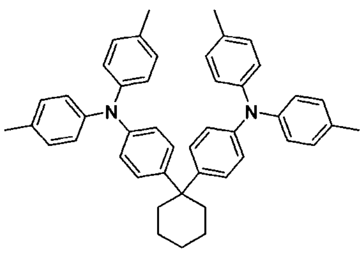
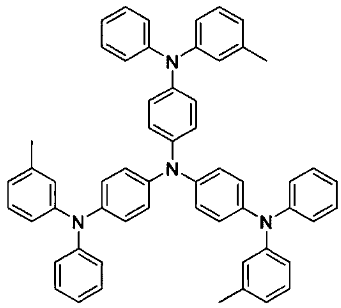
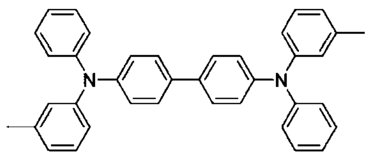

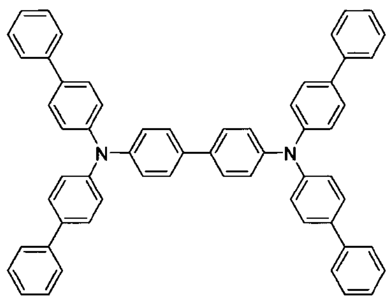
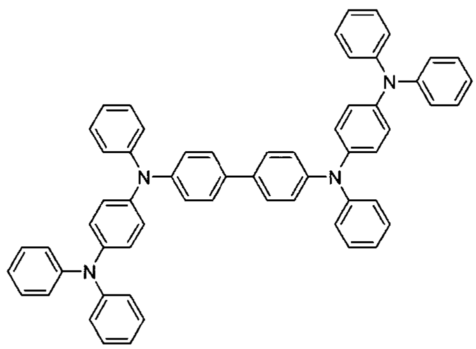

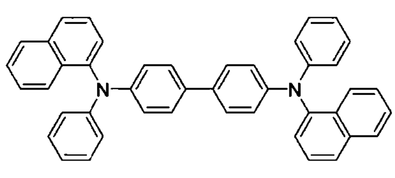
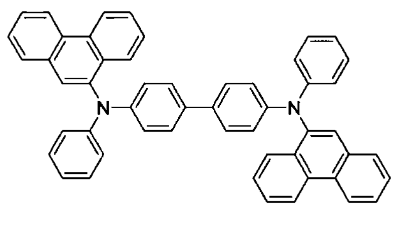

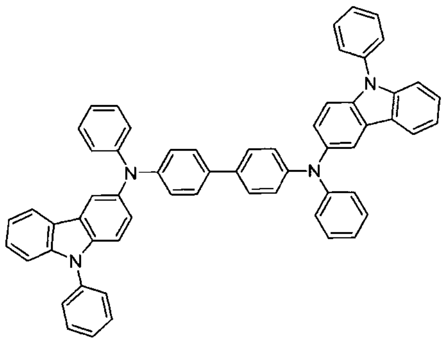
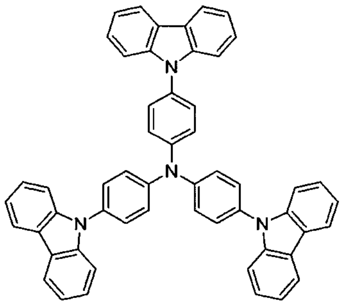
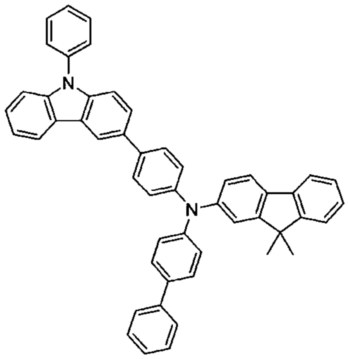

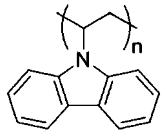
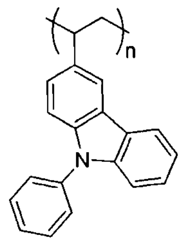
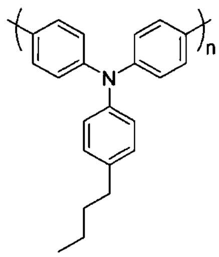

Electron Transporting Layer:

The electron transporting layer contains an electron transporting material. In some embodiments, the electron transporting layer is a single layer. In some embodiments, the electron transporting layer includes a plurality of layers.

In some embodiments, the electron transporting material needs only to have a function of transporting electrons, which are injected from the cathode, to the light-emitting layer. In some embodiments, the electron transporting material also function as a hole barrier material. Examples of the electron transporting layer that can be used in the present invention include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an oxadiazole derivative, an azole derivative, an azine derivative, or a combination thereof, or a polymer thereof. In some embodiments, the electron transporting material is a thiadiazole derivative, or a quinoxaline derivative. In some embodiments, the electron transporting material is a polymer material. Specific examples of preferred compounds for use as the electron transporting material are shown below.

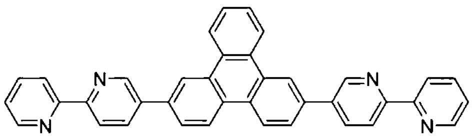
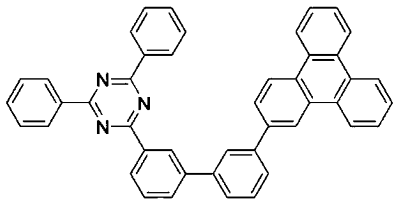

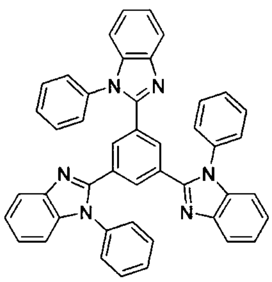
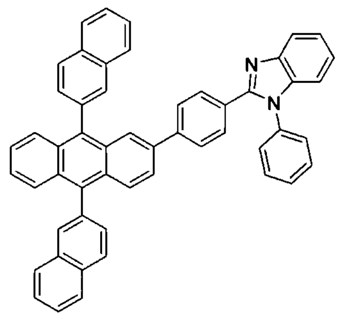
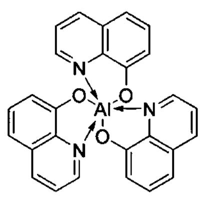

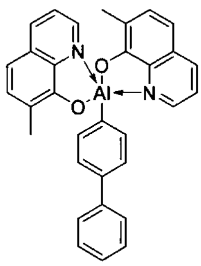
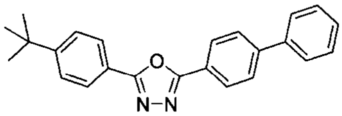
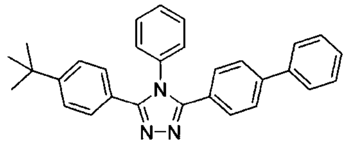

Further, compound examples preferred as a material that can be added to the organic layers are shown. For example, it is conceivable to add these as a stabilization material.

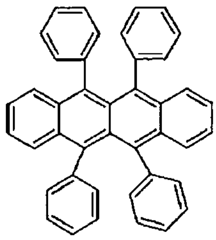
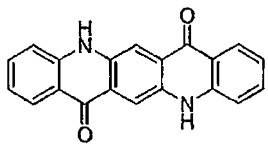
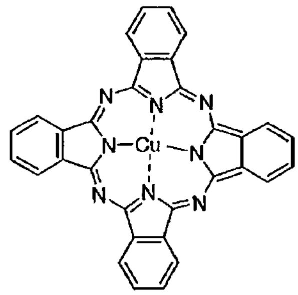

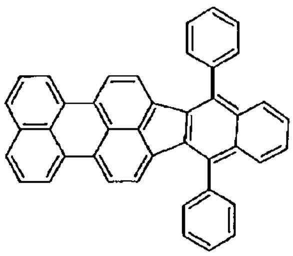
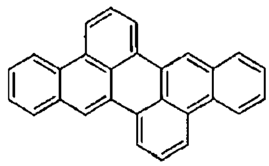

Preferred materials for use in the organic electroluminescent device are specifically shown. However, the materials usable in the present invention should not be limitatively interpreted by the following exemplary compounds. Compounds that are exemplified as materials having a specific function can also be used as materials having any other function.

Devices:

In some embodiments, an light-emitting layer is incorporated into a device. For example, the device includes, but is not limited to an OLED bulb, an OLED lamp, a television screen, a computer monitor, a mobile phone, and a tablet.
In some embodiments, an electronic device includes an OLED including an anode, a cathode, and at least one organic layer including a light-emitting layer between the anode and the cathode.
In some embodiments, compositions described herein can be incorporated into various light-sensitive or light-activated devices, such as OLEDs or opto-electronic devices. In some embodiments, the composition can be useful in facilitating charge transfer or energy transfer within a device and/or as a hole transport material. The device can be, for example, an organic light-emitting diode (OLED), an organic integrated circuit (OIC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

Bulbs or Lamps:

In some embodiments, an electronic device includes an OLED including an anode, a cathode, and at least one organic layer including a light-emitting layer between the anode and the cathode.
In some embodiments, a device includes OLEDs that differ in color. In some embodiments, a device includes an array including a combination of OLEDs. In some embodiments, the combination of OLEDs is a combination of three colors (e.g., RGB). In some embodiments, the combination of OLEDs is a combination of colors that are not red, green, or blue (for

example, orange and yellow green). In some embodiments, the combination of OLEDs is a combination of two, four, or more colors.

In some embodiments, a device is an OLED light including:

a circuit board having a first surface with a mounting surface and an opposing second surface, and defining at least one opening,
at least one OLED on the mounting surface, the at least one OLED configured to emit light, the at least one OLED including an anode, a cathode, and at least one organic layer including a light-emitting layer between the anode and the cathode,
a housing for the circuit board, and
at least one connector arranged at an end of the housing, the housing and the at least one connector defining a package adapted for installation in a light fixture.

In some embodiments, the OLED light includes a plurality of OLEDs mounted on a circuit board such that light emits in a plurality of directions. In some embodiments, a portion of the light emitted in a first direction is deflected to emit in a second direction. In some embodiments, a reflector is used to deflect the light emitted in a first direction.

Displays or Screens:

In some embodiments, the light-emitting layer of the present invention can be used in a screen or a display. In some embodiments, the compounds of the present invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen (which can also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the fine patterning of pixels needed for high resolution displays while optimizing the chemical vapor deposition onto a TFT backplane.

The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etching rate but the depths are varying depending on the halftone pattern. Varying the size and spacing of the halftone pattern allows etching to be inhibited at different rates within the pixel and allow a localized deeper etch needed to create steep vertical bevels.

A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the opening areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Methods of Manufacturing Devices:

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect of the present invention, provided herein is a method of manufacturing an organic light-emitting diode (OLED) display, the method including:

a step of forming a barrier layer on a base substrate of a mother panel,
a step of forming a plurality of display units in units of cell panels on the barrier layer,
a step of forming an encapsulation layer on each of the display units of the cell panels, and
a step of applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps

the mother panel to be softly cut in units of the cell panel.

In some embodiments, the thin film transistor (TFT) layer includes a light-emitting layer, a gate electrode, and a source/drain electrode. Each of the plurality of display units may include a thin film transistor (TFT) layer, a planarization film formed on the TFT layer, and a light-emitting unit formed on the planarization film, in which the organic film applied to the interface portion is formed of a same material as a material of the planarization film and is formed at a same time as the planarization film is formed. In some embodiments, a light-emitting unit is connected to the TFT layer with a passivation layer and a planarization film therebetween and an encapsulation layer that covers and protects the light-emitting unit. In some embodiments of the method of manufacturing, the organic film contacts neither the display units nor the encapsulation layer.

Each of the organic film and the planarization film can include any one of polyimide and acryl. In some embodiments, the barrier layer can be an inorganic film. In some embodiments, the base substrate can be formed of polyimide. The method can further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film can be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit including the TFT layer and the light-emitting unit is referred to as a display unit.

In some embodiments, the encapsulation layer that covers the display unit and prevents penetration of external moisture can be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further includes cutting along the interface portion, in which a groove is formed in the barrier layer, at least a portion of the organic film is formed in the groove, and the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels can be softly cut and cracks can be prevented from occurring in the barrier layer. In one embodiment, the organic film covering the groove at the interface portion and the planarization film are spaced apart from each other. For example, if the organic film and the planarization film are connected to each other as one layer, since

external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below can be appropriately modified unless they deviate from the substance of the present invention. Accordingly, the scope of the present invention is not construed as being limited to the specific examples shown below. The light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optics Corporation, USB2000), a spectroradiometer (available from Topcon Corporation, SR-3), and a streak camera (available from Hamamatsu Photonics K.K., Model C4334). The energies of HOMO and LUMO were measured by photoelectron spectroscopy in air (such as AC-3 manufactured by Riken Keiki Co., Ltd.).

In the following Synthesis Examples, compounds included in the general formula (1) were synthesized.

(Synthesis Example 1) Synthesis of Compound 727

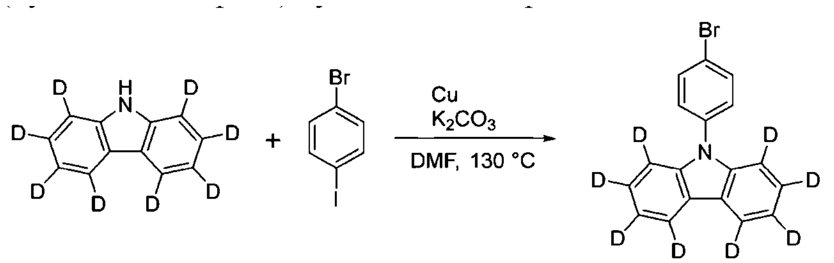


**X1**

Compound X1

Carbazole-1,2,3,4,5,6,7,8-d8 (3.3 g, 18.8 mmol), p-bromoiodobenzene (7.9 g, 27.9 mmol), potassium carbonate (10.2 g, 73.8 mmol), and copper powder (2.4 g, 37.7 mmol) were heated in N,N-dimethylformamide (DMF: 50 mL) at 130°C under a nitrogen atmosphere. After cooling to room temperature, the mixture was filtered through Celite and washed with ethyl acetate. The filtrate was concentrated and then purified by silica gel chromatography (hexane) to obtain 5.7 g (17.2 mmol, yield 92%) of white solid Compound X1.

$^1H$ NMR (400 MHz, $CDCl_3$): δ 7.73 (dt, J = 9.2, 2.0 Hz, 2H) 7.46 (dt, J = 8.8, 2.8 Hz, 2H).
ASAP MS Spectral Analysis: $C_{18}H_4D_8BrN$: theoretical value 329.07, observed value 330.12 [M+H$^+$]

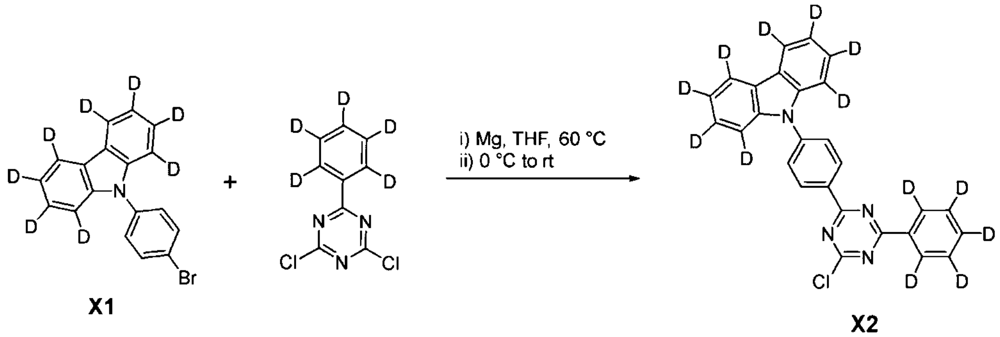


X1

X2

Compound X2

Under a nitrogen stream, a tetrahydrofuran solution (THF solution: 55 mL) of Mg (0.48 g, 19.7 mmol) and Compound X1 (5.5 g, 16.7 mmol) was heated to 60°C and stirred for 3 hours. The obtained brown solution was cooled to room temperature and slowly added dropwise to a THF solution (55 mL) of 2,4-dichloro-6-(phenyl-2,3,4,5,6-$d_5$)-1,3,5-triazine (3.82 g, 16.5 mmol) at 0°C. After the dropwise addition was completed, the mixture was heated to room temperature and stirred for 3 hours. The reaction container was cooled to 0°C, and a saturated aqueous ammonium chloride solution (200 mL) was added, followed by extraction with ethyl acetate. The organic layer was washed with saturated saline and then dried using anhydrous magnesium. The solvent was removed, and the resultant was purified by silica gel chromatography (hexane/toluene = 6:1) to obtain 1.76g (3.92 mmol, yield 23%) of pale yellow solid Compound X2.

$^1$H NMR (400 MHz, $CDCl_3$): δ 8.88 (dt, J = 8.8, 2.0 Hz, 2H), 7.82 (dt, J = 8.4, 2.0 Hz, 2H).
ASAP MS Spectral Analysis: $C_{27}H_4D_{13}ClN_4$: theoretical value 445.20, observed value 446.27 [M+H$^+$]

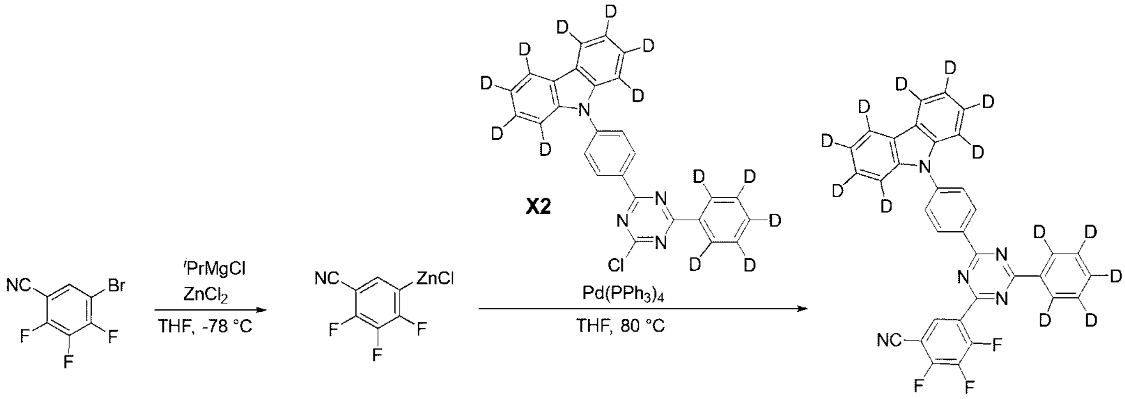


X2

X3

Compound X3

Under a nitrogen stream, a THF solution (1.2 mL, 2.4 mmol) of 2.0M isopropylmagnesium chloride was slowly added dropwise to a THF (8 mL) solution of 5-bromo-2,3,4-trifluorobenzonitrile (0.47 g, 2.0 mmol) at -78°C. After stirring for 1 hour, a THF solution (6 mL, 6 mmol) of 1M zinc chloride was added, and the mixture was further stirred for 30 minutes. The mixture was heated to room temperature, and Compound X2 (0.60 g, 1.3 mmol), tetrakis(triphenylphosphine)palladium(0) (0.79 g, 0.068 mmol), and THF (5 mL) were added thereto. Thereafter, the mixture was heated to 80°C and stirred for 17 hours. After cooling to room temperature, the obtained solid was washed with ion-exchanged water, methanol, ethyl acetate, and hexane to obtain 0.623 g (1.09 mmol, yield 81%) of yellow solid Compound X3.
ASAP MS Spectral Analysis: $C_{34}H_5D_{13}F_3N_5$: theoretical value 566.23, observed value 567.39 [M+H$^+$]

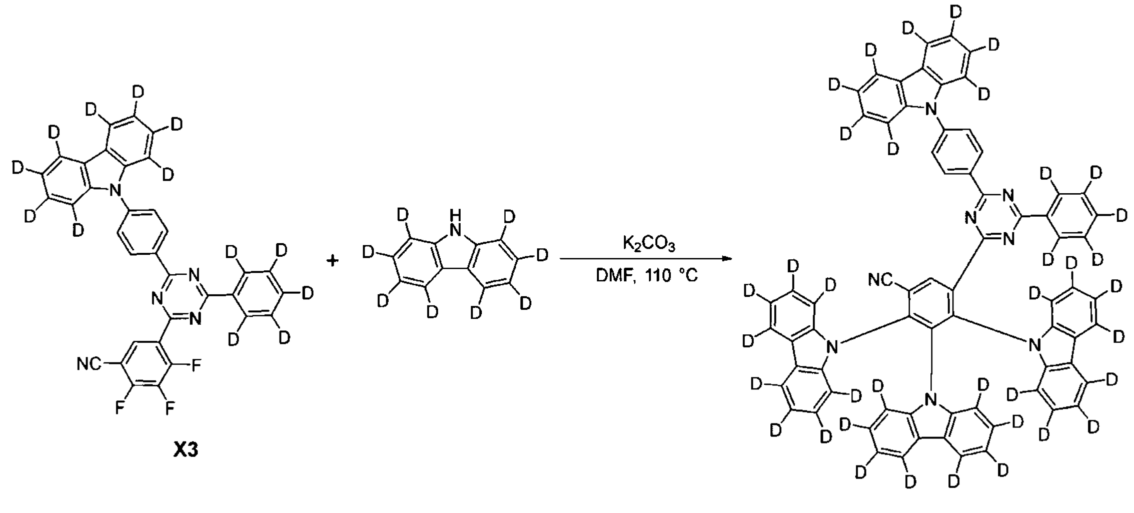


X3

727

Compound 727

Potassium carbonate (0.980 g, 7.06 mmol) was added to a DMF (29 mL) solution of Compound X3 (1.00 g, 1.76 mmol) and carbazole-1,2,3,4,5,6,7,8-d8 (1.08 g, 6.17 mmol), followed by stirring at 110°C for 20 hours under a nitrogen atmosphere. The reaction solution was cooled to room temperature, and a saturated aqueous ammonium chloride solution was added thereto, followed by extraction with methylene chloride. Then, the organic layer was washed with ion-exchanged water and saturated saline, and dried using anhydrous magnesium sulfate. The obtained solid was purified by silica gel chromatography (hexane/methylene chloride = 1:1). The obtained solid was washed with methanol and then reprecipitated with methylene chloride/methanol to obtain 1.08 g (1.05 mmol, yield 59%) of yellow solid Compound 727.

$^1H$ NMR (400 MHz, $CDCl_3$): $\delta$ 9.08 (s, 1H), 8.08 (d, J = 8.8 Hz, 2H), 7.51 (d, J = 8.8 Hz, 2H).
ASAP MS Spectral Analysis: $C_{70}H_5D_{37}N_8$: theoretical value 1031.59, observed value 1032.82 [M+H$^+$]

(Synthesis Example 2) Synthesis of Compound 727(28)

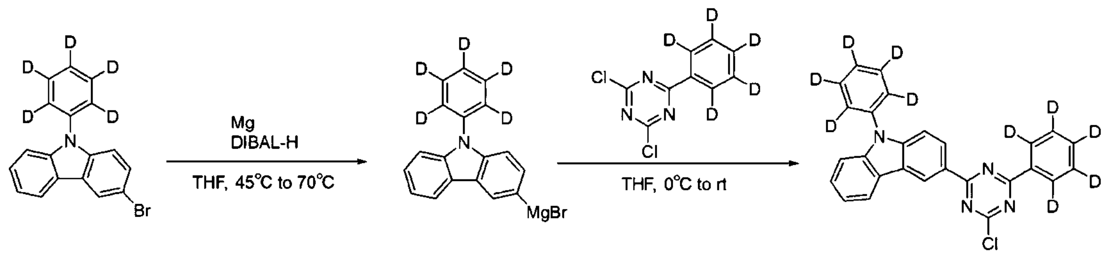


X4

Compound X4

Under a nitrogen stream, a toluene solution (DIBAL-H toluene solution: 0.60 mL, 0.60 mmol) of 1.0M diisobutylaluminum hydride was added to a mixture of Mg (1.53 g, 63 mmol) and THF (20 mL), and then a THF solution (80 mL) of 1.0M 3-bromo-9-(phenyl-2,3,4,5,6-d5)-9H-carbazole (20.3 g, 62 mmol) was slowly added dropwise thereto at 45°C. After the dropwise addition was completed, the mixture was heated to 70°C and further stirred for 2 hours. The obtained brown solution was cooled to room temperature and slowly added dropwise to a THF solution (120 mL) of 2,4-dichloro-6-(phenyl-2,3,4,5,6-d5)-1,3,5-triazine at 0°C. After the dropwise addition was completed, the mixture was returned to room temperature and stirred for 18 hours. The reaction container was cooled to 0°C, a saturated aqueous ammonium chloride solution (100 mL) was added thereto, and the organic phase and the aqueous phase were separated. The organic phase was concentrated and the obtained solid was washed with THF and hexane. The solid was dissolved in hot toluene and then passed through a silica pad, and the filtrate was concentrated. The obtained pale yellow solid was washed with THF and hexane to obtain 13.0 g (29.3 mmol, yield 47%) of pale yellow Compound X4.

$^{1}$H-NMR (400 MHz, DMSO-d6): δ 9.49 (d, J=1.6 Hz, 1H), 8.65 (dd, J=8.8, 1.6 Hz, 1H), 8.55 (d, J=12 Hz, 1H), 7.51-7.54 (m, 2H), 7.388-7.424 (m, 2H).
ASAP MS Spectral Analysis: $C_{27}H_7D_{10}ClN_4$: theoretical value 442.18, observed value 443.39 [M+H$^+$]

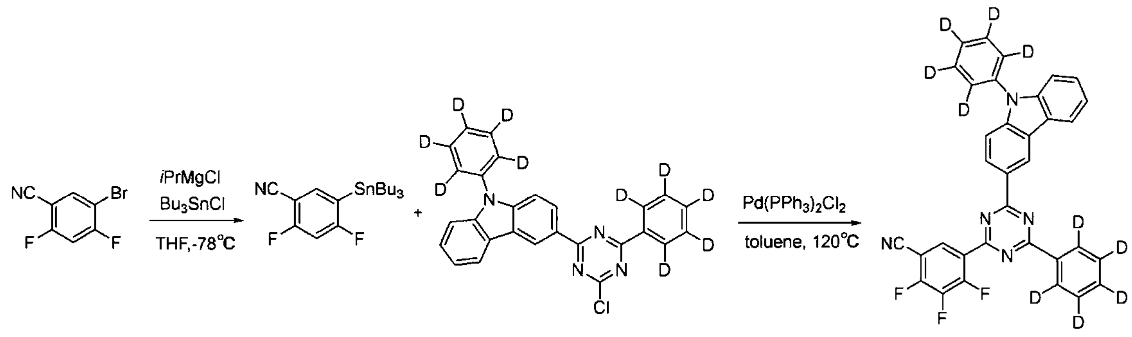


X4 X5

Compound X5

Under a nitrogen stream, a THF solution (5.3 mL, 10.6 mmol) of 2.0M isopropylmagnesium chloride was slowly added dropwise to a THF (100 mL) solution of 5-bromo-2,4-difluorobenzonitrile (2.36 g, 10.0 mmol) at -78°C. After stirring for 1 hour, tributyltin chloride (3.96 g, 12.1 mmol) was added, and the mixture was stirred for 10 minutes, then heated to room temperature and further stirred for 3 hours. The reaction container was cooled to 0°C, and a saturated ammonium chloride solution was added thereto. The obtained reaction solution was extracted with toluene, the organic layer was washed with saturated saline, and dried using anhydrous magnesium. The solvent was removed, and the obtained yellow liquid was dissolved in dehydrated toluene (100 mL), and bis(triphenylphosphine)palladium(II) dichloride (0.71 g, 1.0 mmol) and Compound X4 (4.43 g, 10.0 mmol) were added thereto, followed by stirring at 120°C for 15 hours. The reaction container was returned to room temperature, and the obtained gray solid was washed with toluene and hexane. The solid was dissolved in hot toluene and then passed through a silica pad, and the filtrate was concentrated. The obtained pale yellow solid was washed with a mixed solution of ethyl acetate and hexane to obtain 2.41 g (4.28 mmol, yield 43%) of pale orange solid Compound X5.
ASAP MS Spectral Analysis: $C_{34}H_8D_{10}F_3N_5$: theoretical value 563.21, observed value 564.39 [M+H$^+$]

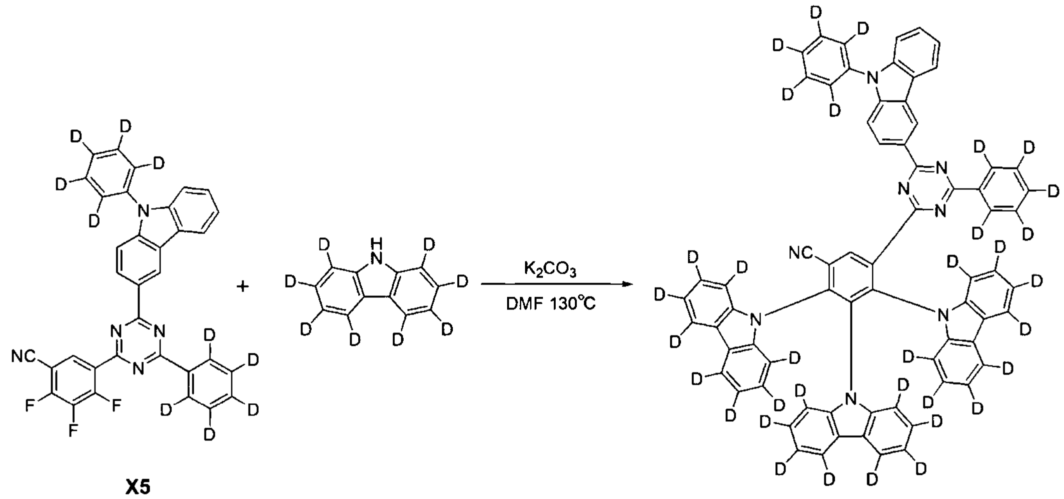


X5

727(28)

Compound 727(28)

Potassium carbonate (1.42 g, 10.3 mmol) was added to a DMF (50 mL) solution of Compound X5 (1.41 g, 2.50 mmol) and carbazole-1,2,3,4,5,6,7,8-d8 (1.49 g, 8.50 mmol), followed by stirring at 130°C for 5 hours. The reaction solution was cooled to room temperature and diluted by adding ethyl acetate. The solution was washed with saturated saline, and dried using anhydrous magnesium sulfate, followed by filtration. The filtrate was concentrated, and the

obtained solid was reprecipitated with ethyl acetate/hexane, and the solid was filtered and washed with hexane and methanol. The crude product was purified by column chromatography (toluene/hexane = 4:1), and the obtained solid was reprecipitated with ethyl acetate/hexane to obtain 1.83 g (1.78 mmol, yield 71%) of pale green Compound 727(28).

$^1$H-NMR (400 MHz, DMSO-d6): δ 9.36 (s, 1H), 9.05 (brs, 1H), 8.31 (d, J=8.4 Hz, 1H), 8.08 (d, J=8.4 Hz, 1H), 7.48-7.52 (m, 2H), 7.37-7.40 (m, 1H), 7.28 (d, J=8.4 Hz, 1H).

ASAP MS Spectral Analysis: $C_{70}H_8D_{34}N_8$: theoretical value 1028.56, observed value 1029.88 [M+H$^+$]

(Synthesis Example 3) Synthesis of Compound 727(252)

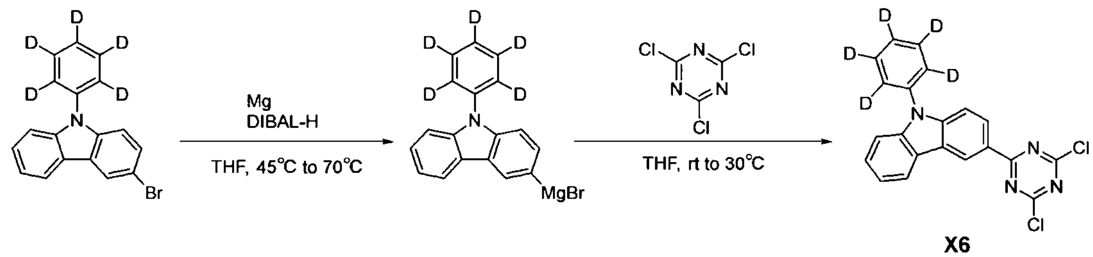


X6

Compound X6

Under a nitrogen stream, a 1.0M DIBAL-H toluene solution (0.21 mL, 0.21 mmol) was added to a mixture of Mg (0.54 g, 22.23 mmol) and THF (21 mL), and then a THF solution (7 mL) of 3.0M 3-bromo-9-(phenyl-2,3,4,5,6-d5)-9H-carbazole (7.2 g, 21.1 mmol) was slowly added dropwise thereto at 45°C. After the dropwise addition was completed, the mixture was heated to 70°C and further stirred for 2 hours. The obtained brown solution was cooled to room temperature and then slowly added dropwise to a THF solution (33.6 mL) of 2,4,6-trichloro-1,3,5-triazine at room temperature. After the dropwise addition was completed, the mixture was stirred at 30°C for 15 hours. A saturated aqueous ammonium chloride solution (30 mL) was added thereto, followed by stirring for 15 hours. The reaction mixture was filtered, and the obtained solid was washed with hexane to obtain 3.3 g (8.3 mmol, yield 40%) of pale yellow solid Compound X6.

$^1$H-NMR (400 MHz, $CDCl_3$): δ 9.33 (d, J=1.6 Hz, 1H), 8.55 (dd, J=2.0, 1.6 Hz, 1H), 8.27 (d, J=7.6 Hz, 1H), 7.37-7.51 (m, 4H).

ASAP MS Spectral Analysis: $C_{21}H_7D_3Cl_2N_4$: theoretical value 395.08, observed value 396.11 [M+H$^+$]

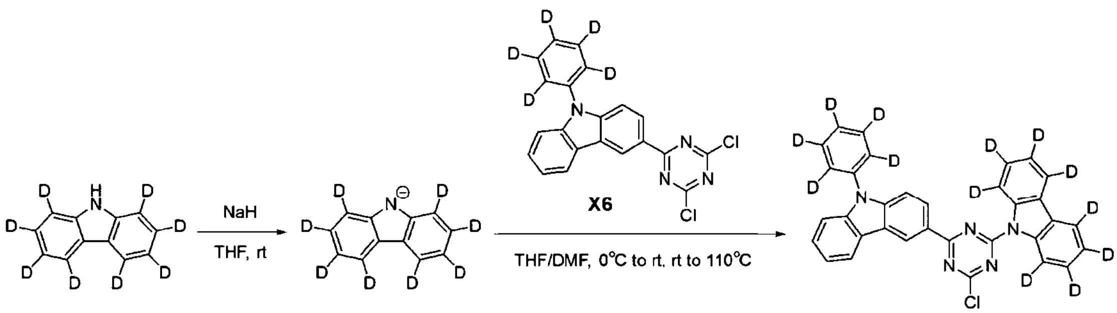


X7

Compound X7

Under a nitrogen gas stream, THF (10 mL) was added to a mixture of carbazole-1,2,3,4,5,6,7,8-d8 (0.63g, 3.60 mmol) and 60 wt% sodium hydride (0.14 g, 3.60 mmol), followed by stirring at room temperature for 1 hour. The obtained reaction mixture was slowly added dropwise to a THF solution (150 mL) of Compound X6 (1.7 g, 4.28 mmol) at 0°C. After the dropwise addition was completed, the mixture was stirred at room temperature for 1 hour. Thereafter, DMF (250 mL) was added thereto, and the mixture was stirred at 100°C for 13 hours and further stirred at 110°C for 4 hours. The reaction solution was returned to room temperature, and a saturated aqueous ammonium chloride solution and methanol were added thereto. The obtained solid was filtered and washed with hexane to obtain 1.97 g (3.68 mmol, yield 86%) of pale yellow solid Compound X7.

$^1$H-NMR (400 MHz, $CDCl_3$): δ 9.44 (d, J=1.6 Hz, 1H), 8.68 (dd, J=2.0, 1.6 Hz, 1H), 8.30 (d, J=3.6 Hz, 1H),

7.37-7.55 (m, 4H).
ASAP MS Spectral Analysis: $C_{33}H_7D_{13}ClN_5$: theoretical value 534.22, observed value 535.33 [M+H$^+$]

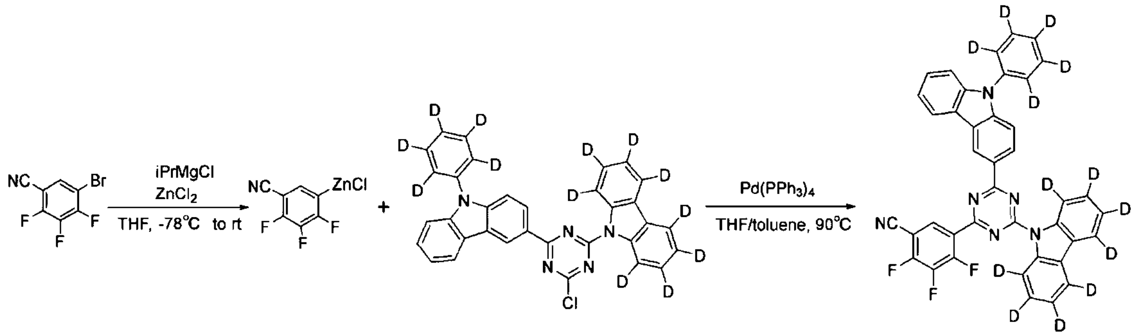


X7

X8

Compound X8

Under a nitrogen stream, a THF solution (4.2 mL, 4.2 mmol) of 1.0M isopropylmagnesium chloride was slowly added dropwise to a THF (15 mL) solution of 5-bromo-2,3,4-trifluorobenzonitrile (0.9 g, 3.81 mmol) at -78°C. After stirring for 30 minutes, a THF solution (11.4 ml, 11.4 mmol) of 1.0 M zinc chloride was added and stirred for 30 minutes. The mixture was then heated to room temperature and stirred for 1 hour. Compound X7 (1.43 g, 2.67 mmol), tetrakis(triphenylphosphine)palladium(0) (0.22 g, 0.19 mmol) and dehydrated toluene (38 mL) were added thereto, followed by stirring at 90°C for 14 hours. The reaction container was returned to room temperature, and a saturated aqueous ammonium chloride solution and methanol were added thereto. The obtained solid was filtered and washed with hexane to obtain 0.64 g (0.97 mmol, yield 37%) of pale yellow solid Compound X8.
ASAP MS Spectral Analysis: $C_{40}H_8D_{13}F_3N_6$: theoretical value 655.26, observed value 656.34 [M+H$^+$]

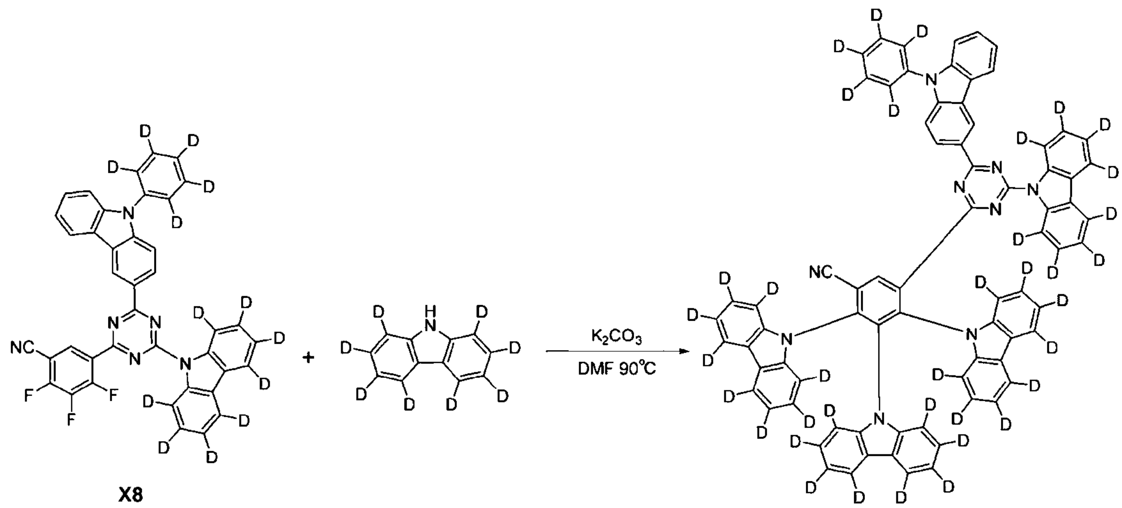


X8

727(252)

Compound 727(252)

Under a nitrogen atmosphere, potassium carbonate (0.65 g, 3.05 mmol) was added to a DMF (20 mL) solution of Compound X8 (0.4 g, 0.61 mmol) and carbazole-1,2,3,4,5,6,7,8-d8 (0.44 g, 2.5 mmol), followed by stirring at 90°C for 41 hours. The reaction solution was cooled to room temperature, and the solution was stirred with saturated saline and methanol, followed by filtration. The filtrate was concentrated, and the obtained solid was dissolved in chloroform and purified by column chromatography (toluene/hexane = 4:1) and column chromatography (toluene/hexane = 2:1). The obtained solid was washed with acetonitrile to obtain 0.40 g (0.356 mmol, yield 58%) of yellow green solid Compound 727(252).

$^1$H-NMR (400 MHz, DMSO-d6): δ 8.98 (s, 1H), 8.55 (s, 1H), 7.90-7.95 (m, 2H), 7.21-7.43 (m, 4H).
ASAP MS Spectral Analysis: $C_{76}H_8D_{37}N_9$: theoretical value 1120.61, observed value 1121.88 [M+H$^+$]

(Example 1) Preparation and Evaluation of Thin Film

Compound 727 was vapor-deposited on a quartz substrate by a vacuum deposition method under conditions of a vacuum degree of less than 1 × $10^{-3}$ Pa to form a neat thin film of Compound 727 having a thickness of 100 nm.
Separately, Compound 727 and H1 having the following structure were vapor-deposited from different vapor deposition sources on a quartz substrate by a vacuum deposition method under conditions of a vacuum degree of less than 1 × $10^{-3}$ Pa to form a doped thin film having a content of Compound 727 of 30% by weight and a thickness of 100 nm.
In the same manner but using Compound 727(28) and Comparative Compound A in place of Compound 727, neat thin films and doped thin films were formed.
The percentage of the delayed fluorescent component and the lifetime (τ2) of the delayed fluorescent component were measured by analyzing the photoluminescence of each doped thin film formed when irradiated with 300 nm excitation light. Also, using the formed neat thin films, the HOMO energy and the LUMO energy were measured. The results are shown in the following Table.

[Table 5]

| | Percentage (%) of delayed fluorescent component | τ2 (nanosecond) | HOMO (eV) | LUMO (eV) |
|---|---|---|---|---|
| Compound 727 | 80 | 2550 | 6.07 | 3.34 |
| Compound 727(28) | 81 | 2687 | 6.02 | 3.53 |
| Comparative Compound A | 78 | 2726 | 6.01 | 3.49 |

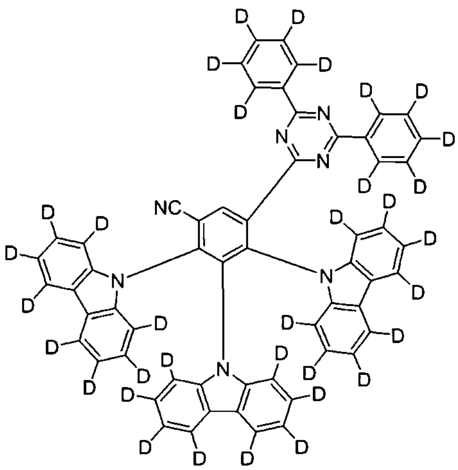

Comparative Compound A

(Example 2) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of 5.0 × $10^{-5}$ Pa. First, HAT-CN was formed to a thickness of 10 nm on the ITO, NPD was formed to a thickness of 30 nm thereon, further TrisPCz was formed to a thickness of 10 nm, and further H1 was formed to a thickness of 5 nm. Next, H1, Compound 727 and a dopant EM1 were co-deposited from different vapor deposition sources to form a layer with a thickness of 40 nm as a light-emitting layer. In the light-emitting layer, the content of H1 was 34.2% by mass, the content of Compound 727 was 65% by mass, and the content of EM1 was 0.8% by mass. Next, after SF3-TRZ was formed at a thickness of 10 nm, Liq and SF3-TRZ were co-deposited from different vapor deposition sources to form a layer with a thickness of 30 nm. The contents of Liq and SF3-TRZ in this layer were 30% by mass and 70% by mass, respectively. Further, Liq was formed to a thickness of 2 nm, and aluminum (Al) was vapor-deposited to a thickness of 100 nm to form a cathode, thereby obtaining an organic electroluminescent device.
An organic electroluminescent device was produced in the same manner using Compound 727(28) instead of Compound 727.
When the external quantum efficiency (EQE) of each organic electroluminescent device was measured, it was

confirmed that all of them exhibited a high value exceeding 20%, and the compound represented by the general formula (1) was useful.

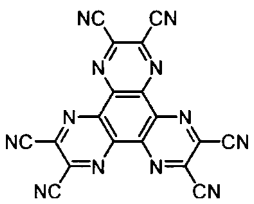
HAT-CN

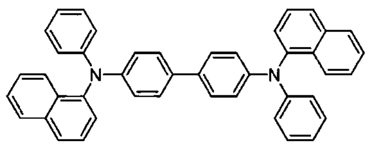
NPD

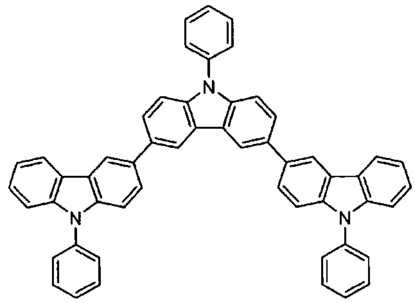
TrisPCz

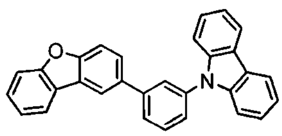
H1

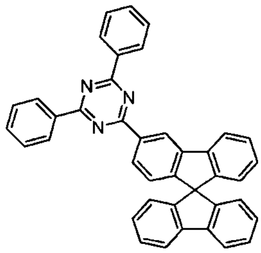
SF3-TRZ

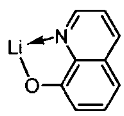
Liq

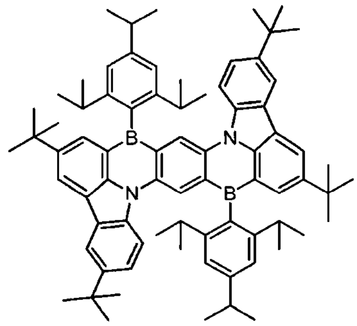
EM1

(Example 3) Evaluation of $\Delta E_{ST}$

Compound 727, Compound 6677, and Comparative Compound B and Comparative Compound C having the following structures that were synthesized in Synthesis Example 1 were each subjected to quantum chemical calculation by B3LYP-631G. The value obtained by structurally optimizing a lowest excited triplet state (T1) was adopted to determine an energy difference ($\Delta E_{ST}$) between a lowest excited singlet state (S1) and the lowest excited triplet state (T1). The results are shown in the following Table.

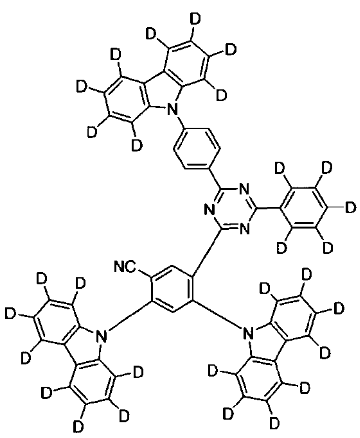
Comparative Compound 6677

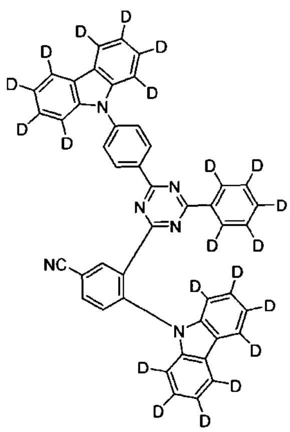
Comparative Compound B

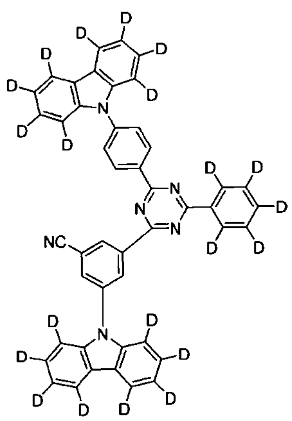
Comparative Compound C

[Table 6]

| | $\Delta E_{ST}$ |
|---|---|
| Compound 727 | 0.043 eV |
| Compound 6677 | 0.249 eV |
| Comparative Compound B | 0.489 eV |
| Comparative Compound C | 0.556 eV |

(Example 4) Evaluation of $\Delta E_{ST}$

Compound 727(28), Compound 6677(28), and Comparative Compound D and Comparative Compound E having the following structures that were synthesized in Synthesis Example 2 were subjected to quantum chemical calculation in the same manner as in Example 3 to determine $\Delta E_{ST}$. The results are shown in the following Table.

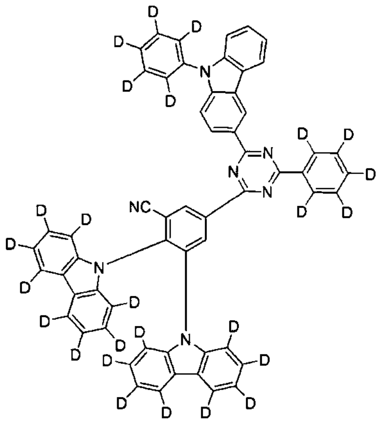

Comparative Compound 6677(28)

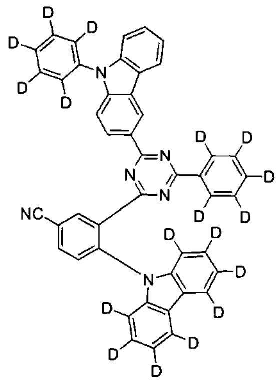

Comparative Compound D

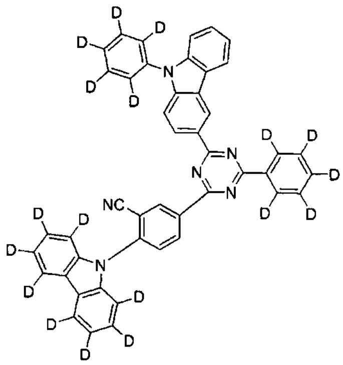

Comparative Compound E

[Table 7]

| | $\Delta E_{ST}$ |
|---|---|
| Compound 727(28) | 0.071 eV |
| Compound 6677(28) | 0.299 eV |
| Comparative Compound D | 0.401 eV |
| Comparative Compound E | 0.500 eV |

The results in Table 6 and Table 7 indicate that the compound represented by the general formula (1) has a $\Delta E_{ST}$ smaller than a $\Delta E_{ST}$ of the comparative compound, and thus is useful as a delayed fluorescent material.

Industrial Applicability

By using a compound represented by the general formula (1), there can be provided an organic light-emitting device having good light emission characteristics. Accordingly, the industrial applicability of the present invention is great.

**Claims**

**1.** A compound represented by the following general formula (1):

General Formula (1)

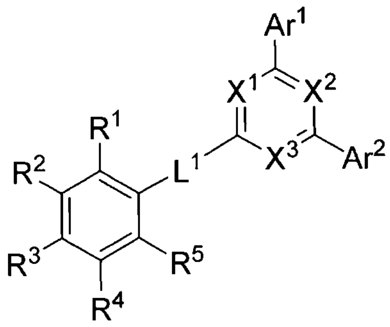

where $R^1$ to $R^5$ each independently represent a hydrogen atom, a deuterium atom, a cyano group, a substituted or unsubstituted alkyl group, a substituted or unsubstituted aryl group, or a donor group, one or more of $R^1$ to $R^5$ represent a cyano group, and two or more of $R^1$ to $R^5$ represent a donor group, and $X^1$ to $X^3$ each independently represent N or C(R), and at least one of $X^1$ to $X^3$ represents N, R represents a hydrogen atom, a deuterium atom, or a substituent, $Ar^1$ and $Ar^2$ each independently represent a substituted or unsubstituted aryl group, or a substituted or unsubstituted heteroaryl group, and at least one of $Ar^1$ and $Ar^2$ represents a group bonding via a benzene ring that has a donor group bonding via a nitrogen atom but has no acceptor group bonding thereto, and $L^1$ represents a single bond or a divalent linking group.

**2.** The compound according to claim 1, wherein only one of $R^1$ to $R^5$ represents a cyano group.

**3.** The compound according to claim 2, wherein $R^2$ represents a cyano group.

**4.** The compound according to claim 1, wherein $Ar^1$ represents a group having a structure represented by the following general formula (c):

General Formula (c)

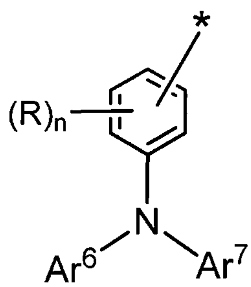

where R's each independently represent a deuterium atom or a donor group, $Ar^6$ and $Ar^7$ each independently represent a substituted or unsubstituted aryl group, or a substituted or unsubstituted heteroaryl group, $Ar^6$ and $Ar^7$ may bond to each other to form a cyclic structure, $Ar^6$ and R may bond to each other to form a cyclic structure, and $Ar^7$ and R may bond to each other to form a cyclic structure, n represents an integer of 0 to 4, and * indicates a bonding position.

**5.** The compound according to claim 1, wherein $Ar^1$ represents a group having a structure represented by the following general formula (d):

General Formula (d)

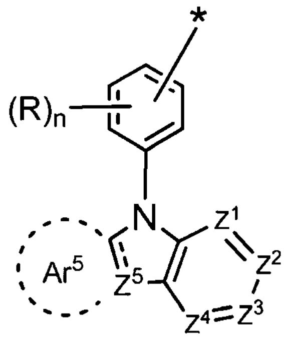

where R's each independently represent a deuterium atom or a donor group, $Z^1$ represents C-$R^{14}$ or N, $Z^2$ represents C-$R^{15}$ or N, $Z^3$ represents C-$R^{16}$ or N, and $Z^4$ represents C-$R^{17}$ or N, $Z^5$ represents C or N, and $Ar^5$ represents a substituted or unsubstituted aromatic ring or a substituted or unsubstituted heteroaromatic ring, $R^{14}$ and $R^{15}$, $R^{15}$ and $R^{16}$, and $R^{16}$ and $R^{17}$ each may bond to each other to form a cyclic structure, n represents an integer of 0 to 4, and * indicates a bonding position.

**6.** The compound according to claim 1, wherein
$Ar^1$ represents a group having a structure represented by the following general formula (f):

General Formula (f)

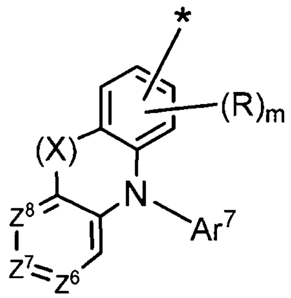

where R's each independently represent a deuterium atom or a donor group, $Z^6$ represents C-$R^{18}$ or N, $Z^7$ represents C-$R^{19}$ or N, $Z^8$ represents C-$R^{20}$ or N, and $R^{18}$ and $R^{19}$, $R^{19}$ and $R^{20}$, and $R^{20}$ and $R^{21}$ each may bond to each other to form a cyclic structure, X represents a single bond when absent, or represents a linking group having a linking chain of one or two atoms, $Ar^7$ represents a substituted or unsubstituted aryl group, or a substituted or unsubstituted heteroaryl group, m represents an integer of 0 to 3, and * indicates a bonding position.

**7.** The compound according to claim 1, wherein
$Ar^2$ represents a substituted or unsubstituted aryl group having no donor group.

**8.** The compound according to claim 1, wherein
$Ar^2$ represents a donor group bonding via a nitrogen atom.

**9.** The compound according to claim 1, wherein
$Ar^2$ represents a group having a structure represented by the following general formula (f):

General Formula (f)

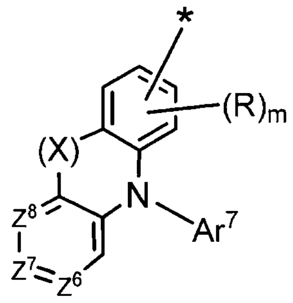

where R's each independently represent a deuterium atom or a donor group, $Z^6$ represents C-$R^{18}$ or N, $Z^7$ represents C-$R^{19}$ or N, $Z^8$ represents C-$R^{20}$ or N, and $R^{18}$ and $R^{19}$, $R^{19}$ and $R^{20}$, and $R^{20}$ and $R^{21}$ each may bond to each other to form a cyclic structure, X represents a single bond when absent, or represents a linking group having a linking chain of one or two atoms, $Ar^7$ represents a substituted or unsubstituted aryl group, or a substituted or unsubstituted heteroaryl group, m represents an integer of 0 to 3, and * indicates a bonding position.

**10.** The compound according to claim 1, wherein
three of $R^1$ to $R^5$ represent a donor group.

**11.** The compound according to claim 1, wherein
the donor group is a substituted or unsubstituted carbazol-9-yl group.

**12.** The compound according to claim 1, wherein
$X^1$ to $X^3$ represent N.

**13.** The compound according to claim 1, wherein
$L^1$ represents a single bond.

**14.** The compound according to claim 1, wherein
$R^1$ represents a hydrogen atom.

**15.** The compound according to claim 1, wherein
the compound has at least one deuterium atom.

**16.** A light-emitting material comprising:
the compound according to any one of claims 1 to 15.

**17.** A delayed fluorescent substance comprising:
the compound according to any one of claims 1 to 15.

**18.** A film comprising:
the compound according to any one of claims 1 to 15.

**19.** An organic semiconductor device comprising:
the compound according to any one of claims 1 to 15.

**20.** An organic light-emitting device comprising:
the compound according to any one of claims 1 to 15.

**21.** The organic light-emitting device according to claim 20, wherein

the device includes a layer containing the compound, and
the layer also contains a host material.

**22.** The organic light-emitting device according to claim 21, wherein

the layer containing the compound further contains a delayed fluorescent material in addition to the compound and the host material, and
the delayed fluorescent material has a lowest excited singlet energy lower than a lowest excited singlet energy of the host material and higher than a lowest excited singlet energy of the compound.

**23.** The organic light-emitting device according to claim 21, wherein

the device includes the layer containing the compound, and
the layer also contains a light-emitting material having a structure different from a structure of the compound.

**24.** The organic light-emitting device according to claim 21, wherein
an amount of light emitted from the compound is largest among the materials contained in the device.

**25.** The organic light-emitting device according to claim 23, wherein
an amount of light emitted from the light-emitting material is larger than an amount of light emitted from the compound.

**26.** The organic light-emitting device according to claim 20, which is an organic electroluminescent device.

**27.** The organic light-emitting device according to claim 20, which emits delayed fluorescence.

**INTERNATIONAL SEARCH REPORT**

International application No.
**PCT/JP2024/007515**

**A. CLASSIFICATION OF SUBJECT MATTER**

***C07D 403/14***(2006.01)i; ***C09K 11/06***(2006.01)i; ***H10K 50/11***(2023.01)i; ***H10K 50/12***(2023.01)i; ***H10K 50/15***(2023.01)i; ***H10K 50/16***(2023.01)i; ***H10K 85/60***(2023.01)i; *H10K 101/20*(2023.01)n; *H10K 101/30*(2023.01)n
FI: C07D403/14 CSP; H10K50/11; H10K50/12; H10K85/60; C09K11/06 690; H10K50/15; H10K50/16; H10K101:30; H10K101:20

According to International Patent Classification (IPC) or to both national classification and IPC

**B. FIELDS SEARCHED**

Minimum documentation searched (classification system followed by classification symbols)

C07D403/14; C09K11/06; H10K50/11; H10K50/12; H10K50/15; H10K50/16; H10K85/60; H10K101/20; H10K101/30

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | CN 114276337 A (JIANGSU MARCH SCIENCE AND TECHNOLOGY CO., LTD.) 05 April 2022 (2022-04-05)<br>claims, paragraphs [0001]-[0065], examples | 1-5, 7, 10-27<br>6, 8-9 |
| X<br>A | CN 114805318 A (JIANGSU SUNERA TECHNOLOGY CO., LTD.) 29 July 2022 (2022-07-29)<br>claims, paragraphs [0001]-[0107], examples | 1-2, 6, 11-27<br>3-5, 7-10 |
| A | JP 2021-519765 A (KYULUX, INC.) 12 August 2021 (2021-08-12)<br>entire text, all drawings | 1-27 |
| A | WO 2019/190223 A1 (LG CHEM, LTD.) 03 October 2019 (2019-10-03)<br>entire text, all drawings | 1-27 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 May 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (July 2022)

**INTERNATIONAL SEARCH REPORT**

International application No.
**PCT/JP2024/007515**

**C. DOCUMENTS CONSIDERED TO BE RELEVANT**

| Category* | Citation of document, with indication, where appropriate, of the relevant passages | Relevant to claim No. |
|---|---|---|
| A | EP 3670508 A1 (CYNORA GMBH) 24 June 2020 (2020-06-24) entire text, all drawings | 1-27 |
| A | HANSCH, Corwin et al., A survey of Hammett substituent constants and resonance and field parameters, Chem. Rev., 1991, 91(2), 165-195, DOI: 10.1021/cr00002a004 tables | 1-27 |
| P, A | WO 2023/090154 A1 (KYULUX INC.) 25 May 2023 (2023-05-25) entire text, all drawings | 1-27 |
| P, A | WO 2023/090288 A1 (KYULUX INC.) 25 May 2023 (2023-05-25) entire text, all drawings | 1-27 |

Form PCT/ISA/210 (second sheet) (July 2022)

**INTERNATIONAL SEARCH REPORT**
**Information on patent family members**

International application No.
**PCT/JP2024/007515**

| Patent document cited in search report | Publication date (day/month/year) | Patent family member(s) | Publication date (day/month/year) |
|---|---|---|---|
| CN 114276337 A | 05 April 2022 | (Family: none) | |
| CN 114805318 A | 29 July 2022 | (Family: none) | |
| JP 2021-519765 A | 12 August 2021 | WO 2019/191665 A1<br>KR 10-2020-0139715 A<br>CN 112272695 A<br>EP 3775100 A1<br>US 2021/0135119 A1 | |
| WO 2019/190223 A1 | 03 October 2019 | CN 111247140 A<br>KR 10-2019-0113661 A | |
| EP 3670508 A1 | 24 June 2020 | (Family: none) | |
| WO 2023/090154 A1 | 25 May 2023 | (Family: none) | |
| WO 2023/090288 A1 | 25 May 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019191665 A1
- WO 2015022974 A
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A

### Non-patent literature cited in the description

- **HANSCH, C.** *Chem. Rev.*, 1991, vol. 91, 165-195